# EUROPEAN PATENT APPLICATION

(11) **EP 2 703 008 A1**
(43) Date of publication of application: **05.03.2014**
(21) Application number: 12306039.4
(22) Date of filing: 31.08.2012
(51) Int. Cl.: A61K 39/395, C07K 16/40, C07K 5/02

(54) **Human antibodies to PCSK9 for use in methods of treating particular groups of subjects**

(71) Applicant: SANOFI, 75008 Paris (FR)
(72) Inventor: The designation of the inventor has not yet been filed
(74) Representative: Schneider, Rudolf

(57) **Abstract**

The present invention relates to methods for treating diseases or conditions in which proprotein convertase subtilisin/kexin type 9 (PCSK9) expression or activity causes an impact by administration of PCSK9-specific antibodies or antigen-binding fragments thereof. The present invention further relates to PCSK9-specific antibodies or antigen-binding fragments thereof for use in the treatment of diseases or conditions in which PCSK9 expression or activity causes an impact.

The present invention also relates to articles of manufacture comprising packaging material, PCSK9-specific antibodies or antigen-binding fragments thereof, and a label or packaging insert indicating which groups of patients can be treated with said antibodies or fragments, which groups of patients must not be treated with said antibodies or fragments, and preferably which dosage regimen should be used.

## Description

The present invention relates to methods for treating diseases or conditions in which proprotein convertase subtilisin/kexin type 9 (PCSK9) expression or activity causes an impact by administration of PCSK9-specific antibodies or antigen-binding fragments thereof. The present invention further relates to PCSK9-specific antibodies or antigen-binding fragments thereof for use in the treatment of diseases or conditions in which PCSK9 expression or activity causes an impact.

The present invention also relates to articles of manufacture comprising packaging material, PCSK9-specific antibodies or antigen-binding fragments thereof, and a label or packaging insert indicating which groups of patients can be treated with said antibodies or fragments, which groups of patients must not be treated with said antibodies or fragments, and/or which dosage regimen should be used.

### BACKGROUND OF THE INVENTION

Proprotein convertase subtilisin/kexin type 9 (PCSK9) is a proprotein convertase belonging to the proteinase K subfamily of the secretory subtilase family. The encoded protein is synthesized as a soluble zymogen that undergoes autocatalytic intramolecular processing in the endoplasmic reticulum. Evidence suggest that PCSK9 increases plasma LDL cholesterol by promoting degradation of the LDL receptor, which mediates LDL endocytosis in the liver, the major route of LDL clearance from circulation. The structure of PCSK9 protein shows that it has a signal sequence, followed by a prodomain, a catalytic domain that contains a conserved triad of residues (D186, H226 and S386), and a C-terminal domain. It is synthesized as a soluble 74-kDa precursor that undergoes autocatalytic cleavage in the ER, generating a 14-kDa prodomain and 60-kDa catalytic fragment. The autocatalytic activity has been shown to be required for secretion. After cleavage the prodomain remains tightly associated with the catalytic domain.

Antibodies to PCSK9 are described in, for example, WO 2008/057457, WO 2008/057458, WO 2008/057459, WO 2008/063382, WO 2008/125623, and US 2008/0008697. Anti-PCSK9 antibodies that are particularly well-suited for practicing the present invention are disclosed in US 2010/0166768 A1, the content of which is hereby incorporated by reference in its entirety.

### TECHNICAL PROBLEMS UNDERLYING THE PRESENT INVENTION

Statins are among the most widely used drugs in the world. Although statins generally exhibit an excellent safety profile, it is desirable to further optimize the safety profile by reducing the already low rate of unwanted side-effects.

Despite the widespread availability of lipid-lowering agents such as statins, approximately 30% of all adult patients treated for hypercholesterolemia in the United States between 1999 and 2006 failed to achieve their recommended LDL-C targets. Reasons for this include poor adherence to therapy, drug-resistance/intolerance and the positive relationship between adverse event rates and increasing dosage. Moreover, since the most effective lipid-lowering agents can only reduce LDL-C levels by up to 55%,target attainment rates in patients that require substantial reductions in LDL-C, such as those with familial hypercholesterolemia, are often significantly lower than might be expected. More effective lipid-lowering agents and treatment regimes are therefore required to improve target attainment rates in these patients.

There is a direct reciprocal relationship between liver function and cholesterol levels: Impaired liver function can cause elevated cholesterol levels and elevated cholesterol levels can contribute to liver damage as well. However, the administration of antibody therapeutics often represents a stress factor for the liver. This is a problem of antibody therapeutics intended for treatment of elevated cholesterol levels as - due to the above correlation of liver function and elevated cholesterol - many of the patients suffering from elevated cholesterol levels also have impaired liver function.

Quite surprisingly, the inventors of the present invention found that the administration of the anti-PCSK9 antibodies or fragments thereof according to present invention is well tolerable not only for patients with healthy or normal liver function but also for patients with moderately impaired liver function. This allows for the effective and safe antibody treatment of large patient groups and frequently renders testing for impaired liver function in advance to the antibody therapy dispensable.

This low liver impact of the anti-PCSK9 antibodies or fragments of present invention, especially when taken together with the fact that the co-administration of the antibodies or fragments enhances the efficacy of a statin therapy and allows for a reduction in the dosage of statins, leads to a synergy in reducing unwanted side-effects when compared with known cholesterol-lowering therapies.

The above overview does not necessarily describe all problems solved by the present invention.

### SUMMARY OF THE INVENTION

In a first aspect, present invention relates to a method for treating a disease or condition in which PCSK9 expression or activity causes an impact comprising
administering a therapeutic amount of an antibody or an antigen-binding fragment thereof which specifically binds hPCSK9 (human proprotein convertase subtilisin/kexin type 9) to a subject in need thereof,
wherein the subject in need thereof falls into one or more of the following groups of subjects:
(i) subjects having a serum LDL cholesterol (LDL-C) level of at least 100 mg/dL, *[at least 130 mg*/*dL, at least 160 mg*/*dL* / *at least 200 mg*/*dL];*
(ii) subjects having a serum HDL-C level of less than 40 mg/dL;
(iii) subjects having a serum cholesterol level of at least 200 mg/dL *[240 mg*/*dL]*;
(iv) subjects having a serum triacylglycerol level of at least 150 mg/dL *[at least 200 mg*/*dL; at least 500 mg*/*dL],* wherein said triacylglycerol level is determined after fasting for at least 8 hours;
(v) subjects being at least 18 years old *[at least 18* /*20* /*25* /*30* /*35* /*40* /*45* /*50* /*55* /*60* /*65* / *70*/*75 years old];*
(vi) subjects being at maximum 75 years old *[75* /*70* /65 / 60 / *55* / *50* / *45* / *40 35*/ *30*/ *25*/ *20 years]*;
(vii) subjects having a BMI of *25 [26* / *27* / *28* / *29* / *30* / *31* / *32* / *33* / *34* / *35* / *36* / *37* / *38* / *39]* or more;
(viii) male subjects;
(ix) female subjects;
(x) subjects in which the administration of said antibody or antigen-binding fragment thereof leads to a reduction in the serum LDL-C level by at least 30 mg/dL *[40 mg*/*dL; 50 mg*/*dL; 60 mg*/*dL; 70 mg*/*dL]* relative to predose level; or
(xi) subjects in which the administration of said antibody or antigen-binding fragment thereof leads to a reduction in the serum LDL-C level by at least 20% *[30%; 40%; 50%; 60%]* relative to predose level
(xii) subjects having mild and/or moderate hepatic impairment
(xiii) subjects with normal hepatic liver function
(xiv) subjects with high cardiovascular risk
(xv) subjects with coronary heart disease (CHD) or a CHD risk equivalent
(xvi) subjects with cardiovascular disease.

In a second aspect, present invention relates to a method for treating a disease or condition in which PCSK9 expression or activity causes an impact comprising

administering a therapeutic amount of an antibody or an antigen-binding fragment thereof which specifically binds hPCSK9 (human proprotein convertase subtilisin/kexin type 9) to a subject in need thereof,

wherein the subject in need thereof does not fall into one or more of the following groups of subjects:
(i) smokers;
(ii) persons being 76 years old or older;
(iii) persons being 17 years or younger;
(iv) persons suffering from hypertension;
(v) women who are pregnant;
(vi) women who are trying to become pregnant;
(vii) women who are breast-feeding;
(viii) persons who have or ever had severe hepatic impairment
(ix) persons who had any unexplained abnormal results from blood tests for liver function;
(x) persons who drink excessive amounts of alcohol;
(xi) persons having kidney problems;
(xii) persons suffering from hypothyroidism;
(xiii) persons suffering from muscle disorders;
(xiv) persons having encountered previous muscular problems during treatment with lipid-lowering medicine;
(xv) persons having serious problems with their breathing;
(xvi) persons taking one or more of the following medicines: medicines altering the way the immune systems works (e.g. ciclosporin or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or
(xvii) persons drinking more than 0.1 L of grapefruit juice per day;
(xviii) persons having a body mass index (BMI) of more than 40;
(xix) persons having a body mass index (BMI) of less than 18;
(xx) persons suffering from type 1 diabetes or type 2 diabetes;
(xxi) persons positive for hepatitis B or hepatitis C; or
(xxii) persons having a known sensitivity to monoclonal antibody therapeutics
(xxiii) persons having acute hepatitis,
(xxiv) persons having hepatic encephalopathy grade 2, 3, and 4,
(xxv) persons having uncontrolled clinically relevant cardiovascular, pulmonary, gastrointestinal, metabolic, hematological, neurological, psychiatric, systemic, ocular, gynecologic (if female), or infectious disease, or signs of acute illness
(xxvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness
and wherein the subject in need thereof preferably falls into one or more of the subject groups of the first aspect.

In a third aspect, present invention relates to a method of lowering cholesterol levels in a subject in need thereof, comprising:
(a) selecting a subject with a blood low density lipoprotein cholesterol (LDL-C) level greater than 100 mg/dL; and
(b) administering to said subject a composition comprising an antibody or antigen binding fragment thereof that specifically binds to human proprotein convertase subtilisin/kexin type 9 (hPCSK9); thereby lowering cholesterol levels in the subject in need thereof,
wherein the subject falls into one or more of the groups of subjects as listed in the first aspect and wherein the subject preferably does not fall into one or more of the groups of subjects as listed in the second aspect.

In a fourth aspect, present invention relates to a method of regulating the LDL level in the blood of a subject comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg,
wherein the subject falls into one or more of the groups of subjects as listed in the first aspect and wherein the subject preferably does not fall into one or more of the groups of subjects as listed in the second aspect.

In a fifth aspect, present invention relates to a method of preventing effects of a (persistently) increased LDL level in the blood comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg,
wherein the subject falls into one or more of the groups of subjects as listed in the first aspect and wherein the subject preferably does not fall into one or more of the groups of subjects as listed in the second aspect.

In a sixth aspect, present invention relates to a antibody or an antigen-binding fragment thereof which specifically binds hPCSK9 (human proprotein convertase subtilisin/kexin type 9) for use in the treatment of a disease or condition in which PCSK9 expression or activity causes an impact,
wherein the antibody or antigen-binding fragment thereof is for administration to a subject falling at least into one of the groups of subjects as listed in the first aspect and wherein the antibody or antigen-binding fragment thereof preferably is not for administration to a subject falling at least into one of the groups of subjects as listed in the second aspect.

In a seventh aspect, present invention relates to a Pharmaceutical composition comprising an antibody or antigen-binding fragment thereof which specifically binds hPCSK9 and a pharmaceutically acceptable excipient for use in a method according to one of the above aspects.

In an eighth aspect, present invention relates to an article of manufacture comprising
(a) a packaging material or container;
(b) an antibody or an antigen-binding fragment thereof which specifically binds hPCSK9; and
(c) a label or packaging insert, wherein the label or packaging insert indicates the following:
   (1) the antibody or antigen-binding fragment thereof can be administered to subjects falling into one or more groups of subjects as recited in the first aspect;
   (2) and the label or packaging insert preferably also indicates that the antibody or antigen-binding fragment thereof is contraindicated for administration to subjects falling into one or more groups of subjects as recited in the second aspect.

In a ninth aspect, present invention relates to a method for preparing an article of manufacture of present invention comprising packaging the antibody , pharmaceutical composition, the liquid or dry formulation or the unit dosage form or forms according to present invention in a container and assembling them with a label or packaging insert.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art to which this invention belongs.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents (for example: patents, patent applications, scientific publications, manufacturer's specifications, instructions, GenBank Accession Number sequence submissions etc.) are cited throughout the text of this specification. Nothing herein is to be construed as an admission that the invention is not entitled to antedate such disclosure by virtue of prior invention. Some of the documents cited herein are characterized as being *"incorporated by reference".* In the event of a conflict between the definitions or teachings of such incorporated references and definitions or teachings recited in the present specification, the text of the present specification takes precedence.

Sequences: All sequences referred to herein are disclosed in the attached sequence listing that, with its whole content and disclosure, is a part of this specification.

The term "about" when used in connection with a numerical value is meant to encompass numerical values within a range having a lower limit that is 5% smaller than the indicated numerical value and having an upper limit that is 5% larger than the indicated numerical value.

The term "human proprotein convertase subtilisin/kexin type 9" or "hPCSK9", as used herein, refers to hPCSK9 having the nucleic acid sequence shown in SEQ ID NO: 754 and the amino acid sequence of SEQ ID NO: 755, or a biologically active fragment thereof.

The terms "specifically binds", "specific binding" or the like, mean that an antibody or antigen-binding fragment thereof forms a complex with an antigen that is relatively stable under physiologic conditions. Specific binding can be characterized by an equilibrium dissociation constant of at least about 1x10⁻⁶ M or less (e.g., a smaller K_{D} denotes a tighter binding). Methods for determining whether two molecules specifically bind are well known in the art and include, for example, equilibrium dialysis, surface plasmon resonance, and the like. An isolated antibody that specifically binds hPCSK9 may, however, exhibit cross-reactivity to other antigens such as PCSK9 molecules from other species. Moreover, multi-specific antibodies (e.g., bispecifics) that bind to hPCSK9 and one or more additional antigens are nonetheless considered antibodies that "specifically bind" hPCSK9, as used herein.

The term "K_{D}", as used herein, is intended to refer to the equilibrium dissociation constant of a particular antibody-antigen interaction. The equilibrium dissociation constant is typically measured in "mol/L" (abbreviated as "M").

By the term "slow off rate", "Koff" or "kd" is meant an antibody that dissociates from hPCSK9 with a rate constant of 1 x 10⁻³ s⁻¹ or less, preferably 1 x 10⁻⁴ s⁻¹ or less, as determined by surface plasmon resonance, e.g., BIACORE™.

The term "high affinity" antibody refers to those mAbs having a binding affinity to hPCSK9 of at least 10⁻¹⁰ M; preferably 10⁻¹¹ M; even more preferably 10⁻¹² M, as measured by surface plasmon resonance, e.g., BIACORE™ or solution-affinity ELISA.

The term "surface plasmon resonance", as used herein, refers to an optical phenomenon that allows for the analysis of real-time biospecific interactions by detection of alterations in protein concentrations within a biosensor matrix, for example using the BIACORE™ system (Pharmacia Biosensor AB, Uppsala, Sweden and Piscataway, N.J.).

An "epitope", also known as antigenic determinant, is the region of an antigen that is recognized by the immune system, specifically by antibodies, B cells, or T cells. As used herein, an "epitope" is the part of an antigen capable of binding to an antibody or antigen-binding fragment thereof as described herein. In this context, the term "binding" preferably relates to a "specific binding", as defined herein. Epitopes usually consist of chemically active surface groupings of molecules such as amino acids, sugar side chains, phosphoryl groups, or sulfonyl groups and may have specific three-dimensional structural characteristics and/or specific charge characteristics. Conformational and non-conformational epitopes can be distinguished in that the binding to the former but not the latter is lost in the presence of denaturing solvents.

A "paratope" is the part of an antibody that specifically binds to the epitope.

The term "antibody", as used herein, is intended to refer to immunoglobulin molecules comprised of four polypeptide chains, two heavy (H) chains and two light (L) chains interconnected by disulfide bonds. The term "antibody" also includes all recombinant forms of antibodies, in particular of the antibodies described herein, e.g. antibodies expressed in prokaryotes, unglycosylated antibodies, and any antigen-binding antibody fragments and derivatives as described below. Each heavy chain is comprised of a heavy chain variable region ("HCVR" or "VH") and a heavy chain constant region (comprised of domains CH1, CH2 and CH3). Each light chain is comprised of a light chain variable region ("LCVR or "VL") and a light chain constant region (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

Substitution of one or more CDR residues or omission of one or more CDRs is also possible. Antibodies have been described in the scientific literature in which one or two CDRs can be dispensed with for binding. Padlan et al. (1995 FASEB J. 9:133-139) analyzed the contact regions between antibodies and their antigens, based on published crystal structures, and concluded that only about one fifth to one third of CDR residues actually contact the antigen. Padlan also found many antibodies in which one or two CDRs had no amino acids in contact with an antigen (see also, Vajdos et al. 2002 J Mol Biol 320:415-428).

CDR residues not contacting antigen can be identified based on previous studies (for example residues H60-H65 in CDRH2 are often not required), from regions of Kabat CDRs lying outside Chothia CDRs, by molecular modeling and/or empirically. If a CDR or residue(s) thereof is omitted, it is usually substituted with an amino acid occupying the corresponding position in another human antibody sequence or a consensus of such sequences. Positions for substitution within CDRs and amino acids to substitute can also be selected empirically. Empirical substitutions can be conservative or non-conservative substitutions.

The term "antigen-binding fragment" of an antibody (or simply "binding portion"), as used herein, refers to one or more fragments of an antibody that retain the ability to specifically bind to hPCSK9. It has been shown that the antigen-binding function of an antibody can be performed by fragments of a full-length antibody. Examples of binding fragments encompassed within the term "antigen-binding fragment" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is a binding-domain immunoglobulin fusion protein comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies. Further examples of "antigen-binding fragments" are so-called microantibodies, which are derived from single CDRs. For example, Heap et al. describe a 17 amino acid residue microantibody derived from the heavy chain CDR3 of an antibody directed against the gp120 envelope glycoprotein of HIV-1 (Heap CJ et al. (2005) J. Gen. Virol. 86:1791-1800). Other examples include small antibody mimetics comprising two or more CDR regions that are fused to each other, preferably by cognate framework regions. Such a small antibody mimetic comprising VH CDR1 and VL CDR3 linked by the cognate VH FR2 has been described by Qiu et al. (Qiu X-Q, et al. (2007) Nature biotechnology 25(8):921-929).

Thus, the term "antibody or antigen-binding fragment thereof', as used herein, refers to immunoglobulin molecules and immunologically active portions of immunoglobulin molecules, i.e. molecules that contain an antigen-binding site that immunospecifically binds an antigen.

Antibodies and antigen-binding fragments thereof usable in the invention may be from any animal origin including birds and mammals. Preferably, the antibodies or fragments are from human, chimpanzee, rodent (e.g. mouse, rat, guinea pig, or rabbit), chicken, turkey, pig, sheep, goat, camel, cow, horse, donkey, cat, or dog origin. It is particularly preferred that the antibodies are of human or murine origin. Antibodies of the invention also include chimeric molecules in which an antibody constant region derived from one species, preferably human, is combined with the antigen binding site derived from another species, e.g. mouse. Moreover antibodies of the invention include humanized molecules in which the antigen binding sites of an antibody derived from a non-human species (e.g. from mouse) are combined with constant and framework regions of human origin. Antibody molecules of the invention particularly also include fully human antibody molecules such as fully human monoclonal antibodies that are generated by in vitro systems such as phage display libraries or are generated in vivo, e.g. in transgenic mice carrying human immunoglobulin genes as transgenes in their genome.

As exemplified herein, antibodies of the invention can be obtained directly from hybridomas which express the antibody, or can be cloned and recombinantly expressed in a host cell (e.g., a CHO cell, or a lymphocytic cell). Further examples of host cells are microorganisms, such as *E. coli,* and fungi, such as yeast. Alternatively, they can be produced recombinantly in a transgenic non-human animal or plant.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another species or class. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human is less likely to elicit an immune response from a human subject when the antibodies are injected than would the constant region from a non-human source. However, the definition is not limited to this particular example.

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen-binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "fully human antibody" refers to an immunoglobulin molecule, in which all variable or constant domains or regions present in the molecule are from one or more human immunoglobulin molecules and comprises a fully human mAb or fragment tereof as well as bispecifics, fusion proteins etc., in which all immunoglobulin-derived domains and regions present in the molecule are derived from human immunoglobulin(s). The terms "fully human antibody" and "human antibody" are used synonymously herin.

Different methods for humanizing antibodies are known to the skilled person, as reviewed by Almagro & Fransson, the content of which is herein incorporated by reference in its entirety (Almagro JC and Fransson J (2008) Frontiers in Bioscience 13:1619-1633). Almagro & Fransson distinguish between rational approaches and empirical approaches. Rational approaches are characterized by generating few variants of the engineered antibody and assessing their binding or any other property of interest. If the designed variants do not produce the expected results, a new cycle of design and binding assessment is initiated. Rational approaches include CDR grafting, Resurfacing, Superhumanization, and Human String Content Optimization. In contrast, empirical approaches are based on the generation of large libraries of humanized variants and selection of the best clones using enrichment technologies or high-throughput screening. Accordingly, empirical approaches are dependent on a reliable selection and/or screening system that is able to search through a vast space of antibody variants. *In vitro* display technologies, such as phage display and ribosome display fulfill these requirements and are well-known to the skilled person. Empirical approaches include FR libraries, Guided selection, Framework-shuffling, and Humaneering.

The term "human antibody", as used herein, is intended to include antibodies having variable and constant regions derived from human germline immunoglobulin sequences. The human mAbs of the invention may include amino acid residues not encoded by human germline immunoglobulin sequences (e.g., mutations introduced by random or site-specific mutagenesis *in vitro* or by somatic mutation *in vivo*), for example in the CDRs and in particular CDR3. However, the term "human antibody", as used herein, is not intended to include mAbs in which CDR sequences derived from the germline of another mammalian species (e.g., mouse), have been grafted onto human FR sequences. Human antibodies of the invention include antibodies isolated from human immunoglobulin libraries or from animals transgenic for one or more human immunoglobulin and that do not express endogenous immunoglobulins, as described for example in U.S. Patent No. 5,939,598 by Kucherlapati & Jakobovits.

The term "monoclonal antibody" as used herein refers to a preparation of antibody molecules of single molecular composition. A monoclonal antibody displays a single binding specificity and affinity for a particular epitope. In one embodiment, the monoclonal antibodies are produced by a hybridoma which includes a B cell obtained from a non-human animal, e.g. mouse, fused to an immortalized cell.

The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g. from a transfectoma, (c) antibodies isolated from a recombinant, combinatorial antibody library, and (d) antibodies prepared, expressed, created or isolated by any other means that involve splicing of immunoglobulin gene sequences to other DNA sequences.

The term "transfectoma", as used herein, includes recombinant eukaryotic host cells expressing an antibody, such as CHO cells, NS/0 cells, HEK293 cells, HEK293T cells, plant cells, or fungi, including yeast cells.

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

Thus, "antibodies and antigen-binding fragments thereof' suitable for use in the present invention include, but are not limited to, polyclonal, monoclonal, monovalent, bispecific, heteroconjugate, multispecific, recombinant, heterologous, heterohybrid, chimeric, humanized (in particular CDR-grafted), deimmunized, or human antibodies, Fab fragments, Fab' fragments, F(ab')₂ fragments, fragments produced by a Fab expression library, Fd, Fv, disulfide-linked Fvs (dsFv), single chain antibodies (e.g. scFv), diabodies or tetrabodies (Holliger P. et al. (1993) Proc. Natl. Acad. Sci. U.S.A. 90(14), 6444-6448), nanobodies (also known as single domain antibodies), anti-idiotypic (anti-Id) antibodies (including, e.g., anti-Id antibodies to antibodies of the invention), and epitope-binding fragments of any of the above.

The antibodies described herein are preferably isolated. An "isolated antibody", as used herein, is intended to refer to an antibody that is substantially free of other mAbs having different antigenic specificities (e.g., an isolated antibody that specifically binds hPCSK9 is substantially free of mAbs that specifically bind antigens other than hPCSK9). An isolated antibody that specifically binds hPCSK9 may, however, have cross-reactivity to other antigens, such as PCSK9 molecules from other species.

As used herein, a "PCSK9 antagonist" denotes a compound that inhibits at least one biological activity of PCSK9, preferably the proteinase activity of PCSK9. Preferred PCSK9 antagonists are characterized in that they bind from 10% to 100% (preferably from 50% to 100%) of the PCSK9 present in the blood when used in stoichiometric amounts. Preferred PCSK9 antagonists of the present invention are neutralizing antibodies.

A "neutralizing antibody", as used herein (or an "antibody that neutralizes PCSK9 activity"), is intended to refer to an antibody whose binding to hPCSK9 results in inhibition of at least one biological activity of PCSK9, preferably inhibition of the proteinase activity of PCSK9. This inhibition of the biological activity of PCSK9 can be assessed by measuring one or more indicators of PCSK9 biological activity by one or more of several standard *in vitro* or *in vivo* assays known in the art. Such assays are described for example in US 2010/0166768 A1, the content of which is hereby incorporated by reference in its entirety.

Since PCSK9 increases plasma LDL cholesterol by promoting degradation of the LDL receptor, the activity of PCSK9 has an effect on several diseases associated with increased plasma LDL cholesterol levels. Accordingly, PCSK9 antagonists, such as neutralizing anti-hPCSK9 antibodies or antigen-binding fragments thereof, are useful to reduce elevated total cholesterol, non-HDL cholesterol, LDL cholesterol, and/or apolipoprotein B100 (ApoB100). Consequently, PCSK9 antagonists are useful for ameliorating, improving, inhibiting or preventing several such diseases, including without limitation hypercholesterolemia, hyperlipidemia, dyslipidemia (such as mixed dyslipidemia), atherosclerosis and cardiovascular diseases.

In the context of present application, the term "a disease or condition in which PCSK9 expression or activity causes an impact" is understood to comprise any disease or condition in which the application or administration of a PCSK-9 inhibitory, activatory, antagonistic, agonistic, neutralizing or blocking molecule, such as an inhibitory, activatory, antagonistic, agonistic, neutralizing or blocking antibody causes an impact and preferably causes an amelioration, improvement, inhibition or prevention of the disease or condition.

Exemplary diseases or conditions that can be ameliorated, improved, inhibited or prevented by administration of an PCSK9 antibody include without limitation hypercholesterolemia, hyperlipidemia, dyslipidemia (such as mixed dyslipidemia), atherosclerosis and cardiovascular disease(s).

In specific embodiments, the anti-PCSK9 antibodies or antigen-binding fragments thereof described herein may be conjugated to a therapeutic moiety ("immunoconjugate"), such as a cytotoxin, a chemotherapeutic drug, an immunosuppressant or a radioisotope.

A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent or degree of similarity may be adjusted upwards to correct for the conservative nature of the substitution. Means for making this adjustment are well known to those of skill in the art. See, e.g., Pearson (1994) Methods Mol. Biol. 24: 307- 331. Examples of groups of amino acids that have side chains with similar chemical properties include
1) aliphatic side chains: glycine, alanine, valine, leucine and isoleucine;
2) aliphatic- hydroxyl side chains: serine and threonine;
3) amide-containing side chains: asparagine and glutamine;
4) aromatic side chains: phenylalanine, tyrosine, and tryptophan;
5) basic side chains: lysine, arginine, and histidine;
6) acidic side chains: aspartate and glutamate, and
7) sulfur-containing side chains: cysteine and methionine.

Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256: 1443-45. A "moderately conservative" replacement is any change having a nonnegative value in the PAM250 log-likelihood matrix. Given the known genetic code, and recombinant and synthetic DNA techniques, the skilled scientist can readily construct DNAs encoding conservative amino acid variants.

As used herein, "non-conservative substitutions" or "non-conservative amino acid exchanges" are defined as exchanges of an amino acid by another amino acid listed in a different group of the seven standard amino acid groups 1) to 7) shown above.

The term "substantial identity" or "substantially identical," when referring to a nucleic acid or fragment thereof, indicates that, when optimally aligned with appropriate nucleotide insertions or deletions with another nucleic acid (or its complementary strand), there is nucleotide sequence identity in at least about 90%, and more preferably at least about 95%, 96%, 97%, 98% or 99% of the nucleotide bases, as measured by any well-known algorithm of sequence identity, such as FASTA, BLAST or GAP, as discussed below.

As applied to polypeptides, the term "substantial similarity" or "substantially similar" means that two peptide sequences, when optimally aligned, such as by the programs GAP or BESTFIT using default gap weights, share at least 80% sequence identity, and preferably at least 90%, 95%, 98% or 99% or 99.5% sequence identity. Preferably, residue positions which are not identical differ by conservative amino acid substitutions.

Sequence similarity for polypeptides is typically measured using sequence analysis software. Protein analysis software matches similar sequences using measures of similarity assigned to various substitutions, deletions and other modifications, including conservative amino acid substitutions. For instance, GCG software contains programs such as GAP and BESTFIT which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA with default or recommended parameters; a program in GCG Version 6.1. FASTA (e.g., FASTA2 and FASTA3) provides alignments and percent sequence identity of the regions of the best overlap between the query and search sequences (Pearson (2000) *supra*). Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215: 403 410 and (1997) Nucleic Acids Res. 25:3389 402, each of which is herein incorporated by reference.

When percentages of sequence identity are referred to in the present application, these percentages are calculated in relation to the full length of the longer sequence, if not specifically indicated otherwise. This calculation in relation to the full length of the longer sequence applies both to nucleic acid sequences and to polypeptide sequences.

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease or disorder means preventing that a disorder occurs in subject.

The terms, "disease", "disorder" and "condition" are used synonymously.

As used herein, the expressions "is for administration" and "is to be administered" have the same meaning as "is prepared to be administered". In other words, the statement that an active compound "is for administration" has to be understood in that said active compound has been formulated and made up into doses so that said active compound is in a state capable of exerting its therapeutic activity.

The terms "therapeutically effective amount" or "therapeutic amount" are intended to mean that amount of a drug or pharmaceutical agent that will elicit the biological or medical response of a tissue, a system, animal or human that is being sought by a researcher, veterinarian, medical doctor or other clinician. The term "prophylactically effective amount" is intended to mean that amount of a pharmaceutical drug that will prevent or reduce the risk of occurrence of the biological or medical event that is sought to be prevented in a tissue, a system, animal or human by a researcher, veterinarian, medical doctor or other clinician. Particularly, the dosage a patient receives can be selected so as to achieve the amount of LDL (low density lipoprotein) cholesterol lowering desired; the dosage a patient receives may also be titrated over time in order to reach a target LDL level. The dosage regimen utilizing an antibody or an antigen-binding fragment thereof as described herein is selected in accordance with a variety of factors including type, species, age, weight, body mass index, sex and medical condition of the patient; the severity of the condition to be treated; the potency of the compound chosen to be administered; the route of administration; the purpose of the administration; and the renal and hepatic function of the patient.

As used herein, a "patient" means any human or non-human animal, such as mammal, reptile or bird who may benefit from a treatment with the antibodies and antigen-biding fragments thereof described herein. Preferably, a "patient" is selected from the group consisting of laboratory animals (e.g. mouse or rat), domestic animals (including e.g. guinea pig, rabbit, chicken, turkey, pig, sheep, goat, camel, cow, horse, donkey, cat, or dog), rodent or primates including chimpanzee, gorilla, bonobo and human beings. It is particularly preferred that the "patient" is a human being.

The terms "subject" or "individual" are used interchangeably herein. As used herein, a "subject" refers to a human or a non-human animal (e.g. a mammal, avian, reptile, fish, amphibian or invertebrate; preferably an individual that can either benefit from one of the different aspects of present invention (e.g. a method of treatment or a drug identified by present methods) or that can be used as laboratory animal for the identification or characterisation of a drug or a method of treatment. The subject can e.g. be a human, a wild-animal, domestic animal or laboratory animal; examples comprise: mammal, e.g. human, non-human primate (chimpanzee, bonobo, gorilla), dog, cat, rodent (e.g. mouse, guinea pig, rat, hamster or rabbit, horse, donkey, cow, sheep, goat, pig, camel; avian, such as duck, dove, turkey, goose or chick; reptile such as: turtle, tortoise, snake, lizard, amphibian such as frog (e.g. Xenopus laevis); fish such as koy or zebrafish; invertebrate such as a worm (e.g. c.elegans) or an insect (such as a fly, e.g. drosophila melanogaster). The term subject also comprises the different morphological developmental stages of avian, fish, reptile or insects, such as egg, pupa, larva or imago. The term "subject" comprises the term "patient". According to a preferred embodiment, the subject is a "patient".

As used herein, "unit dosage form" refers to physically discrete units suitable as unitary dosages for human and/or animal subjects, each unit containing a predetermined quantity of active material (e.g., about 50 to about 500mg of PCSK5 antibody and/or of e.g. 0.05mg to 100 mg HMG-CoA reductase inhibitor and/or about 7 to 15 mg of ezetimibe) calculated to produce the desired therapeutic effect in association with the required pharmaceutical diluent, carrier or vehicle. The unit dosage form can comprise one or more active ingredients. Conceivable are for example unit dosage forms comprising only the antibody or the HMG-CoA reductase inhibitor or another lipid lowering drug. Conceivable are also for example unit dosage forms comprising the antibody and the other lipid lowering drug such as ezetimibe or the antibody and the HMG CoA reductase inhibitor such as a statin or comprising the HMG CoA reductase inhibitor and another lipid lowering drug such as comprising a statin and ezetimibe. One example concerns a unit dosage form comprising one or half an effective dose of the antibody of fragment thereof of present invention (e.g. comprising about 1 ml injection solution comprising 300 mg of the antibody or comprising about 0.5 ml injection solution comprising 50, 100 or 150 mg of the antibody). Another example of a unit dosage form concerns a unit dosage form for oral administration such as a tablet or capsule comprising about 80 mg of atorvastatin. Another example of a unit dosage form concerns a unit dosage form for oral administration such as a tablet or capsule comprising about 10 mg ezetimibe. Another example of a unit dosage form concerns a unit dosage form for oral administration such as a tablet or capsule comprising an effective dose of a statin such as atorvastatin or simvastatin and an effective dose of ezetimibe. The specifications for the novel unit dosage forms of this invention are dictated by and are directly dependent on (a) the unique characteristics of the active material and the particular therapeutic effect to be achieved, and (b) the limitation inherent in the art of compounding such an active material for therapeutic use in animals or humans, as disclosed in this specification, these being features of the present invention. Examples of suitable unit dosage forms in accord with this invention are vials, tablets, capsules, troches, suppositories, powder packets, wafers, cachets, ampules, segregated multiples of any of the foregoing, and other forms as herein described or generally known in the art. One or more such unit dosage forms of the antibody can be comprised in an article of manufacture of present invention, optionally further comprising one or more unit dosage forms of an HMG-CoA reductase inhibitor (e.g. a blister of tablets comprising as active ingredient the HMG-CoA reductase inhibitor) and optionally further comprising one or more unit dosage forms of another lipid lowering drug (e.g. a blister of tablets comprising as active ingredient ezetimibe). One or more such unit dosage forms of the antibody can also be comprised in an article of manufacture of present invention further comprising one or more unit dosage forms, wherein the further comprised unit dosage forms comprise two active ingredients such as an HMG-CoA reductase inhibitor and ezetimibe.

The term "active material" refers to any material with therapeutic activity, such as one or more active ingredients. The active ingredients to be employed as therapeutic agents can be easily prepared in such unit dosage form with the employment of pharmaceutical materials which themselves are available in the art and can be prepared by established procedures.

The following preparations are illustrative of the preparation of the unit dosage forms of the present invention, and not as a limitation thereof. Several dosage forms may be prepared embodying the present invention. For example, a unit dosage per vial may contain 0,5 ml, 1 ml, 2 ml, 3 ml, 4 ml, 5 ml, 6 ml, 7 ml, 8 ml, 9 ml, 10 ml, 15 ml, or 20 ml of PCSK5 antibody or a fragment thereof ranging from about 40 to about 500 mg of PCSK5 antibody. If necessary, these preparations can be adjusted to a desired concentration by adding a sterile diluent to each vial. In one embodiment, the ingredients of formulation of the invention are supplied either separately or mixed together in unit dosage form, for example, as a dry lyophilized powder or water free concentrate in a hermetically sealed container such as a vial, an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The formulations as herein described include bulk drug compositions useful in the manufacture of pharmaceutical compositions (e.g., compositions that are suitable for administration to a subject or patient) which can be used in the preparation of unit dosage forms. In a preferred embodiment, a composition of the invention is a pharmaceutical composition. Such compositions comprise a prophylactically or therapeutically effective amount of one or more prophylactic or therapeutic agents (e.g., an antibody of the invention, an HMG-CoA reductase inhibitor or another prophylactic or therapeutic agent), and a pharmaceutically acceptable carrier. Preferably, the pharmaceutical compositions are formulated to be suitable for the route of administration to a subject.

The active materials, agents or ingredients (e.g. antibodies or fragments thereof and HMG-CoA reductase inhibitors) can be formulated as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

In a specific embodiment, the term "pharmaceutically acceptable" means approved by a regulatory agency of the U.S. Federal or a state government or the EMA (European Medicines Agency) or listed in the U.S. Pharmacopeia Pharmacopeia (United States Pharmacopeia-33/National Formulary-28 Reissue, published by the United States Pharmacopeial Convention, Inc., Rockville Md., publication date: April 2010) or other generally recognized pharmacopeia for use in animals, and more particularly in humans. The term "carrier" refers to a diluent, adjuvant {e.g., Freund's adjuvant (complete and incomplete)), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. For the use of (further) excipients and their use see also "Handbook of _ Pharmaceutical Excipients", fifth edition, R.C.Rowe, P.J. Seskey and S.C. Owen, Pharmaceutical Press, London, Chicago. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The formulation should suit the mode of administration.

Generally, the ingredients of compositions of the invention are supplied either separately or mixed together in a unit dosage form, for example, as a dry formulation for dissolution such as a lyophilized powder, freeze-dried powder or water free concentrate in a hermetically sealed container, such as an ampoule or sachette indicating the quantity of active agent. The ingredients of compositions of the invention can also be supplied as admixed liquid formulation (i.e. injection or infusion solution) in a hermetically sealed container such as an ampoule, sachette, a pre-filled syringe or autoinjector, or a cartridge for a reusable syringe or applicator (e.g. pen or autoinjector). Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The invention also provides that the formulation is packaged in a hermetically sealed container such as an ampoule or sachette indicating the quantity of antibody. In one embodiment, the formulation of the invention comprising an antibody is supplied as a dry formulation, such as a sterilized lyophilized powder, freeze-dried powder, spray -dried powder or water free concentrate in a hermetically sealed container and can be reconstituted, e.g., with water or saline to the appropriate concentration for administration to a subject. In another embodiment the antibody or antigen binding fragment thereof is supplied as a liquid formulation such as an injection or infusion solution. In one embodiment, the formulation of the invention comprising an antibody is supplied as a dry formulation or as a liquid formulation in a hermetically sealed container at a unit dosage of at least 40 mg, at least 50 mg, more preferably at least 75 mg, at least 100 mg, at least 150 mg, at least 200 mg, at least 250 mg, at least 300 mg, at least 350 mg, at least 400 mg, at least 450 mg, or at least 500 mg, of antibody or antigen-binding fragment thereof. The lyophilized formulation of the invention comprising an antibody should be stored at between 2 and 8° C in its original container and the antibody should be administered within 12 hours, preferably within 6 hours, within 5 hours, within 3 hours, or within 1 hour after being reconstituted. The formulation of the invention comprising antibodies can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Adult subjects are characterized as having "hypertension" or a high blood pressure when they have a systolic blood pressure of more than 140 mmHg and/or a diastolic blood pressure of more than 90 mmHg.

Specific populations treatable by the therapeutic methods of the invention include subjects with one or more of the following conditions: subjects indicated for LDL apheresis, subjects with PCSK9-activating mutations (gain of function mutations, "GOF"), subjects with elevated total cholesterol levels, subjects with elevated low-density lipoprotein cholesterol (LDL-C) levels, subjects with primary hypercholesterolemia, such as subjects primary with Familial or Non-Familial Hypercholesterolemia, subjects with heterozygous Familial Hypercholesterolemia (heFH); subjects with hypercholesterolemia, especially primary hypercholesterolemia, who are statin intolerant or statin uncontrolled; and subjects at risk for developing hypercholesterolemia who may be preventably treated. Other indications include hyperlipidemia and dyslipidemia (such as mixed dyslipidemia), especially if associated with secondary causes such as Type 2 diabetes mellitus, cholestatic liver diseases (primary biliary cirrhosis), nephrotic syndrome, hypothyroidism, obesity; and the prevention and treatment of atherosclerosis and cardiovascular diseases, such as coronary heart disease (CHD). The conditions or disorders as listed for the above populations or subjects are conditions or disorders, for which treatment with the antibody of the invention is especially suitable.

However, depending on the severity of the afore-mentioned diseases and conditions, the treatment of subjects with the antibodies and antigen-binding fragments of the invention may be contraindicated for certain diseases and conditions.

The term "adverse effect" (or side-effect) refers to a harmful and undesired effect resulting from a medication. An adverse effect may be termed a "side effect", when judged to be secondary to a main or therapeutic effect. Some adverse effects occur only when starting, increasing or discontinuing a treatment. Adverse effects may cause medical complications of a disease and negatively affect its prognosis. Examples of side effects are allergic reactions, vomiting, headache, or dizziness or any other effect herein described.

The term "statin intolerance" as used herein refers to the inability to tolerate at least two different statins at the lowest approved dose and dose regimen (i.e. for example being intolerant to treatment with atorvastatin and being intolerant to treatment with rosuvastatin and possibly also being intolerant to treatment with one or more other statins).

As used herein, "treat", "treating" or "treatment" of a disease or disorder means accomplishing one or more of the following: (a) reducing the severity and/or duration of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "prevent", "preventing", "prevention", or "prophylaxis" of a disease, condition or disorder means preventing that a disorder, disease or condition occurs in subject.

Elevated total cholesterol levels are understood in the context of present invention to preferably be total cholesterol levels of 200 mg/dL or more, especially 240mg/dL or more. International treatment guidelines recommend lowering LDL-C to <2.0-2.6 mmol/L (<77-100 mg/dL) in patients with established cardiovascular diseases (CVDs) and to <1.8-2.0 mmol/L (<70-77 mg/dL) in high-risk groups such as those with CVDs plus diabetes, smoking, poorly controlled hypertension, metabolic syndrome, or previous myocardial infarction. Elevated LDL-C levels are thus understood in the context of present invention to be LDL-C levels of 77 mg/dL or more (especially for patients with one or more of the following characteristics: established CVDs and one or more of [diabetes, with smoking, poorly controlled hypertension, metabolic syndrome or previous myocardial infarction]) and 100 mg/dL or more (especially for patients with established CVDs) ,130mg/dL or more , or 160 mg/dL or 190mg/dL or more. Low High-density lipoprotein levels (HDL-levels) in the context of present invention are understood to be preferably less than about 40mg/dL.

The terms "uncontrolled by lipid lowering agent(s)" or "not or not adequately controlled by lipid lowering or modifying agents" or "not or not adequately controlled by therapy or treatment with lipid lowering or modifying agents" etc. especially in context of hyperlipidemia, hypercholesterolemia etc., are used synonymously herein and refer to conditions such as hyperlipidemia, wherein treatment with a lipid modifying, e.g. a statin (i.e. regular administration of a statin such as atorvastatin to a patient) does not significantly lower total cholesterol or LDL-C or does not suffice to establish normolipidemic levels for the patient or to establish a lipidemic (e.g. total cholesterol or LDL-C) level that is not a significant risk factor for developing cardiovascular diseases. This means for example that therapy with the lipid modifying or lipid lowering agent does not suffice to establish levels of less than 130 mg/dL in general, or of less than 100 mg/dL (e.g. about ≥77mg/dL to about 100 mg/dL), especially in patients with established cardiovascular diseases, or to establish levels of about less than 77 mg/dL (e.g. about ≥70-77 mg/dL), especially in high-risk groups such as those with CVDs plus diabetes, smoking, poorly controlled hypertension, metabolic syndrome, or previous myocardial infarction.

The terms "lipid modifying therapy" or "lipid lowering therapy" as used herein refer to the treatment or therapy with a lipid modifying or a lipid lowering agent.

The terms "uncontrolled by statins" or "statin-resistant" or "not adequately controlled by statins or by statin therapy" especially in the context of hyperlipidemia, hypercholesterolemia etc., are used synonymously herein and refer to conditions such as hyperlipidemia, wherein treatment with a statin (i.e. regular administration of a statin such as atorvastatin to a patient) does not significantly lower total cholesterol or LDL-C or does not suffice to establish normolipidemic levels for the patient or to establish a lipidemic (e.g. total cholesterol or LDL-C) level that is not a significant risk factor for developing cardiovascular diseases. This means for example that statin-treatment does not suffice to establish levels of less than 130 mg/dL in general, or of less than 100 mg/dL (e.g. about ≥77mg/dL to about 100 mg/dL), especially in patients with established cardiovascular diseases, or to establish levels of about less than 77 mg/dL (e.g. about ≥70-77 mg/dL), especially in high-risk groups such as those with CVDs plus diabetes, smoking, poorly controlled hypertension, metabolic syndrome, or previous myocardial infarction. In the context of present invention, statin resistance preferably relates to atorvastatin resistance.

The terms "Liver function" or "hepatic impairment", as used herein and especially if used in the context of mildly or moderately impaired liver function or mild or moderate hepatic impairment: As used herein, the liver function or hepatic impairment is preferably determined according to the Child-Pugh score. The Child-Pugh score (sometimes the Child-Turcotte-Pugh score) is used to assess the prognosis of chronic liver disease, mainly cirrhosis. Although it was originally used to predict mortality during surgery, it is now frequently used to determine the prognosis, as well as the required strength of treatment and the necessity of liver transplantation. Scoring: The score employs five clinical measures of liver disease. Each measure is scored 1-3, with 3 indicating most severe derangement.

| Measure | 1 point | 2 points | 3 points |
|---|---|---|---|
| Total bilirubin, µmol/l (mg/dl) | <34 (</=2) | 34-50 (2-3) | >50 (>3) |
| Serum albumin, g/l | >35 | 28-35 | <28 |
| PT INR | <1.7 | 1.71-2.30 | > 2.30 |
| Ascites | None | Mild | Moderate to Severe |
| Hepatic encephalopathy | None | Grade I-II (or suppressed with medication) | Grade III-IV (or refractory) |

Different textbooks and publications use different measures. Some older reference works substitute PT prolongation for INR. In primary sclerosing cholangitis (PSC) and primary biliary cirrhosis (PBC), the bilirubin references are changed to reflect the fact that these diseases feature high conjugated bilirubin levels. The upper limit for 1 point is 68 µmol/l (4 mg/dl) and the upper limit for 2 points is 170 µmol/l (10 mg/dl). Interpretation: Chronic liver disease is classified into Child-Pugh class A to C, employing the added score from above.

| Points | Class | One year survival | Two year survival |
|---|---|---|---|
| 5-6 | A | 100% | 85% |
| 7-9 | B | 81% | 57% |
| 10-15 | C | 45% | 35% |

In the context of present invention, the term mild hepatic impairment preferably refers to Child-Pugh Class A, the term moderate hepatic impairment preferably refers to Child-Pugh Class B and the term severe hepatic impairment preferably refers to Child-Pugh Class C. A liver status of 0-4 points in the Child-Pugh Scoring system corresponds to a healthy hepatic liver function or a healthy liver status. There are also other classes of scoring system for determining the severity of impairment of liver function. The skilled artisan knows to which classes of another scoring system a certain Child-Pugh Class corresponds.

In the context of present invention, the term "lipid modifying agent" refers to any agent that is capable of modifying the lipid level in the body, particularly in the plasma and/or liver, lipid modifying agents encompass lipid lowering agents.

The term "lipid lowering agent" or "lipid modifying agent" particularly refers to any agent that is capable of lowering the total cholesterol and/or LDL-C level in the body (particularly in the plasma and/or liver) and/or of increasing HDL-C levels in the body (particularly in the plasma and/or liver) and/or of lowering the triglyceride levels in the body (particularly in the plasma and/or liver).

A lipid lowering or modifying agent can be any synthetic, proteinaceous, nucleic acid or other agent, composition or isolated natural product.

The term "lipid lowering agent" is used synonymously with the term "hypolipidemic agent", or "antihyperlipidemic agent" or "lipid-lowering drug (LLD)" and refers to a diverse group of agents or compositions that can be used in the treatment of one or more hyperlipidemia.

The term lipid lowering agent or LLD particularly includes, but is not limited to any agent that
(1) induces a cellular depletion of cholesterol synthesis by inhibiting 3-hydroxy-3-methylglutaryl (HMG)-coenzyme A (CoA) reductase, such as cerivastatin, atorvastatin, simvastatin, pitavastatin, rosuvastatin, fluvastatin, lovastatin, pravastatin;
(2) inhibits cholesterol uptake, e.g. ezetimibe ;
(3) combinations of any of 1 and 2 such as ezetimibe plus a HMG-CoA inhibitor, ezetimibe plus a statin, ezetimibe with a statin, particularly a fixed combination of ezetimibe with any of the statins as herein desclosed e.g. ezetimibe with atorvastatin or ezetimibe with simvastatin.

The term "cardiovascular disease" as used herein refers to any disease that affects the cardiovascular system, principally cardiac disease, heart disease, vascular diseases of the brain and kidney, and peripheral arterial disease and also comprises coronary heart disease. As used herein, the term particularly includes: coronary heart disease (a disease of the blood vessels supplying the heart muscle), cerebrovascular disease (a disease of the blood vessels supplying the brain), peripheral arterial disease (a disease of blood vessels supplying the arms and legs), rheumatic heart disease (a damage to the heart muscle and heart valves from rheumatic fever, caused by streptococcal bacteria), congenital heart disease (malformations of heart structure existing at birth), deep vein thrombosis (blood clots in the leg veins, which can dislodge and move to the heart and lungs), pulmonary embolism (disloged blood clogs move to the heart lungs and obstruct vessels), heart attack and stroke (both mainly caused by a blockage that prevents blood from flowing to the heart or brain. The most common reason is a build-up of fatty deposits (atherosclerotic plaques) on the inner walls of the blood vessels). Strokes can also be caused by bleeding from a blood vessel in the brain or by blood clots. The terms "cardiovascular disease" and "cardiovascular diseases" are used interchangeably herein.

The term "coronary heart disease" (CHD) as used herein is known in the art and particularly refers to the narrowing or blockage of the coronary arteries, usually caused by atherosclerosis. and can result in a heart attack. Atherosclerosis occurs when cholesterol and other fatty substances accumulate on the inner wall of the arteries. They attract fibrous tissue, blood components, and calcium, and harden into cholesterol-containing artery-clogging plaques. Atherosclerotic plaques often form blood clots that also can block the coronary arteries (coronary thrombosis). Congenital defects and muscle spasms can also block blood flow. Recent research indicates that infection from organisms such as chlamydia bacteria may be responsible for some cases of coronary artery disease. CHD particularly refers to but is not limited to coronary artery disease (CAD), particularly atherosclerotic coronary artery disease.

In the context of present invention, the term "high cardiovascular risk" refers to a high risk of suffering from or developing a cardiovascular disease. Thus, a patient or subject having a high cardiovascular risk or being classified as subject or patient with high cardiovascular risk has a high (e.g. statistically significantly increased risk when compared with other patients or subjects) of suffering from or developing a cardiovascular disease.

A number of major contributing factors increase the risk of developing cardiovascular disease and particularly coronary heart and more particularly coronary artery disease. Subjects having more of these risk factors are more likely to develop cardiovascular disease, particularly CHD and more particularly coronary artery disease (CAD). Major CHD/CAD risk factors are: Heredity, Gender (men have a higher risk up to 60 years of age, then the risk is equal), age (men of 45 years or older, women of 55 years or older have a higher risk), smoking, elevated cholesterol levels (i.e. levels of equal to or more than 240mg/dL total Cholesterol or equal or more than 130-159 mg/dL LDL-Cholesterol, the risk of developing coronary artery disease increases steadily as blood cholesterol levels increase above 160 mg/dL. When a person has other risk factors, the risk multiplies), high blood pressure (It increases the risk of heart attack, stroke, kidney failure, and congestive heart failure. A blood pressure of 140 over 90 or above is considered high. As the numbers rise, high blood pressure goes from Stage 1 (mild) to Stage 4 (very severe). In combination with obesity, smoking, high cholesterol, or diabetes, high blood pressure raises the risk of heart attack or stroke several times.), lack of physical exercise, Type II diabetes (The risk of developing coronary artery disease is seriously increased for diabetics. More than 80% of diabetics die of some type of heart or blood vessel disease.), hormone replacement therapy, obesity, excessive stress.

The term "CHD-risk equivalent" (also known as CHD equivalent) as used herein is known in the art. Subjects who do not have established CHD, but do have a risk for developing major coronary events (myocardial infarction (MI) and coronary death) equal to someone with established CHD (>20% per 10 years according to Framingham risk scoring) or equal to someone who already suffered from a major coronary event, are said to have a CHD risk equivalent. These subjects belong in the highest risk category for primary prevention and have the lowest LDL-C goal (<100 mg/dL). Thus, as used herin, the term "CHD-risk equivalent" refers to a risk of developing a major coronary event that is equal to a subject having established CHD or that is equal to a subject having suffered from a major coronary event and particularly refers to a risk of developing a major coronary event that is >20% per 10 years according to Framingham risk scoring.

CHD risk equivalents include
- clinical forms of noncoronary atherosclerosis such as peripheral arterial disease (PAD), carotid artery disease (transient ischemic attack or stroke of carotid origin, or >50% stenosis on angiography or ultrasound), and abdominal aortic aneurysm (AAA)
- type 1 and type 2 diabetes; however, the differences between the two necessitate different considerations and approaches to treatment
   - In persons with type 1 diabetes, the intensity of LDL-C-lowering therapy depends on clinical judgment. Recent-onset type 1 diabetes need not be considered a CHD risk equivalent; therefore, reduction of LDL-C to <130 mg/dL is sufficient. With increasing duration of type 1 diabetes, however, a lower goal of <100 mg/dL should be considered. Nonetheless, ATP III favors starting drug therapy along with lifestyle therapies when LDL-C levels are >130 mg/dL.
   - For persons with type 2 diabetes, treatment for LDL-C lowering should follow ATP III recommendations for those with established CHD. Clinical judgment should be used as to the intensity of therapy for younger persons with type 2 diabetes, who otherwise are at lower risk; however, consideration should be given to using LDL-C-lowering drugs when LDL-C levels are >130 mg/dL.
- presence of one or more risk factors according to ATP III Framingham risk scoring (high CHD risk occurs at presence of multiple risk factors according to ATP III Framingham risk scoring)
- abdominal aortic aneurysm
- peripheral vascular disease
- carotid artery disease
- a 10-year risk of having heart disease greater than 20% according to the Framingham Cardiac Risk Calculator
- elevated cholesterol levels
- any condition, factor or event leading to a 10-year risk of having heart disease greater than 20% according to the Framingham Cardiac Risk Calculator or ATPIII Framingham risk scoring.

### ASPECTS OF THE INVENTION

The present invention will now be further described. In the following passages different aspects of the invention are defined in more detail and preferred embodiments of the invention will be described. Each aspect and embodiment so defined may be combined with any other aspect or aspects or embodiment or embodiments unless clearly indicated to the contrary. In particular, any feature indicated as being preferred or advantageous may be combined with any other feature or features indicated as being preferred or advantageous, unless clearly indicated to the contrary.

All methods of treatment as herein described, especially the methods according to the first, second third, fourth and fifth aspect should also be understood to relate to the PCSK9 antibody of the present invention (or a formulation, pharmaceutical composition or unit dosage form comprising the antibody) for use in method of treatment according to the first to fifth aspect, i.e. for use in a method according to the first, the second the third, the fourth or the fifth aspect and for use in any combination of said methods. In addition, all methods of treatment as herein described, especially the methods according to the first, second third, fourth and fifth aspect should also be understood to relate to the use of the PCSK9 antibody of the present invention or a formulation, pharmaceutical composition or unit dosage form comprising the antibody in the manufacture of a medicament for treating a disorder according to one of the aspects 1 to 5, i.e. for treating a disorder according to the first, the second the third, the fourth, the fifth or the sixth aspect and any combination of said methods.

In the context of the different aspects of present invention, it is preferred that the subject in need of the treatment or the subject in the pharmaceutical uses of the antibody or the subject in the pharmaceutical uses of the pharmaceutical composition or the subject in the pharmaceutical uses of the unit dosage form or the subject as listed on the label or package insert in the article of manufacture, is a subject falling into one or more of the following subject groups: The subject is a subject having mild hepatic impairment and/or moderate hepatic impairment or a subject with normal hepatic liver function. In addition to this characteristic, the subject can also fall into one or more of the other subject groups of the first aspect and it can also be outside one or more of the subject groups as listed in the second aspect of present invention.

In a first aspect the present invention is directed to a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof falls into one or more of the following groups of subjects:
(i) subjects having a serum LDL cholesterol (LDL-C) level of at least 100 mg/dL, *[at least 130 mg*/*dL, at least 160 mg*/*dL* / *at least 200 mg*/*dL];*
(ii) subjects having a serum HDL-C level of less than 40 mg/dL;
(iii) subjects having a serum cholesterol level of at least 200 mg/dL *[240 mg*/*dL]*;
(iv) subjects having a serum triacylglycerol level of at least 150 mg/dL *[at least 200 mg*/*dL; at least 500 mg*/*dL],* wherein said triacylglycerol level is determined after fasting for at least 8 hours;
(v) subjects being at least 18 years old *[at least 18* /*20* /*25* /*30*/ *35* /*40*/ *45* / *50* / *55* / *60* / *65* / *70* / *75 years old]*;
(vi) subjects being at maximum 75 years old *[75* /*70* /*65* / *60* / *55* / *50* / *45* / *40 35*/ *30*/ *25*/ *20 years];*
(vii) subjects having a BMI of 25 *[26* / *27* / *28* / *29* / *30* / *31* / *32* / *33* / *34* / *35* / *36* / *37* / *38* / *39]* or more;
(viii) male subjects;
(ix) female subjects;
(x) subjects in which the administration of said antibody or antigen-binding fragment thereof leads to a reduction in the serum LDL-C level by at least 30 mg/dL *[40 mg*/*dL; 50 mg*/*dL; 60 mg*/*dL; 70 mg*/*dL]* relative to predose level; or
(xi) subjects in which the administration of said antibody or antigen-binding fragment thereof leads to a reduction in the serum LDL-C level by at least 20% *[30%; 40%*; *50%*; *60%]* relative to predose level
(xii) subjects having mild and/or moderate hepatic impairment
(xiii) subjects with normal hepatic liver function
(xiv) subjects with high cardiovascular risk
(xv) subjects with coronary heart disease or a CHD risk equivalent
(xvi) subjects with cardiovascular disease.

According to one embodiment of the first aspect, the invention concerns a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject
a) having mild and/or moderate hepatic impairment or a subject with normal hepatic liver function and/or
b) a subject with a high cardiovascular risk and/or
c) a subject with cardiovascular disease and/or
d) a subject with coronary heart disease or a CHD risk equivalent.

In this embodiment, the subject preferably also falls into one or more of the categories (i) to (xi) as listed in the first aspect above.

In preferred embodiments of the first and the other aspects of present invention, the subject in need thereof is a subject with mild hepatic impairment. In other preferred embodiments of the first and the other aspects of present invention, the subject in need thereof is a subject with moderate hepatic impairment. In other preferred embodiments of the first and the other aspects of present invention, the subject in need thereof is a subject with normal hepatic liver function.

In further preferred embodiments of the first (the second, third, fourth, fifth, sixth, seventh, eight and ninth) aspect of present invention, the subject in need thereof is a subject with a subject with a cardiovascular disease, high cardiovascular risk, a subject with (established) coronary heart disease (CHD) or a subject with a CHD-risk equivalent. The subject can also be a subject with one or more of high cardiovascular risk, cardiovascular disease, (established) coronary heart disease (CHD), and CHD-risk equivalent AND falling into one or more of the other patient populations as listed in the first aspect, e.g. having hypercholesterolemia, particularly heterozygous familial hypercholesterolemia. Some preferred subjects are thus subjects with hypercholesterolemia, particularly heterozygous familial hypercholesterolemia and having a cardiovascular disease. Other preferred subjects are thus subjects with hypercholesterolemia, particularly heterozygous familial hypercholesterolemia and having a high cardiovascular risk. Other preferred subjects are thus subjects with hypercholesterolemia, particularly heterozygous familial hypercholesterolemia and having a CHD-risk equivalent.

According to one embodiment of the first (as well as the second, third, fourth, fifth, sixth, seventh, eighth or ninth) aspect of present invention, a disease or condition in which PCSK9 expression or activity causes an impact is thus a cardiovascular disease.

According to another embodiment of the first (as well as the second, third, fourth, fifth, sixth, seventh, eighth or ninth) aspect of present invention, a disease or condition in which PCSK9 expression or activity causes an impact is thus high cardiovascular risk.

According to another embodiment of the first (as well as the second, third, fourth, fifth, sixth, seventh, eighth or ninth) aspect of present invention, a disease or condition in which PCSK9 expression or activity causes an impact is thus (established) coronary heart disease (CHD). According to another embodiment of the first (as well as the second, third, fourth, fifth, sixth, seventh, eighth or ninth) aspect of present invention, a disease or condition in which PCSK9 expression or activity causes an impact is thus a CHD-risk equivalent.

A preferred embodiment of the first aspect thus concerns a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function.

In another preferred aspect of the first and the other aspects, the subject falls into one or more further subject groups of the first aspect in addition to being a subject with mild or moderate hepatic impairment or to being a subject with normal hepatic liver function. For example, in further preferred embodiments of the first and the other aspects of present inventinon, the subject in need thereof is a subject having moderate or mild hepatic impairment and having one or more of the following: a cardiovascular disease, a high cardiovascular risk, (established) coronary heart disease (CHD), a CHD-risk equivalent.

According to another preferred embodiment, the subject falls outside one or more of the subject groups according to the second aspect in addition to being a subject with mild or moderate hepatic impairment or to being a subject with normal hepatic liver function and to falling into one or more additional subject groups of the first aspect.

In the context of the different aspects of present invention (i.e the first, the second, the third, the fourth, the fifth, the sixth, the seventh, the eighth and the ninth aspect), the term "a disease or condition in which PCSK9 expression or activity causes an impact" is understood to comprise any disease or condition in which the application of a PCSK-9 antibody causes an impact and preferably causes an amelioration, improvement, inhibition or prevention of the disease or disorder.

Exemplary diseases or conditions that can be ameliorated, improved, inhibited or prevented by administration of an PCSK9 antibody include without limitation hypercholesterolemia, hyperlipidemia, dyslipidemia (such as mixed dyslipidemia), atherosclerosis and cardiovascular disease(s).

According to one embodiment of the the first, the second, the third, the fourth, the fifth, the sixth, the seventh, the eighth and the ninth aspect of present invention, the disease or disorder is hypercholesterolemia, wherein the hypercholesteremia can be any hypercholesteremia, e.g. familal or non-familial hypercholesterolemia.

According to another embodiment of the the first, the second, the third, the fourth, the fifth, the sixth, the seventh, the eighth and the ninth aspect of present invention, the disease or disorder is hyperlipidemia.

According to another embodiment of the the first, the second, the third, the fourth, the fifth, the sixth, the seventh, the eighth and the ninth aspect of present invention, the disease or disorder is dyslipidemia (such as mixed dyslipidemia).

According to another embodiment of the the first, the second, the third, the fourth, the fifth, the sixth, the seventh, the eighth and the ninth aspect of present invention, the disease or disorder is atherosclerosis.

According to another embodiment of the the first, the second, the third, the fourth, the fifth, the sixth, the seventh, the eighth and the ninth aspect of present invention, the disease or disorder is cardiovascular disease.

Most presently available treatment schemes of cardiovascular disease are based upon small molecule therapies, such as the administration of a statin. Statins are widely used for treating patients with existing cardiovascular disease and have been shown to decrease mortality in this target population. However, in some patients, statin treatment is not successful even when high doses of statins are administered, and these patients do not achieve the therapeutic target necessary for reducing the risk of suffering from cardiovascular diseases or necessary for reduction of the severity of the cardiovascular disease of for curing of the cardiovascular disease. Other patients cannot be treated with high doses of statin, possibly because of adverse events connected with statin administration. Other patients cannot be treated with statins at al because of a total contraindication (statin intolerance).

In such patients, there is a high need for complementary (i.e. administration of a drug in addition to another drug) treatment of cardiovascular disease by administration of another drug together with the statin or for a substitutive/alternative (i.e. administration of one drug instead of another drug) treatment instead of statin administration.

This need is fulfilled by the medical use of present PCSK-9 specific antibodies for treating, curing or preventing cardiovascular disease.

The use of present antibodies or fragments thereof in the context of cardiovascular disease is *inter alia* based on the inventors' surprising finding, that the antibodies have an additional synergistic impact on LDL-C lowering and other risk factors of cardiovascular disease on top of that of a statin, when administered together with a statin.

In view of the fact that cardiovascular disease is the leading cause of death in the United States and other countries, there is a great need for alternative or substitutive treatment. A major risk factor of cardiovascular disease is elevated serum lipid levels, such as elevated cholesterol levels. As stated above, the standard treatment of cardiovascular disease is statin administration. The cholesterol (LDL-C and total-C) lowering effect of statins however is limited (attributed to by an increase in PCSK9 expression that is induced by statins). With high lipid (particularly LDL-C and total C) levels as majour risk factor for cardiovascular disease, the efficacy of statin therapy on the treatment of cardiovascular disease must suffer from the same drawback. The PCSK9 antibody of present invention acts via a different mechanism and counteracts this statin-induced increase in PCSK9 expression by inhibiting the PCSK9-LDLR interaction when applied together with a statin. In addition, administration of the PCSK9 antibody of present invention without statin administration, also shows significant lowering of LDL-C levels, thus the antibody of present invention has at least twofold activity when administered with a statin: Once by counteracting the statin-induced PCSK9 expression and second by inhibition of the PCSK9-LDLR interaction, both contributing to the efficacy of the present antibody in lowering LDL-C levels and thus in treating cardiovascular disease. This has been clinically proven since, a significant similar effect on LDL-C levels was observed using different doses of statin (atorvarstatin) together with the PCSK9 antibody of present invention. Thus, patients failing to achieve their LDL-C targets even when being administered high doses of statin (statin-resistant patients) as well as patients that take only low amounts of statin (possibly because of intolerance of high statin doses) can benefit from the co-treatment with the antibody.

When on the other hand the antibody is administered alone, the inhibitory effect of the antibody on the interaction between PCSK9 and LDL-R suffices to significantly lower LDL-C levels: In clinical studies, administration of the PCSK9 antibody alone, without statin administration also exhibited significant effects on the lowering of plasma LDL-C levels. Thus, also patients who are completely contra-indicated for statin treatment will benefit from administration with the PCSK9 antibody of present invention.

The use of the PCSK9 antibody or the fragments thereof of the invention thus offers the possibility to reach statin-resistant or statin-intolerant target populations, because the PCSK9 antibody of present invention has impact on risk factors of cardiovascular disease (LDL-C levels) when administered on top of statins as well as when administered without statins.

Thus, the PCSK9 antibody of present invention offers the possibility a complementary treatment of cardiovascular disease together with statins or a supplementary treatment of cardiovascular disease, where statin administration is contra-indicated.

In addition, the PCSK9 antibody of present invention offers an alternative to the only other presently existing antibody therapeutic available for treatment of patients with cardiovascular disorders (abciximab). Abciximab is an antibody-anticoagulant targeting to inhibit platelet aggregation by blocking of GPIIb/IIIa. As the PCSK9 antibody or fragment therof of present invention acts via a completely different pathway than abciximab, it offers an alternative treatment that is particularly suitable for all target populations being contra-indicated for antiplatelet or anti-coagulation therapy in general or suffering from adverse side effects when using abciximab (such as minor bleeding). This particularly applies to antiplatelet or anticoagulant contra-indicated or abciximab side-effect afflicted patient populations that do not (fully) reach the therapeutic target intended by statin therapy or that are statin resistant.

According to another embodiment of the first and the other aspects of present invention, a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of an HMG-CoA reductase inhibitor is administered to said subject, wherein the HMG-CoA reductase inhibitor is administered in a dosage amount ranging from 0.05 mg to 100 mg. In a preferred embodiment, the HMG-CoA reductase inhibitor is a statin.

According to another embodiment of the first and the other aspects of present invention, another lipid lowering agent is administered in addition to the antibody and the HMG-CoA reductase inhibitor, i.e. the therapy comprises administration of the antibody or fragment therof, the HMG-CoA reductase inhibitor (particularly a statin) and another lipid lowering drug (e.g. a lipid lowering drug as herein described, such as, but not limited to, ezetimibe, particularly ezetimibe in a daily dosage of about 10 mg).

Ezetimibe is a drug that lowers cholesterol by decreasing cholesterol absorption in the intestine. Although ezetimibe was shown to decrease cholesterol levels, the outcome of two clinical trials showed that it did not improve the clinically significant outcomes in the study groups. Thus, ezetimibe is primarily used alone when other cholesterol lowering drugs cannot be used (e.g. due to an intolerance). In addition, it is used together with a statin such as simvastatin in patients, in which statin treatment alone does not suffice for sufficient lowering of the cholesterol levels.

According to another embodiment of the first and the other aspects of present invention, a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of a lipid lowering drug is administered in an effective dosage amount. The lipid lowering drug can be any lipid lowering drug as herein described. According to a preferred embodiment, the lipid lowering drug is ezetimibe, particularly ezetimibe in a daily dosage of about 10 mg.

The combination treatment comprising administering the antibody or fragment therof together with an HMG-CoA reductase inhibitor (particularly a statin) according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with cardiovascular disease, high cardiovascular risk, with CHD or a CHD-risk equivalent.

The combination treatment comprising administering the antibody or fragment therof together with a lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with cardiovascular disease, high cardiovascular risk, subjects with coronary heart disease or a CHD-risk equivalent and most preferred for treatment of subjects with high cardiovascular risk or with CHD or a CHD-risk equivalent.

The combination treatment comprising administering the antibody or fragment therof together with an HMG-CoA reductase inhibitor (particularly a statin) and together with administration of another lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with cardiovascular disease, high cardiovascular risk, subjects with coronary heart disease or a CHD-risk equivalent and most preferred for treatment of subjects with high cardiovascular risk or with CHD or a CHD-risk equivalent.

The combination treatment comprising administration of the antibody or fragment thereof of the invention together with the HMG-CoA reductase inhibitor (particularly a statin and more particularly atorvastatin or simvastatin) and together with another lipid lowering drug (particularly ezetimibe) can be performed according to any suitable administration and dosing regime (particularly as described herein). According to some embodiments of the first, the second, the third, the fourth, the fifth, the sixth, the seventh, the eighth and the ninth aspect, all three components are administered as separate unit dosage forms (e.g. the antibody by injection of a solution, the HMG Co-A reductase inhibitor by oral administration of a suitable unit dosage form (e.g. a tablet) and the other lipid lowering drug also by oral administration of a separate suitable unit dosage form (e.g. a tablet). According to other embodiments of the first, the second, the third, the fourth, the fifth, the sixth, the seventh, the eighth and the ninth aspect, all three components are administered together as one unit dosage form (e.g. all as solution for injection). According to other embodiments of the first, the second, the third, the fourth, the fifth, the sixth, the seventh, the eighth and the ninth aspect, the antibody or fragment thereof is administered as a unit dosage form and the other two components are administered together as one unit dosage form comprising both other components (e.g. a unit dosage form suitable for oral administration comprising the HMG-CoA reductase inhibitor and the other lipid lowering drug, particularly a tablet or capsule comprising a statin and ezetimibe).

According to another embodiment of the first and the other aspects of present invention, the disease or condition is selected from the group consisting of: elevated total cholesterol levels, elevated low-density lipoprotein cholesterol (LDL-C) levels, hypercholesterolemia, particularly hypercholesteremia uncontrolled by statins, severe hypercholesterolemia, refractory hypercholesterolemia, hyperlipidemia, dyslipidemia, such as mixed dyslipidemia, atherosclerosis, cardiovascular diseases, primary hypercholesterolemia, such as primary familial hypercholesterolemia or primary non-familial hypercholesterolemia, hypercholesterolemia (especially primary hypercholesterolemia) uncontrolled by statins (particularly uncontrolled by atorvastatin). Primary hypercholesterolemia or hypercholesterolemia uncontrolled by statins and particularly primary hypercholesterolemia uncontrolled by statins are among the most preferred disorders in the context of the different aspects of present invention.

According to another embodiment of the first and the other aspects of present invention, the subject in need thereof is a subject indicated for LDL apheresis, a subject with PCSK9-activating mutations, a subject with heterozygous Familial Hypercholesterolemia, a subject with primary hypercholesterolemia, a subject with primary hypercholesterolemia who is statin uncontrolled, a subject at risk for developing hypercholesterolemia, a subject with hypercholesterolemia, a subject with hyperlipidemia, a subject with dyslipidemia, such as mixed dyslipidemia, a subject with atherosclerosis or a subject with high cardiovascular risk or a subject with one or more cardiovascular diseases such as CHD or a CHD risk equivalent. Most preferably, the subject in need thereof is a human subject.

Antibodies and antigen-binding fragments thereof that can be used for practicing the first and the other aspects of the present invention are described in the section *"Preferred Antibodies for Practicing the Present Invention".*

According to a preferred embodiment of the first and the other aspects of present invention, the antibody or antigen-binding fragment thereof is a recombinant human antibody or fragment thereof.

According to another preferred embodiment of the first and the other aspects of present invention, the antibody or the antigen-binding fragment thereof is characterized by one or more of the following:
(i) capable of reducing serum total cholesterol at least about 25 to about 35% and sustaining the reduction over at least a 24 day period relative to a predose level;
(ii) capable of reducing serum LDL cholesterol at least about 65-80% and sustaining the reduction over at least a 24 day period relative to a predose level;
(iii) capable of reducing serum triglyceride at least about 25-40% relative to predose level;
(iv) achieves one or more of (i)-(iii) without reducing serum HDL cholesterol or reducing serum HDL cholesterol no more than 5% relative to predose level;
(v) achieves one or more of (i)-(iii) with little or no measurable effect on liver function, as determined by ALT and AST measurements.

According to another preferred embodiment of the first and the other aspects of present invention, the antibody or the antigen-binding fragment thereof comprises
- a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
- a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736.

According to another preferred embodiment of the first and the other aspects of present invention, the antibody or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another preferred embodiment of the first and the other aspects of present invention, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88.

According to another preferred embodiment of the first and the other aspects of present invention, the antibody or antigen-binding fragment thereof competes for binding to hPCSK9 with an antibody comprising heavy and light chain CDR amino acid sequences as shown in SEQ ID NOs: 76, 78, 80, 84, 86, and 88.

According to another preferred embodiment of the first and the other aspects of present invention, a therapeutic amount of an HMG-CoA reductase inhibitor is administered to the subject in a dosage of between 0.05 mg to 100 mg. Preferably, the HMG-CoA reductase inhibitor is a statin. More preferably, the statin is selected from the group consisting of cerivastatin, atorvastatin, simvastatin, pitavastatin, rosuvastatin, fluvastatin, lovastatin, and pravastatin.

Conceivable combinations of antibody or fragment thereof plus HMG-CoA inhibitor (particularly a statin) plus ezetimibe suitable in the context of the first and the other aspects of present invention comprise, but are not limited to antibody or fragment thereof plus ezetimibe plus cerivastatin, antibody or fragment thereof plus ezetimibe plus atorvastatin, antibody or fragment thereof plus ezetimibe plus simvastatin, antibody or fragment thereof plus ezetimibe plus pitavastatin, antibody or fragment thereof plus ezetimibe plus rosuvastatin, antibody or fragment thereof plus ezetimibe plus fluvastatin, antibody or fragment thereof plus ezetimibe plus lovastatin and antibody or fragment thereof plus ezetimibe plus pravastatin. Preferred combinations are antibody or fragment thereof plus ezetimibe plus atorvastatin or antibody or fragment thereof plus ezetimibe plus simvastatin.

In some embodiments of the first and other aspects of the present invention, the HMG-CoA reductase inhibitor is administered three times per day, twice per day, or once per day. In some embodiments of the first and the other aspects of present invention, the HMG-CoA reductase inhibitor is administered every day, every other day, every third day, every fourth day, every fifth day, or every sixth day. In some embodiments of the first and the other aspects of present invention, the HMG-CoA reductase inhibitor is administered every week, every other week, every third week, or every fourth week. In some embodiments of the first and the other aspects of present invention, the HMG-CoA reductase inhibitor is administered in the morning, at noon or in the evening. In preferred embodiments, the HMG-CoA reductase inhibitor is administered once per day, preferably orally, preferably in the evening. These administration schemes can be combined in the different aspects of present invention, e.g. an administration regime would be conceivable with administration of the HMG-CoA inhibitor once a day in the morning, etc. s

According to another preferred embodiment of the first and the other aspects of present invention, the HMG-CoA reductase inhibitor is a statin and the statin is preferably
- cerivastatin administered in a daily dosage of between 0.05 mg and 2 mg, preferably in a daily dosage of 0.2 mg, 0.4 mg, or 0.8 mg;
- atorvastatin administered in a daily dosage of between 2 mg and 100 mg, preferably in a daily dosage of 10 mg, 20 mg, 40 mg, or 80 mg;
- simvastatin administered in a daily dosage of between 2 mg and 100 mg, preferably in a daily dosage of 5 mg, 10 mg, 20 mg, 40 mg, or 80 mg;
- pitavastatin administered in a daily dosage of between 0.2 mg and 100 mg, preferably in a daily dosage of 1 mg, 2 mg, 5 mg, 10 mg, or 20 mg;
- rosuvastatin administered in a daily dosage of between 2 mg and 100 mg, preferably in a daily dosage of 5 mg, 10 mg, 20 mg, or 40 mg;
- fluvastatin administered in a daily dosage of between 2 mg and 100 mg, preferably in a daily dosage of 20 mg, 40 mg, or 80 mg;
- lovastatin administered in a daily dosage of between 2 mg and 100 mg, preferably in a daily dosage of 10 mg, 20 mg, 40 mg, or 80 mg; or
- pravastatin administered in a daily dosage of between 2 mg and 100 mg, preferably in a daily dosage of 10 mg, 20 mg, 40 mg, or 80 mg.

In preferred embodiments of the different aspects of present invention, the statin is atorvastatin, and particularly atorvastatin administered in a daily dosage of between 2 mg and 100 mg, preferably in a daily dosage of 10 mg, 20 mg, 40 mg, or 80 mg.

The additional lipid lowering agent, particularly ezetimibe is preferably administered once a day. Effective daily dosages depend on various factors and the skilled person can readiliy determine the best dosage, conceivably daily dosages are e.g. be about 5 mg, about 8 mg, about 9 mg, about 10 mg, about 11 mg, about 15 mg and particularly about 10 mg.

In preferred embodiments of the first and the other aspects of present invention, the antibody or antigen-binding fragment thereof is administered to the subject every other week, every fourth week or once a month. Administration every fourth week or administration once a month (i.e. once per calendar month, e.g. every first, second etc. day of the month or every first, second or third Monday, Tuesday etc. every month, in contrast to administration every fourth week) is preferred in view of patient compliance. Administration every other week is preferred in view of a very low variation of blood cholesterol levels. Other suitable time schedules for administration of the antibody or antigen-binding fragment thereof include without limitation an administration once per day, every other day, every third day, every fourth day, every fifth day, every sixth day, every week, every third week, every fifth week, every sixth week, every eighth week, every tenth week, and every twelfth week.

According to another preferred embodiment of the first and the other aspects of present invention, the antibody or antigen-binding fragment thereof is a recombinant human antibody or fragment thereof.

In another embodiment of the first and the other aspects, the antibody or antigen-binding fragment thereof is administered in a dosage amount ranging from 50 mg to 300 mg.

In preferred embodiments of the first and the other aspects of present invention, the antibody or antigen-binding fragment thereof is administered in a dosage amount ranging e.g. from about 40 mg to about 500 mg, from about 50 mg to about 500mg, from about 50mg to 300mg or from about 100mg to 200mg. In more preferred embodiments, the antibody or antigen-binding fragment thereof is administered in a dosage amount of about 50 mg, of about 100 mg, of about 150 mg, of about 200 mg, of about 250 mg, of about 300 mg, of about 350mg, of about 400 mg, of about 450 mg or of about 500 mg. Doses of about 50 to about 200 mg, e.g. of about 50 mg, about 100 mg, about 150 mg or about 200 mg are especially suitable for a biweekly dosage regimen (i.e. the application every other week), doses of about 150 mg to about 400 mg, e.g. about 150 mg, about 200 mg, about 250 mg, about 300 mg about 350 mg or about 400 mg are especially suitable for an administration regime with longer intervals, e.g. an administration every third or every fourth week or once a month.

In some embodiments of the first, the second, the third, the fourth, the fifth, the sixth, the seventh, the eighth and the ninth aspect, the antibody or fragment thereof (alone or together with the HMG-CoA reductase inhibitor and/or the other lipid lowering drug) is used as adjunctive therapy together with a diet, particularly a diet suitable for lowering the cholesterol levels (e.g. low-fat diet or low-cholesterol diet or cholesterol lowering diet).

The method of the first aspect can be combined with the method of the second aspect (or that of the third, fourth and fifth aspect), as well as with that of any other aspect. In a preferred embodiment of the first aspect of present invention, the subject in need thereof also does not fall in one or more of the groups of the second aspect.

In a second aspect, present invention relates to a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof does not fall into one or more of the following groups of subjects:
(i) smokers;
(ii) persons being 76 years old or older;
(iii) persons being 17 years or younger;
(iv) persons suffering from hypertension;
(v) women who are pregnant;
(vi) women who are trying to become pregnant;
(vii) women who are breast-feeding;
(viii) persons who have or ever had severe hepatic impairment
(ix) persons who had any unexplained abnormal results from blood tests for liver function;
(x) persons who drink excessive amounts of alcohol;
(xi) persons having kidney problems;
(xii) persons suffering from hypothyroidism;
(xiii) persons suffering from muscle disorders;
(xiv) persons having encountered previous muscular problems during treatment with lipid-lowering medicine;
(xv) persons having serious problems with their breathing;
(xvi) persons taking one or more of the following medicines: medicines altering the way the immune systems works (e.g. ciclosporin or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or
(xvii) persons drinking more than 0.1 L of grapefruit juice per day;
(xviii) persons having a body mass index (BMI) of more than 40;
(xix) persons having a body mass index (BMI) of less than 18;
(xx) persons suffering from type 1 diabetes or type 2 diabetes;
(xxi) persons positive for hepatitis B or hepatitis C; or
(xxii) persons having a known sensitivity to monoclonal antibody therapeutics
(xxiii) persons having acute hepatitis,
(xxiv) persons having hepatic encephalopathy grade 2, 3, or 4,
(xxv) persons having uncontrolled clinically relevant cardiovascular, pulmonary, gastrointestinal, metabolic, hematological, neurological, psychiatric, systemic, ocular, gynecologic (if female), or infectious disease, or signs of acute illness
(xxvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness
and wherein the subject in need thereof preferably falls into one or more of the patient groups of the first aspect and more preferably is a subject having mild and/or moderate hepatic impairment or a subject with normal hepatic liver function

In preferred embodiments of the second and the other aspects of present invention, the disease or condition in which PCSK9 expression or activity causes an impact is ameliorated, improved, inhibited or prevented with a PCSK9 antagonist.

According to another embodiment of the second and the other aspects of present invention a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of an HMG-CoA reductase inhibitor is administered to said subject, wherein the HMG-CoA reductase inhibitor is administered in a dosage amount ranging from 0.05 mg to 100 mg.

According to yet another embodiment of the second and the other aspects of present invention, a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of an HMG-CoA reductase inhibitor is administered to said subject, wherein the HMG-CoA reductase inhibitor is administered in a dosage amount ranging from 0.05 mg to 100 mg. Preferably, the HMG-CoA reductase inhibitor is a statin.

According to another embodiment of the second and the other aspects of present invention, another lipid lowering agent is administered in addition to the antibody and the HMG-CoA reductase inhibitor, i.e. the therapy comprises administration of the antibody or fragment therof, the HMG-CoA reductase inhibitor (particularly a statin) and another lipid lowering drug (e.g. a lipid lowering drug as herein described, such as, but not limited to, ezetimibe, particularly ezetimibe in a daily dosage of about 10 mg).

According to another embodiment of the second and the other aspects of present invention, a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of a lipid lowering drug is administered in an dosage amount effective dosage amount. The lipid lowering drug can be any lipid lowering drug as herein described. According to a preferred embodiment, the lipid lowering drug is ezetimibe, particularly ezetimibe in a daily dosage of about 10 mg.

The combination treatment comprising administering the antibody or fragment therof together with an HMG-CoA reductase inhibitor (particularly a statin) according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

The combination treatment comprising administering the antibody or fragment therof together with a lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

The combination treatment comprising administering the antibody or fragment therof together with an HMG-CoA reductase inhibitor (particularly a statin) and together with administration of another lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

According to another embodiment of the second and the other aspects of present invention, the disease or condition is selected from the group consisting of: elevated total cholesterol levels, elevated low-density lipoprotein cholesterol (LDL-C) levels, hypercholesterolemia, particularly hypercholesteremia uncontrolled by statins, severe hypercholesterolemia, refractory hypercholesterolemia, , hyperlipidemia, dyslipidemia, such as mixed dyslipidemia, atherosclerosis, a subject with high cardiovascular risk or a subject with one or more cardiovascular diseases such as CHD or a CHD risk equivalent, primary hypercholesterolemia, such as primary familial hypercholesterolemia or primary non-familial hypercholesterolemia, hypercholesterolemia (especially primary hypercholesterolemia) uncontrolled by statins (particularly uncontrolled by atorvastatin).

Antibodies and antigen-binding fragments thereof that can be used for practicing the second and the other aspects of the present invention are described in the section *"Preferred*

*Antibodies for Practicing the Present Invention".*

According to a preferred embodiment of the second and the other aspects of present invention, the antibody or antigen-binding fragment thereof is a recombinant human antibody or fragment thereof.

According to another preferred embodiment of the second and the other aspects of present invention, the antibody or the antigen-binding fragment thereof is characterized by one or more of the following:
(i) capable of reducing serum total cholesterol at least about 25 to about 35% and sustaining the reduction over at least a 24 day period relative to a predose level;
(ii) capable of reducing serum LDL cholesterol at least about 65-80% and sustaining the reduction over at least a 24 day period relative to a predose level;
(iii) capable of reducing serum triglyceride at least about 25-40% relative to predose level;
(iv) achieves one or more of (i)-(iii) without reducing serum HDL cholesterol or reducing serum HDL cholesterol no more than 5% relative to predose level;
(v) achieves one or more of (i)-(iii) with little or no measurable effect on liver function, as determined by ALT and AST measurements.

According to another preferred embodiment of the second and the other aspects of present invention, the antibody or the antigen-binding fragment thereof comprises
- a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
- a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736.

According to another preferred embodiment of the second and the other aspects of present invention, the antibody or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another preferred embodiment of the second and the other aspects of present invention, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88.

According to another preferred embodiment of the second and the other aspects of present invention, the antibody or antigen-binding fragment thereof competes for binding to hPCSK9 with an antibody comprising heavy and light chain CDR amino acid sequences as shown in SEQ ID NOs: 76, 78, 80, 84, 86, and 88.

According to another preferred embodiment of the second and the other aspects of present invention, a therapeutic amount of an HMG-CoA reductase inhibitor is administered to the subject in a dosage of between 0.05 mg to 100 mg. Preferably, the HMG-CoA reductase inhibitor is a statin. More preferably, the statin is selected from the group consisting of cerivastatin, atorvastatin, simvastatin, pitavastatin, rosuvastatin, fluvastatin, lovastatin, and pravastatin.

Conceivable combinations of antibody or fragment thereof plus HMG-CoA inhibitor (particularly a statin) plus ezetimibe suitable in the context of the second and the other aspects of present invention, comprise, but are not limited to antibody or fragment thereof plus ezetimibe plus cerivastatin, antibody or fragment thereof plus ezetimibe plus atorvastatin, antibody or fragment thereof plus ezetimibe plus simvastatin, antibody or fragment thereof plus ezetimibe plus pitavastatin, antibody or fragment thereof plus ezetimibe plus rosuvastatin, antibody or fragment thereof plus ezetimibe plus fluvastatin, antibody or fragment thereof plus ezetimibe plus lovastatin and antibody or fragment thereof plus ezetimibe plus pravastatin. Preferred combinations are antibody or fragment thereof plus ezetimibe plus atorvastatin or antibody or fragment thereof plus ezetimibe plus simvastatin.

In some embodiments of the second and other aspects of the invention, the HMG-CoA reductase inhibitor is administered three times per day, twice per day, or once per day. In some embodiments of the second and the other aspects of present invention, the HMG-CoA reductase inhibitor is administered every day, every other day, every third day, every fourth day, every fifth day, or every sixth day. In some embodiments of the second and the other aspects of present invention, the HMG-CoA reductase inhibitor is administered every week, every other week, every third week, or every fourth week. In some embodiments of the second and the other aspects of present invention, the HMG-CoA reductase inhibitor is administered in the morning, at noon or in the evening. In preferred embodiments, the HMG-CoA reductase inhibitor is administered once per day, preferably orally, preferably in the evening.

According to another preferred embodiment of the second and the other aspects of present invention, the HMG-CoA reductase inhibitor is a statin and the statin is preferably
- cerivastatin administered in a daily dosage of between 0.05 mg and 2 mg, preferably in a daily dosage of 0.2 mg, 0.4 mg, or 0.8 mg;
- atorvastatin administered in a daily dosage of between 2 mg and 100 mg, preferably in a daily dosage of 10 mg, 20 mg, 40 mg, or 80 mg;
- simvastatin administered in a daily dosage of between 2 mg and 100 mg, preferably in a daily dosage of 5 mg, 10 mg, 20 mg, 40 mg, or 80 mg;
- pitavastatin administered in a daily dosage of between 0.2 mg and 100 mg, preferably in a daily dosage of 1 mg, 2 mg, 5 mg, 10 mg, or 20 mg;
- rosuvastatin administered in a daily dosage of between 2 mg and 100 mg, preferably in a daily dosage of 5 mg, 10 mg, 20 mg, or 40 mg;
- fluvastatin administered in a daily dosage of between 2 mg and 100 mg, preferably in a daily dosage of 20 mg, 40 mg, or 80 mg;
- lovastatin administered in a daily dosage of between 2 mg and 100 mg, preferably in a daily dosage of 10 mg, 20 mg, 40 mg, or 80 mg; or
- pravastatin administered in a daily dosage of between 2 mg and 100 mg, preferably in a daily dosage of 10 mg, 20 mg, 40 mg, or 80 mg.

In a preferred embodiment of the second and the other aspects of present invention, the statin is atorvastatin and particularly atorvastatin administered in a daily dosage of between 2 mg and 100 mg, preferably in a daily dosage of 10 mg, 20 mg, 40 mg, or 80 mg.

The additional lipid lowering agent, particularly ezetimibe is preferably administered once a day. Effective daily dosages depend on various factors and the skilled person can readiliy determine the best dosage, conceivably daily dosages are e.g. be about 5 mg, about 8 mg, about 9 mg, about 10 mg, about 11 mg, about 15 mg and particularly about 10 mg.

In preferred embodiments of the second and the other aspects of present invention, the antibody or antigen-binding fragment thereof is administered to the subject every other week, every fourth week or once a month. Administration every fourth week or administration once a month (i.e. once per calendar month, e.g. every first, second etc. day of the month or every first, second or third Monday or Tuesday etc. each month, in contrast to administration every fourth week) is preferred in view of patient compliance. Administration every other week is preferred in view of a very low variation of blood cholesterol levels. Other suitable time schedules for administration of the antibody or antigen-binding fragment thereof include without limitation an administration once per day, every other day, every third day, every fourth day, every fifth day, every sixth day, every week, every third week, every fifth week, every sixth week, every eighth week, every tenth week, and every twelfth week.

According to another preferred embodiment of the second and the other aspects of present invention, the antibody or antigen-binding fragment thereof is a recombinant human antibody or fragment thereof.

In another embodiment of the second and the other aspects, the antibody or antigen-binding fragment thereof is administered in a dosage amount ranging from 50 mg to 300 mg.

In preferred embodiments of the second and the other aspects of present invention, the antibody or antigen-binding fragment thereof is administered in a dosage amount ranging e.g. from about 40 mg to about 500 mg, from about 50 mg to about 500mg, from about 50mg to 300mg or from about 100mg to 200mg. In more preferred embodiments, the antibody or antigen-binding fragment thereof is administered in a dosage amount of about 50 mg, of about 100 mg, of about 150 mg, of about 200 mg, of about 250 mg, of about 300 mg, of about 350mg, of about 400 mg, of about 450 mg or of about 500 mg. Doses of about 50 to about 200 mg, e.g. of about 50 mg, about 100 mg, about 150 mg or about 200 mg are especially suitable for a biweekly dosage regimen (i.e. the application every other week), doses of about 150 mg to about 400 mg, e.g. about 150 mg, about 200 mg, about 250 mg, about 300 mg about 350 mg or about 400 mg are especially suitable for an administration regime with longer intervals, e.g. an administration every third or every fourth week or once a month.

The method of the second aspect can be combined with the method of the first aspect (or that of the third, fourth and fifth aspect), as well as with that of any other aspect.

In a third aspect, present invention concerns a method of lowering cholesterol levels in a subject in need thereof, comprising:
(a) selecting a subject with a blood low density lipoprotein cholesterol (LDL-C) level greater than 100 mg/dL; and
(b) administering to said subject a composition comprising an antibody or antigen binding fragment thereof that specifically binds to human proprotein convertase subtilisin/kexin type 9 (hPCSK9); thereby lowering cholesterol levels in the subject in need thereof, wherein the subject falls into one or more of the groups of subjects as listed in the first aspect and wherein the subject preferably does not fall into one or more of the groups of subjects as listed in the second aspect.

According to a preferred embodiment of the third aspect, the method also comprises administration of a therapeutic amount of a lipid lowering drug in an effective dosage amount. The lipid lowering drug can be any lipid lowering drug as herein described. According to a preferred embodiment, the lipid lowering drug is an HMG-CoA reductase inhibitor such as a statin or is ezetimibe, particularly ezetimibe in a daily dosage of about 10 mg.

According to a further preferred embodiment the third aspect comprises thus also administering a therapeutic amount of an HMG-CoA reductase inhibitor (such as a statin) to said subject, wherein the HMG-CoA reductase inhibitor is administered in a dosage amount ranging from 0.05 mg to 100 mg. According to a further preferred embodiment, the third aspect comprises also administering the HMG-CoA reductase inhibitor plus administration of ezetimibe.

According to a fourth aspect, present invention concerns a method of regulating the LDL level in the blood of a subject comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in the first aspect and wherein the subject preferably does not fall into one or more of the groups of subjects as listed in the second aspect.

According to a fifth aspect, the present invention concerns a method of preventing effects of a (persistently) increased LDL level in the blood comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in aspect 1 and wherein the subject preferaboly does not fall into one or more of the groups of subjects as listed in aspect 2.

In further preferred embodiments of the third, fourth, fifth and the other aspects of present invention, the subject in need thereof is a subject with high cardiovascular risk or a subject with (established) coronary heart disease (CHD), cardiovascular disease, or a subject with a CHD-risk equivalent. Preferred subjects are subjects with hypercholesterolemia, particularly heterozygous familial hypercholesterolemia and having a high cardiovascular risk or (established) CHD or a CHD-risk equivalent.

According to further preferred embodiments of the third, fourth and fifth aspect of present invention, the method comprises administration of the antibody or fragment thereof together with a HMG-CoA reductase inhibitor and together with another lipid lowering drug such as described herein, particularly ezetimibe and most particularly ezetimibe in a daily dosage of about 10 mg.

The combination treatment comprising administering the antibody or fragment therof together with an HMG-CoA reductase inhibitor (particularly a statin) according to some embodiments of the third, fourth and fifth and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

The combination treatment comprising administering the antibody or fragment therof together with a lipid lowering drug, particularly ezetimibe, according to some embodiments of the third, fourth and fifth and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

The combination treatment comprising administering the antibody or fragment therof together with an HMG-CoA reductase inhibitor (particularly a statin) and together with administration of another lipid lowering drug, particularly ezetimibe, according to some embodiments of the third, fourth and fifth and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

Conceivable combinations of antibody or fragment thereof plus HMG-CoA inhibitor (particularly a statin) plus ezetimibe for use in the context of the third, fourth and fifth and the other aspects of present invention comprise, but are not limited to antibody or fragment thereof plus ezetimibe plus cerivastatin, antibody or fragment thereof plus ezetimibe plus atorvastatin, antibody or fragment thereof plus ezetimibe plus simvastatin, antibody or fragment thereof plus ezetimibe plus pitavastatin, antibody or fragment thereof plus ezetimibe plus rosuvastatin, antibody or fragment thereof plus ezetimibe plus fluvastatin, antibody or fragment thereof plus ezetimibe plus lovastatin and antibody or fragment thereof plus ezetimibe plus pravastatin. Preferred combinations are antibody or fragment thereof plus ezetimibe plus atorvastatin or antibody or fragment thereof plus ezetimibe plus simvastatin.

The methods of the third, fourth and fifth aspect can be combined with the method of the first and/or the second aspect, as well as with that of any other aspect. In a preferred embodiment of the third, fourth and fifth aspect of present invention, the subject in need thereof falls into one or more of the following patient groups: the subject is a subject having mild and/or moderate hepatic impairment or a subject with normal hepatic liver function, a subject with high cardiovascular risk, a subject with (established) CHD or a subject with a CHD-risk equivalent. According to another preferred embodiment of the third, fourth and fifth aspect, the subject is a subject having mild and/or moderate hepatic impairment or a subject with normal hepatic liver function and/ or falls into one or more of the categories: a subject with high cardiovascular risk, a subject with (established) CHD or a subject with a CHD-risk equivalent , and the subject does not fall into one or more of the groups of subjects as listed in aspect 2.

According to a preferred embodiment of the third, fourth, fifth and the other aspects of present invention, the subject in need thereof has one or more of the following: elevated total cholesterol levels, elevated low-density lipoprotein cholesterol (LDL-C) levels, hypercholesterolemia, particularly hypercholesteremia uncontrolled by statins, severe hypercholesterolemia, refractory hypercholesterolemia, , hyperlipidemia, dyslipidemia, such as mixed dyslipidemia, atherosclerosis, cardiovascular diseases, primary hypercholesterolemia, such as primary familial hypercholesterolemia or primary non-familial hypercholesterolemia, hypercholesterolemia (especially primary hypercholesterolemia) uncontrolled by statins (particularly uncontrolled by atorvastatin).

According to another embodiment of the third, fourth, fifth and the other aspects of present invention, the subject in need thereof is a subject indicated for LDL apheresis, a subject with PCSK9-activating mutations, a subject with heterozygous Familial Hypercholesterolemia, a subject with primary hypercholesterolemia, a subject with primary hypercholesterolemia who is statin uncontrolled, a subject at risk for developing hypercholesterolemia, a subject with hypercholesterolemia, a subject with hyperlipidemia, a subject with dyslipidemia, a subject with atherosclerosis or a subject with high cardiovascular risk or a subject with one or more cardiovascular diseases such as CHD or a CHD risk equivalent . Most preferably, the subject in need thereof is a human subject.

Antibodies and antigen-binding fragments thereof that can be used for practicing the third, fourth, fifth and the other aspects of the present invention are described in the section *"Preferred Antibodies for Practicing the Present Invention".*

According to a preferred embodiment of the third, fourth, fifth and the other aspects of present invention, the antibody or antigen-binding fragment thereof is a recombinant human antibody or fragment thereof.

According to another preferred embodiment of the third, fourth, fifth and the other aspects of present invention, the antibody or the antigen-binding fragment thereof is characterized by one or more of the following:
(i) capable of reducing serum total cholesterol at least about 25 to about 35% and sustaining the reduction over at least a 24 day period relative to a predose level;
(ii) capable of reducing serum LDL cholesterol at least about 65-80% and sustaining the reduction over at least a 24 day period relative to a predose level;
(iii) capable of reducing serum triglyceride at least about 25-40% relative to predose level;
(iv) achieves one or more of (i)-(iii) without reducing serum HDL cholesterol or reducing serum HDL cholesterol no more than 5% relative to predose level;
(v) achieves one or more of (i)-(iii) with little or no measurable effect on liver function, as determined by ALT and AST measurements.

According to another preferred embodiment of the third, fourth, fifth and the other aspects of present invention, the antibody or the antigen-binding fragment thereof comprises
- a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
- a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736.

According to another preferred embodiment of the third, fourth, fifth and the other aspects of present invention, the antibody or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another preferred embodiment of the third, fourth, fifth and the other aspects of present invention, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88.

According to another preferred embodiment of the third, fourth, fifth and the other aspects of present invention, the antibody or antigen-binding fragment thereof competes for binding to hPCSK9 with an antibody comprising heavy and light chain CDR amino acid sequences as shown in SEQ ID NOs: 76, 78, 80, 84, 86, and 88.

According to another preferred embodiment of the third, fourth, fifth and the other aspects of present invention, the HMG-CoA reductase inhibitor is a statin. More preferably, the statin is selected from the group consisting of cerivastatin, atorvastatin, simvastatin, pitavastatin, rosuvastatin, fluvastatin, lovastatin, and pravastatin.

In some embodiments of the third, the fourth, fifth and other aspects of the invention, the HMG-CoA reductase inhibitor is administered three times per day, twice per day, or once per day. In some embodiments of the third, fourth, fifth and the other aspects of present invention, the HMG-CoA reductase inhibitor is administered every day, every other day, every third day, every fourth day, every fifth day, or every sixth day. In some embodiments of the third, fourth and the other aspects of present invention, the HMG-CoA reductase inhibitor is administered every week, every other week, every third week, or every fourth week. In some embodiments of the third, fourth and the other aspects of present invention, the HMG-CoA reductase inhibitor is administered in the morning, at noon or in the evening. In preferred embodiments, the HMG-CoA reductase inhibitor is administered once per day, preferably orally, preferably in the evening.

According to another preferred embodiment of the third, fourth, fifth and the other aspects of present invention, the HMG-CoA reductase inhibitor is a statin and the statin is preferably
- cerivastatin administered in a daily dosage of between 0.05 mg and 2 mg, preferably in a daily dosage of 0.2 mg, 0.4 mg, or 0.8 mg;
- atorvastatin administered in a daily dosage of between 2 mg and 100 mg, preferably in a daily dosage of 10 mg, 20 mg, 40 mg, or 80 mg;
- simvastatin administered in a daily dosage of between 2 mg and 100 mg, preferably in a daily dosage of 5 mg, 10 mg, 20 mg, 40 mg, or 80 mg;
- pitavastatin administered in a daily dosage of between 0.2 mg and 100 mg, preferably in a daily dosage of 1 mg, 2 mg, 5 mg, 10 mg, or 20 mg;
- rosuvastatin administered in a daily dosage of between 2 mg and 100 mg, preferably in a daily dosage of 5 mg, 10 mg, 20 mg, or 40 mg;
- fluvastatin administered in a daily dosage of between 2 mg and 100 mg, preferably in a daily dosage of 20 mg, 40 mg, or 80 mg;
- lovastatin administered in a daily dosage of between 2 mg and 100 mg, preferably in a daily dosage of 10 mg, 20 mg, 40 mg, or 80 mg; or
- pravastatin administered in a daily dosage of between 2 mg and 100 mg, preferably in a daily dosage of 10 mg, 20 mg, 40 mg, or 80 mg.

In a preferred embodiment of the third, fourth, fifth and the other aspects of present invention, the statin is atorvastatin and particularly atorvastatin administered in a daily dosage of between 2 mg and 100 mg, preferably in a daily dosage of 10 mg, 20 mg, 40 mg, or 80 mg.

The additional lipid lowering agent, particularly ezetimibe is preferably administered once a day. Effective daily dosages depend on various factors and the skilled person can readiliy determine the best dosage, conceivable daily dosages are e.g. be about 5 mg, about 8 mg, about 9 mg, about 10 mg, about 11 mg, about 15 mg and particularly about 10 mg.

In preferred embodiments of the third, fourth, fifth and the other aspects of present invention, the antibody or antigen-binding fragment thereof is administered to the subject every other week, every fourth week or once a month. Administration every fourth week or administration once a month (i.e. once per calendar month, e.g. every first, second etc. day of the month or every first, second third Monday, Tuesday etc. each month, in contrast to administration every fourth week) is preferred in view of patient compliance. Administration every other week is preferred in view of a very low variation of blood cholesterol levels. Other suitable time schedules for administration of the antibody or antigen-binding fragment thereof include without limitation an administration once per day, every other day, every third day, every fourth day, every fifth day, every sixth day, every week, every third week, every fifth week, every sixth week, every eighth week, every tenth week, and every twelfth week.

According to another preferred embodiment of the third, fourth, fifth and the other aspects of present invention, the antibody or antigen-binding fragment thereof is a recombinant human antibody or fragment thereof.

In another embodiment of the third, fourth, fifth and the other aspects, the antibody or antigen-binding fragment thereof is administered in a dosage amount ranging from 50 mg to 300 mg.

In preferred embodiments of the third, fourth, fifth and the other aspects of present invention, the antibody or antigen-binding fragment thereof is administered in a dosage amount ranging e.g. from about 40 mg to about 500 mg, from about 50 mg to about 500mg, from about 50mg to 300mg or from about 100mg to 200mg. In more preferred embodiments, the antibody or antigen-binding fragment thereof is administered in a dosage amount of about 50 mg, of about 100 mg, of about 150 mg, of about 200 mg, of about 250 mg, of about 300 mg, of about 350mg, of about 400 mg, of about 450 mg or of about 500 mg. Doses of about 50 to about 200 mg, e.g. of about 50 mg, about 100 mg, about 150 mg or about 200 mg are especially suitable for a biweekly dosage regimen (i.e. the application every other week), doses of about 150 mg to about 400 mg, e.g. about 150 mg, about 200 mg, about 250 mg, about 300 mg about 350 mg or about 400 mg are especially suitable for an administration regime with longer intervals, e.g. an administration every third or every fourth week or once a month.

According to a sixth aspect, present invention concerns an antibody or an antigen-binding fragment thereof which specifically binds hPCSK9 (human proprotein convertase subtilisin/kexin type 9) for use in the treatment of a disease or condition in which PCSK9 expression or activity causes an impact, wherein the antibody or antigen-binding fragment thereof is for administration to a subject falling at least into one of the groups of subjects as listed in the first aspect and/or wherein the antibody or antigen-binding fragment thereof is not for administration to a subject falling at least into one of the groups of subjects as listed in the second aspect.

In a preferred embodiment of the sixth aspect of present invention, the subject in need thereof falls into one or more of the following patient groups: the subject is a subject having mild and/or moderate hepatic impairment or a subject with normal hepatic liver function, a subject with high cardiovascular risk or a subject with (established) CHD or a CHD-risk equivalent. According to another preferred embodiment of the sixth aspect, the subject is a subject having mild and/or moderate hepatic impairment or a subject with normal hepatic liver function, a subject with high cardiovascular risk or a subject with (established) CHD or a CHD-risk equivalent and does not fall into one or more of the groups of subjects as listed in aspect 2.

In a preferred embodiment of the sixth aspect, the antibody or an antigen-binding fragment thereof is for administration in a dose of about 50 to 500 mg.

Antibodies and antigen-binding fragments thereof that can be used for practicing the sixth aspect of the present invention are described in the section *"Preferred Antibodies for Practicing the Present Invention".*

According to another preferred embodiment of the sixth aspect, the antibody or antigen-binding fragment thereof is for administration in a dose of about 50, 75, 100, 150, 200, 250, 300, 350, 400, 450 or 500 mg and preferably of about 75, 100, 150, 200 or 300 mg.

According to another preferred embodiment of the sixth aspect, the disease or condition is selected from the group consisting of: elevated total cholesterol levels, elevated low-density lipoprotein (LDL-C) levels, hypercholesterolemia, hyperlipidemia, dyslipidemia, and atherosclerosis, particularly primary hypercholesterolemia, familial hypercholesterolemia. or hypercholesteremia which is uncontrolled by statins.

According to another preferred embodiment of the sixth aspect, the antibody or antigen-binding fragment thereof is administered to the subject every other week (E2W), every fourth week (E4W) or once a month.

According to another preferred embodiment of the sixth aspect, the antibody or antigen-binding fragment thereof has one or more of the following characteristics:
(i) is for use in the reduction of low-density lipoprotein (LDL-C) levels of at least about -25% to about -40% relative to a predose level with a sustained reduction over at least a 14 day-period, wherein the sustained reduction is preferably at least -25% and more preferably at least -30% relative to a predose level, wherein the antibody or antigen-binding fragment thereof is preferably administered in a dose of about 40 to about 60 mg, about 45 to about 55 mg or about 50 mg E2W;
(ii) is for use in the reduction of low-density lipoprotein (LDL-C) of at least about -50% to about - 65% relative to a predose level with a sustained reduction over at least a 14 day-period, wherein the sustained reduction is preferably at least -40% and more preferably at least -45% relative to a predose level, wherein the antibody or fragment thererof is preferably administered in a dose of about 100 mg E2W;
(iii) is for use in the reduction of low-density lipoprotein (LDL-C) of at least about -60 % to at least about -75% [e.g. at least about -60 %, at least about -65%, at least about -70 or at least about -75%] relative to a predose level with a sustained reduction over at least a 14 day-period, wherein the sustained reduction is preferably at least -55% and more preferably at least -60% relative to a predose level. wherein the antibody or fragment thereof is preferably administered in a dose of about 150 mg E2W;
(iv) is for use in the reduction of low-density lipoprotein (LDL-C) of at least about 40% to about 75% relative to a predose level with a sustained reduction over at least a 28 day period, wherein the sustained reduction is preferably at least -35% and more preferably at least -40% relative to a predose level, wherein the antibody or fragment thereof is preferably administered in a dose of about 200 mg E4W;
(v) is for use in the reduction of low-density lipoprotein (LDL-C) of at least about -50 % to about -75% relative to a predose level with a sustained reduction over at least a 28 day-period, wherein the sustained reduction is preferably at least -40% and more preferably at least -45% relative to a predose level, wherein the antibody or fragment thereof is preferably administered in a dose of about 300 mg E4W;
(vi) is for use in the increase of serum HDL cholesterol levels of at least 2%, at least 2.5%, at least, 3%, at least 3.5%, at least 4%, at least 4.5%, at least 5% or at least 5.5% relative to a predose level;
(vii) s for use in the reduction of serum total cholesterol at least about 25% to about 35% relative to a predose level with sustained reduction over at least a 24 day period;
(viii) is for use in the reduction of serum total cholesterol at least about 65% to about 80% relative to a predose level with sustained reduction over at least a 24 day period;
(ix) is for use in the reduction of serum triglyeride levels at least about 25% to about 40% relative to a predose level;
(x) has little or no measurable effect on liver function, as determined by ALT and AST measurements, or on troponin levels;
(xi) is for use in the increase of one or more of: Total-Cholesterol levels, ApoB levels, non HDL-C levels, Apo-B/ApoA-1 ratio.

According to another preferred embodiment of the sixth aspect, the antibody or antigen-binding fragment thereof is for use together with another lipid lowering agent. The lipid lowering agent can be any lipid lowering agent as herein described.

According to a preferred embodiment of the sixth and the other aspects, the lipid lowering agent is an HMG-CoA reductase inhibitor, wherein the HMG-CoA reductase inhibitor is preferably administered in a dosage amount in the range of about 0.05 mg to about 100 mg and is preferably a statin, wherein the statin is preferably selected from the group consisting of: cerivastatin, atorvastatin, simvastatin, pitavastatin, rosuvastatin, fluvastatin, lovastatin or pravastatin.

According to another preferred embodiment of the sixth and the other aspects, the lipid lowering drug is ezetimibe, particularly ezetimibe in a daily dosage of about 10 mg.

According to another preferred embodiment of the sixth aspect, the antibody or antigen-binding fragment thereof is for use together with two or more lipid lowering agents. The lipid lowering agents can be any lipid lowering agent as herein described, wherein the lipid lowering agents are preferably a HMG-CoA reductase inhibitor (such as a statin) and another lipid lowering agent (such as ezetimibe).

Suitable combinations of antibody or fragment thereof plus HMG-CoA inhibitor (particularly a statin) plus ezetimibe comprise, but are not limited to antibody or fragment thereof plus ezetimibe plus cerivastatin, antibody or fragment thereof plus ezetimibe plus atorvastatin, antibody or fragment thereof plus ezetimibe plus simvastatin, antibody or fragment thereof plus ezetimibe plus pitavastatin, antibody or fragment thereof plus ezetimibe plus rosuvastatin, antibody or fragment thereof plus ezetimibe plus fluvastatin, antibody or fragment thereof plus ezetimibe plus lovastatin and antibody or fragment thereof plus ezetimibe plus pravastatin. Preferred combinations are antibody or fragment thereof plus ezetimibe plus atorvastatin or antibody or fragment thereof plus ezetimibe plus simvastatin.

The use in a combination treatment comprising administering the antibody or fragment therof together with an HMG-CoA reductase inhibitor (particularly a statin) according to some embodiments of the first and the other aspects of present invention is particularly useful for treatment of subjects of the first aspect of present invention, particularly subjects having mild and/or moderate hepatic impairment, subjects with normal hepatic liver function, subjects with high cardiovascular risk, subjects with coronary heart disease, cardiovascular disease or a CHD-risk equivalent and most preferred for treatment of subjects with high cardiovascular risk or with CHD or a CHD-risk equivalent.

The use in a combination treatment comprising administering the antibody or fragment therof together with a lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

The use in a combination treatment comprising administering the antibody or fragment therof together with an HMG-CoA reductase inhibitor (particularly a statin) and together with administration of another lipid lowering drug, particularly ezetimibe, according to some embodiments of the first and the other aspects of present invention is particularly useful for treatment of subjects of the first aspect of present invention, particularly subjects subjects having mild and/or moderate hepatic impairment, subjects with normal hepatic liver function, subjects with high cardiovascular risk, subjects with cardiovascular disease, coronary heart disease or a CHD-risk equivalent and most preferred for treatment of subjects with high cardiovascular risk or with CHD or a CHD-risk equivalent.

According to another preferred embodiment of the sixth aspect, the statin is administered according to one or more of the following dosage or admimistration regimes:
(i) the statin is administered once per day,
(ii) the statin administered at a dosage of about 0,5 to about 100 mg, about 5 to about 90 mg, of about 10, 20, 40 or 80 mg and is preferably atorvastatin.

Further dosage and admimistration regimes of the antibody, antigen fragment therof, the lipid lowering agents such as the HMG-CoA reductase inhibitor or ezetimibe are described at the general description/definitions section and at the other aspects of present invention and preferably at the first, second or eighth aspect.

The antibody of the sixth aspect of present invention exhibits the above properties preferably if administered in combination with an HMG-CoA reductase inhibitor treatment. Preferred embodiments of HMG-CoA reductase inhibitors to be used in conjunction with the antibody of the invention and dosage and administration regimes thereof can be found throughout the specification, particularly as described in aspects 1, 2, 3, 4 or 5.

According to a preferred embodiment of the antibodies and antigen-binding fragments thereof of present invention, particularly of the antibody or antigen-binding fragment according to the sixth aspect, the antibody or antigen binding fragment thereof has one or more of the following characteristics:
(i) The antibody or the antigen-binding fragment comprises the heavy and light chain CDRs of a HCVR/LCVR amino acid sequence pair as shown in SEQ ID NOs: 90/92.
(ii) The antibody or antigen-binding fragment thereof comprises a HCVR/LCVR amino acid sequence pair as shown in SEQ ID NOs: 90/92.
(iii) The antibody or antigen-binding fragment thereof competes for binding to hPCSK9 with an antibody or antigen-binding fragment comprising a HCVR/LCVR amino acid sequence pair as shown in SEQ ID NOs: 90/92.

According to another preferred embodiment of the antibodies and antigen-binding fragments thereof of present invention, particularly of the antibody or antigen-binding fragment according to the sixth aspect, the antibody or antigen binding fragment thereof has one or more of the following characteristics:
(i) overcomes statin resistance in mammals, especially in rodents such as hamster
(ii) increases LDLR expression in mammals, particularly in rodents such as hamster
(iii) decreases of serum LDL-C in rodents such as hamster
(iv) synergistically decreases LDL-C in conjunction with HMG-CoA reductase inhibitor administration, particularly in humans or in rodents such as hamster, wherein the HMG-CoA reductase inhibitor is preferably atorvastatin.

Further suitable characteristics and structural features of the antibody of present invention and particularly of the antibody of the sixth aspect, as well as antibodies and antigen-binding fragments thereof that can be used for practicing the sixth aspect and the other aspects of the present invention are described in the section *"Preferred Antibodies for Practicing the Present Invention".*

The antibody of present invention, such as the antibody according to the sixth aspect, is preferably formulated as a pharmaceutically applicable formulation as known in the art, and specifically as herein described, such as dry formulation for dissolution or liquid formulation.

According to a seventh aspect, present invention concerns a pharmaceutical composition comprising an antibody or antigen-binding fragment thereof which specifically binds hPCSK9 and a pharmaceutically acceptable excipient for use in a method according to one of the aspects 1, 2, 3, 4 or 5.

In a preferred embodiment of the seventh aspect of present invention, the pharmaceutical composition is for use in a method according to the first aspect or the second aspect and preferably for use in a method according to the first and second aspect.

According to a further preferred embodiment, the pharmaceutical composition is for use in method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function.

According to a further preferred embodiment, the pharmaceutical composition is for use in method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function, wherein the subject does not fall into one or more of the groups of subjects as listed in aspect 2.

The antibody as to be used for the subject of the seventh aspect can be as described in the section *"Preferred Antibodies for Practicing the Present Invention".* Further features and further preferred embodiments of the antibody as to be used in the context of the sixth aspect are also described in the fifth aspect.

According to another preferred embodiment, the pharmaceutical composition comprises about about 40 to about 500 mg of the antibody or antigen-binding fragment per dose.

According to another preferred embodiment, the pharmaceutical composition comprises about about 50 mg to about 500mg, about 50 mg to about 300mg, about 50 mg, about 100 mg, about 150 mg, about 200 mg, about 250 mg, about 300 mg, about 350mg, of about 400 mg, about 450 mg or about 500 mg of the antibody or antigen-binding fragment thereof.

According to another preferred embodiment, the pharmaceutical composition comprises about 75, 100, 150, 200 or 300 mg of the antibody or antigen-binding fragment thereof.

According to another preferred embodiment, the pharmaceutical composition comprises an effective dose of an antibody or an antigen-binding fragment thereof which specifically binds hPCSK9 (human proprotein convertase subtilisin/kexin type 9), wherein the dose is sufficient for sustained reduction of low-density lipoprotein (LDL-C) levels over a period of at least 14, at least 15, at least 16, at least 17, at least 18, at least 19, at least 20, at least 21, at least 22, at least 23 , at least 24, at least 25, at least 26, at least 27 or at least 28 days after administration, together with a pharmaceutically acceptable excipient or carrier. According to another preferred embodiment, the dose is sufficient for sustained reduction of LDL-C levels over a period of at least 14 days, at least 28 days or at least 1 month.

According to another preferred embodiment, the pharmaceutical composition comprises an effective amount of an HMG-CoA reductase inhibitor.

According to another preferred embodiment the pharmaceutical composition is arranged together with an effective amount of an HMG-CoA reductase inhibitor.

According to another preferred embodiment, the pharmaceutical composition comprises an effective amount of an HMG-CoA reductase inhibitor and another lipid lowering drug. According to another preferred embodiment the pharmaceutical composition is arranged together with an effective amount of an HMG-CoA reductase inhibitor and another lipid lowering drug.

According to another preferred embodiment, the HMG-CoA reductase inhibitor is a statin, preferably selected from the list consisting or: cerivastatin, atorvastatin, simvastatin, pitavastatin, rosuvastatin, fluvastatin, lovastatin or pravastatin and is preferably atorvastatin.

According to another preferred embodiment, the other lipid lowering drug is ezetimibe, particularly in a dosage amount of about 10 mg or in any other dosage amount as herein described.

According to another preferred embodiment, the pharmaceutical composition comprises about 0.05 mg to about 100 mg, about 0,5 mg to about 100 mg, about 5 mg to about 90 mg, about 10 mg, about 20 mg, about 40 mg or about 80 mg of HMG-CoA reductase inhibitor and preferably of about 10, about 20, about 40 or about 80 mg, and particularly of about 10, about 20, about 40 or about 80 mg of atorvastatin.

According to another preferred embodiment, the pharmaceutical composition comprises an effective dose of HMG-CoA reductase inhibitor for lowering LDL-C levels by administration once per day.

The antibody or antigen-binding fragment thereof to be used for the pharmaceutical composition according to present invention can be any antibody as described herein, such as in the section *"Preferred Antibodies for Practicing the Present Invention"* or in the fifteenth and further aspects.

According to a preferred embodiment, the antibody or antigen-binding fragment thereof has one or more of the following features when administered to a subject, such as a human or non-human mammal:
(a) reduction of low-density lipoprotein (LDL-C) levels of at least about -25% to about -40% relative to a predose level with a sustained reduction over at least a 14 day-period upon administration to a subject, wherein the sustained reduction is preferably at least -25% and more preferably at least -30% relative to a predose level, particularly if administered in a dose of about 40 to about 60 mg, preferably about 45 to about 55 mg and more preferably about 50 mg in a biweekly administration regime (every other week, E2W),
(b) reduction of low-density lipoprotein (LDL-C) of at least about -50% to about -65% relative to a predose level with a sustained reduction over at least a 14 day-period upon administration to a subject, wherein the sustained reduction is preferably at least -40% and more preferably at least -45% relative to a predose level, particularly if administered in a dose of about 100 mg E2W,
(c) reduction of low-density lipoprotein (LDL-C) of at least about -60 % to at least about -75% [e.g. at least about -60 %, at least about -65%, at least about -70 or at least about -75%] relative to a predose level with a sustained reduction over at least a 14 day-period upon administration to a subject, wherein the sustained reduction is preferably at least -55% and more preferably at least -60% relative to a predose level, particularly when administered in a dose of about 150 mg E2W,
(d) reduction of low-density lipoprotein (LDL-C) of at least about 40% to about 75% relative to a predose level with a sustained reduction over at least a 28 day period , wherein the sustained reduction is preferably at least -35% and more preferably at least -40% relative to a predose level, particularly when administered in a dose of about 200 mg E4W,
(e) reduction of low-density lipoprotein (LDL-C) of at least about -50 % to about -75% relative to a predose level with a sustained reduction over at least a 28 day-period upon administration to a subject, wherein the sustained reduction is preferably at least -40% and more preferably at least -45% relative to a predose level, particularly when administered in a dose of about 300 mg E4W,
(f) increase of serum HDL cholesterol levels of at least 2%, at least 2.5%, at least, 3%, at least 3.5%, at least 4%, at least 4.5%, at least 5% or at least 5.5% relative to a predose level upon administration to a subject, particularly when admimistered in a dose of about 150 mg E2W,
(g) little or no measurable effect on troponin levels upon administration to a subject,
(h) increase of one or more of: Total-Cholesterol levels, ApoB levels, non HDL-C levels, Apo-B/ApoA-1 ratio, upon administration to a subject.

According to another preferred embodiment, the antibody or antigen-binding fragment thereof is capable of overcoming statin resistance when administered to a subject with statin-resistant hypercholesterolemia.

According to another preferred embodiment, the antibody or antigen-binding fragment thereof comprises the heavy and light chain CDRs of a HCVR/LCVR amino acid sequence pair as shown in SEQ ID NOs: 90/92 substantially identical sequences having at least 98% or 99% identity therewith.

According to another preferred embodiment, the antibody or antigen-binding fragment thereof comprises a HCVR/LCVR amino acid sequence pair as shown in SEQ ID NOs: 90/92 or a pair of substantially identical sequences having at least 98% or 99% identity therewith.

According to another preferred embodiment, the antibody or antigen-binding fragment thereof competes for binding to hPCSK9 with an antibody or antigen-binding fragment comprising a HCVR/LCVR amino acid sequence pair as shown in SEQ ID NOs: 90/92.

According to another preferred embodiment, the antibody or antigen-binding fragment thereof binds an epitope comprising amino acid residue 238 of hPCSK9 (SEQ ID NO:755).

According to another preferred embodiment, the antibody or antigen-binding fragment thereof binds an epitope comprising one or more of amino acid residues at positions 238, 153, 159 and 343 of hPCSK9 (SEQ ID NO:755).

According to another preferred embodiment, the antibody or antigen-binding fragment thereof binds an epitope which does not comprise an amino acid residue at positions 192, 194, 197 and/or 237 of hPCSK9 (SEQ ID NO:755).

The pharmaceutical composition can be formulated according to any pharmaceutically applicable formulation as known in the art, and specifically as herein described, such as dry formulation for dissolution or liquid formulation. Suitable formulations of antibodies are known in the art and comprise dry formulations (e.g. freeze-dried, spray-dried or lyophilized, water-free concentrate) as well as liquid formulations (e.g. solutions).

The term "carrier" in the context of present invention refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The composition may further contain one or more other active ingredients such as an HMG-CoA reductase inhibitor. The formulation should suit the mode of administration.

According to a preferred embodiment, the pharmaceutical composition comprises the antibody or antigen-binding fragment thereof as dry formulation for dissolution such as a lyophilized powder, freeze-dried or spray-dried powder or water free concentrate.

According to another preferred embodiment, the pharmaceutical composition comprises the antibody or antigen-binding fragment thereof as liquid formulation, e.g. injection or infusion solution.

According to another preferred embodiment, the pharmaceutical composition comprises the HMG-CoA reductase inhibitor as oral or peroral formulation, e.g. capsule or tabled, or as liquid formulation, e.g. suspension, dispersion or solution, e.g. for peroral administration, injection or infusion.

According to another preferred embodiment the pharmaceutical composition is for use in the treatment of a disease or disorder for use in the treatment of a disease or condition in which PCSK9 expression or activity causes an impact or for lowering elevated total cholesterol or elevated LDL-C levels. Further preferred uses, dosage regimens, administration regimens of the antibody or fragment thereof or of the HMG-CoA reductase inhibitor, or populations to be treated with the pharmaceutical composition described in present application, for example at the other aspects of present invention such as the first or second aspect.

The pharmaceutical composition of the antibody as used in the different aspects of present invention, e.g. the sixth or seventh aspect, can e.g. be a liquid formulation as herein described comprising the antibody or antigen-binding fragment thereof of present invention, and preferably comprising about 40 mg to about 200 mg or about 50 to about 200 mg, e.g. about 40 mg, about 50 mg, about 75 mg, at about 100 mg, about 150 mg or about 200 mg of the antibody or antigen-binding fragment thereof per 1 ml volume.

The pharmaceutical composition of the antibody as used in the different aspects of present invention, e.g. the sixth or seventh aspect, can e.g. be a dry formulation as herein described comprising the antibody or antigen-binding fragment thereof of present invention, and preferably comprising about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and more preferably about 50 mg, about 100 mg, about 150 mg, about 200 mg, about 250 mg, about 300 mg and even more preferably about 150 mg, about 200 mg or about 300 mg of the antibody or antigen-binding fragment thereof per dose.

The formulations of present invention can comprise further active ingredients such as an HMG-CoA reductase inhibitor as herein described.

Suitable formulations of antibodies in general are known in the art and comprise dry formulations (e.g. freeze-dried, spray-dried or lyophilized, water-free concentrate) as well as liquid formulations (e.g. solutions). Suitable formulations of statins are as well known in the art and comprise dry formulations as well as liquid formulations, e.g suspensions, dispersions and solutions (for a reference, see e.g. "Statins therapy: a review on conventional and novel formulation approaches" R. Tiwari and K. Pathak, Journal of Pharmacy and Pharmacology, 2011, that is hereby incorporated in entirety).

In preferred embodiments of the sixth, seventh and the other aspects of present invention, the disease or condition in which PCSK9 expression or activity causes an impact is ameliorated, improved, inhibited or prevented with a PCSK9 antagonist.

According to another embodiment of the sixth, seventh and the other aspects of present invention a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of an HMG-CoA reductase inhibitor is administered to said subject, wherein the HMG-CoA reductase inhibitor is administered in a dosage amount ranging from 0.05 mg to 100 mg.

According to yet another embodiment of the sixth, seventh and the other aspects of present invention, a therapeutic amount of an antibody or an antigen-binding fragment thereof which specifically binds hPCSK9 (human proprotein convertase subtilisin/kexin type 9) is administered to a subject in need thereof, wherein the antibody or antigen-binding fragment thereof is administered in a dosage amount ranging from 5 mg to 500 mg, and a therapeutic amount of an HMG-CoA reductase inhibitor is administered to said subject, wherein the HMG-CoA reductase inhibitor is administered in a dosage amount ranging from 0.05 mg to 100 mg.

According to another embodiment of the sixth, seventh and the other aspects of present invention, the disease or condition is selected from the group consisting of: elevated total cholesterol levels, elevated low-density lipoprotein cholesterol (LDL-C) levels, hypercholesterolemia, particularly hypercholesteremia uncontrolled by statins, severe hypercholesterolemia, refractory hypercholesterolemia, , hyperlipidemia, dyslipidemia, atherosclerosis, cardiovascular diseases, primary hypercholesterolemia, such as primary familial hypercholesterolemia or primary non-familial hypercholesterolemia, hypercholesterolemia (especially primary hypercholesterolemia) uncontrolled by statins (particularly uncontrolled by atorvastatin).

According to another embodiment of the sixth, seventh and the other aspects of present invention, the subject in need thereof is a subject indicated for LDL apheresis, a subject with PCSK9-activating mutations, a subject with heterozygous Familial Hypercholesterolemia, a subject with primary hypercholesterolemia, a subject with primary hypercholesterolemia who is statin uncontrolled, a subject at risk for developing hypercholesterolemia, a subject with hypercholesterolemia, a subject with hyperlipidemia, a subject with dyslipidemia, a subject with atherosclerosis or a subject with cardiovascular diseases. Most preferably, the subject in need thereof is a human subject.

Antibodies and antigen-binding fragments thereof that can be used for practicing the sixth, seventh and the other aspects of the present invention are e.g. described in the section *"Preferred Antibodies for Practicing the Present Invention".*

According to a preferred embodiment of the sixth, seventh and the other aspects of present invention, the antibody or antigen-binding fragment thereof is a recombinant human antibody or fragment thereof.

According to another preferred embodiment of the sixth, seventh and the other aspects of present invention, the antibody or the antigen-binding fragment thereof is characterized by one or more of the following:
(i) capable of reducing serum total cholesterol at least about 25 to about 35% and sustaining the reduction over at least a 24 day period relative to a predose level;
(ii) capable of reducing serum LDL cholesterol at least about 65-80% and sustaining the reduction over at least a 24 day period relative to a predose level;
(iii) capable of reducing serum triglyceride at least about 25-40% relative to predose level;
(iv) achieves one or more of (i)-(iii) without reducing serum HDL cholesterol or reducing serum HDL cholesterol no more than 5% relative to predose level;
(v) achieves one or more of (i)-(iii) with little or no measurable effect on liver function, as determined by ALT and AST measurements.

According to another preferred embodiment of the sixth, seventh and the other aspects of present invention, the antibody or the antigen-binding fragment thereof comprises
- a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
- a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736.

According to another preferred embodiment of the sixth, seventh and the other aspects of present invention, the antibody or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.

According to another preferred embodiment of the sixth, seventh and the other aspects of present invention, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88.

According to another preferred embodiment of the sixth, seventh and the other aspects of present invention, the antibody or antigen-binding fragment thereof competes for binding to hPCSK9 with an antibody comprising heavy and light chain CDR amino acid sequences as shown in SEQ ID NOs: 76, 78, 80, 84, 86, and 88.

According to another preferred embodiment of the sixth, seventh and the other aspects of present invention, a therapeutic amount of an HMG-CoA reductase inhibitor is administered to the subject in a dosage of between 0.05 mg to 100 mg. Preferably, the HMG-CoA reductase inhibitor is a statin. More preferably, the statin is selected from the group consisting of cerivastatin, atorvastatin, simvastatin, pitavastatin, rosuvastatin, fluvastatin, lovastatin, and pravastatin.

In some embodiments of the sixth, seventh and other aspects of the invention, the HMG-CoA reductase inhibitor is administered three times per day, twice per day, or once per day. In some embodiments of the sixth, seventh and the other aspects of present invention, the HMG-CoA reductase inhibitor is administered every day, every other day, every third day, every fourth day, every fifth day, or every sixth day. In some embodiments of the sixth, seventh and the other aspects of present invention, the HMG-CoA reductase inhibitor is administered every week, every other week, every third week, or every fourth week. In some embodiments of the sixth, seventh and the other aspects of present invention, the HMG-CoA reductase inhibitor is administered in the morning, at noon or in the evening. In preferred embodiments, the HMG-CoA reductase inhibitor is administered once per day, preferably orally, preferably in the evening.

According to another preferred embodiment of the sixth, seventh and the other aspects of present invention, the HMG-CoA reductase inhibitor is a statin and the statin is preferably
- cerivastatin administered in a daily dosage of between 0.05 mg and 2 mg, preferably in a daily dosage of 0.2 mg, 0.4 mg, or 0.8 mg;
- atorvastatin administered in a daily dosage of between 2 mg and 100 mg, preferably in a daily dosage of 10 mg, 20 mg, 40 mg, or 80 mg;
- simvastatin administered in a daily dosage of between 2 mg and 100 mg, preferably in a daily dosage of 5 mg, 10 mg, 20 mg, 40 mg, or 80 mg;
- pitavastatin administered in a daily dosage of between 0.2 mg and 100 mg, preferably in a daily dosage of 1 mg, 2 mg, 5 mg, 10 mg, or 20 mg;
- rosuvastatin administered in a daily dosage of between 2 mg and 100 mg, preferably in a daily dosage of 5 mg, 10 mg, 20 mg, or 40 mg;
- fluvastatin administered in a daily dosage of between 2 mg and 100 mg, preferably in a daily dosage of 20 mg, 40 mg, or 80 mg;
- lovastatin administered in a daily dosage of between 2 mg and 100 mg, preferably in a daily dosage of 10 mg, 20 mg, 40 mg, or 80 mg; or
- pravastatin administered in a daily dosage of between 2 mg and 100 mg, preferably in a daily dosage of 10 mg, 20 mg, 40 mg, or 80 mg.

In a preferred embodiment of the sixth, seventh or the other aspects of present invention, the statin is atorvastatin and particularly atorvastatin administered in a daily dosage of between 2 mg and 100 mg, preferably in a daily dosage of 10 mg, 20 mg, 40 mg, or 80 mg;

In preferred embodiments of the sixth, seventh and the other aspects of present invention, the antibody or antigen-binding fragment thereof is administered to the subject every other week, every fourth week or once a month. Administration every fourth week or administration once a month (i.e. once per calendar month, e.g. every first, second etc. day of the month or every first, second third Monday, Tuesday etc. each month, in contrast to administration every fourth week) is preferred in view of patient compliance. Administration every other week is preferred in view of a very low variation of blood cholesterol levels. Other suitable time schedules for administration of the antibody or antigen-binding fragment thereof include without limitation an administration once per day, every other day, every third day, every fourth day, every fifth day, every sixth day, every week, every third week, every fifth week, every sixth week, every eighth week, every tenth week, and every twelfth week.

According to another preferred embodiment of the sixth, seventh and the other aspects of present invention, the antibody or antigen-binding fragment thereof is a recombinant human antibody or fragment thereof.

In another embodiment of the sixth, seventh and the other aspects, the antibody or antigen-binding fragment thereof is administered in a dosage amount ranging from 50 mg to 300 mg.

In preferred embodiments of the sixth, seventh and the other aspects of present invention, the antibody or antigen-binding fragment thereof is administered in a dosage amount ranging e.g. from about 40 mg to about 500 mg, from about 50 mg to about 500mg, from about 50mg to 300mg or from about 100mg to 200mg. In more preferred embodiments, the antibody or antigen-binding fragment thereof is administered in a dosage amount of about 50 mg, of about 100 mg, of about 150 mg, of about 200 mg, of about 250 mg, of about 300 mg, of about 350mg, of about 400 mg, of about 450 mg or of about 500 mg. Doses of about 50 to about 200 mg, e.g. of about 50 mg, about 75 mg, about 100 mg, about 150 mg or about 200 mg are especially suitable for a biweekly dosage regimen (i.e. the application every other week), doses of about 150 mg to about 400 mg, e.g. about 150 mg, about 200 mg, about 250 mg, about 300 mg about 350 mg or about 400 mg are especially suitable for an administration regime with longer intervals, e.g. an administration every third or every fourth week or once a month.

According to a further preferred embodiment of the seventh aspect of present invention, the pharmaceutical composition comprising the antibody or fragment thereof is comprised in a unit dosage form as herein described (e.g. as). In this particular embodiment, the present invention relates to a unit dosage form comprising the antibody or fragment thereof for use in a method according to the 1^{st} 2^{nd}, 3^{rd}, 4^{th} or 5^{th} aspect of present invention. In a particularly preferred embodiment, the unit dosage form is for use in a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function. Further embodiments of the unit dosage form can be as e.g. described in the Definitions section or in the eighth aspect of present invention. Further embodiments of the pharmaceutical use of the unit dosage form are e.g. as described in the Definitions section or in the 1^{st}, 2^{nd}, 3^{rd}, 4^{th} or 5^{th} aspect of present invention. Further embodiments of the antibody comprised in the unit dosage form are e.g. described in the section *"Preferred antibodies for practicing present invention"* or in the sixth aspect.

In an eighth aspect, present invention concerns an article of manufacture comprising
(a) a packaging material or container,
(b) an antibody or an antigen-binding fragment thereof which specifically binds hPCSK9;
   and
(c) a label or packaging insert, wherein the label or packaging insert indicates the following:
   (1) the antibody or antigen-binding fragment thereof can be applied for treatment to subjects falling into one or more groups of subjects as recited the first aspect;
   (2) the label or packaging insert preferably further indicates that the application of the antibody or antigen-binding fragment thereof to subjects is contraindicated for subjects belonging to one or more of the groups of subjects as recited in the second aspect.

According to one preferred embodiment of the eighth aspect, the label or packaging insert indicates that the antibody or pharmaceutical composition comprising the antibody can be applied a subject having mild hepatic impairment and/or moderate hepatic impairment or to a subject with normal hepatic liver function and it indicates that the application of the antibody or antigen-binding fragment thereof to subjects is contraindicated for subjects belonging to one or more of the groups of subjects as recited in the second aspect.

According to a further preferred embodiment of the eighth aspect, the label or packaging insert indicates that the antibody or pharmaceutical composition is for use in a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function, a subject having an increased cardiovascular risk, a subject having (established) CHD or a CHD risk equivalent.

According to a further preferred embodiment, the label or packaging insert indicates that the antibody or pharmaceutical composition is for use in a method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function, wherein the subject does not fall into one or more of the groups of subjects as listed in aspect 2.

The antibody or fragment thereof for use in the eighth aspect can be any antibody as herein described, such as the antibody as described in the section *"Preferred antibodies for practicing present invention".*

According to one embodiment, the article of manufacture comprises a pharmaceutical composition comprising the antibody or fragment thereof together with at least one pharmaceutically acceptable excipient or carrier. Different embodiments of the pharmaceutical composition comprising the antibody or fragment thereof for use in the eighth aspect of present invention are listed below and can also be taken from the general Definition section or from the seventh aspect of present invention.

The pharmaceutical composition can be formulated according to any pharmaceutically applicable formulation as known in the art, and specifically as herein described, such as dry formulation for dissolution or liquid formulation. Suitable formulations of antibodies are known in the art and comprise dry formulations (e.g. freeze-dried, spray-dried or lyophilized, water-free concentrate) as well as liquid formulations (e.g. solutions). Suitable formulations of statins are as well known in the art and comprise dry formulations as well as liquid formulations, e.g suspensions, dispersions and solutions (for a reference, see e.g. "Statins therapy: a review on conventional and novel formulation approaches" R. Tiwari and K. Pathak, Journal of Pharmacy and Pharmacology, 2011, that is hereby incorporated in entirety).

The term "carrier" in the context of present invention refers to a diluent, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a prophylactically or therapeutically effective amount of the antibody, preferably in purified form, together with a suitable amount of carrier so as to provide the form for proper administration to the patient. The composition may further contain one or more other active ingredients such as an HMG-CoA reductase inhibitor. The formulation should suit the mode of administration.

According to a preferred embodiment of the eighth and the further aspect of present invention, the antibody or fragment thereof is comprised in a pharmaceutical composition together with a pharmaceutically acceptable excipient. Suitable pharmaceutical compositions for practicing the eighth aspect and the other aspects of present invention are for example described in the definition section of present application or in the context of the seventh aspect of present invention.

According to another preferred embodiment of the eighth aspect of present invention, or of any other aspect comprising a label or packaging insert, the label or packaging insert contains reference to one or more of the methods of treatment according to any of aspects 1 to 5 or one or more of the embodiments of said method.

According to another preferred embodiment of the eighth aspect, the label or packaging insert indicates that the antibody or pharmaceutical composition comprising the antibody can be administered in combination with an HMG-CoA reductase inhibitor such as a statin, and optionally further indicates that the antibody (e.g. together with the HMG-CoA reductase inhibitor such as the statin) can be administered in combination with another lipid modulating or lipid lowering agent such as ezetimibe.

According to yet another embodiment of the eighth aspect of present invention, or of any other aspect comprising a label or packaging insert, the label or packaging insert has one or more of the following features:
(a) the label or packaging insert indicates that patients receiving treatment with said antibody or antigen-binding fragment can be treated for a disease or condition selected from the group consisting of hypercholesterolemia, hyperlipidemia, dyslipidemia, atherosclerosis and cardiovascular diseases such as CHD or a CHD risk equivalent;
(b) the label or packaging insert indicates the treatment of patients with said antibody or antigen-binding fragment thereof together with the application of a lipid lowering agent such as an HMG-CoA reductase inhibitor, and preferably a statin;
(c) the label or packaging insert indicates the treatment of patients with said antibody or antigen-binding fragment thereof together with the application of a lipid lowering agent such as ezetimibe;
(d) the label or packaging insert indicates the treatment of patients with said antibody or antigen-binding fragment thereof together with the application of a lipid lowering agent such as an HMG-CoA reductase inhibitor, and preferably a statin and together with the application of a lipid lowering agent such as ezetimibe, particularly a combination treatment of the antibody or antigen-binding fragment thereof together with a statin and ezetimibe;
(e) the label or packaging insert indicates that the treatment of patients according to (b), (c) or (d) above, is possible for patients belonging to one or more of the subject groups as listed in aspect 1, and preferably is possible for subjects having mild and/or moderate hepatic impairment or subjects having normal hepatic liver function and/or having an increased cardiovascular risk and/or (established) CHD and/or a CHD-risk equivalent;
(f) the label or packaging insert indicates that the treatment of patients with said antibody or antigen-binding fragment thereof according to (b), (c) or (d), is contraindicated for patients belonging to one or more of the subject groups as listed in aspect 2.

According to one embodiment, the article of manufacture comprises another lipid lowering agent as herein described. HMG-CoA reductase inhbitor and preferably an HMG-CoA inhibitor as described in one of the other aspects, such as the first or sixth aspect.

The article of manufacture can for example comprise about 0.05 mg to about 100 mg, about 0,5 mg to about 100 mg, about 5 mg to about 90 mg, about 10 mg, about 20 mg, about 40 mg or about 80 mg of HMG-CoA reductase inhibitor and preferably about 10, about 20, about 40 or about 80 mg of HMG-CoA reductase inhibitor, preferably formulated for oral administration.

The article of manufacture can e.g. comprise one or more effective doses of HMG-CoA reductase inhibitor for lowering LDL-C levels by administration once per day.

The article of manufacture can for example also comprise the additional lipid lowering agent, particularly ezetimibe. The article of manufacture can e.g. comprise one or more effective doses of ezetimibe for lowering LDL-C levels by administration once per day, preferably formulated for oral administration. Effective daily dosages depend on various factors and the skilled person can readiliy determine the best dosage, conceivably daily dosages are e.g. be about 5 mg, about 8 mg, about 9 mg, about 10 mg, about 11 mg, about 15 mg and particularly about 10 mg.

The article of manufacture can also comprise a combination of two or more lipid lowering agents in addition to the antibody or fragment thereof, e.g. the antibody or fragment thereof together with an HMG-CoA reductase inhibitor such as a statin and together with another lipid lowering drug such as ezetimibe.

According to another suitable embodiment, the article of manufacture comprises the antibody as injection solution or as dry formulation as described in present invention.

According to one embodiment of the eighth and the other aspects of present invention, the injection solution of antibody comprises about 40 mg to about 200 mg or about 40 to about 200 mg, e.g. about 40 mg, about 50 mg, about 75 mg, at about 100 mg, about 150 mg or about 200 mg of the antibody or antigen-binding fragment thereof per 1 ml volume.

According to another embodiment of the eighth and the other aspects of present invention, the dry formulation of antibody comprises about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and preferably about 50 mg, about 100 mg, about 150 mg, about 200 mg, about 250 mg, about 300 mg and even more preferably about 150 mg, about 200 mg or about 300 mg of the antibody or antigen-binding fragment thereof per dose or per unit dosage form.

According to another embodiment, the article of manufacture of present invention comprises one or more separate unit dosage forms of the antibody or fragment thereof, the pharmaceutical composition comprising the antibody or fragment thereof, the dry formulation of the antibody or fragment thereof or the injection solution of the antibody or fragment thereof.

As used in the different aspects and embodiments of present invention and in particularly of the eighth aspect, the term "unit dosage form" refers to physically discrete units suitable as unitary dosages for human and animal subjects, each unit containing a predetermined quantity of active material (e.g., about 40mg or about 50mg or about 75 mg to about 500mg of PCSK5 antibody and/or of e.g. 0.05mg to 100 mg HMG-CoA reductase inhibitor and/or about 8 to 12 mg and preferably about 10 mg of ezetimibe) calculated to produce the desired therapeutic effect in association with the required pharmaceutical diluent, carrier or vehicle. The specifications for the unit dosage forms to be used in the context of present invention are dictated by and are directly dependent on (a) the unique characteristics of the active material and the particular therapeutic effect to be achieved, and (b) the limitation inherent in the art of compounding such an active material for therapeutic use in animals or humans, as disclosed in this specification, these being features of the present invention. The term "active material" refers to any material with therapeutic activity, such as one or more active ingredients. The active ingredients to be employed as therapeutic agents can be easily prepared in such unit dosage form with the employment of pharmaceutical materials which themselves are available in the art and can be prepared by established procedures. Preferred active ingredients of present invention are the antibody or fragment thereof or an HMG-CoA reductase inhibitor such as a statin.

Examples of suitable unit dosage forms in accord with the different aspect of this invention and particularly in the context of the eighth aspect of present invention are vials, tablets, capsules, troches, suppositories, powder packets, wafers, cachets, ampules, segregated multiples of any of the foregoing, and other forms as herein described or generally known in the art. One or more such unit dosage forms of the antibody or fragment thereof can be comprised in an article of manufacture of present invention, optionally further comprising one or more unit dosage forms of an HMG-CoA reductase inhibitor (e.g. a blister of tablets comprising as active ingredient the HMG-CoA reductase inhibitor) and optionally further comprising one or more unit dosage forms of a another lipid lowering agents such as ezetimibe (e.g. a blister of tablets or capsules comprising as active ingredient the other lipid lowering drug such as ezetimibe).

According to one embodiment, the article of manufacture of present invention comprises one or more separate unit dosage forms of the antibody or fragment thereof, the pharmaceutical composition comprising the antibody or fragment thereof, the dry formulation of the antibody or fragment thereof or the injection solution of the antibody or fragment thereof and one or more separate unit dosage forms of the HMG-CoA reductase inhibitor. According to another embodiment, the article of manufacture additionally comprises one or more separate unit dosage forms of another lipid modulating or lipid lowering agent such as ezetimibe.

In a preferred embodiment of the eighth aspect, the unit dosage form comprises 40 - about 500 mg of the antibody or an antigen-binding fragment of present invention. According to another preferred embodiment, the unit dosage form comprises about 40 mg, about 50 mg, about 75 mg, about 100 mg, about 150 mg, about 200 mg, about 250 mg, about 300 mg, about 350 mg, about 400 mg, about 450 mg, or about 500 mg and more preferably about 150 mg, about 200 mg or about 300 mg of the antibody or antigen-binding fragment thereof. Further preferred dosages, and dosage regimens are as described elsewhere in the application, such as in the Definition section or in the first, or second to fifth aspect of present invention.

According to a preferred embodiment of the eight and other aspects of present invention, each unit dosage form of HMG-CoA reductase inhibitor comprises about 0.05 mg to about 100 mg HMG-CoA reductase inhibitor.

According to a preferred embodiment of the eight and other aspects of present invention, each unit dosage form of ezetimibe comprises about 5 mg, about 8 mg, about 9 mg, about 10 mg, about 11 mg, about 15 mg and particularly about 10 mg of ezetimibe.

According to another preferred embodiment the HMG-CoA reductase inhibitor is a statin, preferably selected from the list containing: cerivastatin, atorvastatin, simvastatin, pitavastatin, rosuvastatin, fluvastatin, lovastatin or pravastatin and is preferably atorvastatin.

According to another preferred embodiment, each unit dosage form of HMG-CoA reductase inhibitor comprises the HMG-CoA reductase inhibitor, e.g. the statin such as atorvastatin, in an effective dose for administration once per day.

According to another preferred embodiment, the unit dosage form of HMG-CoA reductase inhibitor comprises about 0,5 to about 100 mg, about 5 to about 90 mg, of about 10, 20, 40 or 80 mg HMG-CoA reductase inhibitor and preferably of statin such as atorvastatin. According to another preferred embodiment, the unit dosage form comprising the HMG-CoA inhibitor such as statin or atorvastatin is suitable for oral administration, e.g. a pill, capsule or tablet.

According to another preferred embodiment, the article of manufacture comprises sufficient unit dosage forms of HMG-CoA reductase inhibitor for a daily administration regime over a period of 14 days (i.e. two weeks), four weeks or a month.

According to another preferred embodiment, the unit dosage form comprising the antibody is a sachette, a pre-filled syringe, a pre-filled autoinjector or a cartridge for a reusable syringe or applicator, especially comprising 1 ml or 2 ml of injection solution.

A preferred embodiment of the eight aspect concerns an article of manufacture comprising one or more of the following components:
a) one or more unit dosage forms comprising the antibody or fragment thereof;
b) one or more unit dosage forms comprising the HMG-CoA reductase inhibitor;
c) one or more unit dosage forms of another lipid lowering agents such as ezetimibe;
d) the label or package insert;
e) a device for application of the antibody such as a syringe and instructions for use of the device.

A particularly preferred embodiment of the eighth aspect comprises the features a), c) and d) and another particularly preferred embodiment comprises the features a), b), c) and d) of the article of manufacture of the above-described embodiment.

In some preferred embodiments, the article of manufacture of present invention comprises one or more unit dosage forms comprising the antibody, antigen-binding fragment thereof or pharmaceutical composition as dry formulation for dissolution such as a lyophilized powder, freeze-dried powder or water free concentrate. According to another preferred embodiment, the dry formulation is comprised in a hermetically sealed container such as a vial, an ampoule or sachette. Where the antibody or fragment thereof is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In other preferred embodiments, the article of manufacture of present invention comprises one or more unit dosage forms comprising the antibody, antigen-binding fragment thereof or pharmaceutical composition as liquid formulation, e.g. injection or infusion solution. According to another preferred embodiment, the liquid formulation is comprised in a hermetically sealed container such as a vial, a sachette, a pre-filled syringe, a pre-filled autoinjector or a cartridge for a reusable syringe or applicator.

The following preparations are illustrative of the preparation of the unit dosage forms of the present invention, and not as a limitation thereof. Several dosage forms may be prepared embodying the present invention. For example, a unit dosage per vial may contain 0,5 ml, 1 ml, 2 ml, 3 ml, 4 ml, 5 ml, 6 ml, 7 ml, 8 ml, 9 ml, 10 ml, 15 ml, or 20 ml of PCSK5 antibody or a fragment thereof ranging from about 40 to about 500 mg of PCSK5 antibody. If necessary, these preparations can be adjusted to a desired concentration by adding a sterile diluent to each vial.

According to a preferred embodiment of the eighth aspect, the unit dosage form comprising the antibody comprises about 40 mg, about 50 mg, about 75 mg, at about 100 mg, about 150 mg, about 200 mg, about 250 mg, about 300 mg, about 350 mg, about 400 mg, about 450 mg, or about 500 mg of the antibody or antigen-binding fragment thereof.

The article of manufacture of present invention can also comprise one or more unit dosage forms of the HMG-CoA reductase inhibitor as tablet or capsule or other formulation for oral administration in a hermetically sealed container or blister.

In another preferred embodiment of the article of manufacture of present invention, the quantity of active ingredient is indicated on the hermetically-sealed container (e.g. blister, vial, sachette, pre-filled syringe, pre-filled autoinjector, cartridge etc.) comprising the antibody or fragment thereof or the HMG-CoA reductase inhibitor, or comprising the pharmaceutical composition, formulation or unit dosage form comprising the antibody or fragment thereof or the HMG-CoA reductase inhibitor.

It is further suitable, if the article of manufacture of present invention comprises sufficient unit dosage forms of the antibody and preferably also of the lipid lowering agent(s) for one single administration of antibody and lipid lowering agent(s) or for a two-week (i.e. 14-day) treatment with antibody and lipid lowering agent(s), or for a four week (i.e, 28-day) treatment with antibody and lipid lowering agent(s) or for a one-month treatment with antibody and lipid lowering agent(s).

According to a preferred embodiment, the article of manufacture of present invention comprises thus sufficient unit dosage forms of the antibody and of the HMG-CoA reductase inhibitor, for one single administration of antibody and HMG-CoA reductase inhibitor, or for a two-week (i.e. 14-day) treatment with antibody and HMG-CoA reductase inhibitor, or for a four week (i.e, 28-day) treatment with antibody and HMG-CoA reductase inhibitor or for a one-month treatment with antibody and HMG-CoA reductase inhibitor. The HMG-CoA reductase inhibitor in this embodiment is preferably a statin.

According to another preferred embodiment, the article of manufacture of present invention thus comprises sufficient unit dosage forms of the antibody and of ezetimibe for one single administration of antibody and and of ezetimibe or for a two-week (i.e. 14-day) treatment with antibody and and of ezetimibe or for a four week (i.e, 28-day) treatment with antibody and of ezetimibe or for a one-month treatment with antibody and and of ezetimibe.

According to a preferred embodiment, the article of manufacture of present invention comprises thus sufficient unit dosage forms of the antibody and of the HMG-CoA reductase inhibitor and of ezetimibe, for one single administration of antibody and HMG-CoA reductase inhibitor and of ezetimibe, or for a two-week (i.e. 14-day) treatment with antibody and HMG-CoA reductase inhibitor and of ezetimibe, or for a four week (i.e, 28-day) treatment with antibody and HMG-CoA reductase inhibitor and of ezetimibe or for a one-month treatment with antibody and HMG-CoA reductase inhibitor and of ezetimibe. The HMG-CoA reductase inhibitor in this embodiment is preferably a statin.

Suitable combinations of antibody or fragment thereof plus HMG-CoA inhibitor (particularly a statin) plus ezetimibe for use in the eighth aspect and particularly the above preferred embodiment of the eighth aspect comprise, but are not limited to antibody or fragment thereof plus ezetimibe plus cerivastatin, antibody or fragment thereof plus ezetimibe plus atorvastatin, antibody or fragment thereof plus ezetimibe plus simvastatin, antibody or fragment thereof plus ezetimibe plus pitavastatin, antibody or fragment thereof plus ezetimibe plus rosuvastatin, antibody or fragment thereof plus ezetimibe plus fluvastatin, antibody or fragment thereof plus ezetimibe plus lovastatin and antibody or fragment thereof plus ezetimibe plus pravastatin. Preferred combinations are antibody or fragment thereof plus ezetimibe plus atorvastatin or antibody or fragment thereof plus ezetimibe plus simvastatin.

According to another suitable embodiment of the eighth aspect of present invention, the article of manufacture comprises sufficient unit dosage forms of antibody for a bi-weekly administration regime or a four-weekly administration regime or a monthly administration regime. The article of manufacture can e.g. comprise one unit dosage form of antibody for one single administration or for a two week treatment in a bi-weekly dosage regimen or for a four week treatment with antibody in a four-weekly administration regime or for a one-month treatment with antibody in a monthly administration regime. The article of manufacture can e.g. comprise two unit dosage forms of antibody or for a four week or monthly treatment in a bi-weekly dosage regimen. Further combinations are of course also conceivable and lie within the knowledge of the skilled person.

According to another suitable embodiment of the eighth aspect of present invention, the article of manufacture comprises sufficient unit dosage forms of HMG-CoA reductase inhibitor for a daily administration regime. The article of manufacture can e.g. comprise 14 unit dosage forms of HMG-CoA reductase inhibitor for a two week treatment in a daily administration regime. The article of manufacture can also comprise 28 unit dosage forms of HMG-CoA reductase inhibitor for a four week treatment in a daily administration regime. The article of manufacture can also comprise 30 or 31 unit dosage forms of HMG-CoA reductase inhibitor for a four week or one month treatment in a daily administration regime.

Further preferred embodiments comprise sufficient unit dosage forms of antibody and HMG-CoA inhibitor for the above treatment periods and preferably also in the above administration regimes. E.g. the article of manufacture can e.g. comprise one unit dosage form of antibody for a two week treatment in a bi-weekly dosage regime together with fourteen unit dosage forms of HMG-CoA reductase inhibitor in a daily administration regime. The article of manufacture can also comprise one unit dosage form of antibody for a four week treatment with antibody in a four-weekly administration regime together with 28 unit dosage forms of HMG-CoA reductase inhibitor in a daily administration regime. The article of manufacture can also comprise one unit dosage form of antibody for a one-month treatment with antibody in a monthly administration regime together with 28, 29, 30 or 31 unit dosage forms of HMG-CoA inhibitor for a daily administration regime. The article of manufacture can e.g. also comprise two unit dosage forms of antibody for a four week treatment in a bi-weekly administration regimen together with 28 unit dosage forms of HMG-CoA inhibitor in a daily administration regime. The article of manufacture can e.g. also comprise two unit dosage forms of antibody for a one month treatment in a bi-weekly administration regime together with 28, 29, 30 or 31 unit dosage forms of HMG-CoA inhibitor in a daily administration regime. Further combinations are of course also conceivable and lie within the knowledge of the skilled person.

According to another preferred embodiment, the article of manufacture comprises sufficient unit dosage forms of the antibody and of the HMG-CoA reductase inhibitor...
(a) for one single administration of antibody and HMG-CoA reductase inhibitor, e.g. comprising an ampoule, sachette, vial, cartridge or pre-filled syringe comprising about 50mg, about 75 mg, about 100 mg, about 150 mg, about 200 mg, about 250 mg, about 300 mg, about 350 mg, about 400 mg, about 450 mg or about 500 mg antibody and preferably about 150 mg antibody, about 200 mg antibody or about 300 mg antibody, together with tablet or capsule, e.g. for oral or peroral administration comprising the HMG, CoA-inhibitor, e.g. comprising about 10mg, about 20 mg, about 40 mg or about 80 mg of the HMG CoA-inhibitor such as atorvastatin.
(b) for a two-week (i.e. 14-day) treatment with antibody and HMG-CoA reductase inhibitor, e.g. comprising an ampoule, sachette, vial, cartridge or pre-filled syringe comprising about 50mg, about 75 mg, about 100 mg, about 150 mg, about 200 mg, about 250 mg, about 300 mg, about 350 mg, about 400 mg, about 450 mg or about 500 mg antibody and preferably about 150 mg antibody, about 200 mg antibody or about 300 mg antibody; together with sufficient units comprising of HMG-CoA reductase inhibitor (e.g. tablets or capsules, e.g. for oral or peroral administration) for a 14-day treatment, e.g. 14 units for a once-a day administration regime of HMG-CoA reductase inhibitor or 28 units for a twice-a day administration regime etc, wherein the units per day of HMG CoA-inhibitor preferably comprise about 10mg, about 20 mg, about 40 mg or about 80 mg of the HMG CoA-inhibitor such as atorvastatin. In the case the antibody is to be administered in a dosage of more than 200 mg, two unit dosage forms of antibody together comprising the total dose may be preferable (e.g. two pre-filled syringes comprising about 150 mg of antibody in 1 ml of liquid formulation each for a total administration (e.g. subcutaneous injection) of about 300 mg antibody in two shots) may be preferable (or two units with about 100 mg each for a total administration of about 200 mg antibody, two units with about 175 mg for a total administration of about 350 antibody, etc...).
(c) for a four week (i.e, 28-day) treatment with antibody and HMG-CoA reductase inhibitor, e.g.
   (1) for a E2W administration regimen of the antibody with about 50 to about 200 mg antibody per two weeks: comprising two unit dosage forms (e.g. as exempliefied above) with each about 50 mg, about 100 mg, about 150 mg or about 200 mg antibody or antigen-binding fragment thereof together with 28 unit dosage forms of HMG-CoA reductase inhibitor (as exemplified above) for a daily once a day administration regime or together with 56 unit dosage forms of HMG-CoA reductase inhibitor for a daily twice-a day administration regime, preferably 28 unit dosage forms (e.g. capsules or tablets) of about 10 mg, about 20 mg, about 40 mg or about 80 mg atorvastatin
   (2) for an E4W administration regime of the antibody or fragment thereof with an administration of about 200 mg per four weeks (28 days): e.g. comprising one unit dosage form of the antibody with about 200 mg antibody (e.g. as exemplified above) together with 28 or 56, and preferably 28 unit dosage forms of HMG-CoA reductase inhibitor (e.g. as exemplified above)
   (3) for an E4W administration regime of the antibody with more than 200 mg per four weeks (28 days): comprising two unit dosage forms that together comprise the total dose of antibody (e.g. two pre-filled syringes each comprising 1 ml of liquid antibody formulation with 150 mg antibody each) or comprising one unit dosage form that comprises the total amount of antibody to be administered (e.g. a vial comprising about 300 mg antibody for dissolution or a vial, cartridge or pre-filled syringe comprising about 300 mg of the antibody in liquid formulation (i.e. about 2 ml of liquid formulation, wherein 1 ml of liquid formulation comprises about 150 mg of the antibody); together with 28 or 56, and preferably 28 unit dosage forms of HMG-CoA reductase inhibitor (e.g. as exemplified above)
   (4) for a one-month treatment with antibody and HMG-CoA reductase inhibitor:
      comprising the same numbers of unit dosage forms of antibody as exemplified under (c) for an administration once or twice per month, e.g. every first day of the month or every first Monday etc. of the month for a once a month administration, or e.g. every first and 14^{th} or 15^{th} day of the month for a twice-a-month administration regime; in addition the article of manufacture comprises 31 unit dosage forms of HMG-CoA reductase inhibitor, preferably tablets or capsules arranged in a blister containing a consecutive numbering from 1-31 for the days of the month (wherein the superfluous tablets or capsules for excess days are to be discarded).

The label or package insert of the article of manufacture preferably states for which period or duration of treatment the article of manufacture is intended (e.g. one-time treatment, treatment for two weeks or fourteen days, treatment for four weeks, treatment for one month, treatment for one year etc) and more preferably also indicates the administration regime for the antibody or fragment thereof and - if applicable - the administration regime for the HMG-CoA reductase inhibitor.

The label or packaging insert as applicable for the different aspects and embodiments of the invention, particularly in respect to the different articles of manufacture of the invention, can be any kind of data carrier suitable to be arranged within the package(s) or container(s) or on the outside of the package(s) or container(s) comprised within the article of manufacture. Preferably, the data carrier (i.e. label or, chip, bar code or leaflet or label comprising a bar code etc.) comprises information such as
(i) composition, formulation, concentration and total amount, identity of active ingredient (s) contained in the article of manufacture, i.e. of the antibody or antigen-fragments, HMG-CoA reductase inhibitor, pharmaceutical compositon, unit dosage form or formulation of present invention
(ii) number and composition of unit dosage form contained in the article of manufacture
(iii) indications, contra-indications of the antibody or antigen-fragments, pharmaceutical compositon, unit dosage form or formulation of present invention
(iv) subjects/patients or subject/patient populations indicated or contra-indicated for treatment with the antibody or antigen-fragments, pharmaceutical compositon, unit dosage form or formulation of present invention
(v) instructions for use, dosage regimens and/or administration regimes
(vi) quality information such as information about the lot/batch number of the of the antibody or antigen-fragments, pharmaceutical compositon, unit dosage form or formulation of present invention, the manufacturing or assembly site or the expiry or sell-by date
(vii) information concerning the correct storage or handling of the article of manufacture, of the device for application, or of the antibody or antigen-fragments, pharmaceutical compositon, unit dosage form or formulation of present invention,
(viii) information concerning the composition of the buffer(s), diluent(s), reagent(s), excipients, carriers, formulations of of the antibody or antigen-fragments, pharmaceutical compositon, unit dosage form or formulation of present invention,
(ix) a warning concerning possible consequences when applying unsuitable dosage or administration regimens and/or use in contraindicated indications of patient populations.

In preferred embodiments the label or packaging insert contains reference to a method of treatment or medical use according to the 1^{st}, 2^{nd}, 3^{rd}, 4^{th} or 5^{th} aspect and the embodiments of the 1^{st}, 2^{nd} 3^{rd}, 4^{th} or 5^{th} aspect as described herein.

The article of manufacture of present invention can also be a package comprising the different features of the eighth aspect and/or of the different embodiments thereof as herein described.

In a ninth aspect, present invention concerns a method for preparing or assembling an article of manufacture of present invention comprising packaging the pharmaceutical composition, of the antibody, of the liquid formulation, of the dry formulation or of one or more of the unit dosage forms of present invention in a container, optionally together with one or more of the following: a label or package insert, instructions for use, an application device.

Preferred embodiments (e.g. as concerns the container, the antibody, the label or package insert, the package, the instructions for use or the application device) can be taken from the description of the 1^{st}, 2^{nd}, 3^{rd}, 4^{th}, 5^{th}, 6^{th}, 7^{th} or 8^{th} aspect.

Several aspects of the invention can be combined with each other. For example, the method for treating a disease or condition according to the first aspect and the method for treating a disease or condition according to the 2^{nd}, 3^{rd}, 4^{th}, or 5^{th} aspect can be combined.

The skilled artisan will recognize other preferred embodiments resulting of suitable combinations of different aspects and embodiments of present invention.

### PREFERRED ANTIBODIES FOR PRACTICING THE PRESENT INVENTION

The following section describes functional and structural features of antibodies and antigen-binding fragments thereof that can be used for practicing all aspects of the present invention. Thus, expressions such as "in preferred embodiments", "in some embodiments", "in another preferred embodiment" and similar expressions should be understood as referring to embodiments of the first aspect of the present invention, the second aspect of the present invention, the third aspect of the present invention, the fourth aspect of the present invention, the fifth aspect of the present invention, the sixth aspect of the present invention, the seventh aspect of the present invention, the eighth aspect of the present invention, the ninth aspect of the present invention or to any of the preferred aspects 1-51 as described in this application.

All antibodies or antigen-binding fragments thereof suitable for practicing the present invention specifically bind hPCSK9. In preferred embodiments of any aspect of the present invention, the antibody or antigen-binding fragment thereof is a recombinant human antibody or fragment thereof. In more specific embodiments, the antibody or antigen-binding fragment thereof is a fully human monoclonal antibody or antigen-binding fragment thereof that specifically binds hPCSK9 and neutralizes PCSK9 activity.

The mAbs usable in the present invention can be full-length (e.g., an IgG1 or IgG4 antibody) or may comprise only an antigen-binding portion (e.g., a Fab, F(ab')₂ or scFv fragment), and may be modified to affect functionality, e.g., to eliminate residual effector functions (Reddy et al. (2000) J. Immunol. 164:1925-1933).

In preferred embodiments, the antibodies of present invention are characterized by one or more of the following features upon administration to a subject, preferably a human or non-human mammal and more preferably a human:
- reduction of low density lipoprotein-C (LDL-C) levels of at least about -25% to about - 40% relative to a predose level with a sustained reduction over at least a 14 day-period, wherein the sustained reduction is preferably at least -25% and more preferably at least -30% relative to a predose level, particularly if administered in a dose of about 40 to about 60 mg, preferably about 45 to about 55 mg and more preferably about 50 mg in a biweekly administration regime (every other week, E2W) ,
- reduction of low density lipoprotein-C (LDL-C) of at least about -50% to about -65% relative to a predose level with a sustained reduction over at least a 14 day-period, wherein the sustained reduction is preferably at least -40% and more preferably at least -45% relative to a predose level, particularly if administered in a dose of about 100 mg E2W,
- reduction of low-density lipoprotein-C (LDL-C) of at least about -60 % to at least about -75% [e.g. at least about -60 %, at least about -65%, at least about -70 or at least about -75%] relative to a predose level with a sustained reduction over at least a 14 day-period, wherein the sustained reduction is preferably at least -55% and more preferably at least -60% relative to a predose level, particularly when administered in a dose of about 150 mg E2W,
- reduction of low density lipoprotein-C (LDL-C) of at least about 40% to about 75% relative to a predose level with a sustained reduction over at least a 28 day period, wherein the sustained reduction is preferably at least -35% and more preferably at least -40% relative to a predose level, particularly when administered in a dose of about 200 mg E4W,
- reduction of low density lipoprotein-C (LDL-C) of at least about -50 % to about -75% relative to a predose level with a sustained reduction over at least a 28 day-period, wherein the sustained reduction is preferably at least -40% and more preferably at least -45% relative to a predose level, particularly when administered in a dose of about 300 mg E4W,
- increase of serum HDL cholesterol levels of at least 2%, at least 2.5%, at least, 3%, at least 3.5%, at least 4%, at least 4.5%, at least 5% or at least 5.5% relative to a predose level, particularly when admimistered in a dose of about 150 mg E2W,
- little or no detectable effect on troponin levels,
- Increase of one or more of: Total-Cholesterol levels, ApoB levels, non HDL-C levels, Apo-B/ApoA-1 ratio,

The antibodies according to present invention exhibit the above properties preferably if administered in combination with an HMG-CoA reductase inhibitor treatment. Preferred embodiments of HMG-CoA reductase inhibitors to be used in conjunction with the antibody of the invention and dosage and administration regimes thereof can be found throughout the specification, particularly as described in the aspects related to medical uses and methods of treatment.

According to another preferred embodiment of the antibodies and antigen-binding fragments thereof of present invention, the antibody or antigen binding fragment thereof has one or more of the following characteristics:
- The antibody or the antigen-binding fragment comprises the heavy and light chain CDRs of a HCVR/LCVR amino acid sequence pair as shown in SEQ ID NOs: 90/92.
- The antibody or antigen-binding fragment thereof comprises a HCVR/LCVR amino acid sequence pair as shown in SEQ ID NOs: 90/92.
- The antibody or antigen-binding fragment thereof competes for binding to hPCSK9 with an antibody or antigen-binding fragment comprising a HCVR/LCVR amino acid sequence pair as shown in SEQ ID NOs: 90/92.

According to another preferred embodiment of the antibodies and antigen-binding fragments thereof of present invention, the antibody or antigen binding fragment thereof has one or more of the following characteristics:
- overcomes statin resistance in mammals, especially in rodents such as hamster
- increase in LDLR expression in mammals, particularly in rodents such as hamster
- decreases serum LDL-C in rodents such as hamster
- synergistic decrease of LDL-C in conjunction with HMG-CoA reductase inhibitor administration, particularly in rodents such as hamster, wherein the HMG-CoA reductase inhibitor is preferably Atorvastatin.

In preferred embodiments, the antibody or the antigen-binding fragment thereof is characterized by one or more of the following:
(i) capable of reducing serum total cholesterol at least about 25 to about 35% and sustaining the reduction over at least a 24 day period relative to a predose level, preferably the reduction in serum total cholesterol is at least about 30-40%;
(ii) capable of reducing serum LDL cholesterol at least about 65-80% and sustaining the reduction over at least a 24 day period relative to a predose level;
(iii) capable of reducing serum triglyceride at least about 25-40% relative to predose level;
(iv) achieves one or more of (i)-(iii) without reducing serum HDL cholesterol or reducing serum HDL cholesterol no more than 5% relative to predose level;
(v) achieves one or more of (i)-(iii) with little or no measurable effect on liver function, as determined by ALT and AST measurements.

In preferred embodiments, the antibody or the antigen-binding fragment thereof is characterized by one or more of the following:
(i) capable of reducing serum LDL cholesterol at least about 40-70% and sustaining the reduction over at least a 60 or 90 day period relative to a predose level;
(ii) capable of reducing serum triglyceride at least about 25-40% relative to predose level;
(iii) does not reduce serum HDL cholesterol or reduces serum HDL cholesterol no more than 5% relative to predose level.

In one embodiment, the antibody or the antigen-binding fragment thereof is characterized as binding an epitope comprising amino acid residue 238 of hPCSK9 (SEQ ID NO:755). In a more specific embodiment, the antibody or antigen-binding fragment binds an epitope comprising one or more of amino acid residues at positions 238, 153, 159 and 343 of hPCSK9 (SEQ ID NO:755). In a more specific embodiment, the antibody or fragment thereof is characterized as binding an epitope which does not comprise an amino acid residue at positions 192, 194, 197 and/or 237 of SEQ ID NO:755.

In one embodiment, the antibody or the antigen-binding fragment thereof is characterized as binding an epitope comprising amino acid residue 366 of hPCSK9 (SEQ ID NO:755). In a more specific embodiment, the antibody or antigen-binding fragment binds an epitope comprising one or more of amino acid residues at positions 147, 366 and 380 of hPCSK9 (SEQ ID NO:755). In a more specific embodiment, the antibody or antigen-binding fragment of an antibody is characterized as binding an epitope which does not comprise an amino acid residue at position 215 or 238 of SEQ ID NO:755.

In one embodiment, the antibody or the antigen-binding fragment thereof comprises a heavy chain variable region (HCVR) selected from the group consisting of SEQ ID NO:2, 18, 22, 26, 42, 46, 50, 66, 70, 74, 90, 94, 98, 114, 118, 122, 138, 142, 146, 162, 166, 170, 186, 190, 194, 210, 214, 218, 234, 238, 242, 258, 262, 266, 282, 286, 290, 306, 310, 314, 330, 334, 338, 354, 358, 362, 378, 382, 386, 402, 406, 410, 426, 430, 434, 450, 454, 458, 474, 478, 482, 498, 502, 506, 522, 526, 530, 546, 550, 554, 570, 574, 578, 594, 598, 602, 618, 622, 626, 642, 646, 650, 666, 670, 674, 690, 694, 698, 714, 718, 722, 738 and 742, or a substantially similar sequence thereof having at least 90%, at least 95%, at least 98% or at least 99% sequence identity. In one embodiment, the HCVR comprises an amino acid sequence selected from the group consisting of SEQ ID NO:50, 66, 70, 74, 90, 94, 122, 138, 142, 218, 234, 238, 242, 258, 262, 314, 330 and 334. In a more specific embodiment, the HCVR comprises SEQ ID NO:90 or 218.

In one embodiment, the antibody or the antigen-binding fragment thereof further comprises a light chain variable region (LCVR) selected from the group consisting of SEQ ID NO:10, 20, 24, 34, 44, 48, 58, 68, 72, 82, 92, 96, 106, 116, 120, 130, 140, 144, 154, 164, 168, 178, 188, 192, 202, 212, 216, 226, 236, 240, 250, 260, 264, 274, 284, 288, 298, 308, 312, 322, 332, 336, 346, 356, 360, 370, 380, 384, 394, 404, 408, 418, 428, 432, 442, 452, 456, 466, 476, 480, 490, 500, 504, 514, 524, 528, 538, 548, 552, 562, 572, 576, 586, 596, 600, 610, 620, 624, 634, 644, 648, 658, 668, 672, 682, 692, 696, 706, 716, 720, 730, 740 and 744, or a substantially similar sequence thereof having at least 90%, at least 95%, at least 98% or at least 99% sequence identity. In one embodiment, the LCVR comprises an amino acid sequence selected from the group consisting of SEQ ID NO: 58, 68, 72, 82, 92, 96, 130, 140, 144, 226, 236, 240, 250, 260, 264, 322, 332 and 336. In a more specific embodiment, the LCVR comprises SEQ ID NO:92 or 226.

In specific embodiments, the antibody or the antigen-binding fragment thereof comprises a HCVR and LCVR (HCVR/LCVR) sequence pair selected from the group consisting of SEQ ID NO: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In one embodiment, the HCVR and LCVR sequence pair comprises one of SEQ ID NO: 50/58, 66/68, 70/72, 74/82, 90/92, 94/96, 122/130, 138/140, 142/144, 218/226, 234/236, 238/240, 242/250, 258/260, 262/264, 314/322, 330/332 and 334/336. In preferred embodiments, the antibody or antigen-binding fragment thereof comprises an HCVR amino acid sequence as shown in SEQ ID NO: 90 and an LCVR amino acid sequence as shown in SEQ ID NO: 92. In another preferred embodiment, the antibody or antigen-binding fragment thereof comprises an HCVR amino acid sequence as shown in SEQ ID NO: 218 and an LCVR amino acid sequence as shown in SEQ ID NO: 226.

In preferred embodiments, the antibody or the antigen-binding fragment thereof comprises a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728, or a substantially similar sequence thereof having at least 90%, at least 95%, at least 98% or at least 99% sequence identity; and a light chain CDR3 (LCDR3) domain selected from the group consisting of SEQ ID N0:16, 40, 64, 88, 112, 136, 160, 184, 208, 232, 256, 280, 304, 328, 352, 376, 400, 424, 448, 472, 496, 520, 544, 568, 592, 616, 640, 664, 688, 712 and 736, or substantially similar sequences thereof having at least 90%, at least 95%, at least 98% or at least 99% sequence identity. In one embodiment, the HCDR3/LCDR3 sequence pair is selected from the group consisting of SEQ ID NO:56/64, 80/88, 128/136, 224/232, 248/256 and 320/328. In more preferred embodiments, the antibody or the antigen-binding fragment thereof comprises a HCDR3 domain as shown in SEQ ID NO: 80 and a LCDR3 domain as shown in SEQ ID NO: 88. In another preferred embodiment, the antibody or the antigen-binding fragment thereof comprises a HCDR3 domain as shown in SEQ ID NO: 224 and a LCDR3 domain as shown in SEQ ID NO: 232.

In a further embodiment, the antibody or the antigen-binding fragment thereof further comprises a heavy chain CDR1 (HCDR1) domain selected from the group consisting of SEQ ID NO:4, 28, 52, 76, 100, 124, 148, 172, 196, 220, 244, 268, 292, 316, 340, 364, 388, 412, 436, 460, 484, 508, 532, 556, 580, 604, 628, 652, 676, 700 and 724, or a substantially similar sequence thereof having at least 90%, at least 95%, at least 98% or at least 99% sequence identity; a heavy chain CDR2 (HCDR2) domain selected from the group consisting of SEQ ID NO:6, 30, 54, 78, 102, 126, 150, 174, 198, 222, 246, 270, 294, 318, 342, 366, 390, 414, 438, 462, 486, 510, 534, 558, 582, 606, 630, 654, 678, 702 and 726, or a substantially similar sequence thereof having at least 90%, at least 95%, at least 98% or at least 99% sequence identity; a light chain CDR1 (LCDR1) domain selected from the group consisting of SEQ ID NO:12, 36, 60, 84, 108, 132, 156, 180, 204, 228, 252, 276, 300, 324, 348, 372, 396, 420, 444, 468, 492, 516, 540, 564, 588, 612, 636, 660, 684, 708 and 732, or a substantially similar sequence thereof having at least 90%, at least 95%, at least 98% or at least 99% sequence identity; and a light chain CDR2 (LCDR2) domain selected from the group consisting of SEQ ID NO:14, 38, 62, 86, 110, 134, 158, 182, 206, 230, 254, 278, 302, 326, 350, 374, 398, 422, 446, 470, 494, 518, 542, 566, 590, 614, 638, 662, 686, 710 and 734, or a substantially similar sequence thereof having at least 90%, at least 95%, at least 98% or at least 99% sequence identity. In one embodiment, the heavy and light chain CDR sequences comprise a sequence selected from the group consisting of SEQ ID NO:52, 54, 56, 60, 62, 64; 76, 78, 80, 84, 86, 88; 124, 126, 128, 132, 134, 136; 220, 222, 224, 228, 230, 232; 244, 246, 248, 252, 254, 256; and 316, 318, 320, 324, 326, 328. In more specific embodiments, the CDR sequences comprise SEQ ID NO: 76, 78, 80, 84, 86, 88; or 220, 222, 224, 228, 230, 232. In preferred embodiments, the antibody or antigen-binding fragment thereof comprises heavy and light chain CDR amino acid sequences as shown in SEQ ID NOs: 76, 78, 80, 84, 86 and 88. In another preferred embodiment, the antibody or antigen-binding fragment thereof comprises heavy and light chain CDR amino acid sequences as shown in SEQ ID NOs: 220, 222, 224, 228, 230 and 232.

In a related embodiment, the antibody or antigen-binding fragment thereof comprises heavy and light chain CDR domains contained within heavy and light chain sequence pairs selected from the group consisting of SEQ ID NO: 2/10, 18/20, 22/24, 26/34, 42/44, 46/48, 50/58, 66/68, 70/72, 74/82, 90/92, 94/96, 98/106, 114/116, 118/120, 122/130, 138/140, 142/144, 146/154, 162/164, 166/168, 170/178, 186/188, 190/192, 194/202, 210/212, 214/216, 218/226, 234/236, 238/240, 242/250, 258/260, 262/264, 266/274, 282/284, 286/288, 290/298, 306/308, 310/312, 314/322, 330/332, 334/336, 338/346, 354/356, 358/360, 362/370, 378/380, 382/384, 386/394, 402/404, 406/408, 410/418, 426/428, 430/432, 434/442, 450/452, 454/456, 458/466, 474/476, 478/480, 482/490, 498/500, 502/504, 506/514, 522/524, 526/528, 530/538, 546/548, 550/552, 554/562, 570/572, 574/576, 578/586, 594/596, 598/600, 602/610, 618/620, 622/624, 626/634, 642/644, 646/648, 650/658, 666/668, 670/672, 674/682, 690/692, 694/696, 698/706, 714/716, 718/720, 722/730, 738/740 and 742/744. In one embodiment, the CDR sequences are contained within HCVR and LCVR selected from the amino acid sequence pairs of SEQ ID NO: 50/58, 66/68, 70/72, 74/82, 90/92, 94/96, 122/130, 138/140, 142/144, 218/226, 234/236, 238/240, 242/250, 258/260, 262/264, 314/322, 330/332 and 334/336. In more specific embodiments, the CDR sequences are comprised within HCVR/LCVR sequences selected from SEQ ID NO: 90/92 or 218/226. In preferred embodiments, the antibody or the antigen-binding fragment thereof comprises the heavy and light chain CDRs of an HCVR/LCVR amino acid sequence pair as shown in SEQ ID NOs: 90/92. In another preferred embodiment, the antibody or the antigen-binding fragment thereof comprises the heavy and light chain CDRs of an HCVR/LCVR amino acid sequence pair as shown in SEQ ID NOs: 218/226.

In one specific embodiment, the antibody or the antigen-binding fragment thereof comprises the heavy chain variable region (HCVR), of SEQ ID NO:90 or a substantially similar sequence thereof having at least 90%, at least 95%, at least 98% or at least 99% sequence identity..

In one specific embodiment, the antibody or the antigen-binding fragment thereof further comprises the light chain variable region (LCVR) of SEQ ld NO 92 or a substantially similar sequence thereof having at least 90%, at least 95%, at least 98% or at least 99% sequence identity.

In specific embodiments, the antibody or the antigen-binding fragment thereof comprises HCVR amino acid sequence as shown in SEQ ID NO: 90 and an LCVR amino acid sequence as shown in SEQ ID NO: 92.

In specific embodiments, the antibody or the antigen-binding fragment thereof comprises a heavy chain CDR3 (HCDR3) domain of SEQ ID NO: 80 or a substantially similar sequence thereof having at least 90%, at least 95%, at least 98% or at least 99% sequence identity; and/or a light chain CDR3 (LCDR3) domain of SEQ ID NO: 88, or substantially similar sequences thereof having at least 90%, at least 95%, at least 98% or at least 99% sequence identity. In one embodiment, the HCDR3/LCDR3 sequence pair is SEQ ID NO:80/88 . In more preferred embodiments, the antibody or the antigen-binding fragment thereof comprises a HCDR3 domain as shown in SEQ ID NO: 80 and a LCDR3 domain as shown in SEQ ID NO: 88.

In a further specific embodiment, the antibody or the antigen-binding fragment thereof further comprises the heavy chain CDR1 (HCDR1) domain of SEQ ID NO: 76, or a substantially similar sequence thereof having at least 90%, at least 95%, at least 98% or at least 99% sequence identity; and/or the heavy chain CDR2 (HCDR2) domain of SEQ ID NO: 78 or a substantially similar sequence thereof having at least 90%, at least 95%, at least 98% or at least 99% sequence identity; and/or a light chain CDR1 (LCDR1) domain of SEQ ID NO: 84or a substantially similar sequence thereof having at least 90%, at least 95%, at least 98% or at least 99% sequence identity; and/or a light chain CDR2 (LCDR2) domain of SEQ ID NO: 86, or a substantially similar sequence thereof having at least 90%, at least 95%, at least 98% or at least 99% sequence identity. In one embodiment, the heavy and light chain CDR sequences comprise a sequence selected from the group consisting of SEQ ID NO: 76, 78, 80, 84, 86, 88. In preferred embodiments, the antibody or antigen-binding fragment thereof comprises heavy and light chain CDR amino acid sequences as shown in SEQ ID NOs: 76, 78, 80, 84, 86 and 88.

In another specific embodiment, the antibody or antigen-binding fragment thereof comprises heavy and light chain CDR domains contained within the heavy and light chain sequence pair of SEQ ID NO:, 90/92.

A particularly preferred embodiment concerns an antibody comprising HCVR/LCVR sequences SEQ ID Nos: 90/92 and/or CDR sequences SEQ ID Nos: 76, 78, 80 and/or CDR sequences SEQ ID NO:s 84, 86, 88. Another particularly preferred embodiment concerns an antibody comprising the HCVR/LCVR sequences SEQ ID Nos: 90/92 and the CDR sequences SEQ ID Nos: 76, 78, 80 and the CDR sequences SEQ ID NO:s 84, 86, 88 ("316P").

In one embodiment, the antibody or antigen-binding fragment thereof comprises an HCVR encoded by a nucleic acid sequence selected from the group consisting of SEQ ID NO: 1, 17, 21, 25, 41, 45, 49, 65, 69, 73, 89, 93, 97, 113, 117, 121, 137, 141, 145, 161, 165, 169, 185, 189, 193, 209, 213, 217, 233, 237, 241, 257, 261, 265, 281, 285, 289, 305, 309, 313, 329, 333, 337, 353, 357, 361, 377, 381, 385, 401, 405, 409, 425, 429, 433, 449, 453, 457, 473, 477, 481, 497, 501, 505, 521, 525, 529, 545, 549, 553, 569, 573, 577, 593, 597, 601, 617, 621, 625, 641, 645, 649, 665, 669, 673, 689, 693, 697, 713, 717, 721, 737 and 741, or a substantially identical sequence having at least 90%, at least 95%, at least 98%, or at least 99% sequence identity thereof. In one embodiment, the HCVR is encoded by a nucleic acid sequence selected from the group consisting of SEQ ID NO: 49, 65, 69, 73, 89, 93, 121, 137, 141, 217, 233, 237, 241, 257, 261, 313, 329 and 333. In more specific embodiments, the HCVR is encoded by a nucleic acid sequence selected from the group consisting of SEQ ID NO: 89 and 217.

In one embodiment, the antibody or fragment thereof further comprises an LCVR encoded by a nucleic acid sequence selected from the group consisting of SEQ ID NO: 9, 19, 23, 33, 43, 47, 57, 67, 71, 81, 91, 95, 105, 115, 119, 129, 139, 143, 153, 163, 167, 177, 187, 191, 201, 211, 215, 225, 235, 239, 249, 259, 263, 273, 283, 287, 297, 307, 311, 321, 331, 335, 345, 355, 359, 369, 379, 383, 393, 403, 407, 417, 427, 431, 441, 451, 455, 465, 475, 479, 489, 499, 503, 513, 523, 527, 537, 547, 551, 561, 571, 575, 585, 595, 599, 609, 619, 623, 633, 643, 647, 657, 667, 671, 681, 691, 695, 705, 715, 719, 729, 739 and 743, or a substantially identical sequence having at least 90%, at least 95%, at least 98%, or at least 99% sequence identity thereof. In one embodiment, the LCVR is encoded by a nucleic acid sequence selected from the group consisting of SEQ ID NO: 57, 67, 71, 81, 91, 95, 129, 139, 143, 225, 235, 239, 249, 259, 263, 321, 331 and 335. In more specific embodiments, the LCVR is encoded by a nucleic acid sequence selected from the group consisting of SEQ ID NO: 91 and 225.

In one embodiment, the antibody or antigen-binding fragment thereof comprises an HCDR3 domain encoded by a nucleotide sequence selected from the group consisting of SEQ ID NO:7, 31, 55, 79, 103, 127, 151, 175, 199, 223, 247, 271, 295, 319, 343, 367, 391, 415, 439, 463, 487, 511, 535, 559, 583, 607, 631, 655, 679, 703 and 727, or a substantially identical sequence having at least 90%, at least 95%, at least 98%, or at least 99% sequence identity thereof; and a LCDR3 domain encoded by a nucleotide sequence selected from the group consisting of SEQ ID NO: 15, 39, 63, 87, 111, 135, 159, 183, 207, 231, 255, 279, 303, 327, 351, 375, 399, 423, 447, 471, 495, 519, 543, 567, 591, 615, 639, 663, 687, 711 and 735, or a substantially identical sequence having at least 90%, at least 95%, at least 98%, or at least 99% sequence identity thereof. In one embodiment, the HCDR3 and LCDR3 comprise a sequence pair encoded by the nucleic acid sequence of SEQ ID NO: 55/63, 79/87, 127/135, 223/231, 247/255 and 319/327, respectively. In more specific embodiments, the HCDR3 and LCDR3 comprise a sequence pair encoded by the nucleic acid sequence of SEQ ID NO: 79/87 and 223/231.

In a further embodiment, the antibody or antigen-binding fragment thereof further comprises: an HCDR1 domain encoded by a nucleotide sequence selected from the group consisting of SEQ ID NO: 3, 27, 51, 75, 99, 123, 147, 171, 195, 219, 243, 267, 291, 315, 339, 363, 387, 411, 435, 459, 483, 507, 531, 555, 579, 603, 627, 651, 675, 699 and 723, or a substantially identical sequence having at least 90%, at least 95%, at least 98%, or at least 99% sequence identity thereof; an HCDR2 domain encoded by a nucleotide sequence selected from the group consisting of SEQ ID NO:5, 29, 53, 77, 101, 125, 149, 173, 197, 221, 245, 269, 293, 317, 341, 365, 389, 413, 437, 461, 485, 509, 533, 557, 581, 605, 629, 653, 677, 701 and 725, or a substantially identical sequence having at least 90%, at least 95%, at least 98%, or at least 99% sequence identity thereof; an LCDR1 domain encoded by a nucleotide sequence selected from the group consisting of SEQ ID NO: 11, 35, 59, 83, 107, 131, 155, 179, 203, 227, 251, 275, 299, 323, 347, 371, 395, 419, 443, 467, 491, 515, 539, 563, 587, 611, 635, 659, 683, 707 and 731, or a substantially identical sequence having at least 90%, at least 95%, at least 98%, or at least 99% sequence identity thereof; and an LCDR2 domain encoded by a nucleotide sequence selected from the group consisting of SEQ ID NO: 13, 37, 61, 85, 109, 133, 157, 181, 205, 229, 253, 277, 301, 325, 349, 373, 397, 421, 445, 469, 493, 517, 541, 565, 589, 613, 637, 661, 685, 709 and 733, or a substantially identical sequence having at least 90%, at least 95%, at least 98%, or at least 99% sequence identity thereof. In one embodiment, the heavy and light chain CDR sequences are encoded by the nucleic acid sequences of SEQ ID NO: 51, 53, 55, 59, 61, 63; 75, 77, 79, 83, 85, 87; 123, 125, 127, 131, 133, 135; 219, 221, 223, 227, 229, 231; 243, 245, 247, 251, 253, 255; and 315, 317, 319, 323, 325, 327. In more specific embodiments, the heavy and light chain CDR sequences are encoded by the nucleic acid sequences of SEQ ID NO: 75, 77, 79, 83, 85, 87; and 219, 221, 223, 227, 229, 231.

In a further embodiment, the antibody or antigen-binding fragment thereof comprises an HCDR3 and an LCDR3, wherein HCDR3 comprises an amino acid sequence of the formula X¹ - X² - X³ - X⁴ - X⁵ - X⁶ - X⁷ - X⁸ - X⁹ - X¹⁰ - X¹¹ - X¹² - X¹³ - X¹⁴ - X¹⁵ - X¹⁶ - X¹⁷ - X¹⁸ - X¹⁹ - X²⁰ (SEQ ID NO:747), wherein X¹ is Ala, X² is Arg or Lys, X³ is Asp, X⁴ is Ser or Ile, X⁵ is Asn or Val, X⁶ is Leu or Trp, X⁷ is Gly or Met, X⁸ is Asn or Val, X⁹ is Phe or Tyr, X¹⁰ is Asp, X¹¹ is Leu or Met, X¹² is Asp or absent, X¹³ is Tyr or absent, X¹⁴ is Tyr or absent, X¹⁵ is Tyr or absent, X¹⁶ is Tyr or absent, X¹⁷ is Gly or absent, X¹⁸ is Met or absent, X¹⁹ is Asp or absent, and X²⁰ is Val or absent; and LCDR3 comprises an amino acid sequence of the formula X¹ - X² - X³ - X⁴ - X⁵ - X⁶ - X⁷ - X⁸ - X⁹ (SEQ ID NO:750), wherein X¹ is Gln or Met, X² is Gln, X³ is Tyr or Thr, X⁴ is Tyr or Leu, X⁵ is Thr or Gln, X⁶ is Thr, X⁷ is Pro, X⁸ is Tyr or Leu, and X⁹ is Thr.

In a further embodiment, the antibody or antigen-binding fragment thereof further comprises an HCDR1 sequence of the formula X¹ - X² - X³ - X⁴ - X⁵ - X⁶ - X⁷ - X⁸ (SEQ ID NO:745), wherein X¹ is Gly, X² is Phe, X³ is Thr, X⁴ is Phe, X⁵ is Ser or Asn, X⁶ is Ser or Asn, X⁷ is Tyr or His, and X⁸ is Ala or Trp; a HCDR2 sequence of the formula X¹ - X² - X³ - X⁴ - X⁵ - X⁶ - X7 - X⁸ (SEQ ID NO:746), wherein X¹ is Ile, X² is Ser or Asn, X³ is Gly or Gln, X⁴ is Asp or Ser, X⁵ is Gly, X⁶ is Ser or Gly, X⁷ is Thr or Glu, and X⁸ is Thr or Lys; a LCDR1 sequence of the formula X¹ - X² - X³ - X⁴ - X⁵ - X⁶⁻ X⁷ - X⁸ - X⁹ - X¹⁰ - X¹¹ - X¹² (SEQ ID NO:748) wherein X¹ is Gln, X² is Ser, X³ is Val or Leu, X⁴ is Leu, X⁵ is His or Tyr, X⁶ is Arg or Ser, X⁷ is Ser or Asn, X⁸ is Asn or Gly, X⁹ is Asn, X¹⁰ is Arg or Asn, X¹¹ is Asn or Tyr, and X¹² is Phe or absent; an LCDR2 sequence of the formula X¹ - X² - X³ (SEQ ID NO:749) wherein X¹ is Trp or Leu, X² is Ala or Gly, and X³ is Ser.

In a further embodiment, the antibody or antigen-binding fragment thereof is a human anti-PCSK9 antibody or antigen-binding fragment thereof comprising a heavy chain variable region (HCVR) encoded by nucleotide sequence segments derived from V_{H}, D_{H} and J_{H} germline sequences, and a light chain variable region (LCVR) encoded by nucleotide sequence segments derived from V_{K} and J_{K} germline sequences, wherein the germline sequences are (a) V_{H} gene segment 3-23, D_{H} gene segment 7-27, J_{H} gene segment 2, V_{K} gene segment 4-1 and J_{K} gene segment 2; or (b) V_{H} gene segment 3-7, D_{H} gene segment 2-8, J_{H} gene segment 6, V_{K} gene segment 2-28 and J_{K} gene segment 4.

In preferred embodiments, the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88 or as shown in SEQ ID NOs: 220, 222, 224, 228, 230 and 232.

In preferred embodiments, the antibody or antigen-binding fragment thereof competes for binding to hPCSK9 with an antibody comprising heavy and light chain CDR amino acid sequences as shown in SEQ ID NOs: 76, 78, 80, 84, 86, and 88 or as shown in SEQ ID NOs: 220, 222, 224, 228, 230 and 232.

The invention encompasses anti-PCSK9 antibodies having a modified glycosylation pattern. In some applications, modification to remove undesirable glycosylation sites may be useful, or e.g., removal of a fucose moiety to increase antibody dependent cellular cytotoxicity (ADCC) function (see Shield et al. (2002) JBC 277:26733). In other applications, modification of galactosylation can be made in order to modify complement dependent cytotoxicity (CDC).

Some preferred sequences related to preferred antibodies for practicing present invention:
SEQ ID NO: 76: Gly Phe Thr Phe Asn Asn Tyr Ala
SEQ ID NO:78: Ile Ser Gly Ser Gly Gly Thr Thr
SEQ ID NO: 80: Ala Lys Asp Ser Asn Trp Gly Asn Phe Asp Leu
SEQ ID NO: 84: Gln Ser Val Leu Tyr Arg Ser Asn Asn Arg Asn Phe
SEQ ID NO: 86: Trp Ala Ser
SEQ ID NO: 88: Gln Gln Tyr Tyr Thr Thr Pro Tyr Thr
SEQ ID NO: 90: 

1               5                   10                  15

            20                  25                  30

        35                  40                  45

    50                  55                  60

65                  70                  75                  80

 
                85                  90                  95

            100                 105                 110

        115
SEQ ID NO:92: 

1               5                   10                  15

            20                  25                  30

        35                  40                  45

    50                  55                  60

65                  70                  75                  80

                85                  90                  95

            100                 105                 110

SEQ ID NO: 755 (hPCSK9):


1               5                   10                  15

            20                  25                  30

        35                  40                  45

    50                  55                  60

65                  70                  75                  80

                85                  90                  95

            100                 105                 110

        115                 120                 125

    130                 135                 140

145                 150                 155                 160

                165                 170                 175

            180                 185                 190

        195                 200                 205


    210                 215                 220

225                 230                 235                 240

                245                 250                 255

            260                 265                 270

        275                 280                 285

    290                 295                 300

305                 310                 315                 320

                325                 330                 335

            340                 345                 350

        355                 360                 365

    370                 375                 380

385                 390                 395                 400

                405                 410                 415

            420                 425                 430

        435                 440                 445

    450                 455                 460

465                 470                 475                 480

                485                 490                 495

            500                 505                 510

        515                 520                 525

    530                 535                 540

545                 550                 555                 560

                565                 570                 575

            580                 585                 590

        595                 600                 605

    610                 615                 620

625                 630                 635                 640


                645                 650                 655

            660                 665                 670

        675                 680                 685

    690

### Preparation of Human Antibodies

Methods for generating human antibodies in transgenic mice are known (see for example, US 6,596,541, Regeneron Pharmaceuticals, VELOCIMMUNE™). The VELOCIMMUNE™ technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody. In specific embodiment, the cell is a CHO cell.

Antibodies may be therapeutically useful in blocking a ligand-receptor interaction or inhibiting receptor component interaction, rather than by killing cells through fixation of complement and participation in complement-dependent cytotoxicity (CDC), or killing cells through antibody-dependent cell-mediated cytotoxicity (ADCC). The constant region of an antibody is thus important in the ability of an antibody to fix complement and mediate cell-dependent cytotoxicity. Thus, the isotype of an antibody may be selected on the basis of whether it is desirable for the antibody to mediate cytotoxicity.

Human antibodies can exist in two forms that are associated with hinge heterogeneity. In one form, an antibody molecule comprises a stable four-chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, CH2 or CH3 region which may be desirable, for example, in production, to improve the yield of the desired antibody form.

Generally, a VELOCIMMUNE™ mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. As described below, the antibodies are characterized and selected for desirable characteristics, including affinity, selectivity, epitope, etc. The mouse constant regions are replaced with a desired human constant region to generate the fully human antibody of the invention, for example wild-type or modified IgG1 or IgG4 (for example, SEQ ID NO:751, 752, 753). While the constant region selected may vary according to specific use, high affinity antigen-binding and target specificity characteristics reside in the variable region.

### Epitope Mapping and Related Technologies

To screen for antibodies that bind to a particular epitope (e.g., those which block binding of IgE to its high affinity receptor), a routine cross-blocking assay such as that described Antibodies, Harlow and Lane (Cold Spring Harbor Press, Cold Spring Harb., NY) can be performed. Other methods include alanine scanning mutants, peptide blots (Reineke (2004) Methods Mol Biol 248:443-63) (herein specifically incorporated by reference in its entirety), or peptide cleavage analysis. In addition, methods such as epitope excision, epitope extraction and chemical modification of antigens can be employed (Tomer (2000) Protein Science 9: 487-496) (herein specifically incorporated by reference in its entirety).

The term "epitope" refers to a site on an antigen to which B and/or T cells respond. B-cell epitopes can be formed both from contiguous amino acids or noncontiguous amino acids juxtaposed by tertiary folding of a protein. Epitopes formed from contiguous amino acids are typically retained on exposure to denaturing solvents, whereas epitopes formed by tertiary folding are typically lost on treatment with denaturing solvents. An epitope typically includes at least 3, and more usually, at least 5 or 8-10 amino acids in a unique spatial conformation.

Modification-Assisted Profiling (MAP), also known as Antigen Structure-based Antibody Profiling (ASAP) is a method that categorizes large numbers of monoclonal antibodies (mAbs) directed against the same antigen according to the similarities of the binding profile of each antibody to chemically or enzymatically modified antigen surfaces (US 2004/0101920, herein specifically incorporated by reference in its entirety). Each category may reflect a unique epitope either distinctly different from or partially overlapping with epitope represented by another category. This technology allows rapid filtering of genetically identical mAbs, such that characterization can be focused on genetically distinct mAbs. When applied to hybridoma screening, MAP may facilitate identification of rare hybridoma clones that produce mAbs having the desired characteristics. MAP may be used to sort the anti-PCSK9 mAbs of the invention into groups of mAbs binding different epitopes.

In various embodiments, the anti-hPCSK9 antibody or antigen-binding fragment of an antibody binds an epitope within the catalytic domain, which is about 153 to 425 of SEQ ID NO:755); more specifically, an epitope from about 153 to about 250 or from about 250 to about 425; more specifically, the antibody or antibody fragment of the invention binds an epitope within the fragment from about 153 to about 208, from about 200 to about 260, from about 250 to about 300, from about 275 to about 325, from about 300 to about 360, from about 350 to about 400, and/or from about 375 to about 425.

In various embodiments, the anti-hPCSK9 antibody or antigen-binding fragment of an antibody binds an epitope within the propeptide domain (residues 31 to 152 of SEQ ID NO:755); more specifically, an epitope from about residue 31 to about residue 90 or from about residue 90 to about residue 152; more specifically, the antibody or antibody fragment of the invention binds an epitope within the fragment from about residue 31 to about residue 60, from about residue 60 to about residue 90, from about residue 85 to about residue 110, from about residue 100 to about residue 130, from about residue 125 to about residue 150, from about residue 135 to about residue 152, and/or from about residue 140 to about residue 152.

In some embodiments, the anti-hPCSK9 antibody or antigen-binding fragment of an antibody binds an epitope within the C-terminal domain, (residues 426 to 692 of SEQ ID NO:755); more specifically, an epitope from about residue 426 to about residue 570 or from about residue 570 to about residue 692; more specifically, the antibody or antibody fragment of the invention binds an epitope within the fragment from about residue 450 to about residue 500, from about residue 500 to about residue 550, from about residue 550 to about residue 600, and/or from about residue 600 to about residue 692.

In some embodiments, the antibody or antibody fragment binds an epitope which includes more than one of the enumerated epitopes within the catalytic, propeptide or C-terminal domain, and/or within two or three different domains (for example, epitopes within the catalytic and C-terminal domains, or within the propeptide and catalytic domains, or within the propeptide, catalytic and C-terminal domains.

In some embodiments, the antibody or antigen-binding fragment binds an epitope on hPCSK9 comprising amino acid residue 238 of hPCSK9 (SEQ ID NO:755). Experimental results (see US 2010/0166768) showed that when D238 was mutated, the K_{D} of mAb 316P exhibited >400-fold reduction in binding affinity (∼1 x10⁻⁹ M to ∼410 x10-9 M) and T_{1/2} decreased >30-fold (from ∼37 to ∼1 min). In a specific embodiment, the mutation was D238R. In specific embodiments, the antibody or antigen-binding fragment of the invention binds an epitope of hPCSK9 comprising two or more of amino acid residues at positions 153, 159, 238 and 343.

As shown before (see US 2010/0166768), a mutation in amino acid residue 153, 159 or 343 resulted in about a 5- to 10-fold decrease in affinity or similar shortening in T_{1/2}. In specific embodiments, the mutation was S153R, E159R and/or D343R.

In some embodiments, the antibody or antigen-binding fragment binds an epitope on hPCSK9 comprising amino acid residue 366 of hPCSK9 (SEQ ID NO:755). Experimental results (see US 2010/0166768) showed that when E366 was mutated, the affinity of mAb 300N exhibited about 50-fold decrease (∼0.7 x10⁻⁹ M to ∼36 x10⁻⁹ M) and a similar shortening in T_{1/2} (from ∼120 to ∼2 min). In a specific embodiment, the mutation is E366K.

The present invention includes anti-PCSK9 antibodies that bind to the same epitope as any of the specific exemplary antibodies described herein. Likewise, the present invention also includes anti-PCSK9 antibodies that compete for binding to PCSK9 or a PCSK9 fragment with any of the specific exemplary antibodies described herein.

One can easily determine whether an antibody binds to the same epitope as, or competes for binding with, a reference anti-PCSK9 antibody by using routine methods known in the art. For example, to determine if a test antibody binds to the same epitope as a reference anti-PCSK9 antibody of the invention, the reference antibody is allowed to bind to a PCSK9 protein or peptide under saturating conditions. Next, the ability of a test antibody to bind to the PCSK9 molecule is assessed. If the test antibody is able to bind to PCSK9 following saturation binding with the reference anti-PCSK9 antibody, it can be concluded that the test antibody binds to a different epitope than the reference anti-PCSK9 antibody. On the other hand, if the test antibody is not able to bind to the PCSK9 molecule following saturation binding with the reference anti-PCSK9 antibody, then the test antibody may bind to the same epitope as the epitope bound by the reference anti-PCSK9 antibody of the invention.

To determine if an antibody competes for binding with a reference anti-PCSK9 antibody, the above-described binding methodology is performed in two orientations: In a first orientation, the reference antibody is allowed to bind to a PCSK9 molecule under saturating conditions followed by assessment of binding of the test antibody to the PCSK9 molecule. In a second orientation, the test antibody is allowed to bind to a PCSK9 molecule under saturating conditions followed by assessment of binding of the reference antibody to the PCSK9 molecule. If, in both orientations, only the first (saturating) antibody is capable of binding to the PCSK9 molecule, then it is concluded that the test antibody and the reference antibody compete for binding to PCSK9. As will be appreciated by a person of ordinary skill in the art, an antibody that competes for binding with a reference antibody may not necessarily bind to the identical epitope as the reference antibody, but may sterically block binding of the reference antibody by binding an overlapping or adjacent epitope.

Two antibodies bind to the same or overlapping epitope if each competitively inhibits (blocks) binding of the other to the antigen. That is, a 1-, 5-, 10-, 20- or 1 00-fold excess of one antibody inhibits binding of the other by at least 50% but preferably 75%, 90% or even 99% as measured in a competitive binding assay (see, e.g., Junghans et al., Cancer Res. 1990 50:1495-1502). Alternatively, two antibodies have the same epitope if essentially all amino acid mutations in the antigen that reduce or eliminate binding of one antibody reduce or eliminate binding of the other. Two antibodies have overlapping epitopes if some amino acid mutations that reduce or eliminate binding of one antibody reduce or eliminate binding of the other.

Additional routine experimentation (e.g., peptide mutation and binding analyses) can then be carried out to confirm whether the observed lack of binding of the test antibody is in fact due to binding to the same epitope as the reference antibody or if steric blocking (or another phenomenon) is responsible for the lack of observed binding. Experiments of this sort can be performed using ELISA, RIA, surface plasmon resonance, flow cytometry or any other quantitative or qualitative antibody-binding assay available in the art.

In a specific embodiment, the invention comprises an anti-PCSK9 antibody or antigen binding fragment of an antibody that binds an PCSK9 protein of SEQ ID NO:755, wherein the binding between the antibody or fragment thereof to PCSK9 and a variant PCSK9 protein is less than 50% of the binding between the antibody or fragment and the PCSK9 protein of SEQ ID NO:755. In one specific embodiment, the variant PCSK9 protein comprises at least one mutation of a residue at a position selected from the group consisting of 153, 159, 238 and 343. In a more specific embodiment, the at least one mutation is S153R, E159R, D238R, and/or D343R. In another specific embodiment, the variant PCSK9 protein comprises at least one mutation of a residue at a position selected from the group consisting of 366. In one specific embodiment, the variant PCSK9 protein comprises at least one mutation of a residue at a position selected from the group consisting of 147, 366 and 380. In a more specific embodiment, the mutation is S147F, E366K and V380M.

### Immunoconjugates

The invention encompasses a human anti-PCSK9 monoclonal antibody conjugated to a therapeutic moiety ("immunoconjugate"), such as a cytotoxin, a chemotherapeutic drug, an immunosuppressant or a radioisotope. Cytotoxin agents include any agent that is detrimental to cells. Examples of suitable cytotoxin agents and chemotherapeutic agents for forming immunoconjugates are known in the art, see for example, WO 05/103081.

### Bispecifics

The antibodies of the present invention may be monospecific, bispecific, or multispecific. Multispecific mAbs may be specific for different epitopes of one target polypeptide or may contain antigen-binding domains specific for more than one target polypeptide. See, e.g., Tutt et al. (1991) J. lmmunol. 147:60-69. The human anti-PCSK9 mAbs can be linked to or co-expressed with another functional molecule, e.g., another peptide or protein. For example, an antibody or fragment thereof can be functionally linked (e.g., by chemical coupling, genetic fusion, noncovalent association or otherwise) to one or more other molecular entities, such as another antibody or antibody fragment, to produce a bispecific or a multispecific antibody with a second binding specificity.

An exemplary bi-specific antibody format that can be used in the context of the present invention involves the use of a first immunoglobulin (lg) CH3 domain and a second lg CH3 domain, wherein the first and second Ig CH3 domains differ from one another by at least one amino acid, and wherein at least one amino acid difference reduces binding of the bispecific antibody to Protein A as compared to a bi-specific antibody lacking the amino acid difference. In one embodiment, the first lg CH3 domain binds Protein A and the second lg CH3 domain contains a mutation that reduces or abolishes Protein A binding such as an H95R modification (by IMGT exon numbering; H435R by EU numbering). The second CH3 may further comprise a Y96F modification (by IMGT; Y436F by EU). Further modifications that may be found within the second CH3 include: D16E, L18M, N44S, K52N, V57M, and V82l (by IMGT; D356E, L358M, N384S, K392N, V397M, and V422l by EU) in the case of IgG1 antibodies; N44S, K52N, and V82l (IMGT; N384S, K392N, and V422l by EU) in the case of IgG2 antibodies; and Q15R, N44S, K52N, V57M, R69K, E79Q, and V82I (by IMGT; Q355R, N384S, K392N, V397M, R409K, E419Q, and V422l by EU) in the case of IgG4 antibodies. Variations on the bi-specific antibody format described above are contemplated within the scope of the present invention.

### Bioequivalents

The anti-PCSK9 antibodies and antibody fragments of the present invention encompass proteins having amino acid sequences that vary from those of the described mAbs, but that retain the ability to bind human PCSK9. Such variant mAbs and antibody fragments comprise one or more additions, deletions, or substitutions of amino acids when compared to parent sequence, but exhibit biological activity that is essentially equivalent to that of the described mAbs. Likewise, the anti-PCSK9 antibody-encoding DNA sequences of the present invention encompass sequences that comprise one or more additions, deletions, or substitutions of nucleotides when compared to the disclosed sequence, but that encode an anti-PCSK9 antibody or antibody fragment that is essentially bioequivalent to an anti-PCSK9 antibody or antibody fragment of the invention. Examples of such variant amino acid and DNA sequences are discussed above.

Two antigen-binding proteins, or antibodies, are considered bioequivalent if, for example, they are pharmaceutical equivalents or pharmaceutical alternatives whose rate and extent of absorption do not show a significant difference when administered at the same molar dose under similar experimental conditions, either single does or multiple dose. Some antibodies will be considered equivalents or pharmaceutical alternatives if they are equivalent in the extent of their absorption but not in their rate of absorption and yet may be considered bioequivalent because such differences in the rate of absorption are intentional and are reflected in the labeling, are not essential to the attainment of effective body drug concentrations on, e.g., chronic use, and are considered medically insignificant for the particular drug product studied. In one embodiment, two antigen-binding proteins are bioequivalent if there are no clinically meaningful differences in their safety, purity, and potency.

In one embodiment, two antigen-binding proteins are bioequivalent if a patient can be switched one or more times between the reference product and the biological product without an expected increase in the risk of adverse effects, including a clinically significant change in immunogenicity, or diminished effectiveness, as compared to continued therapy without such switching.

In one embodiment, two antigen-binding proteins are bioequivalent if they both act by a common mechanism or mechanisms of action for the condition or conditions of use, to the extent that such mechanisms are known.

Bioequivalence may be demonstrated by *in vivo* and *in vitro* methods. Bioequivalence measures include, e.g., (a) an *in vivo* test in humans or other mammals, in which the concentration of the antibody or its metabolites is measured in blood, plasma, serum, or other biological fluid as a function of time; (b) an *in vitro* test that has been correlated with and is reasonably predictive of human *in vivo* bioavailability data; (c) an *in vivo* test in humans or other mammals in which the appropriate acute pharmacological effect of the antibody (or its target) is measured as a function of time; and (d) in a well-controlled clinical trial that establishes safety, efficacy, or bioavailability or bioequivalence of an antibody.

Bioequivalent variants of anti-PCSK9 antibodies of the invention may be constructed by, for example, making various substitutions of residues or sequences or deleting terminal or internal residues or sequences not needed for biological activity. For example, cysteine residues not essential for biological activity can be deleted or replaced with other amino acids to prevent formation of unnecessary or incorrect intramolecular disulfide bridges upon renaturation.

### Treatment Population

The invention provides therapeutic methods for treating a human patient in need of a composition of the invention. While modifications in lifestyle and conventional drug treatment are often successful in reducing cholesterol levels, not all patients are able to achieve the recommended target cholesterol levels with such approaches. Various conditions, such as familial hypercholesterolemia (FH), appear to be resistant to lowering of LDL-C levels in spite of aggressive use of conventional therapy. Homozygous and heterozygous familial hypercholesterolemia (hoFH, heFH) is a condition associated with premature atherosclerotic vascular disease. However, patients diagnosed with hoFH are largely unresponsive to conventional drug therapy and have limited treatment options. Specifically, treatment with statins, which reduce LDL-C by inhibiting cholesterol synthesis and upregulating the hepatic LDL receptor, may have little effect in patients whose LDL receptors are non-existent or defective. A mean LDL-C reduction of only less than about 20% has been recently reported in patients with genotype-confirmed hoFH treated with the maximal dose of statins. The addition of ezetimibe 10 mg/day to this regimen resulted in a total reduction of LDL-C levels of 27%, which is still far from optimal. Likewise, many patients are statin non-responsive, poorly controlled with statin therapy, or cannot tolerate statin therapy; in general, these patients are unable to achieve cholesterol control with alternative treatments. There is a large unmet medical need for new treatments that can address the short-comings of current treatment options.

Specific populations treatable by the therapeutic methods of the invention include subjects indicated for LDL apheresis, subjects with PCSK9-activating mutations (gain of function mutations, "GOF"), subjects with heterozygous Familial Hypercholesterolemia (heFH); subjects with primary hypercholesterolemia who are statin intolerant or statin uncontrolled; and subjects at risk for developing hypercholesterolemia who may be preventably treated. Other indications include hyperlipidemia and dyslipidemia (such as mixed dyslipidemia) associated with secondary causes such as Type 2 diabetes mellitus, cholestatic liver diseases (primary biliary cirrhosis), nephrotic syndrome, hypothyroidism, obesity; and the prevention and treatment of atherosclerosis and cardiovascular diseases. However, depending on the severity of the afore-mentioned diseases and conditions, the treatment of subjects with the antibodies and antigen-binding fragments of the invention may be contraindicated for certain diseases and conditions.

### Therapeutic Administration and Formulations

The invention provides therapeutic compositions comprising the anti-PCSK9 antibodies or antigen-binding fragments thereof of the present invention. The administration of therapeutic compositions in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The dose may vary depending upon the age and the size of a subject to be administered, target disease, conditions, route of administration, and the like. When the antibody of the present invention is used for treating various conditions and diseases associated with PCSK9, including hypercholesterolemia, disorders associated with LDL and apolipoprotein B, and lipid metabolism disorders, and the like, in an adult patient, it is advantageous to intravenously administer the antibody of the present invention normally at a single dose of about 0.01 to about 20 mg/kg body weight, more preferably about 0.02 to about 7, about 0.03 to about 5, or about 0.05 to about 3 mg/kg body weight. Depending on the severity of the condition, the frequency and the duration of the treatment can be adjusted.

Various delivery systems are known and can be used to administer the pharmaceutical compositions of the invention, e.g., encapsulation in liposomes, microparticles, microcapsules, recombinant cells capable of expressing the mutant viruses, receptor mediated endocytosis (see, e.g., Wu et al. (1987) J. Biol. Chem. 262:4429-4432). Methods of introduction include, but are not limited to, intradermal, intramuscular, intraperitoneal, intravenous, subcutaneous, intranasal, epidural, and oral or peroral routes. If the antibody of present invention is administered per injection, subcutaneous injection is preferred. Oral or peroral administration is preferred for the HMG-CoA reductase inhibitor, e.g. the statin.

The composition may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. The pharmaceutical composition can be also delivered in a vesicle, in particular a liposome (see Langer (1990) Science 249:1527-1533; Treat et al. (1989) in Liposomes in the Therapy of Infectious Disease and Cancer, Lopez Berestein and Fidler (eds.), Liss, New York, pp. 353-365; Lopez-Berestein, ibid., pp. 317-327; see generally ibid.).

In certain situations, the pharmaceutical composition can be delivered in a controlled release system. In one embodiment, a pump may be used (see Langer, supra; Sefton (1987) CRC Crit. Ref. Biomed. Eng. 14:201). In another embodiment, polymeric materials can be used; see, Medical Applications of Controlled Release, Langer and Wise (eds.), CRC Pres., Boca Raton, Florida (1974). In yet another embodiment, a controlled release system can be placed in proximity of the composition's target, thus requiring only a fraction of the systemic dose (see, e.g., Goodson, in Medical Applications of Controlled Release, supra, vol. 2, pp. 115-138, 1984).

The injectable preparations may include dosage forms for intravenous, subcutaneous, intracutaneous and intramuscular injections, drip infusions, etc. These injectable preparations may be prepared by methods publicly known. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but certainly are not limited to AUTOPEN™ (Owen Mumford, Inc., Woodstock, UK), DISETRONIC™ pen (Disetronic Medical Systems, Burghdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™ , and OPTICLIK™ (sanofi-aventis, Frankfurt, Germany), to name only a few. Examples of disposable pen delivery devices having applications in subcutaneous delivery of a pharmaceutical composition of the present invention include, but certainly are not limited to the SOLOSTAR™ pen (sanofi-aventis), the FLEXPEN™ (Novo Nordisk), and the KWIKPEN™ (Eli Lilly).

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. The amount of the aforesaid antibody contained is generally about 4 to about 500 mg or from about, from about 5 to about 500 mg per dosage form in a unit dose; especially in the form of injection, it is preferred that the aforesaid antibody is contained in about 5 to about 100 mg or about 5 to 400 mg (such as from about 50 to about 200 mg per 1 ml injection solution) and in about 10 to about 250 mg or to about 500 mg for the other dosage forms.

The invention provides therapeutic methods in which the antibody or antibody fragment of the invention is useful to treat hypercholesterolemia associated with a variety of conditions involving hPCSK9. The anti-PCSK9 antibodies or antibody fragments of the invention are particularly useful for the treatment of hypercholesterolemia and the like, particularly in the types of hypercholesteremia as herein described.

In some embodiments, the antibodies or antibody fragments of the invention and as herein described are for use in combination therapies for treatment or prevention of the herein described indications. Combination therapies may include the antibody or fragment of the invention and as herein described together with one or more additional lipid-lowering agent.

Combination of the antibody or fragment of the invention may comprise administration of the antibody or fragment thereof together with one or more of the following lipid lowering agents:
(1) an agent that induces a cellular depletion of cholesterol synthesis by inhibiting 3-hydroxy-3-methylglutaryl (HMG)-coenzyme A (CoA) reductase, such as cerivastatin, atorvastatin, simvastatin, pitavastatin, rosuvastatin, fluvastatin, lovastatin, pravastatin;
(2) an agent that inhibits cholesterol uptake and or bile acid re-absorption, e.g. ezetimibe;
(3) an agent that increases lipoprotein catabolism (such as niacin and activators of the LXR transcription factor that plays a role in cholesterol elimination such as 22-hydroxycholesterol, ezetimibe
(4) any other lipid lowering agent such as an omega-3-fatty acid ethyl estes (for example, omacor), or
(5) combinations of any of 1-4 such as ezetimibe plus a HMG-CoA inhibitor, ezetimibe plus a statin, ezetimibe with a statin, particularly a fixed combination of ezetimibe with any of the statins as herein desclosed e.g. ezetimibe with atorvastatin or ezetimibe with simvastatin; a statin with a bile resin (e.g., ) cholestyramine, colestipol, colesevelam), a combination of niacin plus a statin, particularly a fixed combination of nicacin plus a statin (e.g., niacin with lovastatin); or combinations of any of 1-3 with other lipid lowering agents such as omega-3-fatty acid ethyl esters (for example, omacor).

Combination therapies may for example include the anti-PCSK9 antibody of the invention with, for example, one or more of any agent that (1) induces a cellular depletion of cholesterol synthesis by inhibiting 3-hydroxy-3-methylglutaryl (HMG)-coenzyme A (CoA) reductase, such as cerivastatin, atorvastatin, simvastatin, pitavastatin, rosuvastatin, fluvastatin, lovastatin, pravastatin; (2) inhibits cholesterol uptake and or bile acid re-absorption; (3) increase lipoprotein catabolism (such as niacin) and activators of the LXR transcription factor that plays a role in cholesterol elimination such as 22-hydroxycholesterol or fixed combinations such as ezetimibe plus simvastatin; a statin with a bile resin (e.g., cholestyramine, colestipol, colesevelam), a fixed combination of niacin plus a statin (e.g., niacin with lovastatin); or with other lipid lowering agents such as omega-3-fatty acid ethyl esters (for example, omacor).

The above combination therapies are particularly suitable in the context of the method of the first, second, third, fourth and/or fifth aspect and for the use according to the sixth aspect of present invention. The present invention thus also relates to a pharmaceutical composition for use in the above combination therapy and a pharmaceutical composition comprising (in addition to the antibody or fragment of present invention) one or more of the above-indicated additional lipid lowering agents. The present invention also relates to an article of manufacture of present invention, e.g. according to the eighth aspect of present invention that is adapted for the combination therapy as herein described, e.g. by comprising in addition to the antibody or fragment thereof of present invention one or more of the herein described additional lipid lowering agents and/or by comprising instructions for a use adapted to a particular combination treatment and/or comprising instructions for a dosage or administration regime of the additional lipid lowering agent.

### PREFERRED ASPECTS OF PRESENT INVENTION

In the following, some preferred aspects of present invention will be listed:
1. A method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof falls into one or more of the following groups of subjects:
   (i) subjects having a serum LDL cholesterol (LDL-C) level of at least 100 mg/dL, *[at least 130 mg*/*dL, at least 160 mg*/*dL* / *at least 200 mg*/*dL];*
   (ii) subjects having a serum HDL-C level of less than 40 mg/dL;
   (i) subjects having a serum cholesterol level of at least 200 mg/dL *[240 mg*/*dL];*
   (ii) subjects having a serum triacylglycerol level of at least 150 mg/dL *[at least 200 mg*/*dL; at least* 500 *mg*/*dL],* wherein said triacylglycerol level is determined after fasting for at least 8 hours;
   (iii) subjects being at least 18 years old *[at least* 18 /20 /25 /30/ 35 /40/ 45 / 50 / 55 /*60* / *65* / *70* / *75 years old];*
   (iv) subjects being at maximum 75 years old *[75* /*70* /*65* / *60* / *55* / *50* / *45* / *40 35*/ *30*/ *25*/ *20 years];*
   (v) subjects having a BMI of 25 *[26* / *27* / *28* / *29* / *30* / *31* / *32* / *33* / *34* / *35* / *36* / *37* / *38* / 39] or more;
   (vi) male subjects;
   (vii) female subjects;
   (viii) subjects in which the administration of said antibody or antigen-binding fragment thereof leads to a reduction in the serum LDL-C level by at least 30 mg/dL *[40 mg*/*dL; 50 mg*/*dL; 60 mg*/*dL; 70 mg*/*dL]* relative to predose level; or
   (ix) subjects in which the administration of said antibody or antigen-binding fragment thereof leads to a reduction in the serum LDL-C level by at least 20% *[30%; 40%; 50%; 60%j* relative to predose level
   (x) subjects having mild and/or moderate hepatic impairment
   (xi) subjects with normal hepatic liver function
   (xii) subjects with high cardiovascular risk
   (xiii) subjects with coronary heart disease
   (xiv) subjects with cardiovascular disease.
2. A method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof does not fall into one or more of the following groups of subjects:
   (i) smokers;
   (ii) persons being 76 years old or older;
   (iii) persons being 17 years or younger;
   (iv) persons suffering from hypertension;
   (v) women who are pregnant;
   (vi) women who are trying to become pregnant;
   (vii) women who are breast-feeding;
   (viii) persons who have or ever had severe hepatic impairment
   (ix) persons who had any unexplained abnormal results from blood tests for liver function;
   (x) persons who drink excessive amounts of alcohol;
   (xi) persons having kidney problems;
   (xii) persons suffering from hypothyroidism;
   (xiii) persons suffering from muscle disorders;
   (xiv) persons having encountered previous muscular problems during treatment with lipid-lowering medicine;
   (xv) persons having serious problems with their breathing;
   (xvi) persons taking one or more of the following medicines: medicines altering the way the immune systems works (e.g. ciclosporin or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or
   (xvii) persons drinking more than 0.1 L of grapefruit juice per day;
   (xviii) persons having a body mass index (BMI) of more than 40;
   (xix) persons having a body mass index (BMI) of less than 18;
   (xx) persons suffering from type 1 diabetes or type 2 diabetes;
   (xxi) persons positive for hepatitis B or hepatitis C; or
   (xxii) persons having a known sensitivity to monoclonal antibody therapeutics
   (xxiii) persons having acute hepatitis,
   (xxiv) persons having hepatic encephalopathy grade 2, 3, or 4,
   (xxv) persons having uncontrolled clinically relevant cardiovascular, pulmonary, gastrointestinal, metabolic, hematological, neurological, psychiatric, systemic, ocular, gynecologic (if female), or infectious disease, or signs of acute illnes
   (xxvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness
   and wherein the subject preferably falls into one or more of the subject groups according to aspect 1.
3. A method of lowering cholesterol levels in a subject in need thereof, comprising:
   (a) selecting a subject with a blood low density lipoprotein cholesterol (LDL-C) level greater than 100 mg/dL; and
   (b) administering to said subject a composition comprising an antibody or antigen binding fragment thereof that specifically binds to human proprotein convertase subtilisin/kexin type 9 (hPCSK9); thereby lowering cholesterol levels in the subject in need thereof, wherein the subject falls into one or more of the groups of subjects as listed in aspect 1 and/or wherein the subject does not fall into one or more of the groups of subjects as listed in aspect 2.
4. A method of regulating the LDL level in the blood of a subject comprising:
   - administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
   - administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in aspect 1 and wherein the subject preferably does not fall into one or more of the groups of subjects as listed in aspect 2.
5. A method of preventing effects of a (persistently) increased LDL level in the blood comprising:
   - administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
   - administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in aspect 1 and wherein the subject preferably does not fall into one or more of the groups of subjects as listed in aspect 2.
6. An antibody or an antigen-binding fragment thereof which specifically binds hPCSK9 (human proprotein convertase subtilisin/kexin type 9) for use in the treatment of a disease or condition in which PCSK9 expression or activity causes an impact, wherein the antibody or antigen-binding fragment thereof is for administration to a subject falling at least into one of the groups of subjects as listed in aspect 1 and wherein the antibody or antigen-binding fragment thereof preferably is not for administration to a subject falling at least into one of the groups of subjects as listed in aspect 2.
7. The method according to one of the aspects 1 to 5 or the antibody of aspect 6, wherein the disease or condition in which PCSK9 expression or activity causes an impact is ameliorated, improved, inhibited or prevented with a PCSK9 antagonist.
8. The method according to one of the aspects 1-5 or 7, comprising:
   - administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
   - administering a therapeutic amount of an HMG-CoA reductase inhibitor to said subject, wherein the HMG-CoA reductase inhibitor is administered in a dosage amount ranging from 0.05 mg to 100 mg.
9. The antibody or an antigen-binding fragment thereof according to aspect 6 or 7, wherein the antibody or antigen-binding fragment thereof is for administration in a dosage amount ranging from 5 mg to 500 mg, wherein the antibody or antigen-binding fragment thereof is further for administration in combination with an HMG-CoA reductase inhibitor at a dosage amount ranging from 0.05 mg to 100 mg.
10. The method or the antibody of any one of aspects 1 to 9, wherein the disease or condition in which PCSK9 expression or activity causes an impact is selected from the group consisting of: hypercholesterolemia, hyperlipidemia, dyslipidemia (such as mixed dyslipidemia), atherosclerosis and cardiovascular disease(s).
11. The method or the antibody of any one of aspects 1 to 10, wherein the subject in need thereof is a subject indicated for LDL apheresis, a subject with PCSK9-activating mutations, a subject with heterozygous Familial Hypercholesterolemia, a subject with primary hypercholesterolemia who is statin uncontrolled, a subject at risk for developing hypercholesterolemia, a subject with hypercholesterolemia, hyperlipidemia, dyslipidemia (such as mixed dyslipidemia), atherosclerosis, a high risk of developing or suffering from cardiovascular disease, a subject with a cardiovascular disease such as coronary heart disease or a coronary heart disease risk-equivalent.
12. The method or the antibody of any one of aspects 1 to 11, wherein the antibody or antigen-binding fragment thereof is a recombinant human antibody or fragment thereof.
13. The method or the antibody of any one of aspects 1 to 12, wherein the antibody or the antigen-binding fragment thereof is characterized by one or more of the following:
   (i) capable of reducing serum total cholesterol at least about 25 to about 35% and sustaining the reduction over at least a 24 day period relative to a predose level;
   (ii) capable of reducing serum LDL cholesterol at least about 65-80% and sustaining the reduction over at least a 24 day period relative to a predose level;
   (iii) capable of reducing serum triglyceride at least about 25-40% relative to predose level;
   (iv) achieves one or more of (i)-(iii) without reducing serum HDL cholesterol or reducing serum HDL cholesterol no more than 5% relative to predose level;
   (v) achieves one or more of (i)-(iii) with little or no measurable effect on liver function, as determined by ALT and AST measurements.
14. The method or the antibody of any one of aspects 1 to 13, wherein the antibody or the antigen-binding fragment thereof comprises
   - a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
   - a light chain CDR3 (LCDR3) domain selected from the group consisting of SEQ ID NO:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736.
15. The method or the antibody of any one of aspects 1 to 14, wherein the antibody or the antigen-binding fragment thereof comprises the heavy and light chain CDRs of a HCVR/LCVR amino acid sequence pair as shown in SEQ ID NOs: 90/92, and wherein the antibody or antigen-binding fragment thereof preferably comprises heavy and light chain CDR amino acid sequences as shown in SEQ ID NOs: 76, 78, 80, 84, 86, and 88, and wherein the antibody or antigen-binding fragment thereof more preferably comprises an HCVR amino acid sequence as shown in SEQ ID NO: 90 and an LCVR amino acid sequence as shown in SEQ ID NO: 92.
16. The method or the antibody of any one of aspects 1 to 15, wherein the antibody or antigen-binding fragment thereof binds to the same epitope on hPCSK9 as an antibody comprising heavy and light chain CDR amino acid sequences as shown in SEQ ID NOs: 76, 78, 80, 84, 86, and 88.
17. The method or the antibody of any one of aspects 1 to 16, wherein the antibody or antigen-binding fragment thereof competes for binding to hPCSK9 with an antibody comprising heavy and light chain CDR amino acid sequences as shown in SEQ ID NOs: 76, 78, 80, 84, 86, and 88.
18. The method or the antibody of any one of aspects 1 to 17, further comprising:
   (a) administering a therapeutic amount of an HMG-CoA reductase inhibitor to the subject in a dosage of between 0.05 mg to 100 mg and/or
   (b) administering a therapeutic amount of another lipid lowering agent to the subject, preferably ezetimibe and more preferably ezetimibe in a dosage of about 10 mg .
19. The method or the antibody of aspect 18, wherein the HMG-CoA reductase inhibitor is a statin.
20. The method or the antibody of aspect 19, wherein the statin is selected from the group consisting of cerivastatin, atorvastatin, simvastatin, pitavastatin, rosuvastatin, fluvastatin, lovastatin, and pravastatin and is preferably atorvastatin.
21. The method or the antibody of aspect 20, wherein the statin is
   - cerivastatin administered in a daily dosage of between 0.05 mg and 2 mg;
   - atorvastatin administered in a daily dosage of between 2 mg and 100 mg;
   - simvastatin administered in a daily dosage of between 2 mg and 100 mg;
   - pitavastatin administered in a daily dosage of between 0.2 mg and 100 mg;
   - rosuvastatin administered in a daily dosage of between 2 mg and 100 mg;
   - fluvastatin administered in a daily dosage of between 2 mg and 100 mg;
   - lovastatin administered in a daily dosage of between 2 mg and 100 mg; or
   - pravastatin administered in a daily dosage of between 2 mg and 100 mg.
22. The method or the antibody of any one of aspects 18 to 21, wherein the HMG-CoA reductase inhibitor is administered three times per day, twice per day, or once per day.
23. The method or the antibody of any one of aspects 18 to 22, wherein the HMG-CoA reductase inhibitor is administered every day, every other day, every third day, every fourth day, every fifth day, or every sixth day.
24. The method or the antibody of any one of aspects 18 to 23 wherein the HMG-CoA reductase inhibitor is administered every week, every other week, every third week, or every fourth week.
25. The method or the antibody of any one of aspects 18 to 24 wherein the HMG-CoA reductase inhibitor is administered in the morning, at noon or in the evening.
26. The method or the antibody of any one of aspects 1 to 25, wherein the antibody or antigen-binding fragment thereof is administered to the subject every other week.
27. The method or the antibody of any one of aspects 1 to 26, wherein the antibody or antigen-binding fragment thereof is administered in a dosage amount ranging from 50 mg to 300 mg.
28. The method or the antibody of any one of aspects 1 to 27, wherein the antibody or antigen-binding fragment thereof is a recombinant human antibody or fragment thereof.
29. The method or the antibody of any one of aspects 18 to 28, further comprising:
   administering another lipid lowering drug and preferably administering ezetimibe.
30. Pharmaceutical composition comprising an antibody or antigen-binding fragment thereof which specifically binds hPCSK9 and a pharmaceutically acceptable excipient for use in a method according to one of the aspects 1 to 5 or 7 to 29.
31. Article of manufacture comprising
   (a) a packaging material or container;
   (b) an antibody or an antigen-binding fragment thereof which specifically binds hPCSK9; and
   (c) a label or packaging insert, wherein the label or packaging insert indicates the following:
      (1) the antibody or antigen-binding fragment thereof can be applied for treatment to subjects falling into one or more groups of subjects as recited in aspect 1
      (2) the label or packaging insert preferably further indicates that the application of the antibody or antigen-binding fragment thereof to subjects is contraindicated for subjects belonging to one or more of the groups of subjects as recited in aspect 2.
32. The article of manufacture according to aspect 31, wherein the antibody or fragment thereof is comprised in a pharmaceutical formulation together with a pharmaceutically acceptable excipient.
33. The article of manufacture according to any of aspects 31 or 32, wherein the label or packaging insert contains reference to a method of treatment according to any of aspects 1 to 5, or 7 to 30.
34. The article of manufacture according to one of the aspects 31 to 33, wherein the antibody or antigen-binding fragment is an antibody or antigen-binding fragment as specified in any of aspects 14-17 or 29.
35. The article of manufacture according to any of aspects 31 to 34, wherein the label or packaging insert has one or more of the following features:
   (a) the label or packaging insert indicates that patients receiving treatment with said antibody or antigen-binding fragment can be treated for a disease or condition selected from the group consisting of hypercholesterolemia, hyperlipidemia, dyslipidemia (such as mixed dyslipidemia), atherosclerosis and cardiovascular disease such as coronary heart disease or a CHD-risk equivalent;
   (b) the label or packaging insert indicates the treatment of patients with said antibody or antigen-binding fragment thereof together with the application of a lipid lowering agent such as an HMG-CoA reductase inhibitor, and preferably a statin;
   (c) the label or packaging insert indicates the treatment of patients with said antibody or antigen-binding fragment thereof together with the application of a lipid lowering agent such as ezetimibe;
   (d) the label or packaging insert indicates the treatment of patients with said antibody or antigen-binding fragment thereof together with the application of a lipid lowering agent such as an HMG-CoA reductase inhibitor, and preferably a statin and together with the application of a lipid lowering agent such as ezetimibe, particularly a combination treatment of the antibody or antigen-binding fragment thereof together with a statin and ezetimibe;
   (e) the label or packaging insert indicates that the treatment of patients according to (b), (c) or (d) above, is possible for patients belonging to one or more of the subject groups as listed in aspect 1, and preferably is possible for subjects having mild and/or moderate hepatic impairment or subjects having normal hepatic liver function and/or having an increased cardiovascular risk and/or (established) CHD and/or a CHD-risk equivalent;
   (f) the label or packaging insert indicates that the treatment of patients with said antibody or antigen-binding fragment thereof according to (b), (c) or (d), is contraindicated for patients belonging to one or more of the subject groups as listed in aspect 2.
36. The article of manufacture according to one of the aspects 30 to 34 further comprising an HMG-CoA reductase inhbitor and preferably an HMG-CoA inhibitor according to one of the aspects 20-22.
37. The article of manufacture according to aspect 36, comprising about 0.05 mg to about 100 mg, about 0,5 mg to about 100 mg, about 5 mg to about 90 mg, about 10 mg, about 20 mg, about 40 mg or about 80 mg of HMG-CoA reductase inhibitor and preferably about 10, about 20, about 40 or about 80 mg.
38. The article of manufacture according to one of aspects 36 or 37, comprising an effective dose of HMG-CoA reductase inhibitor for lowering LDL-C levels by administration once per day.
39. The article of manufacture according to one of aspects 31 to 38, comprising the antibody as injection solution and preferably comprising about 40 mg to about 200 mg or about 40 to about 200 mg, e.g. about 40 mg, about 50 mg, about 75 mg, at about 100 mg, about 150 mg or about 200 mg of the antibody or antigen-binding fragment thereof per 1 ml volume.
40. The article of manufacture according to one of aspects 31 to 38, comprising the antibody as dry formulation, preferably comprising about 40 mg to about 500 mg, 50 to about 500 mg, about 50 to about 400, about 50 to about 300 e.g. about 40 mg, about 50 mg, about 75 mg, at about 100 mg, about 150 mg, about 200 mg, about 250 mg, about 300 mg, about 350 mg, about 400 mg, about 450 mg or about 500mg and preferably about 50 mg, about 100 mg, about 150 mg, about 200 mg, about 250 mg, about 300 mg and even more preferably about 150 mg, about 200 mg or about 300 mg of the antibody or antigen-binding fragment thereof per dose.
41. Article of manufacture according to one of the aspects 38 to 40 comprising separate unit dosage forms the antibody, pharmaceutical composition, dry formulation or injection solution and the for the HMG-CoA reductase inhibitor.
42. Article of manufacture according to one of the aspects 34 to 41 comprising one or more of the following components:
   a) one or more unit dosage forms comprising the antibody or fragment thereof;
   b) one or more unit dosage forms comprising the HMG-CoA reductase inhibitor;
   c) one or more unit dosage forms of another lipid lowering agents such as ezetimibe;
   d) the label or package insert;
   e) a device for application of the antibody such as a syringe and instructions for use of the device.
43. Article of manufacture according to one of the aspects 34-42, comprising one or more unit dosage forms comprising the antibody or fragment thereof as dry formulation for dissolution in a hermetically sealed container such as a vial, an ampoule or sachette
44. Article of manufacture according to one of the aspects 34-42, comprising one or more unit dosage forms comprising the antibody or fragment thereof as liquid formulation in a hermetically sealed container such as a vial, a sachette, a pre-filled syringe, a pre-filled autoinjector or a cartridge for a reusable syringe or applicator.
45. Article of manufacture according to one of the aspects 38 to 44, comprising one or more unit dosage forms of the HMG-CoA reductase inhibitor as tablet or capsule or other formulation for oral administration in a hermetically sealed container or blister
46. Article of manufacture according to one of the aspects 43 to 45, wherein the quantity of active ingredient is indicated on the hermetically-sealed container or blister.
47. Article of manufacture according to one of the aspects 42 to 46, comprising sufficient unit dosage forms of the antibody and preferably also of the HMG-CoA reductase inhibitor, for one single administration of antibody and HMG-CoA reductase inhibitor, for a two-week (i.e. 14-day) treatment with antibody and HMG-CoA reductase inhibitor, for a four week (i.e, 28-day) treatment with antibody and HMG-CoA reductase inhibitor or for a one-month treatment with antibody and HMG-CoA reductase inhibitor.
48. Article of manufacture according to one of the aspects 42 to 47, comprising sufficient unit dosage forms of antibody for a bi-weekly administration regime or a four-weekly administration regime or a monthly administration regime.
49. Article of manufacture according to one of the aspects 42 to 48 comprising sufficient unit dosage forms of HMG-CoA reductase inhibitor for a daily administration regime.
50. The article of manufacture according to one of the aspects 38 to 49, comprising an a further lipid lowering drug in an effective dose for lowering LDL-C levels and/or increasing HDL-C levels and/or lowering trygliceride levels in addition to the antibody or fragment thereof and the HMG-CoA reductase inhibitor and wherein the further lipid lowering drug is preferably ezetimibe.
51. A method for preparing an article of manufacture according to one of the aspects 34-498 comprising packaging the antibody, pharmaceutical composition, the liquid or dry formulation or the unit dosage form or forms according to one of the previous aspects in a container and assembling them with a label or packaging insert according to one of the previous aspects.
52. A method for treating a disease or condition in which PCSK9 expression or activity causes an impact according to to one of the aspects 1 to 29 comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject having mild and/or moderate hepatic impairment or a subject with normal hepatic liver function.
53. Unit dosage form comprising an antibody or an antigen-binding fragment thereof which specifically binds hPCSK9 (human proprotein convertase subtilisin/kexin type 9) for use in a method according to one of the aspects 1 to 29, wherein the antibody preferably is an antibody according to one of the aspects 6 to 29.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used but some experimental errors and deviations should be accounted for. Unless indicated otherwise, molecular weight is average molecular weight, temperature is in degrees Centigrade, and pressure is at or near atmospheric.

### Study 1

### Title: Pharmacokinetic and tolerability of antibody 316P in subjects with hepatic failure and in healthy subjects

This is a 12-week open-label, pharmacokinetic and tolerability study of antibody 316P, administered as a single subcutaneous dose in subjects with mild and moderate hepatic impairment, and in matched subjects with normal hepatic function. The study is designed as a phase 1, open label, single arm (arm-type: experimental), parallel study on subjects/patients with mild and moderate hepatic impairment and in matched subjects with normal hepatic function. The antibody 316P is to be administered subcutaneously in a dosage of 75 mg.

Therapeutic Area of the study is nutritional and metabolic disease, MedDRA Preferred Term: Hypercholesterolemia

Primary Aim is to to study the effect of mild or moderate hepatic impairment on the pharmacokinetics of antibody 316P.

Secondary aim is to assess the safety and tolerability of antibody 316P in subjects with mild and moderate hepatic impairment and in matched subjects with normal hepatic function and to assess the pharmacodynamic profile of antibody 316P in patients with hepatic impairment and in matched subjects with normal hepatic function.

Primary purpose of the study is basic science, primary focus of the study is pharmacokinetics. Scope of the trial is the assessment of safety and pharmacokinetics of antibody 316P.

### Study population:

- Healthy volunteers, both genders, minimum age 18 years, maximum age: 75 years

### Inclusion criteria:

- Patients: mild and moderate hepatic impairment based on Child-Pugh score, stable chronic liver disease
- Healthy subjects with normal hepatic function

### Exclusion criteria:

- age <18 or >75
- Patients:
   o acute hepatitis, hepatic encephalopathy grade 2, 3, and 4, Uncontrolled clinically relevant cardiovascular, pulmonary, gastrointestinal, metabolic, ematological, neurological, psychiatric, systemic, ocular, gynecologic (if female), or infectious disease, or signs of acute illness.
- Healthy subjects :
   o history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness.

### Specific vulnerable populations:

- women of child-bearing potential using contraception

Planned number of subjects/patients to be included or randomized: 24

Approximate number of subjects/patients per age range: 16 adults (18-64 years) and 8 elderly (≥65 years)

Investigational medicinal products: antibody 316P

Pharmaceutical Form: Solution for injection

Route for administration: subcutaneous

Plans for treatment or care after the end of the trial: not different from the expected normal treatment of the condition. The end of the trial is to be the last visit of the last subject/patient.

Primary endpoint: pharmacokinetics (serum concentrations) up to 12 weeks

Secondary endpoint: safety and tolerability up to 12 weeks

Duration of study period per patient: screening: up to 3 weeks, study duration per patient: about 12 weeks (excluding screening)

### Active compounds: Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

**Study 2:** Efficacy and Safety of antibody 316P versus placebo on top of lipid-modifying therapy in patients with high cardiovascular risk and hypercholesterolemia

This is a randomized, double-blind, placebo-controlled, parallel group study to evaluate the efficacy and safety of antibody 316P in ihgh cardiovascular risk patients with hypercholesterolemia not adequately controlled with their lipid-modifying therapy.

### Therapeutic area: Nutritional and metabolic diseases, particularly hypercholesterolaemia

Primary subject is to demonstrate the reduction of low-density lipoprotein cholesterol (LDL-C) by antibody 316P as add-on therapy to stable maximally tolerated daily statin therapy with or without other lipid-modifying therapy (LMT) in comparison with placebo after 24 weeks of treatment in high cardiovascular (CV) risk patients with hypercholesterolemia.

### Secondary subjects are:

To evaluate the effect of antibody 316P in comparison with placebo on LDL-C after 12 weeks of treatment
to evaluate the effect of antibody 316P on other lipid parameter
to evaluate the safety and tolerability of antibody 316P

An optional pharmacogenetic sub-study will be conducted to identify genetic associations with clinical or biomarker response to PCSK9 inhibition, hyperlipidemia, or cardiovascular disease.

Study design is a randomized, parallel, double blind phase 3 study.

Administration is antibody 316P versus placebo via subcutanous injection.

Study population are patients of both gender with minimum age of 18 years and no upper age-limit.
- Inclusion criteria:
   Patients with hypercholesterolemia and established coronary heart disease (CHD) or CHD risk equivalents who are not adequately controlled with a maximally tolerated daily dose of statin with or without other LMT (lipid modifying treatment) , both at stable dose for at least 4 weeks to 6 weeks prior to screening (Week -2)
- Exclusion criteria:
   Age < 18 or legal age of adulthood, whichever is greater
   Patients without established coronary heart disease (CHD) or CHD risk equivalent LDL-C <70 mg/dL (<1.81 mmol/L) at the screening visit and patients with an history of documented cardiovascular disease
   LDL-C <100 mg/dL (<2.59 mmol/L) at the screening visit and patients without history of documented cardiovascular disease
   Not on a stable dose of LMT (including statin) for at least 4 Weeks and/or fenofibrate for at least 6 weeks, as applicable, prior to the screening visit (Week -2) and from screening to randomization.
   Fasting serum triglycerides >400 mg/dL (>4.52 mmol/L) during the screening period.
   Specific vulnerable population: Women of child-bearing potential using contraception
   Investigational medicinal product is antibody 316P formulated as solution for injection. Injection is subcutaneous and control is a matching placebo.

### Active compounds: Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Primary endpoint is percent change of LDL-C from baseline to week 24. Secondary endpoint is the percent change in LDL-C from baseline to weeks 12 and 52. Further secondary endpoint is the percent change in other lipid parameters from baseline to weeks 12, 24 and 52.

Duration of the study per subject/patient is 2 weeks, screening period: 2 weeks, double-blind treatment period: 52 weeks, follow-up period: 8 weeks

**Study 3 -** Efficacy and Safety of antibody 316P versus Ezetimibe on top of statin in high cardiovascular risk patients with hypercholesterolemia.

This is a randomized, double-blind, parallel group study to evaluate the efficacy and safety of antibody 316P versus ezetimibe in high cardiovascular risk patients with hypercholesterolemia not adequately controlled with their statin therapy

### Therapeutic area: Nutritional and metabolic diseases, preferably: Hypercholesterolemia

Primary Study objective is to demonstrate the reduction of low-density lipoprotein cholesterol (LDL-C) by antibody 316P as add-on therapy to stable maximally tolerated daily statin therapy in comparison with ezetimibe 10 mg daily after 24 weeks of treatment in patients with hypercholesterolemia at high cardiovascular (CV) risk.

Secondary objectives are:
To evaluate the effect of antibody 316P 75 mg in comparison with ezetimibe on LDL-C after 12 weeks of treatment
To evaluate the effect of antibody 316P on other lipid parameters.
To evaluate the safety and tolerability of antibody 316P

An optional pharmacogenetic sub-study is conducted to identify genetic associations with clinical or biomarker response to PCSK9 inhibition, hyperlipidemia, or cardiovascular disease.

Study design: This is a randomized, parallel, double blind phase 3 study. The study has two arms with one arm receiving antibody 316P as injection through subcutaneous administration and receiving an ezetimibe placebo administered orally (316P plus ezetimibe-placebo). The second arm receives a 316P placebo as injection through subcutaneous administration and receives ezetimibe administered orally (316P-placebo plus ezetimibe).

Study population are patients of both gender with a minimum age of 18 years and no upper age limit (∼330 adults of 18-64 years of age, ∼330 elderly with 65 years of age or older).

Inclusion criteria: Patients with hypercholesterolemia and established coronary heart disease (CHD) or CHD risk equivalents who are not adequately controlled with a maximally tolerated daily dose of statin at stable dose for at least 4 weeks prior to the screening visit (Week -2).

Exclusion criteria:
Age < 18 years or under legal age of adulthood whatever is higher
Patients without established CHD or CHD risk equivalents
LDL-C <70 mg/dL (<1.81 mmol/L) at the screening visit (Week-2) and patients with an history of documented CV disease as described in section 7.1)
LDL-C <100 mg/dL (<2.59 mmol/L) at the screening visit (Week-2) and patients without history of documented CV disease as described in section 7.1)
Fasting serum triglycerides >400 mg/dL (>4.52 mmol/L) during the screening period.

### Specific vulnerable population: Women of child-bearing potential using contraception.

Investigational medicinal products are antibody 316P as solution for injection with subcutaneous route of administration and ezetimibe as encapsulated tablets for oral administration. The placebo for 316P is in a matching formulation as injection solution for subcutaneous administration, the placebo for ezetimibe is in a matching formulation as capsules for oral administration.

Active compounds:
Antibody 316P:
   Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

### Ezetimibe:

Formula of ezetimibe:

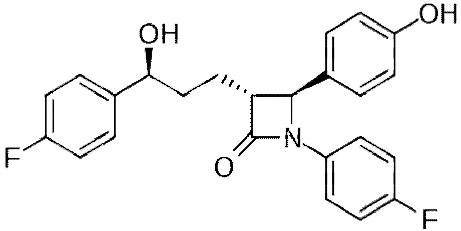

Primary endpoint is the percent change in LDL-C from baseline to week 24.

Secondary endpoints are the percent change in LDL-C from baseline to week 12 and the percent change in other lipid parameters at weeks 24 and 12.

Study duration per subject/patient is 114 weeks with a screening period of 2 weeks, a double-blind treatement period of 104 weeks and a follow-up period: 8 weeks.

**Study 4 -** Efficacy and Safety of antibody 316P versus placebo on top of lipid-modifying therapy in patients with heterozygous familial hypercholesterolemia

This is a randomized, double-blind, placebo-controlled, parallel group study to evaluate the efficacy and safety of antibody 316P in patients with heterozygous familial hypercholesterolemia and LDL-C higher or equal to 160mg/dL with their lipid-modifying therapy

Therapeutic area: Nutritional and metabolic diseases, preferably: Hypercholesterolemia Primary objective: To evaluate the effect of antibody 316P on low-density lipoprotein cholesterol (LDL-C) levels after 24 weeks of treatment in comparison with placebo.

Secondary objectives:
To evaluate the effect of antibody 316P in comparison with placebo on LDL-C at other time points
To evaluate the effects of antibody 316P on other lipid parameters
To evaluate the safety and tolerability of antibody 316P

### Study design: This is a randomized, parallel, double blind phase 3 study.

Study arms:
First arm (experimental): Injection of antibody 316P through subcutaneous (SC) administration. Background statin therapy or other lipid lowering therapy is administered according to site investigator discretion as background therapy.
Second arm (Placebo Comparator): injection of placebo through subcutaneous (SC) administration. Background statin therapy or other lipid lowering therapy will be administered according to site investigator discretion as background therapy.

### Study population: Minimum age of 18 years without upper age limit.

Inclusion criteria: Patients with heterozygous familial hypercholesterolemia who are not adequately controlled with their lipid-modifying therapy.

Exclusion criteria:
Age < 18 years
LDL-C < 160 mg/dL (< 4.14 mmol/L) at the screening visit (Week-2).
Fasting serum triglycerides > 400 mg/dL (> 4.52 mmol/L) during the screening period.
Known history of homozygous familial hypercholesterolemia.

### Specific vulnerable population: Women of child-bearing potential using contraception.

Number of subjects/patients: ∼200 (∼180 adults with age of 18-64 years and ∼20 elderly with age of 65 years or more).

Investigational medicinal product is antibody 316P as solution for injection with subcutaneous administration, the placebo is equally formulated as for injection with subcutaneous administration.

### Active compounds: Antibody 316P

Antibody 316P is a fully human antibody comprising a HCVR as shown in SEQ ID NO: 90 and LCVR as shown in SEQ ID NO: 92 of the sequence listing. The CDR sequences are shown in SEQ ID NOs: 76, 78, and 80 (CDR1, CDR2, CDR3 of the heavy chain) as well as in SEQ ID NOs: 84, 86, and 88 (CDR1, CDR2, CDR3 of the light chain).

Primary endpoint: percent change in LDL-C from baseline to week 24.

Secondary endpoints: Percent change in LDL-C from baseline up to week 78 and percent change in other lipid parameters from baseline up to week 78.

Duration of study per subject/patient: The maximum study duration will be 88 weeks per patient, including a 78-week randomized treatment period.

## Claims

1. A method for treating a disease or condition in which PCSK9 expression or activity causes an impact comprising administering a therapeutic amount of an antibody or an antigen-binding fragment thereof which specifically binds hPCSK9 (human proprotein convertase subtilisin/kexin type 9) to a subject in need thereof, wherein the subject in need thereof is a subject
a) having mild and/or moderate hepatic impairment or a subject with normal hepatic liver function and/or
b) a subject with a high cardiovascular risk and/or
c) a subject with coronary heart disease.

2. The method according to claim 1, wherein the subject in need thereof does not fall into one or more of the following groups of subjects:
(i) smokers;
(ii) persons being 76 years old or older;
(iii) persons being 17 years or younger;
(iv) persons suffering from hypertension;
(v) women who are pregnant;
(vi) women who are trying to become pregnant;
(vii) women who are breast-feeding;
(viii) persons who have or ever had severe hepatic impairment
(ix) persons who had any unexplained abnormal results from blood tests for liver function;
(x) persons who drink excessive amounts of alcohol;
(xi) persons having kidney problems;
(xii) persons suffering from hypothyroidism;
(xiii) persons suffering from muscle disorders;
(xiv) persons having encountered previous muscular problems during treatment with lipid-lowering medicine;
(xv) persons having serious problems with their breathing;
(xvi) persons taking one or more of the following medicines: medicines altering the way the immune systems works (e.g. ciclosporin or antihistamines), antibiotics or antifungal medicines (e.g. erythromycin, clarithromycin, ketoconazole, itraconazole, rifampicin, fusidic acid), medicines regulating lipid levels (e.g. gemfibrozil, colestipol), calcium channel blockers (e.g. verapamil, diltiazem), medicines regulating the heart rhythm (digoxin, amiodarone), protease inhibitors used in the treatment of HIV (e.g. nelfinavir), warfarin, oral contraceptives, antacids or St. John's Wort; or
(xvii) persons drinking more than 0.1 L of grapefruit juice per day;
(xviii) persons having a body mass index (BMI) of more than 40;
(xix) persons having a body mass index (BMI) of less than 18;
(xx) persons suffering from type 1 diabetes or type 2 diabetes;
(xxi) persons positive for hepatitis B or hepatitis C;
(xxii) persons having a known sensitivity to monoclonal antibody therapeutics
(xxiii) persons having acute hepatitis,
(xxiv) persons having hepatic encephalopathy grade 2, 3, and 4,
(xxv) persons having uncontrolled clinically relevant cardiovascular, pulmonary, gastrointestinal, metabolic, hematological, neurological, psychiatric, systemic, ocular, gynecologic (if female), or infectious disease, or signs of acute illness (xxvi) persons having history or presence of clinically relevant cardiovascular, pulmonary, gastrointestinal, hepatic, renal, metabolic, hematological, neurological, osteomuscular, articular, psychiatric, systemic, ocular, gynecologic (if female), or infectious disease, or signs of acute illness.

3. A method of lowering cholesterol levels in a subject in need thereof, comprising:
(a) selecting a subject with a blood low density lipoprotein cholesterol (LDL-C) level greater than 100 mg/dL; and
(b) administering to said subject a composition comprising an antibody or antigen binding fragment thereof that specifically binds to human proprotein convertase subtilisin/kexin type 9 (hPCSK9); thereby lowering cholesterol levels in the subject in need thereof, wherein the subject falls into one or more of the groups of subjects as listed in claim 1 and wherein the subject preferably does not fall into one or more of the groups of subjects as listed in claim 2.

4. A method of regulating the LDL level in the blood of a subject comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in claim 1 and wherein the subject preferably does not fall into one or more of the groups of subjects as listed in claim 2.

5. A method of preventing effects of a (persistently) increased LDL level in the blood comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg, wherein the subject falls into one or more of the groups of subjects as listed in claim 1 and wherein the subject preferably does not fall into one or more of the groups of subjects as listed in claim 2.

6. An antibody or an antigen-binding fragment thereof which specifically binds hPCSK9 (human proprotein convertase subtilisin/kexin type 9) for use in the treatment of a disease or condition in which PCSK9 expression or activity causes an impact, wherein the antibody or antigen-binding fragment thereof is for administration to a subject falling at least into one of the groups of subjects as listed in claim 1 and wherein the antibody or antigen-binding fragment thereof preferably is not for administration to a subject falling at least into one of the groups of subjects as listed in claim 2.

7. The method according to one of the claims 1 to 5 or the antibody of claim 6, wherein the disease or condition in which PCSK9 expression or activity causes an impact is ameliorated, improved, inhibited or prevented with a PCSK9 antagonist.

8. The method according to one of the claims 1-5 or 7, comprising:
- administering a therapeutic amount of an antibody or an antigen-binding fragment thereof which specifically binds hPCSK9 (human proprotein convertase subtilisin/kexin type 9) to a subject in need thereof, wherein the antibody or antigen-binding fragment thereof is administered in a dosage amount ranging from 5 mg to 500 mg, and
- administering a therapeutic amount of an HMG-CoA reductase inhibitor to said subject, wherein the HMG-CoA reductase inhibitor is administered in a dosage amount ranging from 0.05 mg to 100 mg and/or
- administering a therapeutic amount of another lipid lowering drug to said subject, wherein the other lipid lowering drug is preferably ezetimibe.

9. The antibody or an antigen-binding fragment thereof according to claim 6 or7, wherein the antibody or antigen-binding fragment thereof is for administration in a dosage amount ranging from 5 mg to 500 mg, wherein the antibody or antigen-binding fragment thereof is further for administration in combination with an HMG-CoA reductase inhibitor at a dosage amount ranging from 0.05 mg to 100 mg.

10. The method or the antibody of any one of claims 1 to 9, wherein the disease or condition in which PCSK9 expression or activity causes an impact is selected from the group consisting of: hypercholesterolemia, hyperlipidemia, dyslipidemia, atherosclerosis and cardiovascular diseases.

11. The method or the antibody of any one of claims 1 to 10 wherein the antibody or the antigen-binding fragment thereof comprises
- a heavy chain CDR3 (HCDR3) domain selected from the group consisting of SEQ ID NO:8, 32, 56, 80, 104, 128, 152, 176, 200, 224, 248, 272, 296, 320, 344, 368, 392, 416, 440, 464, 488, 512, 536, 560, 584, 608, 632, 656, 680, 704 and 728; and
- a light chain CDR3 (LCDR3) domain selected from the group consisting of SEQ ID N0:16, 40, 64, 88, 112, 136, 160, 184, 208, 232, 256, 280, 304, 328, 352, 376, 400, 424, 448, 472, 496, 520, 544, 568, 592, 616, 639, 664, 688, 712 and 736 or
- the heavy and light chain CDRs of a HCVR/LCVR amino acid sequence pair as shown in SEQ ID NOs: 90/92
- or heavy and light chain CDR amino acid sequences as shown in SEQ ID NOs: 76, 78, 80, 84, 86, and 88
- or an HCVR amino acid sequence as shown in SEQ ID NO: 90 and an LCVR amino acid sequence as shown in SEQ ID NO: 92.

12. The method or the antibody of any one of claims 1 to 11, further comprising:
administering a therapeutic amount of an HMG-CoA reductase inhibitor to the subject in a dosage of between 0.05 mg to 100 mg and optionally comprising administration of another lipid lowering drug in an effective amount for decreasing LDL-C levels and/or
increasing HDL-C levels and/or decreasing trygliceride levels.

13. Pharmaceutical composition comprising an antibody or antigen-binding fragment thereof which specifically binds hPCSK9 and a pharmaceutically acceptable excipient for use in a method according to one of the claims 1 to 5 or 7 to 12.

14. Article of manufacture comprising
(a) a packaging material or container;
(b) an antibody or an antigen-binding fragment thereof which specifically binds hPCSK9; and
(c) a label or packaging insert, wherein the label or packaging insert indicates the following:
(1) the antibody or antigen-binding fragment thereof can be applied for treatment to subjects falling into one or more groups of subjects as recited in claim 1
(2) the label or packaging insert preferably also indicates that the antibody or antigen-binding fragment thereof is contraindicated for treatment of subjects and/or should not be applied to subjects falling into one or more groups of subjects as recited in claim 2.

15. A method for preparing an article of manufacture according to claim 14 comprising packaging the antibody , pharmaceutical composition, the liquid or dry formulation or the unit dosage form or forms according to one of the previous claims in a container and assembling them with a label or packaging insert.


SEQUENCE LISTING

<110> SANOFI

<120> HUMAN ANTIBODIES TO PCSK9 FOR USE IN METHODS OF TREATING PARTICULAR GROUPS
OF SUBJECTS

<130> DE2012-130

<160> 758

<170> FastSEQ for Windows Version 4.0

<210> 1
<211> 351
<212> DNA
<213> Artificial Sequence

<220>
<223> Synthetic

<400> 1
caggtccagc tggtgcagtc tgggggaggc ttggtacagc ctggggggtc cctgagactc 60
tcctgtgcag cctctggatt tactctaagt agttacgaca tgcactgggt ccgccaatct 120
acaggaaaag gtctggagtg ggtctcagct attggttcta ccggtgacac atactatcca 180
ggctccgtga agggccgatt caccatcacc agagaaaaag ccaagaactc cgtgtatctt 240
caaatgaaca gcctgagagc cggggacacg gctgtgtatt actgtgtaag agaggggtgg 300
gaggtaccct ttgactactg gggccaggga accctggtca ctgtctcctc a 351

<210> 2
<211> 117
<212> PRT
<213> Artificial Sequence

<220>
<223> Synthetic

<400> 2
Gln Val Gln Leu Val Gln Ser Gly Gly Gly Leu Val Gln Pro Gly Gly
 1               5                   10                  15
Ser Leu Arg Leu Ser Cys Ala Ala Ser Gly Phe Thr Leu Ser Ser Tyr
            20                  25                  30
Asp Met His Trp Val Arg Gln Ser Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Thr Arg Glu Lys Ala Lys Asn Ser Val Tyr Leu
65                  70                  75                  80
Gln Met Asn Ser Leu Arg Ala Gly Asp Thr Ala Val Tyr Tyr Cys Val
                85                  90                  95
Arg Glu Gly Trp Glu Val Pro Phe Asp Tyr Trp Gly Gln Gly Thr Leu
            100                 105                 110
Val Thr Val Ser Ser
        115

<210> 3
<211> 24
<212> DNA
<213> Artificial Sequence

<220>
<223> Synthetic

<400> 3
ggatttactc taagtagtta cgac                                        24

<210> 4
<211> 8
<212> PRT
<213> Artificial Sequence

<220>
<223> Synthetic

<400> 4
Gly Phe Thr Leu Ser Ser Tyr Asp
1               5


<210> 5
<211> 21
<212> DNA
<213> Artificial Sequence

<220>
<223> Synthetic

<400> 5
attggttcta ccggtgacac a                                                21

<210> 6
<211> 7
<212> PRT
<213> Artificial Sequence

<220>
<223> Synthetic

<400> 6
Ile Gly Ser Thr Gly Asp Thr
1               5


<210> 7
<211> 33
<212> DNA
<213> Artificial Sequence

<220>
<223> Synthetic

<400> 7
gtaagagagg ggtgggaggt accctttgac tac                                   33

<210> 8
<211> 11
<212> PRT
<213> Artificial Sequence

<220>
<223> Synthetic

<400> 8
Val Arg Glu Gly Trp Glu Val Pro Phe Asp Tyr
1               5                   10


<210> 9
<211> 327
<212> DNA
<213> Artificial Sequence

<220>
<223> Synthetic

<400> 9
gacatccaga tgacccagtc tccagccacc ctgtctgtgt ctccagggga aagagccgcc 60
ctctcctgca gggccagtca gagtgttagc agcaacttag cctggtacca ccagaaacct 120
ggccaggctc ccaggctcct catctatggt gcatccacca gggccactgg tatcccagcc 180
aggttcagtg gcattgggtc tgggacagag ttcactctca ttatcagcag cctgcagtct 240
gaagattttg cattttattt ctgtcagcag tataataact ggcctccatt cactttcggc 300
cctgggacca aggtggagat caaacga 327

<210> 10
<211> 109
<212> PRT
<213> Artificial Sequence

<220>
<223> Synthetic

<400> 10
Asp Ile Gln Met Thr Gln Ser Pro Ala Thr Leu Ser Val Ser Pro Gly
1               5                   10                  15
Glu Arg Ala Ala Leu Ser Cys Arg Ala Ser Gln Ser Val Ser Ser Asn
            20                  25                  30
Leu Ala Trp Tyr His Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ile Gly Ser Gly Thr Glu Phe Thr Leu Ile Ile Ser Ser Leu Gln Ser
65                  70                  75                  80
Glu Asp Phe Ala Phe Tyr Phe Cys Gln Gln Tyr Asn Asn Trp Pro Pro
                85                  90                  95
Phe Thr Phe Gly Pro Gly Thr Lys Val Glu Ile Lys Arg
            100                 105

<210> 11
<211> 18
<212> DNA
<213> Artificial Sequence

<220>
<223> Synthetic

<400> 11
cagagtgtta gcagcaac 18

<210> 12
<211> 6
<212> PRT
<213> Artificial Sequence

<220>
<223> Synthetic

<400> 12
Gln Ser Val Ser Ser Asn
1               5

<210> 13
<211> 9
<212> DNA
<213> Artificial Sequence

<220>
<223> Synthetic

<400> 13
ggtgcatcc 9

<210> 14
<211> 3
<212> PRT
<213> Artificial Sequence

<220>
<223> Synthetic

<400> 14
Gly Ala Ser
1

<210> 15
<211> 30
<212> DNA
<213> Artificial Sequence

<220>
<223> Synthetic

<400> 15
cagcagtata ataactggcc tccattcact                                        30

<210> 16
<211> 10
<212> PRT
<213> Artificial Sequence

<220>
<223> Synthetic

<400> 16
Gln Gln Tyr Asn Asn Trp Pro Pro Phe Thr
1               5                   10

<210> 17
<211> 351
<212> DNA
<213> Artificial Sequence

<220>
<223> Synthetic

<400> 17
gaggtgcagc tggtggagtc tgggggaggc ttggtacagc ctggggggtc cctgagactc 60
tcctgtgcag cctctggatt tactctaagt agttacgaca tgcactgggt ccgccaatct 120
acaggaaaag gtctggagtg ggtctcagct attggttcta ccggtgacac atactatcca 180
ggctccgtga agggccgatt caccatcacc agagaaaaag ccaagaactc cgtgtatctt 240
caaatgaaca gcctgagagc cggggacacg gctgtgtatt actgtgtaag agaggggtgg 300
gaggtaccct ttgactactg gggccaggga accctggtca ccgtctcctc a             351

<210> 18
<211> 117
<212> PRT
<213> Artificial Sequence

<220>
<223> Synthetic

<400> 18
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ser Thr Gly Lys Gly Leu Glu Trp Val
        35                  40                  45
Ser Ala Ile Gly Ser Thr Gly Asp Thr Tyr Tyr Pro Gly Ser Val Lys

50                  55                  60
Gly Arg Phe Thr Ile Thr Arg Glu Lys Ala Lys Asn Ser Val Tyr Leu
65                  70                  75                  80
Gln Met Asn Ser Leu Arg Ala Gly Asp Thr Ala Val Tyr Tyr Cys Val
                85                  90                  95
Arg Glu Gly Trp Glu Val Pro Phe Asp Tyr Trp Gly Gln Gly Thr Leu
            100                 105                 110
Val Thr Val Ser Ser
        115

<210> 19
<211> 324
<212> DNA
<213> Artificial Sequence

<220>
<223> Synthetic

<400> 19
gaaatagtga tgacgcagtc tccagccacc ctgtctgtgt ctccagggga aagagccgcc 60
ctctcctgca gggccagtca gagtgttagc agcaacttag cctggtacca ccagaaacct 120
ggccaggctc ccaggctcct catctatggt gcatccacca gggccactgg tatcccagcc 180
aggttcagtg gcattgggtc tgggacagag ttcactctca ttatcagcag cctgcagtct 240
gaagattttg catttatatt ctgtcagcag tataataact ggcctccatt cactttcggc 300
cctgggacca aagtggatat caaa                                         324

<210> 20
<211> 108
<212> PRT
<213> Artificial Sequence

<220>
<223> Synthetic

<400> 20
Glu Ile Val Met Thr Gln Ser Pro Ala Thr Leu Ser Val Ser Pro Gly
1               5                   10                  15
Glu Arg Ala Ala Leu Ser Cys Arg Ala Ser Gln Ser Val Ser Ser Asn
            20                  25                  30
Leu Ala Trp Tyr His Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ile Gly Ser Gly Thr Glu Phe Thr Leu Ile Ile Ser Ser Leu Gln Ser
65                  70                  75                  80
Glu Asp Phe Ala Phe Tyr Phe Cys Gln Gln Tyr Asn Asn Trp Pro Pro
                85                  90                  95
Phe Thr Phe Gly Pro Gly Thr Lys Val Asp Ile Lys
            100                 105

<210> 21
<211> 351
<212> DNA
<213> Artificial Sequence

<220>
<223> Synthetic

<400> 21
gaggtgcagc tggtggagtc tgggggaggc ttggtacagc ctggggggtc cctgagactc 60
tcctgtgcag cctctggatt tactctaagt agttacgaca tgcactgggt ccgccaagct 120
acaggaaaag gtctggagtg ggtctcagct attggttcta ccggtgacac atactatcca 180
ggctccgtga agggccgatt caccatctcc agagaaaatg ccaagaactc cttgtatctt 240
caaatgaaca gcctgagagc cggggacacg gctgtgtatt actgtgtaag agaggggtgg 300
gaggtaccct ttgactactg gggccaggga accctggtca ccgtctcctc a           351

<210> 22

<211> 117
<212> PRT
<213> Artificial Sequence

<220>
<223> Synthetic

<400> 22
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Val
                85                  90                  95
Arg Glu Gly Trp Glu Val Pro Phe Asp Tyr Trp Gly Gln Gly Thr Leu
            100                 105                 110
Val Thr Val Ser Ser
        115


<210> 23
<211> 324
<212> DNA
<213> Artificial Sequence

<220>
<223> Synthetic

<400> 23
gaaatagtga tgacgcagtc tccagccacc ctgtctgtgt ctccagggga aagagccacc 60
ctctcctgca gggccagtca gagtgttagc agcaacttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataact ggcctccatt cactttcggc 300
cctgggacca aagtggatat caaa                                        324

<210> 24
<211> 108
<212> PRT
<213> Artificial Sequence

<220>
<223> Synthetic

<400> 24
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105


<210> 25
<211> 342

<212> DNA
<213> Artificial Sequence

<220>
<223> Synthetic

<400> 25
caggtgcagc tggtgcagtc tgggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatacattat 180
ggagactccg tgaggggccg aatcatcata tccagagaca attccgagaa cacgttgtat 240
ctggaaatga acagcctgag agccgaggac acggcaatgt actattgtgc gagagagaag 300
ggtttagact ggggccaggg aaccacggtc accgtctcct ca 342

<210> 26
<211> 114
<212> PRT
<213> Artificial Sequence

<220>
<223> Synthetic

<400> 26
Gln Val Gln Leu Val Gln Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile His Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Met Tyr Tyr Cys
                85                  90                  95
Ala Arg Glu Lys Gly Leu Asp Trp Gly Gln Gly Thr Thr Val Thr Val
            100                 105                 110
Ser Ser

<210> 27
<211> 24
<212> DNA
<213> Artificial Sequence

<220>
<223> Synthetic

<400> 27
ggattcacct tcagtagcta tggc 24

<210> 28
<211> 8
<212> PRT
<213> Artificial Sequence

<220>
<223> Synthetic

<400> 28
Gly Phe Thr Phe Ser Ser Tyr Gly
1               5

<210> 29
<211> 24
<212> DNA
<213> Artificial Sequence

<220>
<223> Synthetic

<400> 29
ataggatttg atggaagtaa tata                                              24

<210> 30
<211> 8
<212> PRT
<213> Artificial Sequence

<220>
<223> Synthetic

<400> 30
Ile Gly Phe Asp Gly Ser Asn Ile
1               5


<210> 31
<211> 21
<212> DNA
<213> Artificial Sequence

<220>
<223> Synthetic

<400> 31
gcgagagaga agggtttaga c                                                 21

<210> 32
<211> 7
<212> PRT
<213> Artificial Sequence

<220>
<223> Synthetic

<400> 32
Ala Arg Glu Lys Gly Leu Asp
1               5


<210> 33
<211> 321
<212> DNA
<213> Artificial Sequence

<220>
<223> Synthetic

<400> 33
gccatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300
accaaggtgg aaatcaaacg a                                                 321

<210> 34
<211> 107
<212> PRT
<213> Artificial Sequence

<220>
<223> Synthetic

<400> 34

Ala Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Tyr Thr
                85                  90                  95
Phe Gly Gln Gly Thr Lys Val Glu Ile Lys Arg
            100                 105

<210> 35
<211> 18
<212> DNA
<213> Artificial Sequence

<220>
<223> Synthetic

<400> 35
cagagtatta gtagctgg                                                    18

<210> 36
<211> 6
<212> PRT
<213> Artificial Sequence

<220>
<223> Synthetic

<400> 36
Gln Ser Ile Ser Ser Trp
1               5

<210> 37
<211> 9
<212> DNA
<213> Artificial Sequence

<220>
<223> Synthetic

<400> 37
aaggcgtct                                                              9

<210> 38
<211> 3
<212> PRT
<213> Artificial Sequence

<220>
<223> Synthetic

<400> 38
Lys Ala Ser
1

<210> 39
<211> 24
<212> DNA
<213> Artificial Sequence

<220>
<223> Synthetic

<400> 39
caacagtata atagttatta cact                                              24

<210> 40
<211> 8
<212> PRT
<213> Artificial Sequence

<220>
<223> Synthetic

<400> 40
Gln Gln Tyr Asn Ser Tyr Tyr Thr
1               5

<210> 41
<211> 342
<212> DNA
<213> Artificial Sequence

<220>
<223> Synthetic

<400> 41
   caggtgcagc tggtggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcgttt ataggatttg atggaagtaa tatacattat 180
   ggagactccg tgaggggccg aatcatcata tccagagaca attccgagaa cacgttgtat 240
   ctggaaatga acagcctgag agccgaggac acggcaatgt actattgtgc gagagagaag 300
   ggtttagact ggggccaggg aaccctggtc accgtctcct ca                        342

<210> 42
<211> 114
<212> PRT
<213> Artificial Sequence

<220>
<223> Synthetic

<400> 42
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile His Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Met Tyr Tyr Cys
                85                  90                  95
Ala Arg Glu Lys Gly Leu Asp Trp Gly Gln Gly Thr Leu Val Thr Val
            100                 105                 110
Ser Ser

<210> 43
<211> 318
<212> DNA
<213> Artificial Sequence

<220>
<223> Synthetic

<400> 43
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300
accaagctgg agatcaaa 318

<210> 44
<211> 106
<212> PRT
<213> Artificial Sequence

<220>
<223> Synthetic

<400> 44
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Tyr Thr
                85                  90                  95
Phe Gly Gln Gly Thr Lys Leu Glu Ile Lys
            100                 105

<210> 45
<211> 342
<212> DNA
<213> Artificial Sequence

<220>
<223> Synthetic

<400> 45
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt ataggatttg atggaagtaa tatatactat 180
gcagactccg tgaagggccg attcaccatc tccagagaca attccaagaa cacgctgtat 240
ctgcaaatga acagcctgag agctgaggac acggctgtgt attactgtgc gagagagaag 300
ggtttagact ggggccaggg aaccctggtc accgtctcct ca 342

<210> 46
<211> 114
<212> PRT
<213> Artificial Sequence

<220>
<223> Synthetic

<400> 46
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Gly Phe Asp Gly Ser Asn Ile Tyr Tyr Ala Asp Ser Val
    50                  55                  60
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
Ala Arg Glu Lys Gly Leu Asp Trp Gly Gln Gly Thr Leu Val Thr Val
            100                 105                 110
Ser Ser

<210> 47
<211> 319
<212> DNA
<213> Artificial Sequence

<220>
<223> Synthetic

<400> 47
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attacacttt tggccagggg 300
accaagctgg agatcaaac                                               319

<210> 48
<211> 106
<212> PRT
<213> Artificial Sequence

<220>
<223> Synthetic

<400> 48
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Tyr Thr
                85                  90                  95
Phe Gly Gln Gly Thr Lys Leu Glu Ile Lys
            100                 105

<210> 49
<211> 342
<212> DNA
<213> Artificial Sequence

<220>
<223> Synthetic

<400> 49
caggtgcagc tgcaggagtc tgggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatatattat 180
ggagactccg tgaggggccg aatcatcata tccagagaca attccgagaa cacgttgtat 240
ctggaaatga acagcctgag agccgaggac acggcagtgt attattgtgc gagagagaag 300
ggtttagact ggggccaggg aaccctggtc actgtctcct ca                     342

<210> 50
<211> 114
<212> PRT
<213> Artificial Sequence

<220>
<223> Synthetic

<400> 50
Gln Val Gln Leu Gln Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile Tyr Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Val Tyr Tyr Cys
                85                  90                  95
Ala Arg Glu Lys Gly Leu Asp Trp Gly Gln Gly Thr Leu Val Thr Val
            100                 105                 110
Ser Ser

<210> 51
<211> 24
<212> DNA
<213> Artificial Sequence

<220>
<223> Synthetic

<400> 51
ggattcacct tcagtagcta tggc                                          24

<210> 52
<211> 8
<212> PRT
<213> Artificial Sequence

<220>
<223> Synthetic

<400> 52
Gly Phe Thr Phe Ser Ser Tyr Gly
1               5

<210> 53
<211> 24
<212> DNA
<213> Artificial Sequence

<220>
<223> Synthetic

<400> 53
ataggatttg atggaagtaa tata                                          24

<210> 54
<211> 8
<212> PRT
<213> Artificial Sequence

<220>
<223> Synthetic

<400> 54
Ile Gly Phe Asp Gly Ser Asn Ile
1               5

<210> 55
<211> 21
<212> DNA
<213> Artificial Sequence

<220>
<223> Synthetic

<400> 55
gcgagagaga agggtttaga c                                                21

<210> 56
<211> 7
<212> PRT
<213> Artificial Sequence

<220>
<223> Synthetic

<400> 56
Ala Arg Glu Lys Gly Leu Asp
1               5

<210> 57
<211> 342
<212> DNA
<213> Artificial Sequence

<220>
<223> Synthetic

<400> 57
gccatccaga tgacccagtc tccagactcc ctggctgtgt ctctgggcga gagggccacc 60
atcaactgca agtccagcca gagtgttttt cacacctcca acaataagaa ctacttagtt 120
tggtatcagc agaaaccagg acagcctcct aagttgctcc tttactgggc ctctacccgg 180
gaatccgggg tccctgaccg attcagtggc agcgggtctg ggacagattt cactctcacc 240
atcagcagcc tgcaggctga agatgtggca aattattact gtcaccaata ttacagtatt 300
ccgtggacgt tcggccaagg gaccaaggtg gagatcaaac ga                         342

<210> 58
<211> 114
<212> PRT
<213> Artificial Sequence

<220>
<223> Synthetic

<400> 58
Ala Ile Gln Met Thr Gln Ser Pro Asp Ser Leu Ala Val Ser Leu Gly
1               5                   10                  15
Glu Arg Ala Thr Ile Asn Cys Lys Ser Ser Gln Ser Val Phe His Thr
            20                  25                  30
Ser Asn Asn Lys Asn Tyr Leu Val Trp Tyr Gln Gln Lys Pro Gly Gln
        35                  40                  45
Pro Pro Lys Leu Leu Leu Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Asn Tyr Tyr Cys His Gln
                85                  90                  95
Tyr Tyr Ser Ile Pro Trp Thr Phe Gly Gln Gly Thr Lys Val Glu Ile
            100                 105                 110
Lys Arg

<210> 59
<211> 36
<212> DNA
<213> Artificial Sequence

<220>
<223> Synthetic

<400> 59
cagagtgttt ttcacacctc caacaataag aactac                                36

<210> 60
<211> 12
<212> PRT
<213> Artificial Sequence

<220>
<223> Synthetic

<400> 60
Gln Ser Val Phe His Thr Ser Asn Asn Lys Asn Tyr
1               5                   10

<210> 61
<211> 9
<212> DNA
<213> Artificial Sequence

<220>
<223> Synthetic

<400> 61
tgggcctct                                                              9

<210> 62
<211> 3
<212> PRT
<213> Artificial Sequence

<220>
<223> Synthetic

<400> 62
Trp Ala Ser
1

<210> 63
<211> 27
<212> DNA
<213> Artificial Sequence

<220>
<223> Synthetic

<400> 63
caccaatatt acagtattcc gtggacg                                          27

<210> 64
<211> 9
<212> PRT
<213> Artificial Sequence

<220>
<223> Synthetic

<400> 64
His Gln Tyr Tyr Ser Ile Pro Trp Thr

1                   5


<210> 65
<211> 342
<212> DNA
<213> Artificial Sequence

<220>
<223> Synthetic

<400> 65
caggtgcagc tggtggagtc tgggggaggc gtggtccagc ctgggaggtc cctgagactc 60
tcctgtgcag cgtctggatt caccttcagt agctatggca tgcactgggt ccgccaggct 120
ccaggcaagg ggctggagtg ggtggcgttt ataggatttg atggaagtaa tatatattat 180
ggagactccg tgaggggccg aatcatcata tccagagaca attccgagaa cacgttgtat 240
ctggaaatga acagcctgag agccgaggac acggcagtgt attattgtgc gagagagaag 300
ggtttagact ggggccaggg aaccctggtc accgtctcct ca                    342

<210> 66
<211> 114
<212> PRT
<213> Artificial Sequence

<220>
<223> Synthetic

<400> 66
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Phe Ile Gly Phe Asp Gly Ser Asn Ile Tyr Tyr Gly Asp Ser Val
    50                  55                  60
Arg Gly Arg Ile Ile Ile Ser Arg Asp Asn Ser Glu Asn Thr Leu Tyr
65                  70                  75                  80
Leu Glu Met Asn Ser Leu Arg Ala Glu Asp Thr Ala Val Tyr Tyr Cys
                85                  90                  95
Ala Arg Glu Lys Gly Leu Asp Trp Gly Gln Gly Thr Leu Val Thr Val
            100                 105                 110
Ser Ser



<210> 67
<211> 339
<212> DNA
<213> Artificial Sequence

<220>
<223> Synthetic

<400> 67
gacatcgtga tgacccagtc tccagactcc ctggctgtgt ctctgggcga gagggccacc 60
atcaactgca agtccagcca gagtgttttt cacacctcca acaataagaa ctacttagtt 120
tggtatcagc agaaaccagg acagcctcct aagttgctcc tttactgggc ctctacccgg 180
gaatccgggg tccctgaccg attcagtggc agcgggtctg ggacagattt cactctcacc 240
atcagcagcc tgcaggctga agatgtggca atttattact gtcaccaata ttacagtatt 300
ccgtggacgt tcggccaagg gaccaaggtg gaaatcaaa                        339

<210> 68
<211> 113
<212> PRT
<213> Artificial Sequence

<220>
<223> Synthetic

<400> 68
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Phe His Thr
            20                  25                  30
Ser Asn Asn Lys Asn Tyr Leu Val Trp Tyr Gln Gln Lys Pro Gly Gln
        35                  40                  45
Pro Pro Lys Leu Leu Leu Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Asn Tyr Tyr Cys His Gln
                85                  90                  95
Tyr Tyr Ser Ile Pro Trp Thr Phe Gly Gln Gly Thr Lys Val Glu Ile
            100                 105                 110
Lys

<210> 69
<211> 342
<212> DNA
<213> Artificial Sequence

<220>
<223> Synthetic

<400> 69
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt ataggatttg atggaagtaa tatatactat 180
gcagactccg tgaagggccg attcaccatc tccagagaca attccaagaa cacgctgtat 240
ctgcaaatga acagcctgag agctgaggac acggctgtgt attactgtgc gagagagaag 300
ggtttagact ggggccaggg aaccctggtc accgtctcct ca                    342

<210> 70
<211> 114
<212> PRT
<213> Artificial Sequence

<220>
<223> Synthetic

<400> 70
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Gly Phe Asp Gly Ser Asn Ile Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Glu Lys Gly Leu Asp Trp Gly Gln Gly Thr Leu Val Thr Val
            100                 105                 110
Ser Ser

<210> 71
<211> 339
<212> DNA
<213> Artificial Sequence

<220>

<223> Synthetic

<400> 71
gacatcgtga tgacccagtc tccagactcc ctggctgtgt ctctgggcga gagggccacc 60
atcaactgca agtccagcca gagtgttttt cacacctcca acaataagaa ctacttagct 120
tggtaccagc agaaaccagg acagcctcct aagctgctca tttactgggc ctctacccgg 180
gaatccgggg tccctgaccg attcagtggc agcgggtctg ggacagattt cactctcacc 240
atcagcagcc tgcaggctga agatgtggca gtttattact gtcaccaata ttacagtatt 300
ccgtggacgt tcggccaagg gaccaaggtg gaaatcaaa 339

<210> 72
<211> 113
<212> PRT
<213> Artificial Sequence

<220>
<223> Synthetic

<400> 72
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Phe His Thr
            20                  25                  30
Ser Asn Asn Lys Asn Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys His Gln
                85                  90                  95
Tyr Tyr Ser Ile Pro Trp Thr Phe Gly Gln Gly Thr Lys Val Glu Ile
            100                 105                 110
Lys

<210> 73
<211> 354
<212> DNA
<213> Artificial Sequence

<220>
<223> Synthetic

<400> 73
gaagtgcagc tggtgcagtc tgggggaggc ttggtacagc ctggggggtc cctgagactc 60
tcctgtgcag cctctggatt cacctttaac aactatgcca tgaactgggt ccgccaggct 120
ccagggaagg gactggactg ggtctcaact attagtggta gcggtggtac tacaaactac 180
gcagactccg tgaagggccg tttcattatt tcccgagaca gttccaaaca cacgctgtat 240
ctgcaaatga acagcctgag agccgaggac acggccgtat attactgtgc gaaagattct 300
aactggggaa atttcgatct ctggggccgt ggcaccacgg tcactgtctc ctca 354

<210> 74
<211> 118
<212> PRT
<213> Artificial Sequence

<220>
<223> Synthetic

<400> 74
Glu Val Gln Leu Val Gln Ser Gly Gly Gly Leu Val Gln Pro Gly Gly
1               5                   10                  15
Ser Leu Arg Leu Ser Cys Ala Ala Ser Gly Phe Thr Phe Asn Asn Tyr
            20                  25                  30
Ala Met Asn Trp Val Arg Gln Ala Pro Gly Lys Gly Leu Asp Trp Val
        35                  40                  45
Ser Thr Ile Ser Gly Ser Gly Gly Thr Thr Asn Tyr Ala Asp Ser Val

50                  55                  60
Lys Gly Arg Phe Ile Ile Ser Arg Asp Ser Ser Lys His Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Ser Asn Trp Gly Asn Phe Asp Leu Trp Gly Arg Gly Thr
            100                 105                 110
Thr Val Thr Val Ser Ser
        115

<210> 75
<211> 24
<212> DNA
<213> Artificial Sequence

<220>
<223> Synthetic

<400> 75
ggattcacct ttaacaacta tgcc                                              24

<210> 76
<211> 8
<212> PRT
<213> Artificial Sequence

<220>
<223> Synthetic

<400> 76
Gly Phe Thr Phe Asn Asn Tyr Ala
1               5

<210> 77
<211> 24
<212> DNA
<213> Artificial Sequence

<220>
<223> Synthetic

<400> 77
attagtggta gcggtggtac taca                                              24

<210> 78
<211> 8
<212> PRT
<213> Artificial Sequence

<220>
<223> Synthetic

<400> 78
Ile Ser Gly Ser Gly Gly Thr Thr
1               5

<210> 79
<211> 33
<212> DNA
<213> Artificial Sequence

<220>
<223> Synthetic

<400> 79
gcgaaagatt ctaactgggg aaatttcgat ctc                                    33

<210> 80
<211> 11
<212> PRT
<213> Artificial Sequence

<220>
<223> Synthetic

<400> 80
Ala Lys Asp Ser Asn Trp Gly Asn Phe Asp Leu
1               5                   10

<210> 81
<211> 342
<212> DNA
<213> Artificial Sequence

<220>
<223> Synthetic

<400> 81
gacatccaga tgacccagtc tccagactcc ctggctgtgt ctctgggcga gagggccacc 60
atcaactgca agtccagcca gagtgtttta tacaggtcca acaataggaa cttcttaggt 120
tggtaccagc agaaaccagg gcagcctcct aatctactca tttactgggc atctacccgg 180
gaatccgggg tccctgaccg attcagtggc agcgggtctg ggacagattt cactctcacc 240
atcagcagcc tgcaggctga agatgtggca gtttattact gtcaacaata ttatactact 300
ccgtacactt ttggccaggg gaccaaggtg gaaatcaaac ga                    342

<210> 82
<211> 114
<212> PRT
<213> Artificial Sequence

<220>
<223> Synthetic

<400> 82
Asp Ile Gln Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Arg
            20                  25                  30
Ser Asn Asn Arg Asn Phe Leu Gly Trp Tyr Gln Gln Lys Pro Gly Gln
        35                  40                  45
Pro Pro Asn Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Thr Thr Pro Tyr Thr Phe Gly Gln Gly Thr Lys Val Glu Ile
            100                 105                 110
Lys Arg

<210> 83
<211> 36
<212> DNA
<213> Artificial Sequence

<220>
<223> Synthetic

<400> 83
cagagtgttt tatacaggtc caacaatagg aacttc                           36

<210> 84

<211> 12
<212> PRT
<213> Artificial Sequence

<220>
<223> Synthetic

<400> 84
Gln Ser Val Leu Tyr Arg Ser Asn Asn Arg Asn Phe
1               5                   10


<210> 85
<211> 9
<212> DNA
<213> Artificial Sequence

<220>
<223> Synthetic

<400> 85
tgggcatct                                                               9

<210> 86
<211> 3
<212> PRT
<213> Artificial Sequence

<220>
<223> Synthetic

<400> 86
Trp Ala Ser
1


<210> 87
<211> 27
<212> DNA
<213> Artificial Sequence

<220>
<223> Synthetic

<400> 87
caacaatatt atactactcc gtacact                                          27

<210> 88
<211> 9
<212> PRT
<213> Artificial Sequence

<220>
<223> Synthetic

<400> 88
Gln Gln Tyr Tyr Thr Thr Pro Tyr Thr
1               5


<210> 89
<211> 354
<212> DNA
<213> Artificial Sequence

<220>
<223> Synthetic

<400> 89

gaggtgcagc tggtggagtc tgggggaggc ttggtacagc ctggggggtc cctgagactc 60
tcctgtgcag cctctggatt cacctttaac aactatgcca tgaactgggt ccgccaggct 120
ccaggaaagg gactggactg ggtctcaact attagtggta gcggtggtac tacaaactac 180
gcagactccg tgaagggccg tttcattatt tcccgagaca gttccaaaca cacgctgtat 240
ctgcaaatga acagcctgag agccgaggac acggccgtat attactgtgc gaaagattct 300
aactggggaa atttcgatct ctggggccgt ggcaccctgg tcactgtctc ctca 354

<210> 90
<211> 118
<212> PRT
<213> Artificial Sequence

<220>
<223> Synthetic

<400> 90

<210> 91
<211> 339
<212> DNA
<213> Artificial Sequence

<220>
<223> Synthetic

<400> 91
gacatcgtga tgacccagtc tccagactcc ctggctgtgt ctctgggcga gagggccacc 60
atcaactgca agtccagcca gagtgtttta tacaggtcca acaataggaa cttcttaggt 120
tggtaccagc agaaaccagg gcagcctcct aatctactca tttactgggc atctacccgg 180
gaatccgggg tccctgaccg attcagtggc agcgggtctg ggacagattt cactctcacc 240
atcagcagcc tgcaggctga agatgtggca gtttattact gtcaacaata ttatactact 300
ccgtacactt ttggccaggg gaccaagctg gagatcaaa 339

<210> 92
<211> 113
<212> PRT
<213> Artificial Sequence

<220>
<223> Synthetic

<400> 92

<210> 93
<211> 354
<212> DNA
<213> Artificial Sequence

<220>
<223> Synthetic

<400> 93
gaggtgcagc tggtggagtc tgggggaggc ttggtacagc ctggggggtc cctgagactc 60
tcctgtgcag cctctggatt cacctttaac aactatgcca tgagctgggt ccgccaggct 120
ccagggaagg ggctggagtg ggtctcagct attagtggta gcggtggtac tacatactac 180
gcagactccg tgaagggccg gttcaccatc tccagagaca attccaagaa cacgctgtat 240
ctgcaaatga acagcctgag agccgaggac acggccgtat attactgtgc gaaagattct 300
aactggggaa atttcgatct ctggggccgt ggcaccctgg tcactgtctc ctca       354

<210> 94
<211> 118
<212> PRT
<213> Artificial Sequence

<220>
<223> Synthetic

<400> 94
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Asn Asn Tyr
            20                  25                  30
Ala Met Ser Trp Val Arg Gln Ala Pro Gly Lys Gly Leu Glu Trp Val
        35                  40                  45
Ser Ala Ile Ser Gly Ser Gly Gly Thr Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Ser Asn Trp Gly Asn Phe Asp Leu Trp Gly Arg Gly Thr
            100                 105                 110
Leu Val Thr Val Ser Ser
        115

<210> 95
<211> 339
<212> DNA
<213> Artificial Sequence

<220>
<223> Synthetic

<400> 95
gacatcgtga tgacccagtc tccagactcc ctggctgtgt ctctgggcga gagggccacc 60
atcaactgca agtccagcca gagtgtttta tacaggtcca acaataggaa cttcttagct 120
tggtaccagc agaaaccagg acagcctcct aagctgctca tttactgggc atctacccgg 180
gaatccgggg tccctgaccg attcagtggc agcgggtctg ggacagattt cactctcacc 240
atcagcagcc tgcaggctga agatgtggca gtttattact gtcaacaata ttatactact 300
ccgtacactt ttggccaggg gaccaagctg gagatcaaa                        339

<210> 96
<211> 113

<212> PRT
<213> Artificial Sequence

<220>
<223> Synthetic

<400> 96
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Arg
            20                  25                  30
Ser Asn Asn Arg Asn Phe Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Thr Thr Pro Tyr Thr Phe Gly Gln Gly Thr Lys Leu Glu Ile
            100                 105                 110
Lys

<210> 97
<211> 351
<212> DNA
<213> Artificial Sequence

<220>
<223> Synthetic

<400> 97
caggtgcagc tggtgcagtc tgggggaggc ttggtacagc ctggggggtc cctgagactc 60
tcctgtgcag tctctggatt caccctcagt agctacgata tgcactgggt ccgccaacct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
gacgtaccct ttgacttctg gggccaggga accctggtca ccgtctcctc a          351

<210> 98
<211> 117
<212> PRT
<213> Artificial Sequence

<220>
<223> Synthetic

<400> 98
Gln Val Gln Leu Val Gln Ser Gly Gly Gly Leu Val Gln Pro Gly Gly
1               5                   10                  15
Ser Leu Arg Leu Ser Cys Ala Val Ser Gly Phe Thr Leu Ser Ser Tyr
            20                  25                  30
Asp Met His Trp Val Arg Gln Pro Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Trp Asp Val Pro Phe Asp Phe Trp Gly Gln Gly Thr Leu
            100                 105                 110
Val Thr Val Ser Ser
        115

<210> 99

<211> 24
<212> DNA
<213> Artificial Sequence

<220>
<223> Synthetic

<400> 99
ggattcaccc tcagtagcta cgat                                              24

<210> 100
<211> 8
<212> PRT
<213> Artificial Sequence

<220>
<223> Synthetic

<400> 100
Gly Phe Thr Leu Ser Ser Tyr Asp
1               5


<210> 101
<211> 21
<212> DNA
<213> Artificial Sequence

<220>
<223> Synthetic

<400> 101
attggttcta ctggtgacac a                                                 21

<210> 102
<211> 7
<212> PRT
<213> Artificial Sequence

<220>
<223> Synthetic

<400> 102
Ile Gly Ser Thr Gly Asp Thr
1               5


<210> 103
<211> 33
<212> DNA
<213> Artificial Sequence

<220>
<223> Synthetic

<400> 103
gcaagagagg gatgggacgt accctttgac ttc                                    33

<210> 104
<211> 11
<212> PRT
<213> Artificial Sequence

<220>
<223> Synthetic

<400> 104
Ala Arg Glu Gly Trp Asp Val Pro Phe Asp Phe
1               5                   10

<210> 105
<211> 324
<212> DNA
<213> Artificial Sequence

<220>
<223> Synthetic

<400> 105
gccatccagt tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
cggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggagatcaa acga 324

<210> 106
<211> 108
<212> PRT
<213> Artificial Sequence

<220>
<223> Synthetic

<400> 106
Ala Ile Gln Leu Thr Gln Ser Pro Ser Ser Leu Ser Ala Ser Val Gly
1               5                   10                  15
Asp Arg Val Thr Ile Thr Cys Arg Ala Ser Gln Asp Ile Arg Asn Asp
            20                  25                  30
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Asp Tyr Asn Tyr Pro Trp
                85                  90                  95
Thr Phe Gly Gln Gly Thr Lys Val Glu Ile Lys Arg
            100                 105

<210> 107
<211> 18
<212> DNA
<213> Artificial Sequence

<220>
<223> Synthetic

<400> 107
caggacatta gaaatgat 18

<210> 108
<211> 6
<212> PRT
<213> Artificial Sequence

<220>
<223> Synthetic

<400> 108
Gln Asp Ile Arg Asn Asp
1               5

<210> 109
<211> 9

<212> DNA
<213> Artificial Sequence

<220>
<223> Synthetic

<400> 109
gctgcatcc                                                              9

<210> 110
<211> 3
<212> PRT
<213> Artificial Sequence

<220>
<223> Synthetic

<400> 110
Ala Ala Ser
1


<210> 111
<211> 27
<212> DNA
<213> Artificial Sequence

<220>
<223> Synthetic

<400> 111
ctacaagatt acaattaccc gtggacg                                          27

<210> 112
<211> 9
<212> PRT
<213> Artificial Sequence

<220>
<223> Synthetic

<400> 112
Leu Gln Asp Tyr Asn Tyr Pro Trp Thr
1               5


<210> 113
<211> 351
<212> DNA
<213> Artificial Sequence

<220>
<223> Synthetic

<400> 113
gaggtgcagc tggtggagtc tgggggaggc ttggtacagc ctggggggtc cctgagactc 60
tcctgtgcag tctctggatt caccctcagt agctacgata tgcactgggt ccgccaacct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
gacgtaccct ttgacttctg gggccaggga accctggtca ccgtctcctc a              351

<210> 114
<211> 117
<212> PRT
<213> Artificial Sequence

<220>
<223> Synthetic

<400> 114
Glu Val Gln Leu Val Glu Ser Gly Gly Gly Leu Val Gln Pro Gly Gly
1               5                   10                  15
Ser Leu Arg Leu Ser Cys Ala Val Ser Gly Phe Thr Leu Ser Ser Tyr
            20                  25                  30
Asp Met His Trp Val Arg Gln Pro Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Trp Asp Val Pro Phe Asp Phe Trp Gly Gln Gly Thr Leu
            100                 105                 110
Val Thr Val Ser Ser
        115

<210> 115
<211> 321
<212> DNA
<213> Artificial Sequence

<220>
<223> Synthetic

<400> 115
gccatccaga tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
cggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggaaatcaa a                                           321

<210> 116
<211> 107
<212> PRT
<213> Artificial Sequence

<220>
<223> Synthetic

<400> 116
Ala Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Arg Asn Asp
            20                  25                  30
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Asp Tyr Asn Tyr Pro Trp
                85                  90                  95
Thr Phe Gly Gln Gly Thr Lys Val Glu Ile Lys
            100                 105

<210> 117
<211> 351
<212> DNA
<213> Artificial Sequence

<220>
<223> Synthetic

<400> 117
gaggtgcagc tggtggagtc tgggggaggc ttggtacagc ctggggggtc cctgagactc 60
tcctgtgcag cctctggatt caccctcagt agctacgata tgcactgggt ccgccaagct 120
acaggaaaag gtctggagtg ggtctcagct attggttcta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggatgg 300
gacgtaccct ttgacttctg gggccaggga accctggtca ccgtctcctc a 351

<210> 118
<211> 117
<212> PRT
<213> Artificial Sequence

<220>
<223> Synthetic

<400> 118
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Trp Asp Val Pro Phe Asp Phe Trp Gly Gln Gly Thr Leu
            100                 105                 110
Val Thr Val Ser Ser
        115


<210> 119
<211> 321
<212> DNA
<213> Artificial Sequence

<220>
<223> Synthetic

<400> 119
gccatccaga tgacccagtc tccatcctcc ctgtctgcat ctgtaggaga cagagtcacc 60
atcacttgcc gggcaagtca ggacattaga aatgatttag gctggtatca gcagaaacca 120
gggaaagccc ctaagctcct gatctatgct gcatccagtt tacaaagtgg ggtcccatca 180
aggttcagcg gcagtggatc tggcacagat ttcactctca ccatcagcag cctgcagcct 240
gaagattttg caacttatta ctgtctacaa gattacaatt acccgtggac gttcggccaa 300
gggaccaagg tggaaatcaa a 321

<210> 120
<211> 107
<212> PRT
<213> Artificial Sequence

<220>
<223> Synthetic

<400> 120
Ala Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Arg Asn Asp
            20                  25                  30
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro

65                  70                  75                  80
Glu Asp Phe Ala Thr Tyr Tyr Cys Leu Gln Asp Tyr Asn Tyr Pro Trp
                85                  90                  95
Thr Phe Gly Gln Gly Thr Lys Val Glu Ile Lys
            100                 105

<210> 121
<211> 384
<212> DNA
<213> Artificial Sequence

<220>
<223> Synthetic

<400> 121
caggtgcagc tgcaggagtc ggggccagga ctggtgaagc cttcggagac cctgtccctc 60
acctgcactg tctctgggga ctccatcaat acttactact ggagctggtt ccggcagccc 120
ccagggaagg gactggagtg gattgggtat atctattata gtggaaccac caactacaac 180
ccctccctca agagtcgagt caccatatca atagacacgc ccaggaacca gttctccctg 240
aagctgatct ctgtgaccgc agcggacacg gccgtgtatt actgtgcgag agagaggatt 300
actatgattc ggggagttac cctctactat tactcctacg gtatggacgt ctggggccaa 360
gggaccacgg tcaccgtctc ctca                                        384

<210> 122
<211> 128
<212> PRT
<213> Artificial Sequence

<220>
<223> Synthetic

<400> 122
Gln Val Gln Leu Gln Glu Ser Gly Pro Gly Leu Val Lys Pro Ser Glu
1               5                   10                  15
Thr Leu Ser Leu Thr Cys Thr Val Ser Gly Asp Ser Ile Asn Thr Tyr
            20                  25                  30
Tyr Trp Ser Trp Phe Arg Gln Pro Pro Gly Lys Gly Leu Glu Trp Ile
        35                  40                  45
Gly Tyr Ile Tyr Tyr Ser Gly Thr Thr Asn Tyr Asn Pro Ser Leu Lys
    50                  55                  60
Ser Arg Val Thr Ile Ser Ile Asp Thr Pro Arg Asn Gln Phe Ser Leu
65                  70                  75                  80
Lys Leu Ile Ser Val Thr Ala Ala Asp Thr Ala Val Tyr Tyr Cys Ala
                85                  90                  95
Arg Glu Arg Ile Thr Met Ile Arg Gly Val Thr Leu Tyr Tyr Tyr Ser
            100                 105                 110
Tyr Gly Met Asp Val Trp Gly Gln Gly Thr Thr Val Thr Val Ser Ser
        115                 120                 125

<210> 123
<211> 24
<212> DNA
<213> Artificial Sequence

<220>
<223> Synthetic

<400> 123
ggggactcca tcaatactta ctac                                        24

<210> 124
<211> 8
<212> PRT
<213> Artificial Sequence

<220>
<223> Synthetic

<400> 124
Gly Asp Ser Ile Asn Thr Tyr Tyr
1               5

<210> 125
<211> 21
<212> DNA
<213> Artificial Sequence

<220>
<223> Synthetic

<400> 125
atctattata gtggaaccac c                                                21

<210> 126
<211> 7
<212> PRT
<213> Artificial Sequence

<220>
<223> Synthetic

<400> 126
Ile Tyr Tyr Ser Gly Thr Thr
1               5

<210> 127
<211> 66
<212> DNA
<213> Artificial Sequence

<220>
<223> Synthetic

<400> 127
gcgagagaga ggattactat gattcgggga gttaccctct actattactc ctacggtatg 60
gacgtc                                                                 66

<210> 128
<211> 22
<212> PRT
<213> Artificial Sequence

<220>
<223> Synthetic

<400> 128
Ala Arg Glu Arg Ile Thr Met Ile Arg Gly Val Thr Leu Tyr Tyr Tyr
1               5                   10                  15
Ser Tyr Gly Met Asp Val
            20

<210> 129
<211> 324
<212> DNA
<213> Artificial Sequence

<220>
<223> Synthetic

<400> 129
gacatccaga tgacccagtc tccatccttc ctgtctgcat ctgtaggaga cagagtcacc 60
atcacttgct gggccagtca ggacattagc agttatttag cctggtatca gcaaaaacca 120
gggatagccc ctaagctcct gatctatgct gcatccactt tgcaaagtgg ggtcccatca 180

aggttcggcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa acga 324

<210> 130
<211> 108
<212> PRT
<213> Artificial Sequence

<220>
<223> Synthetic

<400> 130
Asp Ile Gln Met Thr Gln Ser Pro Ser Phe Leu Ser Ala Ser Val Gly
1               5                   10                  15
Asp Arg Val Thr Ile Thr Cys Trp Ala Ser Gln Asp Ile Ser Ser Tyr
            20                  25                  30
Leu Ala Trp Tyr Gln Gln Lys Pro Gly Ile Ala Pro Lys Leu Leu Ile
        35                  40                  45
Tyr Ala Ala Ser Thr Leu Gln Ser Gly Val Pro Ser Arg Phe Gly Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ser Tyr Pro Arg
                85                  90                  95
Thr Phe Gly Gln Gly Thr Lys Val Glu Ile Lys Arg
            100                 105

<210> 131
<211> 18
<212> DNA
<213> Artificial Sequence

<220>
<223> Synthetic

<400> 131
caggacatta gcagttat 18

<210> 132
<211> 6
<212> PRT
<213> Artificial Sequence

<220>
<223> Synthetic

<400> 132
Gln Asp Ile Ser Ser Tyr
1               5

<210> 133
<211> 9
<212> DNA
<213> Artificial Sequence

<220>
<223> Synthetic

<400> 133
gctgcatcc 9

<210> 134
<211> 3
<212> PRT
<213> Artificial Sequence

<220>
<223> Synthetic

<400> 134
Ala Ala Ser
1

<210> 135
<211> 27
<212> DNA
<213> Artificial Sequence

<220>
<223> Synthetic

<400> 135
caacagctta atagttaccc tcggacg                                          27

<210> 136
<211> 9
<212> PRT
<213> Artificial Sequence

<220>
<223> Synthetic

<400> 136
Gln Gln Leu Asn Ser Tyr Pro Arg Thr
1               5

<210> 137
<211> 384
<212> DNA
<213> Artificial Sequence

<220>
<223> Synthetic

<400> 137
caggtgcagc tgcaggagtc ggggccagga ctggtgaagc cttcggagac cctgtccctc 60
acctgcactg tctctgggga ctccatcaat acttactact ggagctggtt ccggcagccc 120
ccagggaagg gactggagtg gattgggtat atctattata gtggaaccac caactacaac 180
ccctccctca agagtcgagt caccatatca atagacacgc ccaggaacca gttctccctg 240
aagctgatct ctgtgaccgc agcggacacg gccgtgtatt actgtgcgag agagaggatt 300
actatgattc ggggagttac cctctactat tactcctacg gtatggacgt ctggggccaa 360
gggaccacgg tcaccgtctc ctca                                             384

<210> 138
<211> 128
<212> PRT
<213> Artificial Sequence

<220>
<223> Synthetic

<400> 138
Gln Val Gln Leu Gln Glu Ser Gly Pro Gly Leu Val Lys Pro Ser Glu
1               5                   10                  15
Thr Leu Ser Leu Thr Cys Thr Val Ser Gly Asp Ser Ile Asn Thr Tyr
            20                  25                  30
Tyr Trp Ser Trp Phe Arg Gln Pro Pro Gly Lys Gly Leu Glu Trp Ile
        35                  40                  45
Gly Tyr Ile Tyr Tyr Ser Gly Thr Thr Asn Tyr Asn Pro Ser Leu Lys
    50                  55                  60
Ser Arg Val Thr Ile Ser Ile Asp Thr Pro Arg Asn Gln Phe Ser Leu
65                  70                  75                  80
Lys Leu Ile Ser Val Thr Ala Ala Asp Thr Ala Val Tyr Tyr Cys Ala

                85                  90                  95
Arg Glu Arg Ile Thr Met Ile Arg Gly Val Thr Leu Tyr Tyr Tyr Ser
            100                 105                 110
Tyr Gly Met Asp Val Trp Gly Gln Gly Thr Thr Val Thr Val Ser Ser
        115                 120                 125

<210> 139
<211> 321
<212> DNA
<213> Artificial Sequence

<220>
<223> Synthetic

<400> 139
gacatccaga tgacccagtc tccatccttc ctgtctgcat ctgtaggaga cagagtcacc 60
atcacttgct gggccagtca ggacattagc agttatttag cctggtatca gcaaaaacca 120
gggatagccc ctaagctcct gatctatgct gcatccactt tgcaaagtgg ggtcccatca 180
aggttcggcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa a                                           321

<210> 140
<211> 107
<212> PRT
<213> Artificial Sequence

<220>
<223> Synthetic

<400> 140
Asp Ile Gln Met Thr Gln Ser Pro Ser Phe Leu Ser Ala Ser Val Gly
1               5                   10                  15
Asp Arg Val Thr Ile Thr Cys Trp Ala Ser Gln Asp Ile Ser Ser Tyr
            20                  25                  30
Leu Ala Trp Tyr Gln Gln Lys Pro Gly Ile Ala Pro Lys Leu Leu Ile
        35                  40                  45
Tyr Ala Ala Ser Thr Leu Gln Ser Gly Val Pro Ser Arg Phe Gly Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ser Tyr Pro Arg
                85                  90                  95
Thr Phe Gly Gln Gly Thr Lys Val Glu Ile Lys
            100                 105

<210> 141
<211> 384
<212> DNA
<213> Artificial Sequence

<220>
<223> Synthetic

<400> 141
caggtgcagc tgcaggagtc gggcccagga ctggtgaagc cttcggagac cctgtccctc 60
acctgcactg tctctgggga ctccatcaat acttactact ggagctggat ccggcagccc 120
ccagggaagg gactggagtg gattgggtat atctattata gtggaaccac caactacaac 180
ccctccctca agagtcgagt caccatatca gtagacacgt ccaagaacca gttctccctg 240
aagctgagct ctgtgaccgc tgcggacacg gccgtgtatt actgtgcgag agagaggatt 300
actatgattc ggggagttac cctctactat tactcctacg gtatggacgt ctggggccaa 360
gggaccacgg tcaccgtctc ctca                                        384

<210> 142
<211> 128
<212> PRT
<213> Artificial Sequence

<220>
<223> Synthetic

<400> 142
Gln Val Gln Leu Gln Glu Ser Gly Pro Gly Leu Val Lys Pro Ser Glu
1               5                   10                  15
Thr Leu Ser Leu Thr Cys Thr Val Ser Gly Asp Ser Ile Asn Thr Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Thr Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg Glu Arg Ile Thr Met Ile Arg Gly Val Thr Leu Tyr Tyr Tyr Ser
            100                 105                 110
Tyr Gly Met Asp Val Trp Gly Gln Gly Thr Thr Val Thr Val Ser Ser
        115                 120                 125

<210> 143
<211> 321
<212> DNA
<213> Artificial Sequence

<220>
<223> Synthetic

<400> 143
gacatccaga tgacccagtc tccatcctcc ctgtctgcat ctgtaggaga cagagtcacc 60
atcacttgcc gggcaagtca ggacattagc agttatttag gctggtatca gcagaaacca 120
gggaaagccc ctaagcgcct gatctatgct gcatccagtt tgcaaagtgg ggtcccatca 180
aggttcagcg gcagtggatc tgggacagaa ttcactctca caatcagcag cctgcagcct 240
gaagattttg caacttatta ctgtcaacag cttaatagtt accctcggac gttcggccaa 300
gggaccaagg tggaaatcaa a                                           321

<210> 144
<211> 107
<212> PRT
<213> Artificial Sequence

<220>
<223> Synthetic

<400> 144
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Ser Ser Tyr
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ser Tyr Pro Arg
                85                  90                  95
Thr Phe Gly Gln Gly Thr Lys Val Glu Ile Lys
            100                 105

<210> 145
<211> 378
<212> DNA
<213> Artificial Sequence

<220>
<223> Synthetic

<400> 145
caggtgcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag gacttgagtt aatgggatgg attagtggtt acaatggtaa cacaaactat 180
gcacaagaac tccaggccag agtcaccatg accacagaca catccacgag cacagcctac 240
atggagctga ggaacctgag atctgacgac acggccgtat attactgtgc gagagataga 300
gtcgttgtag cagctgctaa ttactacttt tattctatgg acgtctgggg ccaagggacc 360
acggtcaccg tctcctca 378

<210> 146
<211> 126
<212> PRT
<213> Artificial Sequence

<220>
<223> Synthetic

<400> 146
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Gly Ile Ser Trp Val Arg Gln Ala Pro Gly Gln Gly Leu Glu Leu Met
        35                  40                  45
Gly Trp Ile Ser Gly Tyr Asn Gly Asn Thr Asn Tyr Ala Gln Glu Leu
    50                  55                  60
Gln Ala Arg Val Thr Met Thr Thr Asp Thr Ser Thr Ser Thr Ala Tyr
65                  70                  75                  80
Met Glu Leu Arg Asn Leu Arg Ser Asp Asp Thr Ala Val Tyr Tyr Cys
                85                  90                  95
Ala Arg Asp Arg Val Val Val Ala Ala Ala Asn Tyr Tyr Phe Tyr Ser
            100                 105                 110
Met Asp Val Trp Gly Gln Gly Thr Thr Val Thr Val Ser Ser
        115                 120                 125

<210> 147
<211> 24
<212> DNA
<213> Artificial Sequence

<220>
<223> Synthetic

<400> 147
ggttacacct ttaccaacta tggt 24

<210> 148
<211> 8
<212> PRT
<213> Artificial Sequence

<220>
<223> Synthetic

<400> 148
Gly Tyr Thr Phe Thr Asn Tyr Gly
1               5

<210> 149
<211> 24
<212> DNA
<213> Artificial Sequence

<220>

<223> Synthetic

<400> 149
attagtggtt acaatggtaa caca                                              24

<210> 150
<211> 8
<212> PRT
<213> Artificial Sequence

<220>
<223> Synthetic

<400> 150
Ile Ser Gly Tyr Asn Gly Asn Thr
1               5

<210> 151
<211> 57
<212> DNA
<213> Artificial Sequence

<220>
<223> Synthetic

<400> 151
gcgagagata gagtcgttgt agcagctgct aattactact tttattctat ggacgtc          57

<210> 152
<211> 19
<212> PRT
<213> Artificial Sequence

<220>
<223> Synthetic

<400> 152
Ala Arg Asp Arg Val Val Val Ala Ala Ala Asn Tyr Tyr Phe Tyr Ser
1               5                   10                  15
Met Asp Val

<210> 153
<211> 339
<212> DNA
<213> Artificial Sequence

<220>
<223> Synthetic

<400> 153
gccatccaga tgacccagtc tccactctcc ctgtccgtca cccttggaca gccggcctcc      60
atctcctgca ggtctagtca aagcctcgta tacagtgatg gagacaccta cttgaattgg     120
tttcagcaga ggccaggcca atctccaagg cgcctaattt ataaggtttc taaccgggac     180
tctggggtcc cagacagatt cagcggcagt gggtcaggca ctgctttcac actgaaaatc     240
agcggggtgg aggccgagga tgttggggtt tactactgca tgcaagctac acactggcct     300
cggacgttcg gccaagggac caaggtggaa atcaaacga                            339

<210> 154
<211> 113
<212> PRT
<213> Artificial Sequence

<220>
<223> Synthetic

<400> 154

Ala Ile Gln Met Thr Gln Ser Pro Leu Ser Leu Ser Val Thr Leu Gly
1               5                   10                  15
Gln Pro Ala Ser Ile Ser Cys Arg Ser Ser Gln Ser Leu Val Tyr Ser
            20                  25                  30
Asp Gly Asp Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Ala Phe Thr Leu Lys Ile
65                  70                  75                  80
Ser Gly Val Glu Ala Glu Asp Val Gly Val Tyr Tyr Cys Met Gln Ala
                85                  90                  95
Thr His Trp Pro Arg Thr Phe Gly Gln Gly Thr Lys Val Glu Ile Lys
            100                 105                 110
Arg

<210> 155
<211> 33
<212> DNA
<213> Artificial Sequence

<220>
<223> Synthetic

<400> 155
caaagcctcg tatacagtga tggagacacc tac                                33

<210> 156
<211> 11
<212> PRT
<213> Artificial Sequence

<220>
<223> Synthetic

<400> 156
Gln Ser Leu Val Tyr Ser Asp Gly Asp Thr Tyr
1               5                   10

<210> 157
<211> 9
<212> DNA
<213> Artificial Sequence

<220>
<223> Synthetic

<400> 157
aaggtttct                                                            9

<210> 158
<211> 3
<212> PRT
<213> Artificial Sequence

<220>
<223> Synthetic

<400> 158
Lys Val Ser
1

<210> 159
<211> 27
<212> DNA

<213> Artificial Sequence

<220>
<223> Synthetic

<400> 159
atgcaagcta cacactggcc tcggacg                                          27

<210> 160
<211> 9
<212> PRT
<213> Artificial Sequence

<220>
<223> Synthetic

<400> 160
Met Gln Ala Thr His Trp Pro Arg Thr
1               5

<210> 161
<211> 378
<212> DNA
<213> Artificial Sequence

<220>
<223> Synthetic

<400> 161
caggttcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag gacttgagtt aatgggatgg attagtggtt acaatggtaa cacaaactat 180
gcacaagaac tccaggccag agtcaccatg accacagaca catccacgag cacagcctac 240
atggagctga ggaacctgag atctgacgac acggccgtat attactgtgc gagagataga 300
gtcgttgtag cagctgctaa ttactacttt tattctatgg acgtctgggg ccaagggacc 360
acggtcaccg tctcctca                                                378

<210> 162
<211> 126
<212> PRT
<213> Artificial Sequence

<220>
<223> Synthetic

<400> 162
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Gly Ile Ser Trp Val Arg Gln Ala Pro Gly Gln Gly Leu Glu Leu Met
        35                  40                  45
Gly Trp Ile Ser Gly Tyr Asn Gly Asn Thr Asn Tyr Ala Gln Glu Leu
    50                  55                  60
Gln Ala Arg Val Thr Met Thr Thr Asp Thr Ser Thr Ser Thr Ala Tyr
65                  70                  75                  80
Met Glu Leu Arg Asn Leu Arg Ser Asp Asp Thr Ala Val Tyr Tyr Cys
                85                  90                  95
Ala Arg Asp Arg Val Val Val Ala Ala Ala Asn Tyr Tyr Phe Tyr Ser
            100                 105                 110
Met Asp Val Trp Gly Gln Gly Thr Thr Val Thr Val Ser Ser
        115                 120                 125

<210> 163
<211> 336
<212> DNA
<213> Artificial Sequence

<220>
<223> Synthetic

<400> 163
gatgttgtga tgactcagtc tccactctcc ctgtccgtca cccttggaca gccggcctcc 60
atctcctgca ggtctagtca aagcctcgta tacagtgatg gagacaccta cttgaattgg 120
tttcagcaga ggccaggcca atctccaagg cgcctaattt ataaggtttc taaccgggac 180
tctggggtcc cagacagatt cagcggcagt gggtcaggca ctgctttcac actgaaaatc 240
agcggggtgg aggccgagga tgttggggtt tactactgca tgcaagctac acactggcct 300
cggacgttcg gccaagggac caaggtggaa atcaaa 336

<210> 164
<211> 112
<212> PRT
<213> Artificial Sequence

<220>
<223> Synthetic

<400> 164
Asp Val Val Met Thr Gln Ser Pro Leu Ser Leu Ser Val Thr Leu Gly
1               5                   10                  15
Gln Pro Ala Ser Ile Ser Cys Arg Ser Ser Gln Ser Leu Val Tyr Ser
            20                  25                  30
Asp Gly Asp Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Ala Phe Thr Leu Lys Ile
65                  70                  75                  80
Ser Gly Val Glu Ala Glu Asp Val Gly Val Tyr Tyr Cys Met Gln Ala
                85                  90                  95
Thr His Trp Pro Arg Thr Phe Gly Gln Gly Thr Lys Val Glu Ile Lys
            100                 105                 110

<210> 165
<211> 378
<212> DNA
<213> Artificial Sequence

<220>
<223> Synthetic

<400> 165
caggttcagc tggtgcagtc tggagctgag gtgaagaagc ctggggcctc agtgaaggtc 60
tcctgcaagg cttctggtta cacctttacc aactatggta tcagctgggt gcgacaggcc 120
cctggacaag ggcttgagtg gatgggatgg attagtggtt acaatggtaa cacaaactat 180
gcacagaagc tccagggcag agtcaccatg accacagaca catccacgag cacagcctac 240
atggagctga ggagcctgag atctgacgac acggccgtgt attactgtgc gagagataga 300
gtcgttgtag cagctgctaa ttactacttt tattctatgg acgtctgggg ccaagggacc 360
acggtcaccg tctcctca 378

<210> 166
<211> 126
<212> PRT
<213> Artificial Sequence

<220>
<223> Synthetic

<400> 166
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Gly Ile Ser Trp Val Arg Gln Ala Pro Gly Gln Gly Leu Glu Trp Met
        35                  40                  45

Gly Trp Ile Ser Gly Tyr Asn Gly Asn Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Asp Arg Val Val Val Ala Ala Ala Asn Tyr Tyr Phe Tyr Ser
            100                 105                 110
Met Asp Val Trp Gly Gln Gly Thr Thr Val Thr Val Ser Ser
        115                 120                 125

<210> 167
<211> 336
<212> DNA
<213> Artificial Sequence

<220>
<223> Synthetic

<400> 167
gatgttgtga tgactcagtc tccactctcc ctgcccgtca cccttggaca gccggcctcc 60
atctcctgca ggtctagtca aagcctcgta tacagtgatg gagacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaagctac acactggcct 300
cggacgttcg gccaagggac caaggtggaa atcaaa                           336

<210> 168
<211> 112
<212> PRT
<213> Artificial Sequence

<220>
<223> Synthetic

<400> 168
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asp Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Thr His Trp Pro Arg Thr Phe Gly Gln Gly Thr Lys Val Glu Ile Lys
            100                 105                 110

<210> 169
<211> 375
<212> DNA
<213> Artificial Sequence

<220>
<223> Synthetic

<400> 169
caggtccact tgaaggagtc tggtcctacg ctggtgaaac ccacacagac cctcacgctg 60
acctgcacct tctctggatt ctcactcatc actagtggag tgggtgtggg ctggattcgt 120
cagccccccg gaaaggccct ggagtggctt gcactcattt attggaatgg tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca                                                   375

<210> 170
<211> 125
<212> PRT
<213> Artificial Sequence

<220>
<223> Synthetic

<400> 170
Gln Val His Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ile Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Gly Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile Thr Glu Thr Ser Tyr Tyr Phe Tyr Tyr Gly Met
            100                 105                 110
Asp Val Trp Gly Gln Gly Thr Thr Val Thr Val Ser Ser
        115                 120                 125


<210> 171
<211> 30
<212> DNA
<213> Artificial Sequence

<220>
<223> Synthetic

<400> 171
ggattctcac tcatcactag tggagtgggt                                        30

<210> 172
<211> 10
<212> PRT
<213> Artificial Sequence

<220>
<223> Synthetic

<400> 172
Gly Phe Ser Leu Ile Thr Ser Gly Val Gly
1               5                   10


<210> 173
<211> 21
<212> DNA
<213> Artificial Sequence

<220>
<223> Synthetic

<400> 173
atttattgga atggtgataa g                                                 21

<210> 174
<211> 7
<212> PRT
<213> Artificial Sequence

<220>

<223> Synthetic

<400> 174
Ile Tyr Trp Asn Gly Asp Lys
1               5

<210> 175
<211> 51
<212> DNA
<213> Artificial Sequence

<220>
<223> Synthetic

<400> 175
gcacacagga taactgaaac tagttactac ttctactacg gtatggacgt c          51

<210> 176
<211> 17
<212> PRT
<213> Artificial Sequence

<220>
<223> Synthetic

<400> 176
Ala His Arg Ile Thr Glu Thr Ser Tyr Tyr Phe Tyr Tyr Gly Met Asp
1               5                   10                  15
Val

<210> 177
<211> 339
<212> DNA
<213> Artificial Sequence

<220>
<223> Synthetic

<400> 177
gacatccaga tgacccagtc tccactctcc ctgcccgtca cccctggaga gccggcctcc 60
atctcctgca ggtctagtca gagcctcctg catagtcatg gatacgacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggaa atcaaacga                        339

<210> 178
<211> 113
<212> PRT
<213> Artificial Sequence

<220>
<223> Synthetic

<400> 178
Asp Ile Gln Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asp Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala

85                  90                  95
Leu Gln Thr Pro Leu Thr Phe Gly Gly Gly Thr Lys Val Glu Ile Lys
            100                 105                 110
Arg

<210> 179
<211> 33
<212> DNA
<213> Artificial Sequence

<220>
<223> Synthetic

<400> 179
cagagcctcc tgcatagtca tggatacgac tat                                    33

<210> 180
<211> 11
<212> PRT
<213> Artificial Sequence

<220>
<223> Synthetic

<400> 180
Gln Ser Leu Leu His Ser His Gly Tyr Asp Tyr
1               5                   10

<210> 181
<211> 9
<212> DNA
<213> Artificial Sequence

<220>
<223> Synthetic

<400> 181
ttgggttct                                                               9

<210> 182
<211> 3
<212> PRT
<213> Artificial Sequence

<220>
<223> Synthetic

<400> 182
Leu Gly Ser
1

<210> 183
<211> 27
<212> DNA
<213> Artificial Sequence

<220>
<223> Synthetic

<400> 183
atgcaagctc tacaaactcc gctcact                                           27

<210> 184
<211> 9
<212> PRT

<213> Artificial Sequence

<220>
<223> Synthetic

<400> 184
Met Gln Ala Leu Gln Thr Pro Leu Thr
1               5


<210> 185
<211> 375
<212> DNA
<213> Artificial Sequence

<220>
<223> Synthetic

<400> 185
cagatcacct tgaaggagtc tggtcctacg ctggtgaaac ccacacagac cctcacgctg 60
acctgcacct tctctggatt ctcactcatc actagtggag tgggtgtggg ctggattcgt 120
cagccccccg gaaaggccct ggagtggctt gcactcattt attggaatgg tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca                                                  375

<210> 186
<211> 125
<212> PRT
<213> Artificial Sequence

<220>
<223> Synthetic

<400> 186
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ile Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Gly Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile Thr Glu Thr Ser Tyr Tyr Phe Tyr Tyr Gly Met
            100                 105                 110
Asp Val Trp Gly Gln Gly Thr Thr Val Thr Val Ser Ser
        115                 120                 125


<210> 187
<211> 336
<212> DNA
<213> Artificial Sequence

<220>
<223> Synthetic

<400> 187
gatattgtga tgactcagtc tccactctcc ctgcccgtca cccctggaga gccggcctcc 60
atctcctgca ggtctagtca gagcctcctg catagtcatg gatacgacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggag atcaaa                           336

<210> 188
<211> 112
<212> PRT
<213> Artificial Sequence

<220>
<223> Synthetic

<400> 188
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asp Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110


<210> 189
<211> 375
<212> DNA
<213> Artificial Sequence

<220>
<223> Synthetic

<400> 189
cagatcacct tgaaggagtc tggtcctacg ctggtgaaac ccacacagac cctcacgctg 60
acctgcacct tctctggatt ctcactcatc actagtggag tgggtgtggg ctggatccgt 120
cagcccccag gaaaggccct ggagtggctt gcactcattt attggaatgg tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacagg 300
ataactgaaa ctagttacta cttctactac ggtatggacg tctggggcca agggaccacg 360
gtcaccgtct cctca                                                  375

<210> 190
<211> 125
<212> PRT
<213> Artificial Sequence

<220>
<223> Synthetic

<400> 190
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ile Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Gly Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile Thr Glu Thr Ser Tyr Tyr Phe Tyr Tyr Gly Met
            100                 105                 110
Asp Val Trp Gly Gln Gly Thr Thr Val Thr Val Ser Ser
        115                 120                 125

<210> 191
<211> 336
<212> DNA
<213> Artificial Sequence

<220>
<223> Synthetic

<400> 191
gatattgtga tgactcagtc tccactctcc ctgcccgtca cccctggaga gccggcctcc 60
atctcctgca ggtctagtca gagcctcctg catagtcatg gatacgacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactccg 300
ctcactttcg gcggagggac caaggtggag atcaaa 336

<210> 192
<211> 112
<212> PRT
<213> Artificial Sequence

<220>
<223> Synthetic

<400> 192
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asp Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 193
<211> 375
<212> DNA
<213> Artificial Sequence

<220>
<223> Synthetic

<400> 193
cagatcacct tgaaggagtc tggtcctact ctggtgaaac ccacacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggta 240
gtccttacaa tgaccaacat ggaccctgtg gacacagcca catattactg tgcacacaga 300
catgacagct cgtcctacta cttctactac ggtatggacg tctggggcca agggatcacg 360
gtcaccgtct cctca 375

<210> 194
<211> 125
<212> PRT
<213> Artificial Sequence

<220>
<223> Synthetic

<400> 194
Gln Ile Thr Leu Lys Glu Ser Gly Pro Thr Leu Val Lys Pro Ser Gln

 1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Ser Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Ile Thr Val Thr Val Ser Ser
        115                 120                 125

<210> 195
<211> 30
<212> DNA
<213> Artificial Sequence

<220>
<223> Synthetic

<400> 195
gggttctcac tcagcactag tggagtgggt                                        30

<210> 196
<211> 10
<212> PRT
<213> Artificial Sequence

<220>
<223> Synthetic

<400> 196
Gly Phe Ser Leu Ser Thr Ser Gly Val Gly
1               5                   10

<210> 197
<211> 21
<212> DNA
<213> Artificial Sequence

<220>
<223> Synthetic

<400> 197
atttattgga attctgataa g                                                 21

<210> 198
<211> 7
<212> PRT
<213> Artificial Sequence

<220>
<223> Synthetic

<400> 198
Ile Tyr Trp Asn Ser Asp Lys
1               5

<210> 199
<211> 51
<212> DNA
<213> Artificial Sequence

<220>
<223> Synthetic

<400> 199
gcacacagac atgacagctc gtcctactac ttctactacg gtatggacgt c 51

<210> 200
<211> 17
<212> PRT
<213> Artificial Sequence

<220>
<223> Synthetic

<400> 200
Ala His Arg His Asp Ser Ser Ser Tyr Tyr Phe Tyr Tyr Gly Met Asp
1               5                   10                  15
Val

<210> 201
<211> 339
<212> DNA
<213> Artificial Sequence

<220>
<223> Synthetic

<400> 201
gacatccaga tgacccagtc tccgctctcc ctgcccgtca cccctggaga gccggcctcc 60
atctcctgca ggtctagtca gagcctcctc catagtcatg gatacaacta tttggattgg 120
tacctgcaga agccagggca gtctccacaa ctcctgatct atttgggttc taatcgggcc 180
tccggggtcc ctgacaggtt cagtggcggt ggatcaggca cagattttac actgaaaatc 240
agcagagtgg aggctgagga tgttgggatt tattactgca tgcaagctct acagactcct 300
ctcactttcg gcggagggac caaggtggag atcaaacga 339

<210> 202
<211> 113
<212> PRT
<213> Artificial Sequence

<220>
<223> Synthetic

<400> 202
Asp Ile Gln Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Gly Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Lys
            100                 105                 110
Arg

<210> 203
<211> 33
<212> DNA
<213> Artificial Sequence

<220>
<223> Synthetic

<400> 203
cagagcctcc tccatagtca tggatacaac tat                                    33

<210> 204
<211> 11
<212> PRT
<213> Artificial Sequence

<220>
<223> Synthetic

<400> 204
Gln Ser Leu Leu His Ser His Gly Tyr Asn Tyr
1               5                   10


<210> 205
<211> 9
<212> DNA
<213> Artificial Sequence

<220>
<223> Synthetic

<400> 205
ttgggttct                                                               9

<210> 206
<211> 3
<212> PRT
<213> Artificial Sequence

<220>
<223> Synthetic

<400> 206
Leu Gly Ser
1


<210> 207
<211> 27
<212> DNA
<213> Artificial Sequence

<220>
<223> Synthetic

<400> 207
atgcaagctc tacagactcc tctcact                                           27

<210> 208
<211> 9
<212> PRT
<213> Artificial Sequence

<220>
<223> Synthetic

<400> 208
Met Gln Ala Leu Gln Thr Pro Leu Thr
1               5


<210> 209

<211> 375
<212> DNA
<213> Artificial Sequence

<220>
<223> Synthetic

<400> 209
cagatcacct tgaaggagtc tggtcctact ctggtgaaac cctcacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggta 240
gtccttacaa tgaccaacat ggaccctgtg gacacagcca catattactg tgcacacaga 300
catgacagct cgtcctacta cttctactac ggtatggacg tctggggcca agggaccacg 360
gtcaccgtct cctca 375

<210> 210
<211> 125
<212> PRT
<213> Artificial Sequence

<220>
<223> Synthetic

<400> 210
Gln Ile Thr Leu Lys Glu Ser Gly Pro Thr Leu Val Lys Pro Ser Gln
1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Ser Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Thr Thr Val Thr Val Ser Ser
        115                 120                 125

<210> 211
<211> 336
<212> DNA
<213> Artificial Sequence

<220>
<223> Synthetic

<400> 211
gatattgtga tgactcagtc tccgctctcc ctgcccgtca cccctggaga gccggcctcc 60
atctcctgca ggtctagtca gagcctcctc catagtcatg gatacaacta tttggattgg 120
tacctgcaga agccagggca gtctccacaa ctcctgatct atttgggttc taatcgggcc 180
tccggggtcc ctgacaggtt cagtggcggt ggatcaggca cagattttac actgaaaatc 240
agcagagtgg aggctgagga tgttgggatt tattactgca tgcaagctct acagactcct 300
ctcactttcg gcggagggac caaggtggag atcaaa 336

<210> 212
<211> 112
<212> PRT
<213> Artificial Sequence

<220>
<223> Synthetic

<400> 212
Asp Ile Val Met Thr Gln Ser Pro Leu Ser Leu Pro Val Thr Pro Gly

1               5                   10                  15
Glu Pro Ala Ser Ile Ser Cys Arg Ser Ser Gln Ser Leu Leu His Ser
            20                  25                  30
His Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Gly Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 213
<211> 375
<212> DNA
<213> Artificial Sequence

<220>
<223> Synthetic

<400> 213
cagatcacct tgaaggagtc tggtcctacg ctggtgaaac ccacacagac cctcacgctg 60
acctgcacct tctctgggtt ctcactcagc actagtggag tgggtgtggg ctggatccgt 120
cagcccccag gaaaggccct ggagtggctt gcactcattt attggaattc tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacaga 300
catgacagct cgtcctacta cttctactac ggtatggacg tctggggcca agggaccacg 360
gtcaccgtct cctca                                                  375

<210> 214
<211> 125
<212> PRT
<213> Artificial Sequence

<220>
<223> Synthetic

<400> 214
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Ser Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg His Asp Ser Ser Ser Tyr Tyr Phe Tyr Tyr Gly Met
            100                 105                 110
Asp Val Trp Gly Gln Gly Thr Thr Val Thr Val Ser Ser
        115                 120                 125

<210> 215
<211> 336
<212> DNA
<213> Artificial Sequence

<220>
<223> Synthetic

<400> 215
gatattgtga tgactcagtc tccactctcc ctgcccgtca cccctggaga gccggcctcc 60

atctcctgca ggtctagtca gagcctcctc catagtcatg gatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acagactcct 300
ctcactttcg gcggagggac caaggtggag atcaaa 336

<210> 216
<211> 112
<212> PRT
<213> Artificial Sequence

<220>
<223> Synthetic

<400> 216
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
His Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 217
<211> 381
<212> DNA
<213> Artificial Sequence

<220>
<223> Synthetic

<400> 217
gagatgcaac tggtggagtc tgggggaggc ttggtccagc ctggggggtc cctgagactc 60
tcctgtgcag cctctggatt cacctttagt agtcactgga tgaagtgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgttt 240
ctgcaaatga acagcctgag agccgaggac acggctgtgt attactgtgc gagagatatt 300
gtactaatgg tctatgatat ggactactac tactacggta tggacgtctg gggccaaggg 360
accacggtca ccgtctcctc a 381

<210> 218
<211> 127
<212> PRT
<213> Artificial Sequence

<220>
<223> Synthetic

<400> 218
Glu Met Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser His
            20                  25                  30
Trp Met Lys Trp Val Arg Gln Ala Pro Gly Lys Gly Leu Glu Trp Val
        35                  40                  45
Ala Asn Ile Asn Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Phe
65                  70                  75                  80
Leu Gln Met Asn Ser Leu Arg Ala Glu Asp Thr Ala Val Tyr Tyr Cys
                85                  90                  95

Ala Arg Asp Ile Val Leu Met Val Tyr Asp Met Asp Tyr Tyr Tyr Tyr
            100                 105                 110
Gly Met Asp Val Trp Gly Gln Gly Thr Thr Val Thr Val Ser Ser
        115                 120                 125

<210> 219
<211> 24
<212> DNA
<213> Artificial Sequence

<220>
<223> Synthetic

<400> 219
ggattcacct ttagtagtca ctgg                                              24

<210> 220
<211> 8
<212> PRT
<213> Artificial Sequence

<220>
<223> Synthetic

<400> 220
Gly Phe Thr Phe Ser Ser His Trp
1               5

<210> 221
<211> 24
<212> DNA
<213> Artificial Sequence

<220>
<223> Synthetic

<400> 221
ataaaccaag atggaagtga gaaa                                              24

<210> 222
<211> 8
<212> PRT
<213> Artificial Sequence

<220>
<223> Synthetic

<400> 222
Ile Asn Gln Asp Gly Ser Glu Lys
1               5

<210> 223
<211> 60
<212> DNA
<213> Artificial Sequence

<220>
<223> Synthetic

<400> 223
gcgagagata ttgtactaat ggtctatgat atggactact actactacgg tatggacgtc 60

<210> 224
<211> 20
<212> PRT

<213> Artificial Sequence

<220>
<223> Synthetic

<400> 224
Ala Arg Asp Ile Val Leu Met Val Tyr Asp Met Asp Tyr Tyr Tyr Tyr
1               5                   10                  15
Gly Met Asp Val
            20


<210> 225
<211> 336
<212> DNA
<213> Artificial Sequence

<220>
<223> Synthetic

<400> 225
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa                           336

<210> 226
<211> 112
<212> PRT
<213> Artificial Sequence

<220>
<223> Synthetic

<400> 226
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Asn Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Thr
                85                  90                  95
Leu Gln Thr Pro Leu Thr Phe Gly Gly Gly Thr Lys Val Glu Ile Lys
            100                 105                 110


<210> 227
<211> 33
<212> DNA
<213> Artificial Sequence

<220>
<223> Synthetic

<400> 227
cagagcctcc tgcatagtaa tggaaacaac tat                              33

<210> 228
<211> 11
<212> PRT
<213> Artificial Sequence

<220>
<223> Synthetic

<400> 228
Ser Ser Gln Ser Leu Leu His Ser Asn Gly Asn
1               5                   10


<210> 229
<211> 9
<212> DNA
<213> Artificial Sequence

<220>
<223> Synthetic

<400> 229
ttgggttct                                                              9

<210> 230
<211> 3
<212> PRT
<213> Artificial Sequence

<220>
<223> Synthetic

<400> 230
Leu Gly Ser
1


<210> 231
<211> 27
<212> DNA
<213> Artificial Sequence

<220>
<223> Synthetic

<400> 231
atgcaaactc tacaaactcc gctcact                                          27

<210> 232
<211> 9
<212> PRT
<213> Artificial Sequence

<220>
<223> Synthetic

<400> 232
Met Gln Thr Leu Gln Thr Pro Leu Thr
1               5


<210> 233
<211> 381
<212> DNA
<213> Artificial Sequence

<220>
<223> Synthetic

<400> 233
gaggtgcagc tggtggagtc tgggggaggc ttggtccagc ctggggggtc cctgagactc 60
tcctgtgcag cctctggatt cacctttagt agtcactgga tgaagtgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgttt 240

ctgcaaatga acagcctgag agccgaggac acggctgtgt attactgtgc gagagatatt 300
gtactaatgg tctatgatat ggactactac tactacggta tggacgtctg gggccaaggg 360
accacggtca ccgtctcctc a 381

<210> 234
<211> 127
<212> PRT
<213> Artificial Sequence

<220>
<223> Synthetic

<400> 234
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser His
            20                  25                  30
Trp Met Lys Trp Val Arg Gln Ala Pro Gly Lys Gly Leu Glu Trp Val
        35                  40                  45
Ala Asn Ile Asn Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Phe
65                  70                  75                  80
Leu Gln Met Asn Ser Leu Arg Ala Glu Asp Thr Ala Val Tyr Tyr Cys
                85                  90                  95
Ala Arg Asp Ile Val Leu Met Val Tyr Asp Met Asp Tyr Tyr Tyr Tyr
            100                 105                 110
Gly Met Asp Val Trp Gly Gln Gly Thr Thr Val Thr Val Ser Ser
        115                 120                 125

<210> 235
<211> 336
<212> DNA
<213> Artificial Sequence

<220>
<223> Synthetic

<400> 235
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa 336

<210> 236
<211> 112
<212> PRT
<213> Artificial Sequence

<220>
<223> Synthetic

<400> 236
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Asn Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Thr
                85                  90                  95
Leu Gln Thr Pro Leu Thr Phe Gly Gly Gly Thr Lys Val Glu Ile Lys

            100                 105                 110

<210> 237
<211> 381
<212> DNA
<213> Artificial Sequence

<220>
<223> Synthetic

<400> 237
gaggtgcagc tggtggagtc tgggggaggc ttggtccagc ctggggggtc cctgagactc 60
tcctgtgcag cctctggatt cacctttagt agtcactgga tgagctgggt ccgccaggct 120
ccagggaagg ggctggagtg ggtggccaac ataaaccaag atggaagtga gaaatactat 180
gtggactctg tgaagggccg attcaccatc tccagagaca acgccaagaa ctcactgtat 240
ctgcaaatga acagcctgag agccgaggac acggctgtgt attactgtgc gagagatatt 300
gtactaatgg tctatgatat ggactactac tactacggta tggacgtctg gggccaaggg 360
accacggtca ccgtctcctc a 381

<210> 238
<211> 127
<212> PRT
<213> Artificial Sequence

<220>
<223> Synthetic

<400> 238
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser His
            20                  25                  30
Trp Met Ser Trp Val Arg Gln Ala Pro Gly Lys Gly Leu Glu Trp Val
        35                  40                  45
Ala Asn Ile Asn Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Ile Val Leu Met Val Tyr Asp Met Asp Tyr Tyr Tyr Tyr
            100                 105                 110
Gly Met Asp Val Trp Gly Gln Gly Thr Thr Val Thr Val Ser Ser
        115                 120                 125

<210> 239
<211> 336
<212> DNA
<213> Artificial Sequence

<220>
<223> Synthetic

<400> 239
gatattgtga tgactcagtc tccactctcc ctgcccgtca cccctggaga gccggcctcc 60
atctcctgca ggtctagtca gagcctcctg catagtaatg gaaacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaaactct acaaactccg 300
ctcactttcg gcggagggac caaggtggag atcaaa 336

<210> 240
<211> 112
<212> PRT
<213> Artificial Sequence

<220>

<223> Synthetic

<400> 240
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Asn Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Thr
                85                  90                  95
Leu Gln Thr Pro Leu Thr Phe Gly Gly Gly Thr Lys Val Glu Ile Lys
            100                 105                 110


<210> 241
<211> 381
<212> DNA
<213> Artificial Sequence

<220>
<223> Synthetic

<400> 241
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a                                           381

<210> 242
<211> 127
<212> PRT
<213> Artificial Sequence

<220>
<223> Synthetic

<400> 242
Gln Val Gln Leu Val Glu Ser Gly Gly Gly Val Val Gln Pro Gly Arg
1               5                   10                  15
Ser Leu Arg Leu Ser Cys Ala Val Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Ala Ile Ser Tyr Asp Gly Ser Asn Lys Tyr Tyr Val Asp Ser Val
    50                  55                  60
Lys Gly Arg Phe Thr Ile Ser Arg Asp Asn Ser Lys Lys Thr Leu Tyr
65                  70                  75                  80
Leu Gln Met Asn Ser Leu Arg Ala Glu Asp Thr Ala Val Tyr Asn Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125


<210> 243
<211> 24
<212> DNA
<213> Artificial Sequence

<220>

<223> Synthetic

<400> 243
ggattcacct tcagtagcta tggc                                              24

<210> 244
<211> 8
<212> PRT
<213> Artificial Sequence

<220>
<223> Synthetic

<400> 244
Gly Phe Thr Phe Ser Ser Tyr Gly
1               5


<210> 245
<211> 24
<212> DNA
<213> Artificial Sequence

<220>
<223> Synthetic

<400> 245
atatcatatg atggaagtaa taaa                                              24

<210> 246
<211> 8
<212> PRT
<213> Artificial Sequence

<220>
<223> Synthetic

<400> 246
Ile Ser Tyr Asp Gly Ser Asn Lys
1               5


<210> 247
<211> 60
<212> DNA
<213> Artificial Sequence

<220>
<223> Synthetic

<400> 247
gcgaaaaata ttgtactagt gatgtatgat atagactatc actactatgg gatggacgtc 60


<210> 248
<211> 20
<212> PRT
<213> Artificial Sequence

<220>
<223> Synthetic

<400> 248
Ala Lys Asn Ile Val Leu Val Met Tyr Asp Ile Asp Tyr His Tyr Tyr
1               5                   10                  15
Gly Met Asp Val
            20

<210> 249
<211> 336
<212> DNA
<213> Artificial Sequence

<220>
<223> Synthetic

<400> 249
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaga 336

<210> 250
<211> 112
<212> PRT
<213> Artificial Sequence

<220>
<223> Synthetic

<400> 250
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Arg
            100                 105                 110

<210> 251
<211> 33
<212> DNA
<213> Artificial Sequence

<220>
<223> Synthetic

<400> 251
cagagcctcc tgcatagtaa tggatacaac tat 33

<210> 252
<211> 11
<212> PRT
<213> Artificial Sequence

<220>
<223> Synthetic

<400> 252
Gln Ser Leu Leu His Ser Asn Gly Tyr Asn Tyr
1               5                   10

<210> 253
<211> 9
<212> DNA
<213> Artificial Sequence

<220>
<223> Synthetic

<400> 253
ttgggtttt                                                              9

<210> 254
<211> 3
<212> PRT
<213> Artificial Sequence

<220>
<223> Synthetic

<400> 254
Leu Gly Phe
1

<210> 255
<211> 27
<212> DNA
<213> Artificial Sequence

<220>
<223> Synthetic

<400> 255
atgcaagctc tacaaactcc tctcact                                          27

<210> 256
<211> 9
<212> PRT
<213> Artificial Sequence

<220>
<223> Synthetic

<400> 256
Met Gln Ala Leu Gln Thr Pro Leu Thr
 1               5

<210> 257
<211> 381
<212> DNA
<213> Artificial Sequence

<220>
<223> Synthetic

<400> 257
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a                                                381

<210> 258
<211> 127
<212> PRT
<213> Artificial Sequence

<220>
<223> Synthetic

<400> 258
Gln Val Gln Leu Val Glu Ser Gly Gly Gly Val Val Gln Pro Gly Arg
1               5                   10                  15
Ser Leu Arg Leu Ser Cys Ala Val Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Ala Ile Ser Tyr Asp Gly Ser Asn Lys Tyr Tyr Val Asp Ser Val
    50                  55                  60
Lys Gly Arg Phe Thr Ile Ser Arg Asp Asn Ser Lys Lys Thr Leu Tyr
65                  70                  75                  80
Leu Gln Met Asn Ser Leu Arg Ala Glu Asp Thr Ala Val Tyr Asn Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125

<210> 259
<211> 336
<212> DNA
<213> Artificial Sequence

<220>
<223> Synthetic

<400> 259
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa                           336

<210> 260
<211> 112
<212> PRT
<213> Artificial Sequence

<220>
<223> Synthetic

<400> 260
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 261
<211> 381
<212> DNA
<213> Artificial Sequence

<220>
<223> Synthetic

<400> 261

caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt atatcatatg atggaagtaa taaatactat 180
gcagactccg tgaagggccg attcaccatc tccagagaca attccaagaa cacgctgtat 240
ctgcaaatga acagcctgag agctgaggac acggctgtgt attactgtgc gaaaaatatt 300
gtactagtga tgtatgatat agactatcac tactatggga tggacgtctg gggccaaggg 360
accacggtca ccgtctcctc a 381

<210> 262
<211> 127
<212> PRT
<213> Artificial Sequence

<220>
<223> Synthetic

<400> 262
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125

<210> 263
<211> 336
<212> DNA
<213> Artificial Sequence

<220>
<223> Synthetic

<400> 263
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa 336

<210> 264
<211> 112
<212> PRT
<213> Artificial Sequence

<220>
<223> Synthetic

<400> 264
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile

65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 265
<211> 381
<212> DNA
<213> Artificial Sequence

<220>
<223> Synthetic

<400> 265
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a                                           381

<210> 266
<211> 127
<212> PRT
<213> Artificial Sequence

<220>
<223> Synthetic

<400> 266
Gln Val Gln Leu Val Glu Ser Gly Gly Gly Val Val Gln Pro Gly Arg
1               5                   10                  15
Ser Leu Arg Leu Ser Cys Ala Val Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Ala Ile Ser Tyr Asp Gly Ser Asn Lys Tyr Tyr Val Asp Ser Val
    50                  55                  60
Lys Gly Arg Phe Thr Ile Ser Arg Asp Asn Ser Lys Lys Thr Leu Tyr
65                  70                  75                  80
Leu Gln Met Asn Ser Leu Arg Ala Glu Asp Thr Ala Val Tyr Asn Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125

<210> 267
<211> 24
<212> DNA
<213> Artificial Sequence

<220>
<223> Synthetic

<400> 267
ggattcacct tcagtagcta tggc                                        24

<210> 268
<211> 8
<212> PRT
<213> Artificial Sequence

<220>
<223> Synthetic

<400> 268
Gly Phe Thr Phe Ser Ser Tyr Gly
1               5

<210> 269
<211> 24
<212> DNA
<213> Artificial Sequence

<220>
<223> Synthetic

<400> 269
atatcatatg atggaagtaa taaa                                              24

<210> 270
<211> 8
<212> PRT
<213> Artificial Sequence

<220>
<223> Synthetic

<400> 270
Ile Ser Tyr Asp Gly Ser Asn Lys
1               5

<210> 271
<211> 60
<212> DNA
<213> Artificial Sequence

<220>
<223> Synthetic

<400> 271
gcgaaaaata ttgtactagt gatgtatgat atagactatc actactatgg gatggacgtc 60

<210> 272
<211> 20
<212> PRT
<213> Artificial Sequence

<220>
<223> Synthetic

<400> 272
Ala Lys Asn Ile Val Leu Val Met Tyr Asp Ile Asp Tyr His Tyr Tyr
1               5                   10                  15
Gly Met Asp Val
            20

<210> 273
<211> 336
<212> DNA
<213> Artificial Sequence

<220>
<223> Synthetic

<400> 273
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180

tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaga 336

<210> 274
<211> 112
<212> PRT
<213> Artificial Sequence

<220>
<223> Synthetic

<400> 274
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Arg
            100                 105                 110

<210> 275
<211> 33
<212> DNA
<213> Artificial Sequence

<220>
<223> Synthetic

<400> 275
cagagcctcc tgcatagtaa tggatacaac tat 33

<210> 276
<211> 11
<212> PRT
<213> Artificial Sequence

<220>
<223> Synthetic

<400> 276
Gln Ser Leu Leu His Ser Asn Gly Tyr Asn Tyr
1               5                   10

<210> 277
<211> 9
<212> DNA
<213> Artificial Sequence

<220>
<223> Synthetic

<400> 277
ttgggtttt 9

<210> 278
<211> 3
<212> PRT
<213> Artificial Sequence

<220>
<223> Synthetic

<400> 278
Leu Gly Phe
1



<210> 279
<211> 27
<212> DNA
<213> Artificial Sequence

<220>
<223> Synthetic

<400> 279
atgcaagctc tacaaactcc tctcact                                          27

<210> 280
<211> 9
<212> PRT
<213> Artificial Sequence

<220>
<223> Synthetic

<400> 280
Met Gln Ala Leu Gln Thr Pro Leu Thr
1               5



<210> 281
<211> 381
<212> DNA
<213> Artificial Sequence

<220>
<223> Synthetic

<400> 281
caggtgcagc tggtggagtc tgggggaggc gtggtccagc ctgggaggtc cctgagactc 60
tcctgtgcag tctctggatt caccttcagt agctatggca tgcactgggt ccgccaggct 120
ccaggcaagg ggctggagtg ggtggcagct atatcatatg atggaagtaa taaatactat 180
gtagactccg tgaagggccg attcaccatc tccagagaca attccaagaa aacgctgtat 240
ctgcaaatga acagcctgag agctgaggac acggctgtgt ataattgtgc gaaaaatatt 300
gtactagtga tgtatgatat agactatcac tactatggga tggacgtctg gggccaaggg 360
accacggtca ccgtctcctc a                                                381

<210> 282
<211> 127
<212> PRT
<213> Artificial Sequence

<220>
<223> Synthetic

<400> 282
Gln Val Gln Leu Val Glu Ser Gly Gly Gly Val Val Gln Pro Gly Arg
1               5                   10                  15
Ser Leu Arg Leu Ser Cys Ala Val Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Ala Ile Ser Tyr Asp Gly Ser Asn Lys Tyr Tyr Val Asp Ser Val
    50                  55                  60
Lys Gly Arg Phe Thr Ile Ser Arg Asp Asn Ser Lys Lys Thr Leu Tyr
65                  70                  75                  80
Leu Gln Met Asn Ser Leu Arg Ala Glu Asp Thr Ala Val Tyr Asn Cys

85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125

<210> 283
<211> 336
<212> DNA
<213> Artificial Sequence

<220>
<223> Synthetic

<400> 283
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacaa ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa                           336

<210> 284
<211> 112
<212> PRT
<213> Artificial Sequence

<220>
<223> Synthetic

<400> 284
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 285
<211> 381
<212> DNA
<213> Artificial Sequence

<220>
<223> Synthetic

<400> 285
caggtgcagc tggtggagtc tgggggaggc gtggtccagc ctgggaggtc cctgagactc 60
tcctgtgcag cctctggatt caccttcagt agctatggca tgcactgggt ccgccaggct 120
ccaggcaagg ggctggagtg ggtggcagtt atatcatatg atggaagtaa taaatactat 180
gcagactccg tgaagggccg attcaccatc tccagagaca attccaagaa cacgctgtat 240
ctgcaaatga acagcctgag agctgaggac acggctgtgt attactgtgc gaaaaatatt 300
gtactagtga tgtatgatat agactatcac tactatggga tggacgtctg ggggcaaggg 360
accacggtca ccgtctcctc a                                           381

<210> 286
<211> 127
<212> PRT
<213> Artificial Sequence

<220>
<223> Synthetic

<400> 286
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Asn Ile Val Leu Val Met Tyr Asp Ile Asp Tyr His Tyr Tyr
            100                 105                 110
Gly Met Asp Val Trp Gly Gln Gly Thr Thr Val Thr Val Ser Ser
        115                 120                 125


<210> 287
<211> 336
<212> DNA
<213> Artificial Sequence

<220>
<223> Synthetic

<400> 287
gatattgtga tgactcagtc tccactctcc ctgcccgtca cccctggaga gccggcctcc 60
atctcctgca ggtctagtca gagcctcctg catagtaatg gatacaacta tttggattgg 120
tacctgcaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa                           336

<210> 288
<211> 112
<212> PRT
<213> Artificial Sequence

<220>
<223> Synthetic

<400> 288
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110


<210> 289
<211> 372
<212> DNA
<213> Artificial Sequence

<220>
<223> Synthetic

<400> 289
cagatcacct tgaaggagtc tggtcctacg ctggtaaaac ccacacagac cctcacgctg 60
acctgcacct tctctgggtt ctcactcagc gctagtggag tgggtgtggg ctggttccgt 120
cagcccccag gaaaggccct ggagtggctt gcactcattt attggaatga tgataagcgt 180
tacagcccat ctctaaagaa cagcctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct ggggccaagg gaccacggtc 360
accgtctcct ca 372

<210> 290
<211> 124
<212> PRT
<213> Artificial Sequence

<220>
<223> Synthetic

<400> 290
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Ala Ser
            20                  25                  30
Gly Val Gly Val Gly Trp Phe Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60
Leu Lys Asn Ser Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile His Leu Trp Ser Tyr Phe Tyr Tyr Gly Met Asp
            100                 105                 110
Val Trp Gly Gln Gly Thr Thr Val Thr Val Ser Ser
        115                 120

<210> 291
<211> 30
<212> DNA
<213> Artificial Sequence

<220>
<223> Synthetic

<400> 291
gggttctcac tcagcgctag tggagtgggt 30

<210> 292
<211> 10
<212> PRT
<213> Artificial Sequence

<220>
<223> Synthetic

<400> 292
Gly Phe Ser Leu Ser Ala Ser Gly Val Gly
1               5                   10

<210> 293
<211> 21
<212> DNA
<213> Artificial Sequence

<220>

<223> Synthetic

<400> 293
atttattgga atgatgataa g                                                21

<210> 294
<211> 7
<212> PRT
<213> Artificial Sequence

<220>
<223> Synthetic

<400> 294
Ile Tyr Trp Asn Asp Asp Lys
1               5


<210> 295
<211> 48
<212> DNA
<213> Artificial Sequence

<220>
<223> Synthetic

<400> 295
gcacacagaa tacatctatg gtcctacttc tactacggta tggacgtc                   48

<210> 296
<211> 16
<212> PRT
<213> Artificial Sequence

<220>
<223> Synthetic

<400> 296
Ala His Arg Ile His Leu Trp Ser Tyr Phe Tyr Tyr Gly Met Asp Val
1               5                   10                  15


<210> 297
<211> 336
<212> DNA
<213> Artificial Sequence

<220>
<223> Synthetic

<400> 297
gatattgtga tgactcagtc tccactctcc ctgcccgtca cccctggaga gccggcctcc 60
atctcctgca ggtctagtca gactctcctg catagtaatg gatacaacta tttcgattgg 120
tacctgcaga agccagggca gtctccacag ctcctgatct atttgggttc taatcgggcc 180
tccggggtcc ctgacagatt cagtggcagt ggatcaggca cagattttac actgaaaatc 240
agcagagtgg aggctgagga tgttggaatt tattactgca tgcaagctct acaaactcct 300
ctcactttcg gcggagggac caaggtggag atcaga                                336

<210> 298
<211> 112
<212> PRT
<213> Artificial Sequence

<220>
<223> Synthetic

<400> 298
Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Thr Leu Leu His Ser
            20                  25                  30
Asn Gly Tyr Asn Tyr Phe Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Arg
            100                 105                 110

<210> 299
<211> 33
<212> DNA
<213> Artificial Sequence

<220>
<223> Synthetic

<400> 299
cagactctcc tgcatagtaa tggatacaac tat                                33

<210> 300
<211> 11
<212> PRT
<213> Artificial Sequence

<220>
<223> Synthetic

<400> 300
Gln Thr Leu Leu His Ser Asn Gly Tyr Asn Tyr
1               5                   10

<210> 301
<211> 9
<212> DNA
<213> Artificial Sequence

<220>
<223> Synthetic

<400> 301
ttgggttct                                                            9

<210> 302
<211> 3
<212> PRT
<213> Artificial Sequence

<220>
<223> Synthetic

<400> 302
Leu Gly Ser
1

<210> 303
<211> 27
<212> DNA
<213> Artificial Sequence

<220>
<223> Synthetic

<400> 303
atgcaagctc tacaaactcc tctcact                                          27

<210> 304
<211> 9
<212> PRT
<213> Artificial Sequence

<220>
<223> Synthetic

<400> 304
Met Gln Ala Leu Gln Thr Pro Leu Thr
1               5

<210> 305
<211> 372
<212> DNA
<213> Artificial Sequence

<220>
<223> Synthetic

<400> 305
cagatcacct tgaaggagtc tggtcctacg ctggtaaaac ccacacagac cctcacgctg 60
acctgcacct tctctgggtt ctcactcagc gctagtggag tgggtgtggg ctggttccgt 120
cagcccccag gaaaggccct ggagtggctt gcactcattt attggaatga tgataagcgt 180
tacagcccat ctctaaagaa cagcctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct ggggccaagg gaccacggtc 360
accgtctcct ca                                                          372

<210> 306
<211> 124
<212> PRT
<213> Artificial Sequence

<220>
<223> Synthetic

<400> 306
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Ala Ser
            20                  25                  30
Gly Val Gly Val Gly Trp Phe Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60
Leu Lys Asn Ser Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg Ile His Leu Trp Ser Tyr Phe Tyr Tyr Gly Met Asp
            100                 105                 110
Val Trp Gly Gln Gly Thr Thr Val Thr Val Ser Ser
        115                 120

<210> 307
<211> 336
<212> DNA
<213> Artificial Sequence

<220>
<223> Synthetic

<400> 307
gatattgtga tgactcagtc tccactctcc ctgcccgtca cccctggaga gccggcctcc 60
atctcctgca ggtctagtca gactctcctg catagtaatg gatacaacta tttcgattgg 120
tacctgcaga agccagggca gtctccacag ctcctgatct atttgggttc taatcgggcc 180
tccggggtcc ctgacagatt cagtggcagt ggatcaggca cagattttac actgaaaatc 240
agcagagtgg aggctgagga tgttggaatt tattactgca tgcaagctct acaaactcct 300
ctcactttcg gcggagggac caaggtggag atcaaa 336

<210> 308
<211> 112
<212> PRT
<213> Artificial Sequence

<220>
<223> Synthetic

<400> 308
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Thr Leu Leu His Ser
            20                  25                  30
Asn Gly Tyr Asn Tyr Phe Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Ile Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 309
<211> 372
<212> DNA
<213> Artificial Sequence

<220>
<223> Synthetic

<400> 309
cagatcacct tgaaggagtc tggtcctacg ctggtgaaac ccacacagac cctcacgctg 60
acctgcacct tctctgggtt ctcactcagc gctagtggag tgggtgtggg ctggatccgt 120
cagcccccag gaaaggccct ggagtggctt gcactcattt attggaatga tgataagcgc 180
tacagcccat ctctgaagag caggctcacc atcaccaagg acacctccaa aaaccaggtg 240
gtccttacaa tgaccaacat ggaccctgtg gacacagcca catattactg tgcacacaga 300
atacatctat ggtcctactt ctactacggt atggacgtct gggggcaagg gaccacggtc 360
accgtctcct ca 372

<210> 310
<211> 124
<212> PRT
<213> Artificial Sequence

<220>
<223> Synthetic

<400> 310
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Ala Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60
Leu Lys Ser Arg Leu Thr Ile Thr Lys Asp Thr Ser Lys Asn Gln Val
65                  70                  75                  80

Val Leu Thr Met Thr Asn Met Asp Pro Val Asp Thr Ala Thr Tyr Tyr
                85                  90                  95
Cys Ala His Arg Ile His Leu Trp Ser Tyr Phe Tyr Tyr Gly Met Asp
            100                 105                 110
Val Trp Gly Gln Gly Thr Thr Val Thr Val Ser Ser
        115                 120

<210> 311
<211> 336
<212> DNA
<213> Artificial Sequence

<220>
<223> Synthetic

<400> 311
gatattgtga tgactcagtc tccactctcc ctgcccgtca cccctggaga gccggcctcc 60
atctcctgca ggtctagtca gactctcctg catagtaatg gatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct acaaactcct 300
ctcactttcg gcggagggac caaggtggag atcaaa                           336

<210> 312
<211> 112
<212> PRT
<213> Artificial Sequence

<220>
<223> Synthetic

<400> 312
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Thr Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Leu Thr Phe Gly Gly Gly Thr Lys Val Glu Ile Lys
            100                 105                 110

<210> 313
<211> 381
<212> DNA
<213> Artificial Sequence

<220>
<223> Synthetic

<400> 313
caggttcagc tggtgcagtc tggacctgag gtgaagaagc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattcc tactacgtta tggacgtctg gggccaaggg 360
accacggtca ccgtctcctc a                                           381

<210> 314
<211> 127
<212> PRT

<213> Artificial Sequence

<220>
<223> Synthetic

<400> 314
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Asn Tyr Ser Tyr Tyr
            100                 105                 110
Val Met Asp Val Trp Gly Gln Gly Thr Thr Val Thr Val Ser Ser
        115                 120                 125


<210> 315
<211> 24
<212> DNA
<213> Artificial Sequence

<220>
<223> Synthetic

<400> 315
ggttacacct ttaccaccta tggt                                              24

<210> 316
<211> 8
<212> PRT
<213> Artificial Sequence

<220>
<223> Synthetic

<400> 316
Gly Tyr Thr Phe Thr Thr Tyr Gly
1               5


<210> 317
<211> 24
<212> DNA
<213> Artificial Sequence

<220>
<223> Synthetic

<400> 317
atcagcggtt acaatggtaa aaca                                              24

<210> 318
<211> 8
<212> PRT
<213> Artificial Sequence

<220>
<223> Synthetic

<400> 318
Ile Ser Gly Tyr Asn Gly Lys Thr

1                 5


<210> 319
<211> 60
<212> DNA
<213> Artificial Sequence

<220>
<223> Synthetic

<400> 319
tcgagagatc gtttagtagt accacctgcc cttaattatt cctactacgt tatggacgtc 60


<210> 320
<211> 20
<212> PRT
<213> Artificial Sequence

<220>
<223> Synthetic

<400> 320
Ser Arg Asp Arg Leu Val Val Pro Pro Ala Leu Asn Tyr Ser Tyr Tyr
1               5                   10                  15
Val Met Asp Val
            20


<210> 321
<211> 336
<212> DNA
<213> Artificial Sequence

<220>
<223> Synthetic

<400> 321
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tctcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 322
<211> 112
<212> PRT
<213> Artificial Sequence

<220>
<223> Synthetic

<400> 322
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Ser Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 323
<211> 33
<212> DNA
<213> Artificial Sequence

<220>
<223> Synthetic

<400> 323
caaagcctcg tatacagtga tggaaacacc tac                                    33

<210> 324
<211> 11
<212> PRT
<213> Artificial Sequence

<220>
<223> Synthetic

<400> 324
Gln Ser Leu Val Tyr Ser Asp Gly Asn Thr Tyr
1               5                   10

<210> 325
<211> 9
<212> DNA
<213> Artificial Sequence

<220>
<223> Synthetic

<400> 325
aaggtttct                                                               9

<210> 326
<211> 3
<212> PRT
<213> Artificial Sequence

<220>
<223> Synthetic

<400> 326
Lys Val Ser
1

<210> 327
<211> 27
<212> DNA
<213> Artificial Sequence

<220>
<223> Synthetic

<400> 327
atgcaaggta cacactggcc gtacact                                           27

<210> 328
<211> 9
<212> PRT
<213> Artificial Sequence

<220>
<223> Synthetic

<400> 328
Met Gln Gly Thr His Trp Pro Tyr Thr
1               5

<210> 329
<211> 381
<212> DNA
<213> Artificial Sequence

<220>
<223> Synthetic

<400> 329
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattcc tactacgtta tggacgtctg gggccaaggg 360
accacggtca ccgtctcctc a                                           381

<210> 330
<211> 127
<212> PRT
<213> Artificial Sequence

<220>
<223> Synthetic

<400> 330
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Asn Tyr Ser Tyr Tyr
            100                 105                 110
Val Met Asp Val Trp Gly Gln Gly Thr Thr Val Thr Val Ser Ser
        115                 120                 125

<210> 331
<211> 336
<212> DNA
<213> Artificial Sequence

<220>
<223> Synthetic

<400> 331
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tctcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 332
<211> 112
<212> PRT
<213> Artificial Sequence

<220>
<223> Synthetic

<400> 332
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Ser Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 333
<211> 381
<212> DNA
<213> Artificial Sequence

<220>
<223> Synthetic

<400> 333
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct taattattcc tactacgtta tggacgtctg ggggcaaggg 360
accacggtca ccgtctcctc a                                           381

<210> 334
<211> 127
<212> PRT
<213> Artificial Sequence

<220>
<223> Synthetic

<400> 334
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Asn Tyr Ser Tyr Tyr
            100                 105                 110
Val Met Asp Val Trp Gly Gln Gly Thr Thr Val Thr Val Ser Ser
        115                 120                 125


<210> 335
<211> 336
<212> DNA
<213> Artificial Sequence

<220>
<223> Synthetic

<400> 335
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 336
<211> 112
<212> PRT
<213> Artificial Sequence

<220>
<223> Synthetic

<400> 336
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 337
<211> 354
<212> DNA
<213> Artificial Sequence

<220>
<223> Synthetic

<400> 337
gaggtgcagc tggtggagtc tgggggaggc ctggtcaagc ctggggggtc cctgagactc 60
tcctgtgcag cctctggatt caccttcagt agctatagca tggactgggt ccgccaggct 120
ccagggaagg ggctggagtg ggtctcatcc attagtagta gtagtagtta catatactac 180
gcagactctg tgaagggccg attcaccatc tccagagaca ccgccaagaa ctcactgtat 240
ctgcaaatga acagcctgag agacgaggac acggctgttt attactgtgc gagagagggc 300
agtagcagac tttttgacta ctggggccag ggaaccctgg tcaccgtctc ctca 354

<210> 338
<211> 118
<212> PRT
<213> Artificial Sequence

<220>
<223> Synthetic

<400> 338
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asp Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val

Lys Gly Arg Phe Thr Ile Ser Arg Asp Thr Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg Glu Gly Ser Ser Arg Leu Phe Asp Tyr Trp Gly Gln Gly Thr
            100                 105                 110
Leu Val Thr Val Ser Ser
        115

<210> 339
<211> 24
<212> DNA
<213> Artificial Sequence

<220>
<223> Synthetic

<400> 339
ggattcacct tcagtagcta tagc                                              24

<210> 340
<211> 8
<212> PRT
<213> Artificial Sequence

<220>
<223> Synthetic

<400> 340
Gly Phe Thr Phe Ser Ser Tyr Ser
1               5

<210> 341
<211> 24
<212> DNA
<213> Artificial Sequence

<220>
<223> Synthetic

<400> 341
attagtagta gtagtagtta cata                                              24

<210> 342
<211> 8
<212> PRT
<213> Artificial Sequence

<220>
<223> Synthetic

<400> 342
Ile Ser Ser Ser Ser Ser Tyr Ile
1               5

<210> 343
<211> 33
<212> DNA
<213> Artificial Sequence

<220>
<223> Synthetic

<400> 343
gcgagagagg gcagtagcag actttttgac tac                                    33

<210> 344
<211> 11
<212> PRT
<213> Artificial Sequence

<220>
<223> Synthetic

<400> 344
Ala Arg Glu Gly Ser Ser Arg Leu Phe Asp Tyr
1               5                   10


<210> 345
<211> 321
<212> DNA
<213> Artificial Sequence

<220>
<223> Synthetic

<400> 345
gacatccaga tgacccagtc tccttccacc ctgtctgcat ctgtaggaga cagagtcacc 60
atcacttgcc gggccagtca gagtattagt agctggttgg cctggtatca gcagagacca 120
gggaaagccc ctaagctcct gatctataag gcgtctagtt tagaaggtgg agtcccatca 180
aggttcagcg gcagtggatc tgggacagaa ttcactctca ccatcagcag cctgcagcct 240
gaggattttg caacttatta ctgccaacag tataatagtt attggtacac ttttggccag 300
gggaccaagc tggagatcaa a                                           321

<210> 346
<211> 107
<212> PRT
<213> Artificial Sequence

<220>
<223> Synthetic

<400> 346
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Arg Pro Gly Lys Ala Pro Lys Leu Leu Ile
        35                  40                  45
Tyr Lys Ala Ser Ser Leu Glu Gly Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Tyr Asn Ser Tyr Trp Tyr
                85                  90                  95
Thr Phe Gly Gln Gly Thr Lys Leu Glu Ile Lys
            100                 105


<210> 347
<211> 18
<212> DNA
<213> Artificial Sequence

<220>
<223> Synthetic

<400> 347
cagagtatta gtagctgg                                               18

<210> 348
<211> 6
<212> PRT

<213> Artificial Sequence

<220>
<223> Synthetic

<400> 348
Gln Ser Ile Ser Ser Trp
1               5


<210> 349
<211> 9
<212> DNA
<213> Artificial Sequence

<220>
<223> Synthetic

<400> 349
aaggcgtct                                                              9

<210> 350
<211> 3
<212> PRT
<213> Artificial Sequence

<220>
<223> Synthetic

<400> 350
Lys Ala Ser
1


<210> 351
<211> 27
<212> DNA
<213> Artificial Sequence

<220>
<223> Synthetic

<400> 351
caacagtata atagttattg gtacact                                          27

<210> 352
<211> 9
<212> PRT
<213> Artificial Sequence

<220>
<223> Synthetic

<400> 352
Gln Gln Tyr Asn Ser Tyr Trp Tyr Thr
1               5


<210> 353
<211> 354
<212> DNA
<213> Artificial Sequence

<220>
<223> Synthetic

<400> 353
gaggtgcagc tggtggagtc tgggggaggc ctggtcaagc ctggggggtc cctgagactc 60
tcctgtgcag cctctggatt caccttcagt agctatagca tggactgggt ccgccaggct 120

ccagggaagg ggctggagtg ggtctcatcc attagtagta gtagtagtta catatactac 180
gcagactctg tgaagggccg attcaccatc tccagagaca ccgccaagaa ctcactgtat 240
ctgcaaatga acagcctgag agacgaggac acggctgttt attactgtgc gagagagggc 300
agtagcagac tttttgacta ctggggccag ggaaccctgg tcaccgtctc ctca 354

<210> 354
<211> 118
<212> PRT
<213> Artificial Sequence

<220>
<223> Synthetic

<400> 354
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asp Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Thr Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg Glu Gly Ser Ser Arg Leu Phe Asp Tyr Trp Gly Gln Gly Thr
            100                 105                 110
Leu Val Thr Val Ser Ser
        115

<210> 355
<211> 321
<212> DNA
<213> Artificial Sequence

<220>
<223> Synthetic

<400> 355
gacatccaga tgacccagtc tccttccacc ctgtctgcat ctgtaggaga cagagtcacc 60
atcacttgcc gggccagtca gagtattagt agctggttgg cctggtatca gcagagacca 120
gggaaagccc ctaagctcct gatctataag gcgtctagtt tagaaggtgg agtcccatca 180
aggttcagcg gcagtggatc tgggacagaa ttcactctca ccatcagcag cctgcagcct 240
gaggattttg caacttatta ctgccaacag tataatagtt attggtacac ttttggccag 300
gggaccaagc tggagatcaa a 321

<210> 356
<211> 107
<212> PRT
<213> Artificial Sequence

<220>
<223> Synthetic

<400> 356
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Arg Pro Gly Lys Ala Pro Lys Leu Leu Ile
        35                  40                  45
Tyr Lys Ala Ser Ser Leu Glu Gly Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Tyr Asn Ser Tyr Trp Tyr
                85                  90                  95

Thr Phe Gly Gln Gly Thr Lys Leu Glu Ile Lys
            100                 105

<210> 357
<211> 354
<212> DNA
<213> Artificial Sequence

<220>
<223> Synthetic

<400> 357
gaggtgcagc tggtggagtc tgggggaggc ctggtcaagc ctggggggtc cctgagactc 60
tcctgtgcag cctctggatt caccttcagt agctatagca tgaactgggt ccgccaggct 120
ccagggaagg ggctggagtg ggtctcatcc attagtagta gtagtagtta catatactac 180
gcagactcag tgaagggccg attcaccatc tccagagaca acgccaagaa ctcactgtat 240
ctgcaaatga acagcctgag agccgaggac acggctgtgt attactgtgc gagagagggc 300
agtagcagac tttttgacta ctggggccaa ggaaccctgg tcaccgtctc ctca       354

<210> 358
<211> 118
<212> PRT
<213> Artificial Sequence

<220>
<223> Synthetic

<400> 358
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asn Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Glu Gly Ser Ser Arg Leu Phe Asp Tyr Trp Gly Gln Gly Thr
            100                 105                 110
Leu Val Thr Val Ser Ser
        115

<210> 359
<211> 321
<212> DNA
<213> Artificial Sequence

<220>
<223> Synthetic

<400> 359
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tataatagtt attggtacac ttttggccag 300
gggaccaagc tggagatcaa a                                           321

<210> 360
<211> 107
<212> PRT
<213> Artificial Sequence

<220>

<223> Synthetic

<400> 360
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Trp Tyr
                85                  90                  95
Thr Phe Gly Gln Gly Thr Lys Leu Glu Ile Lys
            100                 105


<210> 361
<211> 384
<212> DNA
<213> Artificial Sequence

<220>
<223> Synthetic

<400> 361
caggtgcacc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtcgc ctca                                        384

<210> 362
<211> 128
<212> PRT
<213> Artificial Sequence

<220>
<223> Synthetic

<400> 362
Gln Val His Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ala Ser
        115                 120                 125


<210> 363
<211> 24
<212> DNA
<213> Artificial Sequence

<220>

<223> Synthetic

<400> 363
ggattcacct tcagtgacca ctac                                              24

<210> 364
<211> 8
<212> PRT
<213> Artificial Sequence

<220>
<223> Synthetic

<400> 364
Gly Phe Thr Phe Ser Asp His Tyr
1               5


<210> 365
<211> 24
<212> DNA
<213> Artificial Sequence

<220>
<223> Synthetic

<400> 365
attagtaatg atggtggtac caaa                                              24

<210> 366
<211> 8
<212> PRT
<213> Artificial Sequence

<220>
<223> Synthetic

<400> 366
Ile Ser Asn Asp Gly Gly Thr Lys
1               5


<210> 367
<211> 63
<212> DNA
<213> Artificial Sequence

<220>
<223> Synthetic

<400> 367
gcgagagatc agggatatat tggctacgac tcgtattatt actattccta cggtatggac 60
gtc                                                                     63

<210> 368
<211> 21
<212> PRT
<213> Artificial Sequence

<220>
<223> Synthetic

<400> 368
Ala Arg Asp Gln Gly Tyr Ile Gly Tyr Asp Ser Tyr Tyr Tyr Tyr Ser
1               5                   10                  15
Tyr Gly Met Asp Val
            20

<210> 369
<211> 321
<212> DNA
<213> Artificial Sequence

<220>
<223> Synthetic

<400> 369
aaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tctcggcgga 300
gggaccaagg tggagatcaa g 321

<210> 370
<211> 107
<212> PRT
<213> Artificial Sequence

<220>
<223> Synthetic

<400> 370
Lys Ile Val Leu Thr Gln Ser Pro Gly Thr Leu Pro Leu Phe Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Ser Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Gly Leu Glu
65                  70                  75                  80
Pro Glu Asp Phe Glu Val Tyr Tyr Cys Gln Val Tyr Gly Asn Ser Leu
                85                  90                  95
Thr Leu Gly Gly Gly Thr Lys Val Glu Ile Lys
            100                 105

<210> 371
<211> 21
<212> DNA
<213> Artificial Sequence

<220>
<223> Synthetic

<400> 371
cagagtgtta acaacaaatt c 21

<210> 372
<211> 7
<212> PRT
<213> Artificial Sequence

<220>
<223> Synthetic

<400> 372
Gln Ser Val Asn Asn Lys Phe
1               5

<210> 373
<211> 9
<212> DNA
<213> Artificial Sequence

<220>
<223> Synthetic

<400> 373
ggtgcatcc                                                              9

<210> 374
<211> 3
<212> PRT
<213> Artificial Sequence

<220>
<223> Synthetic

<400> 374
Gly Ala Ser
1


<210> 375
<211> 24
<212> DNA
<213> Artificial Sequence

<220>
<223> Synthetic

<400> 375
caagtatatg gtaactcact cact                                            24

<210> 376
<211> 8
<212> PRT
<213> Artificial Sequence

<220>
<223> Synthetic

<400> 376
Gln Val Tyr Gly Asn Ser Leu Thr
 1               5


<210> 377
<211> 384
<212> DNA
<213> Artificial Sequence

<220>
<223> Synthetic

<400> 377
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtctc ctca                                            384

<210> 378
<211> 128
<212> PRT
<213> Artificial Sequence

<220>
<223> Synthetic

<400> 378
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125

<210> 379
<211> 321
<212> DNA
<213> Artificial Sequence

<220>
<223> Synthetic

<400> 379
gaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tctcggcgga 300
gggaccaagg tggagatcaa a                                           321

<210> 380
<211> 107
<212> PRT
<213> Artificial Sequence

<220>
<223> Synthetic

<400> 380
Glu Ile Val Leu Thr Gln Ser Pro Gly Thr Leu Pro Leu Phe Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Ser Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Gly Leu Glu
65                  70                  75                  80
Pro Glu Asp Phe Glu Val Tyr Tyr Cys Gln Val Tyr Gly Asn Ser Leu
                85                  90                  95
Thr Leu Gly Gly Gly Thr Lys Val Glu Ile Lys
            100                 105

<210> 381
<211> 384
<212> DNA
<213> Artificial Sequence

<220>
<223> Synthetic

<400> 381

caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg ggtttcatac attagtaatg atggtggtac caaatactac 180
gcagactctg tgaagggccg attcaccatc tccagggaca acgccaagaa ctcactgtat 240
ctgcaaatga acagcctgag agccgaggac acggccgtgt attactgtgc gagagatcag 300
ggatatattg gctacgactc gtattattac tattcctacg gtatggacgt ctgggggcaa 360
gggaccacgg tcaccgtctc ctca 384

<210> 382
<211> 128
<212> PRT
<213> Artificial Sequence

<220>
<223> Synthetic

<400> 382
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Val
        35                  40                  45
Ser Tyr Ile Ser Asn Asp Gly Gly Thr Lys Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125

<210> 383
<211> 321
<212> DNA
<213> Artificial Sequence

<220>
<223> Synthetic

<400> 383
gaaattgtgt tgacgcagtc tccaggcacc ctgtctttgt ctccagggga aagagccacc 60
ctctcctgca gggccagtca gagtgttaac aacaaattct tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcaa gtatatggta actcactcac tttcggcgga 300
gggaccaagg tggagatcaa a 321

<210> 384
<211> 107
<212> PRT
<213> Artificial Sequence

<220>
<223> Synthetic

<400> 384
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu

65                  70                  75                  80
Pro Glu Asp Phe Ala Val Tyr Tyr Cys Gln Val Tyr Gly Asn Ser Leu
                85                  90                  95
Thr Phe Gly Gly Gly Thr Lys Val Glu Ile Lys
            100                 105

<210> 385
<211> 360
<212> DNA
<213> Artificial Sequence

<220>
<223> Synthetic

<400> 385
gaggtgcaga aggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 386
<211> 120
<212> PRT
<213> Artificial Sequence

<220>
<223> Synthetic

<400> 386
Glu Val Gln Lys Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 387
<211> 24
<212> DNA
<213> Artificial Sequence

<220>
<223> Synthetic

<400> 387
ggattcacct tcagtactta taac                                        24

<210> 388
<211> 8
<212> PRT
<213> Artificial Sequence

<220>
<223> Synthetic

<400> 388
Gly Phe Thr Phe Ser Thr Tyr Asn
1               5

<210> 389
<211> 24
<212> DNA
<213> Artificial Sequence

<220>
<223> Synthetic

<400> 389
attaggagta gtagtaatta cata                                              24

<210> 390
<211> 8
<212> PRT
<213> Artificial Sequence

<220>
<223> Synthetic

<400> 390
Ile Arg Ser Ser Ser Asn Tyr Ile
1               5

<210> 391
<211> 39
<212> DNA
<213> Artificial Sequence

<220>
<223> Synthetic

<400> 391
gcgagagatg gcagcagttg gtacgactac tctgactac                              39

<210> 392
<211> 13
<212> PRT
<213> Artificial Sequence

<220>
<223> Synthetic

<400> 392
Ala Arg Asp Gly Ser Ser Trp Tyr Asp Tyr Ser Asp Tyr
1               5                   10

<210> 393
<211> 321
<212> DNA
<213> Artificial Sequence

<220>
<223> Synthetic

<400> 393
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                                 321

<210> 394
<211> 107
<212> PRT
<213> Artificial Sequence

<220>
<223> Synthetic

<400> 394
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105


<210> 395
<211> 18
<212> DNA
<213> Artificial Sequence

<220>
<223> Synthetic

<400> 395
cagagtatta gtagctgg                                                    18

<210> 396
<211> 6
<212> PRT
<213> Artificial Sequence

<220>
<223> Synthetic

<400> 396
Gln Ser Ile Ser Ser Trp
1               5


<210> 397
<211> 9
<212> DNA
<213> Artificial Sequence

<220>
<223> Synthetic

<400> 397
aaggcgtct                                                              9

<210> 398
<211> 3
<212> PRT
<213> Artificial Sequence

<220>
<223> Synthetic

<400> 398
Lys Ala Ser
1

<210> 399
<211> 27
<212> DNA
<213> Artificial Sequence

<220>
<223> Synthetic

<400> 399
caacagtata ttagttattc tcggacg 27

<210> 400
<211> 9
<212> PRT
<213> Artificial Sequence

<220>
<223> Synthetic

<400> 400
Gln Gln Tyr Ile Ser Tyr Ser Arg Thr
1               5

<210> 401
<211> 360
<212> DNA
<213> Artificial Sequence

<220>
<223> Synthetic

<400> 401
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 402
<211> 120
<212> PRT
<213> Artificial Sequence

<220>
<223> Synthetic

<400> 402
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110

Gly Thr Leu Val Thr Val Ser Ser
        115                 120

<210> 403
<211> 321
<212> DNA
<213> Artificial Sequence

<220>
<223> Synthetic

<400> 403
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 404
<211> 107
<212> PRT
<213> Artificial Sequence

<220>
<223> Synthetic

<400> 404
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 405
<211> 360
<212> DNA
<213> Artificial Sequence

<220>
<223> Synthetic

<400> 405
gaggtgcagc tggtggagtc tgggggaggc ctggtcaagc ctggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccaaggaa ccctggtcac cgtctcctca 360

<210> 406
<211> 120
<212> PRT
<213> Artificial Sequence

<220>
<223> Synthetic

<400> 406
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 407
<211> 321
<212> DNA
<213> Artificial Sequence

<220>
<223> Synthetic

<400> 407
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a                                           321

<210> 408
<211> 107
<212> PRT
<213> Artificial Sequence

<220>
<223> Synthetic

<400> 408
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 409
<211> 360
<212> DNA
<213> Artificial Sequence

<220>
<223> Synthetic

<400> 409
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 410
<211> 120
<212> PRT
<213> Artificial Sequence

<220>
<223> Synthetic

<400> 410
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 411
<211> 24
<212> DNA
<213> Artificial Sequence

<220>
<223> Synthetic

<400> 411
ggattcacct tcagtactta taac                                          24

<210> 412
<211> 8
<212> PRT
<213> Artificial Sequence

<220>
<223> Synthetic

<400> 412
Gly Phe Thr Phe Ser Thr Tyr Asn
1               5

<210> 413
<211> 24
<212> DNA
<213> Artificial Sequence

<220>
<223> Synthetic

<400> 413

attaggagta gtagtaatta cata                                              24

<210> 414
<211> 8
<212> PRT
<213> Artificial Sequence

<220>
<223> Synthetic

<400> 414
Ile Arg Ser Ser Ser Asn Tyr Ile
1               5


<210> 415
<211> 39
<212> DNA
<213> Artificial Sequence

<220>
<223> Synthetic

<400> 415
gcgagagatg gcagcagttg gtacgactac tctgactac                             39

<210> 416
<211> 13
<212> PRT
<213> Artificial Sequence

<220>
<223> Synthetic

<400> 416
Ala Arg Asp Gly Ser Ser Trp Tyr Asp Tyr Ser Asp Tyr
1               5                   10


<210> 417
<211> 321
<212> DNA
<213> Artificial Sequence

<220>
<223> Synthetic

<400> 417
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                                321

<210> 418
<211> 107
<212> PRT
<213> Artificial Sequence

<220>
<223> Synthetic

<400> 418
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile

                35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 419
<211> 18
<212> DNA
<213> Artificial Sequence

<220>
<223> Synthetic

<400> 419
cagagtatta gtagctgg                                                    18

<210> 420
<211> 6
<212> PRT
<213> Artificial Sequence

<220>
<223> Synthetic

<400> 420
Gln Ser Ile Ser Ser Trp
1               5

<210> 421
<211> 9
<212> DNA
<213> Artificial Sequence

<220>
<223> Synthetic

<400> 421
aaggcgtct                                                              9

<210> 422
<211> 3
<212> PRT
<213> Artificial Sequence

<220>
<223> Synthetic

<400> 422
Lys Ala Ser
1

<210> 423
<211> 27
<212> DNA
<213> Artificial Sequence

<220>
<223> Synthetic

<400> 423
caacagtata ttagttattc tcggacg                                          27

<210> 424
<211> 9
<212> PRT
<213> Artificial Sequence

<220>
<223> Synthetic

<400> 424
Gln Gln Tyr Ile Ser Tyr Ser Arg Thr
1               5


<210> 425
<211> 360
<212> DNA
<213> Artificial Sequence

<220>
<223> Synthetic

<400> 425
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360


<210> 426
<211> 120
<212> PRT
<213> Artificial Sequence

<220>
<223> Synthetic

<400> 426
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 427
<211> 321
<212> DNA
<213> Artificial Sequence

<220>
<223> Synthetic

<400> 427
gacatccaga tgacccagtc tccttccacc ctgtctgcat ctgtaggaga cagagtcacc 60
atcacttgcc gggccagtca gagtattagt agctggttgg cctggtatca acagatacca 120

gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a 321

<210> 428
<211> 107
<212> PRT
<213> Artificial Sequence

<220>
<223> Synthetic

<400> 428
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 429
<211> 360
<212> DNA
<213> Artificial Sequence

<220>
<223> Synthetic

<400> 429
gaggtgcagc tggtggagtc tgggggaggc ctggtcaagc ctggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccaaggaa ccctggtcac cgtctcctca 360

<210> 430
<211> 120
<212> PRT
<213> Artificial Sequence

<220>
<223> Synthetic

<400> 430
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln

                100                 105                 110
Gly Thr Leu Val Thr Val Ser Ser
        115                 120

<210> 431
<211> 321
<212> DNA
<213> Artificial Sequence

<220>
<223> Synthetic

<400> 431
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a                                           321

<210> 432
<211> 107
<212> PRT
<213> Artificial Sequence

<220>
<223> Synthetic

<400> 432
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 433
<211> 360
<212> DNA
<213> Artificial Sequence

<220>
<223> Synthetic

<400> 433
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg ggctggagtg ggtctcatcc attaggagta gtagtaatta catatactac 180
gcagactcag tgaagggccg attcaccatc tccagagaca acgccaagag ttcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360

<210> 434
<211> 120
<212> PRT
<213> Artificial Sequence

<220>

<223> Synthetic

<400> 434
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Ser Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 435
<211> 24
<212> DNA
<213> Artificial Sequence

<220>
<223> Synthetic

<400> 435
ggattcacct tcagtactta taac                                              24

<210> 436
<211> 8
<212> PRT
<213> Artificial Sequence

<220>
<223> Synthetic

<400> 436
Gly Phe Thr Phe Ser Thr Tyr Asn
1               5


<210> 437
<211> 24
<212> DNA
<213> Artificial Sequence

<220>
<223> Synthetic

<400> 437
attaggagta gtagtaatta cata                                              24

<210> 438
<211> 8
<212> PRT
<213> Artificial Sequence

<220>
<223> Synthetic

<400> 438
Ile Arg Ser Ser Ser Asn Tyr Ile
1               5

<210> 439
<211> 39
<212> DNA
<213> Artificial Sequence

<220>
<223> Synthetic

<400> 439
gcgagagatg gcagcagttg gtacgactac tctgactac                              39

<210> 440
<211> 13
<212> PRT
<213> Artificial Sequence

<220>
<223> Synthetic

<400> 440
Ala Arg Asp Gly Ser Ser Trp Tyr Asp Tyr Ser Asp Tyr
1               5                   10


<210> 441
<211> 321
<212> DNA
<213> Artificial Sequence

<220>
<223> Synthetic

<400> 441
gacatccaga tgacccagtc tccttccacc ctgtctgcat ctgtaggaga cagagtcacc 60
atcacttgcc gggccagtca gagtattagt agctggttgg cctggtatca acaggtacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 442
<211> 107
<212> PRT
<213> Artificial Sequence

<220>
<223> Synthetic

<400> 442
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Val Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105


<210> 443
<211> 18
<212> DNA
<213> Artificial Sequence

<220>
<223> Synthetic

<400> 443
cagagtatta gtagctgg                                                    18

<210> 444
<211> 6
<212> PRT
<213> Artificial Sequence

<220>
<223> Synthetic

<400> 444
Gln Ser Ile Ser Ser Trp
1               5


<210> 445
<211> 9
<212> DNA
<213> Artificial Sequence

<220>
<223> Synthetic

<400> 445
aaggcgtct                                                              9

<210> 446
<211> 3
<212> PRT
<213> Artificial Sequence

<220>
<223> Synthetic

<400> 446
Lys Ala Ser
1


<210> 447
<211> 27
<212> DNA
<213> Artificial Sequence

<220>
<223> Synthetic

<400> 447
caacagtata ttagttattc tcggacg                                          27

<210> 448
<211> 9
<212> PRT
<213> Artificial Sequence

<220>
<223> Synthetic

<400> 448
Gln Gln Tyr Ile Ser Tyr Ser Arg Thr
1               5


<210> 449

<211> 360
<212> DNA
<213> Artificial Sequence

<220>
<223> Synthetic

<400> 449
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagag ttcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360


<210> 450
<211> 120
<212> PRT
<213> Artificial Sequence

<220>
<223> Synthetic

<400> 450
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Ser Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 451
<211> 321
<212> DNA
<213> Artificial Sequence

<220>
<223> Synthetic

<400> 451
gacatccaga tgacccagtc tccttccacc ctgtctgcat ctgtaggaga cagagtcacc 60
atcacttgcc gggccagtca gagtattagt agctggttgg cctggtatca acaggtacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 452
<211> 107
<212> PRT
<213> Artificial Sequence

<220>
<223> Synthetic

<400> 452
Asp Ile Gln Met Thr Gln Ser Pro Ser Thr Leu Ser Ala Ser Val Gly

1               5                   10                  15
Asp Arg Val Thr Ile Thr Cys Arg Ala Ser Gln Ser Ile Ser Ser Trp
            20                  25                  30
Leu Ala Trp Tyr Gln Gln Val Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 453
<211> 360
<212> DNA
<213> Artificial Sequence

<220>
<223> Synthetic

<400> 453
gaggtgcagc tggtggagtc tgggggaggc ctggtcaagc ctggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccagggaa ccctggtcac cgtctcctca 360

<210> 454
<211> 120
<212> PRT
<213> Artificial Sequence

<220>
<223> Synthetic

<400> 454
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 455
<211> 321
<212> DNA
<213> Artificial Sequence

<220>
<223> Synthetic

<400> 455
gacatccaga tgacccagtc tccttccacc ctgtctgcat ctgtaggaga cagagtcacc 60

atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a 321

<210> 456
<211> 107
<212> PRT
<213> Artificial Sequence

<220>
<223> Synthetic

<400> 456
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 457
<211> 360
<212> DNA
<213> Artificial Sequence

<220>
<223> Synthetic

<400> 457
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 458
<211> 120
<212> PRT
<213> Artificial Sequence

<220>
<223> Synthetic

<400> 458
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95

Ala Arg Asp Gly Ser Ser Trp Tyr Asp Tyr Ser Asp Tyr Trp Gly Gln
            100                 105                 110
Gly Thr Leu Val Thr Val Ser Ser
        115                 120

<210> 459
<211> 24
<212> DNA
<213> Artificial Sequence

<220>
<223> Synthetic

<400> 459
ggattcacct tcagtactta taac                                              24

<210> 460
<211> 8
<212> PRT
<213> Artificial Sequence

<220>
<223> Synthetic

<400> 460
Gly Phe Thr Phe Ser Thr Tyr Asn
1               5

<210> 461
<211> 24
<212> DNA
<213> Artificial Sequence

<220>
<223> Synthetic

<400> 461
attaggagta gtagtaatta cata                                              24

<210> 462
<211> 8
<212> PRT
<213> Artificial Sequence

<220>
<223> Synthetic

<400> 462
Ile Arg Ser Ser Ser Asn Tyr Ile
1               5

<210> 463
<211> 39
<212> DNA
<213> Artificial Sequence

<220>
<223> Synthetic

<400> 463
gcgagagatg gcagcagttg gtacgactac tctgactac                              39

<210> 464
<211> 13
<212> PRT
<213> Artificial Sequence

<220>
<223> Synthetic

<400> 464
Ala Arg Asp Gly Ser Ser Trp Tyr Asp Tyr Ser Asp Tyr
1               5                   10

<210> 465
<211> 321
<212> DNA
<213> Artificial Sequence

<220>
<223> Synthetic

<400> 465
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 466
<211> 107
<212> PRT
<213> Artificial Sequence

<220>
<223> Synthetic

<400> 466
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 467
<211> 18
<212> DNA
<213> Artificial Sequence

<220>
<223> Synthetic

<400> 467
cagagtatta gtagctgg                                               18

<210> 468
<211> 6
<212> PRT
<213> Artificial Sequence

<220>
<223> Synthetic

<400> 468
Gln Ser Ile Ser Ser Trp
1               5

<210> 469
<211> 9
<212> DNA
<213> Artificial Sequence

<220>
<223> Synthetic

<400> 469
aaggcgtct                                                               9

<210> 470
<211> 3
<212> PRT
<213> Artificial Sequence

<220>
<223> Synthetic

<400> 470
Lys Ala Ser
1

<210> 471
<211> 27
<212> DNA
<213> Artificial Sequence

<220>
<223> Synthetic

<400> 471
caacagtata ttagttattc tcggacg                                          27

<210> 472
<211> 9
<212> PRT
<213> Artificial Sequence

<220>
<223> Synthetic

<400> 472
Gln Gln Tyr Ile Ser Tyr Ser Arg Thr
1               5

<210> 473
<211> 360
<212> DNA
<213> Artificial Sequence

<220>
<223> Synthetic

<400> 473
gaggtgcagc tggtggagtc tgggggaggc ctggtcaagc cgggggggtc cctgagactc 60
tcctgtacag cctctggatt caccttcagt acttataaca tgaattgggt ccgccaggct 120
ccagggaagg gactggagtg ggtctcatcc attaggagta gtagtaatta catatactac 180
gcagactcag tgaagggccg attcaccatc tccagagaca acgccaagaa ttcactgtat 240
ctgcaaatga acagcctgag agccgatgac acggctgtgt attactgtgc gagagatggc 300
agcagttggt acgactactc tgactactgg ggccagggaa ccctggtcac cgtctcctca 360

<210> 474
<211> 120
<212> PRT
<213> Artificial Sequence

<220>
<223> Synthetic

<400> 474
Glu Val Gln Leu Val Glu Ser Gly Gly Gly Leu Val Lys Pro Gly Gly
1               5                   10                  15
Ser Leu Arg Leu Ser Cys Thr Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Asp Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 475
<211> 321
<212> DNA
<213> Artificial Sequence

<220>
<223> Synthetic

<400> 475
gacatccaga tgacccagtc tccttccacc ctgtctgcat ctgtaggaga cagagtcacc 60
atcacttgcc gggccagtca gagtattagt agctggttgg cctggtatca acagatacca 120
gggaaagccc ctaaactcct gatctataag gcgtctagtt tagaaaatgg ggtcccatca 180
aggttcagcg gcagtggatc tgggacagaa ttcactctca tcatcagcag cctgcagcct 240
gatgattttg caacttatta ctgccaacag tatattagtt attctcggac gttcggccaa 300
gggaccaagg tggaaatcaa a                                           321

<210> 476
<211> 107
<212> PRT
<213> Artificial Sequence

<220>
<223> Synthetic

<400> 476
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ile Pro Gly Lys Ala Pro Lys Leu Leu Ile
        35                  40                  45
Tyr Lys Ala Ser Ser Leu Glu Asn Gly Val Pro Ser Arg Phe Ser Gly
    50                  55                  60
Ser Gly Ser Gly Thr Glu Phe Thr Leu Ile Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 477
<211> 360
<212> DNA
<213> Artificial Sequence

<220>
<223> Synthetic

<400> 477
gaggtgcagc tggtggagtc tgggggaggc ctggtcaagc ctggggggtc cctgagactc 60
tcctgtgcag cctctggatt caccttcagt acttataaca tgaactgggt ccgccaggct 120
ccagggaagg ggctggagtg ggtctcatcc attaggagta gtagtaatta catatactac 180
gcagactcag tgaagggccg attcaccatc tccagagaca acgccaagaa ctcactgtat 240
ctgcaaatga acagcctgag agccgaggac acggctgtgt attactgtgc gagagatggc 300
agcagttggt acgactactc tgactactgg ggccaaggaa ccctggtcac cgtctcctca 360


<210> 478
<211> 120
<212> PRT
<213> Artificial Sequence

<220>
<223> Synthetic

<400> 478
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Thr Tyr
            20                  25                  30
Asn Met Asn Trp Val Arg Gln Ala Pro Gly Lys Gly Leu Glu Trp Val
        35                  40                  45
Ser Ser Ile Arg Ser Ser Ser Asn Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gly Ser Ser Trp Tyr Asp Tyr Ser Asp Tyr Trp Gly Gln
            100                 105                 110
Gly Thr Leu Val Thr Val Ser Ser
        115                 120


<210> 479
<211> 321
<212> DNA
<213> Artificial Sequence

<220>
<223> Synthetic

<400> 479
gacatccaga tgacccagtc tccttccacc ctgtctgcat ctgtaggaga cagagtcacc 60
atcacttgcc gggccagtca gagtattagt agctggttgg cctggtatca gcagaaacca 120
gggaaagccc ctaagctcct gatctataag gcgtctagtt tagaaagtgg ggtcccatca 180
aggttcagcg gcagtggatc tgggacagaa ttcactctca ccatcagcag cctgcagcct 240
gatgattttg caacttatta ctgccaacag tatattagtt attctcggac gttcggccaa 300
gggaccaagg tggaaatcaa a                                           321

<210> 480
<211> 107
<212> PRT
<213> Artificial Sequence

<220>
<223> Synthetic

<400> 480

Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Ile Ser Tyr Ser Arg
                85                  90                  95
Thr Phe Gly Gln Gly Thr Lys Val Glu Ile Lys
            100                 105

<210> 481
<211> 354
<212> DNA
<213> Artificial Sequence

<220>
<223> Synthetic

<400> 481
gaggtgcaac tagtggagtc tgggggaggc ttggtacagc ctggggggtc cctgagactc 60
tcctgtgtag tctctggatt caccttcggt gactacgaca tgcactgggt ccgtcaagct 120
acaggaagag gtctggagtg ggtctcaggt attgctcctg ctggtgacac atcctataca 180
ggctccgtga agggccgatt caccatctcc agagagaatg ccaagaactc cttgcatctt 240
caaatgaaca gcctgacaac cggggacacg gctatatatt attgtgctag agaggatata 300
gcagtgcctg gttttgatta ctggggccag ggaaccctgg tcaccgtctc ctca       354

<210> 482
<211> 118
<212> PRT
<213> Artificial Sequence

<220>
<223> Synthetic

<400> 482
Glu Val Gln Leu Val Glu Ser Gly Gly Gly Leu Val Gln Pro Gly Gly
1               5                   10                  15
Ser Leu Arg Leu Ser Cys Val Val Ser Gly Phe Thr Phe Gly Asp Tyr
            20                  25                  30
Asp Met His Trp Val Arg Gln Ala Thr Gly Arg Gly Leu Glu Trp Val
        35                  40                  45
Ser Gly Ile Ala Pro Ala Gly Asp Thr Ser Tyr Thr Gly Ser Val Lys
    50                  55                  60
Gly Arg Phe Thr Ile Ser Arg Glu Asn Ala Lys Asn Ser Leu His Leu
65                  70                  75                  80
Gln Met Asn Ser Leu Thr Thr Gly Asp Thr Ala Ile Tyr Tyr Cys Ala
                85                  90                  95
Arg Glu Asp Ile Ala Val Pro Gly Phe Asp Tyr Trp Gly Gln Gly Thr
            100                 105                 110
Leu Val Thr Val Ser Ser
        115

<210> 483
<211> 24
<212> DNA
<213> Artificial Sequence

<220>
<223> Synthetic

<400> 483
ggattcacct tcggtgacta cgac                                        24

<210> 484
<211> 8
<212> PRT
<213> Artificial Sequence

<220>
<223> Synthetic

<400> 484
Gly Phe Thr Phe Gly Asp Tyr Asp
1               5


<210> 485
<211> 21
<212> DNA
<213> Artificial Sequence

<220>
<223> Synthetic

<400> 485
attgctcctg ctggtgacac a                                                21

<210> 486
<211> 7
<212> PRT
<213> Artificial Sequence

<220>
<223> Synthetic

<400> 486
Ile Ala Pro Ala Gly Asp Thr
1               5


<210> 487
<211> 36
<212> DNA
<213> Artificial Sequence

<220>
<223> Synthetic

<400> 487
gctagagagg atatagcagt gcctggtttt gattac                                36

<210> 488
<211> 12
<212> PRT
<213> Artificial Sequence

<220>
<223> Synthetic

<400> 488
Ala Arg Glu Asp Ile Ala Val Pro Gly Phe Asp Tyr
1               5                   10


<210> 489
<211> 324
<212> DNA
<213> Artificial Sequence

<220>
<223> Synthetic

<400> 489
gaaatagtga tgacgcagtc tccagccacc ctgtctgtgt ctccagggga acgaggcacc 60
ctctcctgca gggccagtca gagtgttagc agcaacttag cctggtacca gcagaaacct 120
ggccaggctc ccagactcct catctatggt gcatccacga gggccactgg cttcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggattt caaa 324

<210> 490
<211> 108
<212> PRT
<213> Artificial Sequence

<220>
<223> Synthetic

<400> 490
Glu Ile Val Met Thr Gln Ser Pro Ala Thr Leu Ser Val Ser Pro Gly
1               5                   10                  15
Glu Arg Gly Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Phe Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Lys Trp Pro Pro
                85                  90                  95
Phe Thr Phe Gly Pro Gly Thr Lys Val Asp Phe Lys
            100                 105

<210> 491
<211> 18
<212> DNA
<213> Artificial Sequence

<220>
<223> Synthetic

<400> 491
cagagtgtta gcagcaac 18

<210> 492
<211> 6
<212> PRT
<213> Artificial Sequence

<220>
<223> Synthetic

<400> 492
Gln Ser Val Ser Ser Asn
1               5

<210> 493
<211> 9
<212> DNA
<213> Artificial Sequence

<220>
<223> Synthetic

<400> 493
ggtgcatcc 9

<210> 494
<211> 3
<212> PRT
<213> Artificial Sequence

<220>
<223> Synthetic

<400> 494
Gly Ala Ser
1

<210> 495
<211> 30
<212> DNA
<213> Artificial Sequence

<220>
<223> Synthetic

<400> 495
cagcagtata ataagtggcc tccgttcact                                        30

<210> 496
<211> 10
<212> PRT
<213> Artificial Sequence

<220>
<223> Synthetic

<400> 496
Gln Gln Tyr Asn Lys Trp Pro Pro Phe Thr
1               5                   10

<210> 497
<211> 354
<212> DNA
<213> Artificial Sequence

<220>
<223> Synthetic

<400> 497
gaggtgcaac tagtggagtc tgggggaggc ttggtacagc ctggggggtc cctgagactc 60
tcctgtgtag tctctggatt caccttcggt gactacgaca tgcactgggt ccgtcaagct 120
acaggaagag gtctggagtg ggtctcaggt attgctcctg ctggtgacac atcctataca 180
ggctccgtga agggccgatt caccatctcc agagagaatg ccaagaactc cttgcatctt 240
caaatgaaca gcctgacaac cggggacacg gctatatatt attgtgctag agaggatata 300
gcagtgcctg gttttgatta ctggggccag ggaaccctgg tcaccgtctc ctca          354

<210> 498
<211> 118
<212> PRT
<213> Artificial Sequence

<220>
<223> Synthetic

<400> 498
Glu Val Gln Leu Val Glu Ser Gly Gly Gly Leu Val Gln Pro Gly Gly
1               5                   10                  15
Ser Leu Arg Leu Ser Cys Val Val Ser Gly Phe Thr Phe Gly Asp Tyr
            20                  25                  30
Asp Met His Trp Val Arg Gln Ala Thr Gly Arg Gly Leu Glu Trp Val
        35                  40                  45
Ser Gly Ile Ala Pro Ala Gly Asp Thr Ser Tyr Thr Gly Ser Val Lys

50                  55                  60
Gly Arg Phe Thr Ile Ser Arg Glu Asn Ala Lys Asn Ser Leu His Leu
65                  70                  75                  80
Gln Met Asn Ser Leu Thr Thr Gly Asp Thr Ala Ile Tyr Tyr Cys Ala
                85                  90                  95
Arg Glu Asp Ile Ala Val Pro Gly Phe Asp Tyr Trp Gly Gln Gly Thr
            100                 105                 110
Leu Val Thr Val Ser Ser
        115

<210> 499
<211> 324
<212> DNA
<213> Artificial Sequence

<220>
<223> Synthetic

<400> 499
gaaatagtga tgacgcagtc tccagccacc ctgtctgtgt ctccagggga acgaggcacc 60
ctctcctgca gggccagtca gagtgttagc agcaacttag cctggtacca gcagaaacct 120
ggccaggctc ccagactcct catctatggt gcatccacga gggccactgg cttcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggatat caaa                                         324

<210> 500
<211> 108
<212> PRT
<213> Artificial Sequence

<220>
<223> Synthetic

<400> 500
Glu Ile Val Met Thr Gln Ser Pro Ala Thr Leu Ser Val Ser Pro Gly
1               5                   10                  15
Glu Arg Gly Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Phe Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Lys Trp Pro Pro
                85                  90                  95
Phe Thr Phe Gly Pro Gly Thr Lys Val Asp Ile Lys
            100                 105

<210> 501
<211> 354
<212> DNA
<213> Artificial Sequence

<220>
<223> Synthetic

<400> 501
gaggtgcagc tggtggagtc tgggggaggc ttggtacagc ctggggggtc cctgagactc 60
tcctgtgcag cctctggatt caccttcggt gactacgaca tgcactgggt ccgccaagct 120
acaggaaaag gtctggagtg ggtctcagct attgctcctg ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgctag agaggatata 300
gcagtgcctg gttttgatta ctggggccaa ggaaccctgg tcaccgtctc ctca       354

<210> 502

<211> 118
<212> PRT
<213> Artificial Sequence

<220>
<223> Synthetic

<400> 502
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Gly Asp Tyr
            20                  25                  30
Asp Met His Trp Val Arg Gln Ala Thr Gly Lys Gly Leu Glu Trp Val
        35                  40                  45
Ser Ala Ile Ala Pro Ala Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Asp Ile Ala Val Pro Gly Phe Asp Tyr Trp Gly Gln Gly Thr
            100                 105                 110
Leu Val Thr Val Ser Ser
        115


<210> 503
<211> 324
<212> DNA
<213> Artificial Sequence

<220>
<223> Synthetic

<400> 503
gaaatagtga tgacgcagtc tccagccacc ctgtctgtgt ctccagggga aagagccacc 60
ctctcctgca gggccagtca gagtgttagc agcaacttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataagt ggcctccgtt cactttcggc 300
cctgggacca aagtggatat caaa                                        324

<210> 504
<211> 108
<212> PRT
<213> Artificial Sequence

<220>
<223> Synthetic

<400> 504
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Lys Trp Pro Pro
                85                  90                  95
Phe Thr Phe Gly Pro Gly Thr Lys Val Asp Ile Lys
            100                 105


<210> 505
<211> 378

<212> DNA
<213> Artificial Sequence

<220>
<223> Synthetic

<400> 505
caaattctgc tggtgcaatc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca 378

<210> 506
<211> 126
<212> PRT
<213> Artificial Sequence

<220>
<223> Synthetic

<400> 506
Gln Ile Leu Leu Val Gln Ser Gly Pro Glu Val Lys Glu Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Val Pro Gly Gln Gly Leu Glu Trp Met
        35                  40                  45
Gly Trp Val Ser Ala Tyr Asn Gly His Thr Asn Tyr Ala His Glu Val
    50                  55                  60
Gln Gly Arg Val Thr Met Thr Thr Asp Thr Ser Thr Thr Thr Ala Tyr
65                  70                  75                  80
Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 507
<211> 24
<212> DNA
<213> Artificial Sequence

<220>
<223> Synthetic

<400> 507
ggttacacct ttaccaacta cgct 24

<210> 508
<211> 8
<212> PRT
<213> Artificial Sequence

<220>
<223> Synthetic

<400> 508
Gly Tyr Thr Phe Thr Asn Tyr Ala
1               5

<210> 509
<211> 24
<212> DNA

<213> Artificial Sequence

<220>
<223> Synthetic

<400> 509
gtcagcgctt acaatggtca caca                                              24

<210> 510
<211> 8
<212> PRT
<213> Artificial Sequence

<220>
<223> Synthetic

<400> 510
Val Ser Ala Tyr Asn Gly His Thr
 1               5


<210> 511
<211> 57
<212> DNA
<213> Artificial Sequence

<220>
<223> Synthetic

<400> 511
gcgagagggg gtgtagtcgt gccagttgct ccccacttct acaacggtat ggacgtc    57

<210> 512
<211> 19
<212> PRT
<213> Artificial Sequence

<220>
<223> Synthetic

<400> 512
Ala Arg Gly Gly Val Val Val Pro Val Ala Pro His Phe Tyr Asn Gly
 1               5                   10                  15
Met Asp Val



<210> 513
<211> 336
<212> DNA
<213> Artificial Sequence

<220>
<223> Synthetic

<400> 513
gatattgtga tgactcagtt tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttag gccaagggac caaggtggaa atcaaa                           336

<210> 514
<211> 112
<212> PRT
<213> Artificial Sequence

<220>

<223> Synthetic

<400> 514
Asp Ile Val Met Thr Gln Phe Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Lys Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Leu Gly Gln Gly Thr Lys Val Glu Ile Lys
            100                 105                 110


<210> 515
<211> 33
<212> DNA
<213> Artificial Sequence

<220>
<223> Synthetic

<400> 515
cagagcctcc tgcatattaa tgaatacaac tat                                33

<210> 516
<211> 11
<212> PRT
<213> Artificial Sequence

<220>
<223> Synthetic

<400> 516
Gln Ser Leu Leu His Ile Asn Glu Tyr Asn Tyr
1               5                   10


<210> 517
<211> 9
<212> DNA
<213> Artificial Sequence

<220>
<223> Synthetic

<400> 517
ttgggtttt                                                            9

<210> 518
<211> 3
<212> PRT
<213> Artificial Sequence

<220>
<223> Synthetic

<400> 518
Leu Gly Phe
1


<210> 519
<211> 27

<212> DNA
<213> Artificial Sequence

<220>
<223> Synthetic

<400> 519
atgcaagctc ttcaaactcc gtggacg                                          27

<210> 520
<211> 9
<212> PRT
<213> Artificial Sequence

<220>
<223> Synthetic

<400> 520
Met Gln Ala Leu Gln Thr Pro Trp Thr
1               5

<210> 521
<211> 378
<212> DNA
<213> Artificial Sequence

<220>
<223> Synthetic

<400> 521
caggttcagc tggtgcagtc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca                                                    378

<210> 522
<211> 126
<212> PRT
<213> Artificial Sequence

<220>
<223> Synthetic

<400> 522
Gln Val Gln Leu Val Gln Ser Gly Pro Glu Val Lys Glu Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Val Pro Gly Gln Gly Leu Glu Trp Met
        35                  40                  45
Gly Trp Val Ser Ala Tyr Asn Gly His Thr Asn Tyr Ala His Glu Val
    50                  55                  60
Gln Gly Arg Val Thr Met Thr Thr Asp Thr Ser Thr Thr Thr Ala Tyr
65                  70                  75                  80
Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 523
<211> 336
<212> DNA

<213> Artificial Sequence

<220>
<223> Synthetic

<400> 523
gatattgtga tgactcagtc tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttag gccaagggac caaggtggaa atcaaa 336

<210> 524
<211> 112
<212> PRT
<213> Artificial Sequence

<220>
<223> Synthetic

<400> 524
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Lys Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Leu Gly Gln Gly Thr Lys Val Glu Ile Lys
            100                 105                 110

<210> 525
<211> 378
<212> DNA
<213> Artificial Sequence

<220>
<223> Synthetic

<400> 525
caggttcagc tggtgcagtc tggagctgag gtgaagaagc ctggggcctc agtgaaggtc 60
tcctgcaagg cttctggtta cacctttacc aactacgcta tcagctgggt gcgacaggcc 120
cctggacaag ggcttgagtg gatgggatgg gtcagcgctt acaatggtca cacaaactat 180
gcacagaagc tccagggcag agtcaccatg accacagaca catccacgag cacagcctac 240
atggagctga ggagcctgag atctgacgac acggccgtgt attactgtgc gagagggggt 300
gtagtcgtgc cagttgctcc ccacttctac aacggtatgg acgtctgggg gcaagggacc 360
acggtcaccg tctcctca 378

<210> 526
<211> 126
<212> PRT
<213> Artificial Sequence

<220>
<223> Synthetic

<400> 526
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Ala Pro Gly Gln Gly Leu Glu Trp Met

        35                  40                  45
Gly Trp Val Ser Ala Tyr Asn Gly His Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 527
<211> 336
<212> DNA
<213> Artificial Sequence

<220>
<223> Synthetic

<400> 527
gatattgtga tgactcagtc tccactctcc ctgcccgtca cccctggaga gccggcctcc 60
atctcctgca ggtctagtca gagcctcctg catattaatg aatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct tcaaactccg 300
tggacgttcg gccaagggac caaggtggaa atcaaa 336

<210> 528
<211> 112
<212> PRT
<213> Artificial Sequence

<220>
<223> Synthetic

<400> 528
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Phe Gly Gln Gly Thr Lys Val Glu Ile Lys
            100                 105                 110

<210> 529
<211> 351
<212> DNA
<213> Artificial Sequence

<220>
<223> Synthetic

<400> 529
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga gcccgggtca ccgtctcctc a 351

<210> 530
<211> 117
<212> PRT
<213> Artificial Sequence

<220>
<223> Synthetic

<400> 530
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Ala Arg
            100                 105                 110
Val Thr Val Ser Ser
        115


<210> 531
<211> 24
<212> DNA
<213> Artificial Sequence

<220>
<223> Synthetic

<400> 531
ggattcaccc taagtagcta cgac                                              24

<210> 532
<211> 8
<212> PRT
<213> Artificial Sequence

<220>
<223> Synthetic

<400> 532
Gly Phe Thr Leu Ser Ser Tyr Asp
1               5


<210> 533
<211> 21
<212> DNA
<213> Artificial Sequence

<220>
<223> Synthetic

<400> 533
attggcagta ctggtgacac a                                                 21

<210> 534
<211> 7
<212> PRT
<213> Artificial Sequence

<220>

<223> Synthetic

<400> 534
Ile Gly Ser Thr Gly Asp Thr
1               5


<210> 535
<211> 33
<212> DNA
<213> Artificial Sequence

<220>
<223> Synthetic

<400> 535
gcaagagagg gaataagaac accctatgat tat                                    33

<210> 536
<211> 11
<212> PRT
<213> Artificial Sequence

<220>
<223> Synthetic

<400> 536
Ala Arg Glu Gly Ile Arg Thr Pro Tyr Asp Tyr
1               5                   10


<210> 537
<211> 324
<212> DNA
<213> Artificial Sequence

<220>
<223> Synthetic

<400> 537
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa                                              324

<210> 538
<211> 108
<212> PRT
<213> Artificial Sequence

<220>
<223> Synthetic

<400> 538
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Val Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys

            100                 105

<210> 539
<211> 18
<212> DNA
<213> Artificial Sequence

<220>
<223> Synthetic

<400> 539
cagagtgtta gcagcaat 18

<210> 540
<211> 6
<212> PRT
<213> Artificial Sequence

<220>
<223> Synthetic

<400> 540
Gln Ser Val Ser Ser Asn
1               5

<210> 541
<211> 9
<212> DNA
<213> Artificial Sequence

<220>
<223> Synthetic

<400> 541
ggtgcatcc 9

<210> 542
<211> 3
<212> PRT
<213> Artificial Sequence

<220>
<223> Synthetic

<400> 542
Gly Ala Ser
1

<210> 543
<211> 30
<212> DNA
<213> Artificial Sequence

<220>
<223> Synthetic

<400> 543
cagcagtata ataattggcc tccattcact 30

<210> 544
<211> 10
<212> PRT
<213> Artificial Sequence

<220>
<223> Synthetic

<400> 544
Gln Gln Tyr Asn Asn Trp Pro Pro Phe Thr
1               5                   10

<210> 545
<211> 351
<212> DNA
<213> Artificial Sequence

<220>
<223> Synthetic

<400> 545
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga accctggtca ccgtctcctc a          351

<210> 546
<211> 117
<212> PRT
<213> Artificial Sequence

<220>
<223> Synthetic

<400> 546
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115

<210> 547
<211> 324
<212> DNA
<213> Artificial Sequence

<220>
<223> Synthetic

<400> 547
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa                                        324

<210> 548
<211> 108
<212> PRT
<213> Artificial Sequence

<220>
<223> Synthetic

<400> 548
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Val Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105

<210> 549
<211> 351
<212> DNA
<213> Artificial Sequence

<220>
<223> Synthetic

<400> 549
gaggtgcagc tggtggagtc tgggggaggc ttggtacagc ctggggggtc cctgagactc 60
tcctgtgcag cctctggatt caccctaagt agctacgaca tgcactgggt ccgccaagct 120
acaggaaaag gtctggagtg ggtctcagct attggcagta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggaata 300
agaacaccct atgattattg gggccaagga accctggtca ccgtctcctc a         351

<210> 550
<211> 117
<212> PRT
<213> Artificial Sequence

<220>
<223> Synthetic

<400> 550
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115

<210> 551
<211> 324
<212> DNA
<213> Artificial Sequence

<220>
<223> Synthetic

<400> 551
gaaatagtga tgacgcagtc tccagccacc ctgtctgtgt ctccagggga aagagccacc 60
ctctcctgca gggccagtca gagtgttagc agcaatttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataatt ggcctccatt cactttcggc 300
cctgggacca aagtggatat caaa 324

<210> 552
<211> 108
<212> PRT
<213> Artificial Sequence

<220>
<223> Synthetic

<400> 552
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105

<210> 553
<211> 351
<212> DNA
<213> Artificial Sequence

<220>
<223> Synthetic

<400> 553
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180
ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga gcccgggtca ccgtctcctc a 351

<210> 554
<211> 117
<212> PRT
<213> Artificial Sequence

<220>
<223> Synthetic

<400> 554
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60

Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Ala Arg
            100                 105                 110
Val Thr Val Ser Ser
        115

<210> 555
<211> 24
<212> DNA
<213> Artificial Sequence

<220>
<223> Synthetic

<400> 555
ggattcaccc taagtagcta cgac                                              24

<210> 556
<211> 8
<212> PRT
<213> Artificial Sequence

<220>
<223> Synthetic

<400> 556
Gly Phe Thr Leu Ser Ser Tyr Asp
1               5

<210> 557
<211> 21
<212> DNA
<213> Artificial Sequence

<220>
<223> Synthetic

<400> 557
attggcagta ctggtgacac a                                                 21

<210> 558
<211> 7
<212> PRT
<213> Artificial Sequence

<220>
<223> Synthetic

<400> 558
Ile Gly Ser Thr Gly Asp Thr
1               5

<210> 559
<211> 33
<212> DNA
<213> Artificial Sequence

<220>
<223> Synthetic

<400> 559
gcaagagagg gaataagaac accctatgat tat                                    33

<210> 560
<211> 11
<212> PRT
<213> Artificial Sequence

<220>
<223> Synthetic

<400> 560
Ala Arg Glu Gly Ile Arg Thr Pro Tyr Asp Tyr
1               5                   10


<210> 561
<211> 324
<212> DNA
<213> Artificial Sequence

<220>
<223> Synthetic

<400> 561
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa                                        324

<210> 562
<211> 108
<212> PRT
<213> Artificial Sequence

<220>
<223> Synthetic

<400> 562
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Val Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105


<210> 563
<211> 18
<212> DNA
<213> Artificial Sequence

<220>
<223> Synthetic

<400> 563
cagagtgtta gcagcaat                                               18

<210> 564
<211> 6
<212> PRT
<213> Artificial Sequence

<220>
<223> Synthetic

<400> 564
Gln Ser Val Ser Ser Asn
1               5


<210> 565
<211> 9
<212> DNA
<213> Artificial Sequence

<220>
<223> Synthetic

<400> 565
ggtgcatcc                                                               9

<210> 566
<211> 3
<212> PRT
<213> Artificial Sequence

<220>
<223> Synthetic

<400> 566
Gly Ala Ser
1


<210> 567
<211> 30
<212> DNA
<213> Artificial Sequence

<220>
<223> Synthetic

<400> 567
cagcagtata ataattggcc tccattcact                                       30

<210> 568
<211> 10
<212> PRT
<213> Artificial Sequence

<220>
<223> Synthetic

<400> 568
Gln Gln Tyr Asn Asn Trp Pro Pro Phe Thr
1               5                   10


<210> 569
<211> 351
<212> DNA
<213> Artificial Sequence

<220>
<223> Synthetic

<400> 569
gaggtgcagc tggtggagtc tgggggaggc ttggtacagc ctggggggtc cctgagactc 60
tcctgtgcag cctctggatt caccctaagt agctacgaca tgcactgggt ccgccaagca 120
acaggaaaag gtctggagtg ggtctcagct attggcagta ctggtgacac atactataca 180

ggctccgtga tgggccgatt caccatctcc agagacgctg ccaaaaactc cttctatctt 240
gaaatgaaca gcctgagagt cggggacacg gctgtatatt actgtgcaag agagggaata 300
agaacaccct atgattattg gggccaggga accctggtca ccgtctcctc a 351

<210> 570
<211> 117
<212> PRT
<213> Artificial Sequence

<220>
<223> Synthetic

<400> 570
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Thr Gly Ser Val Met
    50                  55                  60
Gly Arg Phe Thr Ile Ser Arg Asp Ala Ala Lys Asn Ser Phe Tyr Leu
65                  70                  75                  80
Glu Met Asn Ser Leu Arg Val Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115

<210> 571
<211> 324
<212> DNA
<213> Artificial Sequence

<220>
<223> Synthetic

<400> 571
gaaatagtga tgacgcagtc tccagccacc ctgtctgtgt ctccagggga aagagccacc 60
ctctcctgca gggccagtca gagtgttagc agcaatgtag cctggtacca gcagaaacct 120
ggccaggctc ccaggctcct catctatggt gcatccacca gggccactgg tatcccagcc 180
aggttcagtg gcagtgggtc tgggacagaa ttcactctca ccatcagcag cctgcagtct 240
gaagattttg cagtttatta ctgtcagcag tataataatt ggcctccatt cactttcggc 300
cctgggacca aagtggatat caaa 324

<210> 572
<211> 108
<212> PRT
<213> Artificial Sequence

<220>
<223> Synthetic

<400> 572
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Val Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys

100                 105


<210> 573
<211> 351
<212> DNA
<213> Artificial Sequence

<220>
<223> Synthetic

<400> 573
gaggtgcagc tggtggagtc tgggggaggc ttggtacagc ctggggggtc cctgagactc 60
tcctgtgcag cctctggatt caccctaagt agctacgaca tgcactgggt ccgccaagct 120
acaggaaaag gtctggagtg ggtctcagct attggcagta ctggtgacac atactatcca 180
ggctccgtga agggccgatt caccatctcc agagaaaatg ccaagaactc cttgtatctt 240
caaatgaaca gcctgagagc cggggacacg gctgtgtatt actgtgcaag agagggaata 300
agaacaccct atgattattg gggccaagga accctggtca ccgtctcctc a          351

<210> 574
<211> 117
<212> PRT
<213> Artificial Sequence

<220>
<223> Synthetic

<400> 574
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Leu Ser Ser Tyr
            20                  25                  30
Asp Met His Trp Val Arg Gln Ala Thr Gly Lys Gly Leu Glu Trp Val
        35                  40                  45
Ser Ala Ile Gly Ser Thr Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg Glu Gly Ile Arg Thr Pro Tyr Asp Tyr Trp Gly Gln Gly Thr Leu
            100                 105                 110
Val Thr Val Ser Ser
        115


<210> 575
<211> 324
<212> DNA
<213> Artificial Sequence

<220>
<223> Synthetic

<400> 575
gaaatagtga tgacgcagtc tccagccacc ctgtctgtgt ctccagggga aagagccacc 60
ctctcctgca gggccagtca gagtgttagc agcaatttag cctggtacca gcagaaacct 120
ggccaggctc ccaggctcct catctatggt gcatccacca gggccactgg tatcccagcc 180
aggttcagtg gcagtgggtc tgggacagag ttcactctca ccatcagcag cctgcagtct 240
gaagattttg cagtttatta ctgtcagcag tataataatt ggcctccatt cactttcggc 300
cctgggacca aagtggatat caaa                                         324

<210> 576
<211> 108
<212> PRT
<213> Artificial Sequence

<220>
<223> Synthetic

<400> 576
Glu Ile Val Met Thr Gln Ser Pro Ala Thr Leu Ser Val Ser Pro Gly
1               5                   10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro Pro
                85                  90                  95
Phe Thr Phe Gly Pro Gly Thr Lys Val Asp Ile Lys
            100                 105

<210> 577
<211> 363
<212> DNA
<213> Artificial Sequence

<220>
<223> Synthetic

<400> 577
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
gcggactctg tgaagggccg attcaccatc tccagagaca acgccaagca ctccctgtat 240
ctgcaaatga acagtctgag acctgaggac acggccttgt attactgtgt aaaagaggtg 300
actacgggat actactacgg tatggacgtc tggggccaag ggaccacggt caccgtctcc 360
tca                                                               363

<210> 578
<211> 121
<212> PRT
<213> Artificial Sequence

<220>
<223> Synthetic

<400> 578
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Asn Trp Asn Ser Gly Ser Ile Gly Tyr Ala Asp Ser Val
    50                  55                  60
Lys Gly Arg Phe Thr Ile Ser Arg Asp Asn Ala Lys His Ser Leu Tyr
65                  70                  75                  80
Leu Gln Met Asn Ser Leu Arg Pro Glu Asp Thr Ala Leu Tyr Tyr Cys
                85                  90                  95
Val Lys Glu Val Thr Thr Gly Tyr Tyr Tyr Gly Met Asp Val Trp Gly
            100                 105                 110
Gln Gly Thr Thr Val Thr Val Ser Ser
        115                 120

<210> 579
<211> 24
<212> DNA
<213> Artificial Sequence

<220>
<223> Synthetic

<400> 579
ggattcacct ttgatgatta tgcc                                              24

<210> 580
<211> 8
<212> PRT
<213> Artificial Sequence

<220>
<223> Synthetic

<400> 580
Gly Phe Thr Phe Asp Asp Tyr Ala
1               5

<210> 581
<211> 24
<212> DNA
<213> Artificial Sequence

<220>
<223> Synthetic

<400> 581
attaattgga acagtggtag cata                                              24

<210> 582
<211> 8
<212> PRT
<213> Artificial Sequence

<220>
<223> Synthetic

<400> 582
Ile Asn Trp Asn Ser Gly Ser Ile
1               5

<210> 583
<211> 42
<212> DNA
<213> Artificial Sequence

<220>
<223> Synthetic

<400> 583
gtaaaagagg tgactacggg atactactac ggtatggacg tc                          42

<210> 584
<211> 14
<212> PRT
<213> Artificial Sequence

<220>
<223> Synthetic

<400> 584
Val Lys Glu Val Thr Thr Gly Tyr Tyr Tyr Gly Met Asp Val
1               5                   10

<210> 585
<211> 321
<212> DNA
<213> Artificial Sequence

<220>
<223> Synthetic

<400> 585
gacatccagt tgacccagtc tccatccttc ctgtctgcat ctgtaggaga cagagtcacc 60
atcacttgct gggccagtca gggcattagc agttatttag cctggtatca gaaaaaacca 120
gggaaagccc ctaacctcct gatctatgat gcatccactt tgcaaagtgg ggtcccatca 180
aggttcagcg gcagtggatc tgggacagaa ttcactctca cactcagcag cctgcagcct 240
gaagattttg caacttatta ctgtcaacag cttaatattt acccattcac tttcggccct 300
gggaccaaag tggatatcaa a 321

<210> 586
<211> 107
<212> PRT
<213> Artificial Sequence

<220>
<223> Synthetic

<400> 586
Asp Ile Gln Leu Thr Gln Ser Pro Ser Phe Leu Ser Ala Ser Val Gly
1               5                   10                  15
Asp Arg Val Thr Ile Thr Cys Trp Ala Ser Gln Gly Ile Ser Ser Tyr
            20                  25                  30
Leu Ala Trp Tyr Gln Lys Lys Pro Gly Lys Ala Pro Asn Leu Leu Ile
        35                  40                  45
Tyr Asp Ala Ser Thr Leu Gln Ser Gly Val Pro Ser Arg Phe Ser Gly
    50                  55                  60
Ser Gly Ser Gly Thr Glu Phe Thr Leu Thr Leu Ser Ser Leu Gln Pro
65                  70                  75                  80
Glu Asp Phe Ala Thr Tyr Tyr Cys Gln Gln Leu Asn Ile Tyr Pro Phe
                85                  90                  95
Thr Phe Gly Pro Gly Thr Lys Val Asp Ile Lys
            100                 105

<210> 587
<211> 18
<212> DNA
<213> Artificial Sequence

<220>
<223> Synthetic

<400> 587
cagggcatta gcagttat 18

<210> 588
<211> 6
<212> PRT
<213> Artificial Sequence

<220>
<223> Synthetic

<400> 588
Gln Gly Ile Ser Ser Tyr
1               5

<210> 589
<211> 9
<212> DNA
<213> Artificial Sequence

<220>
<223> Synthetic

<400> 589
gatgcatcc                                                                9

<210> 590
<211> 3
<212> PRT
<213> Artificial Sequence

<220>
<223> Synthetic

<400> 590
Asp Ala Ser
1

<210> 591
<211> 27
<212> DNA
<213> Artificial Sequence

<220>
<223> Synthetic

<400> 591
caacagctta atatttaccc attcact                                           27

<210> 592
<211> 9
<212> PRT
<213> Artificial Sequence

<220>
<223> Synthetic

<400> 592
Gln Gln Leu Asn Ile Tyr Pro Phe Thr
1               5

<210> 593
<211> 363
<212> DNA
<213> Artificial Sequence

<220>
<223> Synthetic

<400> 593
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
gcggactctg tgaagggccg attcaccatc tccagagaca acgccaagca ctccctgtat 240
ctgcaaatga acagtctgag acctgaggac acggccttgt attactgtgt aaaagaggtg 300
actacgggat actactacgg tatggacgtc tggggccaag ggaccacggt caccgtctcc 360
tca                                                                     363

<210> 594
<211> 121
<212> PRT
<213> Artificial Sequence

<220>
<223> Synthetic

<400> 594
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr

20                  25                  30
Ala Met His Trp Val Arg Gln Ala Pro Gly Lys Gly Leu Glu Trp Val
        35                  40                  45
Ser Gly Ile Asn Trp Asn Ser Gly Ser Ile Gly Tyr Ala Asp Ser Val
    50                  55                  60
Lys Gly Arg Phe Thr Ile Ser Arg Asp Asn Ala Lys His Ser Leu Tyr
65                  70                  75                  80
Leu Gln Met Asn Ser Leu Arg Pro Glu Asp Thr Ala Leu Tyr Tyr Cys
                85                  90                  95
Val Lys Glu Val Thr Thr Gly Tyr Tyr Tyr Gly Met Asp Val Trp Gly
            100                 105                 110
Gln Gly Thr Thr Val Thr Val Ser Ser
        115                 120

<210> 595
<211> 321
<212> DNA
<213> Artificial Sequence

<220>
<223> Synthetic

<400> 595
gacatccagt tgacccagtc tccatccttc ctgtctgcat ctgtaggaga cagagtcacc 60
atcacttgct gggccagtca gggcattagc agttatttag cctggtatca gaaaaaacca 120
gggaaagccc ctaacctcct gatctatgat gcatccactt tgcaaagtgg ggtcccatca 180
aggttcagcg gcagtggatc tgggacagaa ttcactctca cactcagcag cctgcagcct 240
gaagattttg caacttatta ctgtcaacag cttaatattt acccattcac tttcggccct 300
gggaccaaag tggatatcaa a                                           321

<210> 596
<211> 107
<212> PRT
<213> Artificial Sequence

<220>
<223> Synthetic

<400> 596
Asp Ile Gln Leu Thr Gln Ser Pro Ser Phe Leu Ser Ala Ser Val Gly
1               5                   10                  15
Asp Arg Val Thr Ile Thr Cys Trp Ala Ser Gln Gly Ile Ser Ser Tyr
            20                  25                  30
Leu Ala Trp Tyr Gln Lys Lys Pro Gly Lys Ala Pro Asn Leu Leu Ile
        35                  40                  45
Tyr Asp Ala Ser Thr Leu Gln Ser Gly Val Pro Ser Arg Phe Ser Gly
    50                  55                  60
Ser Gly Ser Gly Thr Glu Phe Thr Leu Thr Leu Ser Ser Leu Gln Pro
65                  70                  75                  80
Glu Asp Phe Ala Thr Tyr Tyr Cys Gln Gln Leu Asn Ile Tyr Pro Phe
                85                  90                  95
Thr Phe Gly Pro Gly Thr Lys Val Asp Ile Lys
            100                 105

<210> 597
<211> 363
<212> DNA
<213> Artificial Sequence

<220>
<223> Synthetic

<400> 597
gaagtgcagc tggtggagtc tgggggaggc ttggtacagc ctggcaggtc cctgagactc 60
tcctgtgcag cctctggatt cacctttgat gattatgcca tgcactgggt ccggcaagct 120
ccagggaagg gcctggagtg ggtctcaggt attaattgga acagtggtag cataggctat 180
gcggactctg tgaagggccg attcaccatc tccagagaca acgccaagaa ctccctgtat 240

ctgcaaatga acagtctgag agctgaggac acggccttgt attactgtgt aaaagaggtg 300
actacgggat actactacgg tatggacgtc tgggggcaag ggaccacggt caccgtctcc 360
tca 363

<210> 598
<211> 121
<212> PRT
<213> Artificial Sequence

<220>
<223> Synthetic

<400> 598
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Asn Trp Asn Ser Gly Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr Tyr Cys
                85                  90                  95
Val Lys Glu Val Thr Thr Gly Tyr Tyr Tyr Gly Met Asp Val Trp Gly
            100                 105                 110
Gln Gly Thr Thr Val Thr Val Ser Ser
        115                 120

<210> 599
<211> 321
<212> DNA
<213> Artificial Sequence

<220>
<223> Synthetic

<400> 599
gacatccagt tgacccagtc tccatccttc ctgtctgcat ctgtaggaga cagagtcacc 60
atcacttgcc gggccagtca gggcattagc agttatttag cctggtatca gcaaaaacca 120
gggaaagccc ctaagctcct gatctatgat gcatccactt tgcaaagtgg ggtcccatca 180
aggttcagcg gcagtggatc tgggacagaa ttcactctca caatcagcag cctgcagcct 240
gaagattttg caacttatta ctgtcaacag cttaatattt acccattcac tttcggccct 300
gggaccaaag tggatatcaa a 321

<210> 600
<211> 107
<212> PRT
<213> Artificial Sequence

<220>
<223> Synthetic

<400> 600
Asp Ile Gln Leu Thr Gln Ser Pro Ser Phe Leu Ser Ala Ser Val Gly
1               5                   10                  15
Asp Arg Val Thr Ile Thr Cys Arg Ala Ser Gln Gly Ile Ser Ser Tyr
            20                  25                  30
Leu Ala Trp Tyr Gln Gln Lys Pro Gly Lys Ala Pro Lys Leu Leu Ile
        35                  40                  45
Tyr Asp Ala Ser Thr Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ile Tyr Pro Phe
                85                  90                  95
Thr Phe Gly Pro Gly Thr Lys Val Asp Ile Lys

            100                 105

<210> 601
<211> 366
<212> DNA
<213> Artificial Sequence

<220>
<223> Synthetic

<400> 601
gaggtgcagt tgttggagtc tgggggaggc ttggtacagc ctggggggtc cctgagactc 60
tcctgtgcag cctctggatt cacgtttagt agctatgcca tgaactgggt ccgccaggct 120
ccagggaagg ggctggattg ggtctcaggt atcagtggta atggtggtag cacctactac 180
gcagactccg tgaagggccg gttcaccatc tccagagaca tttccaagaa cacgctgtat 240
gtgcaaatgc acagcctgag agtcgaggac acggccgttt actactgtgc gaaagcccgt 300
tattacgatt tttggggggg gaatttcgat ctctggggcc gtggcaccca ggtcactgtc 360
tcctca 366

<210> 602
<211> 122
<212> PRT
<213> Artificial Sequence

<220>
<223> Synthetic

<400> 602
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Asn Trp Val Arg Gln Ala Pro Gly Lys Gly Leu Asp Trp Val
        35                  40                  45
Ser Gly Ile Ser Gly Asn Gly Gly Ser Thr Tyr Tyr Ala Asp Ser Val
    50                  55                  60
Lys Gly Arg Phe Thr Ile Ser Arg Asp Ile Ser Lys Asn Thr Leu Tyr
65                  70                  75                  80
Val Gln Met His Ser Leu Arg Val Glu Asp Thr Ala Val Tyr Tyr Cys
                85                  90                  95
Ala Lys Ala Arg Tyr Tyr Asp Phe Trp Gly Gly Asn Phe Asp Leu Trp
            100                 105                 110
Gly Arg Gly Thr Gln Val Thr Val Ser Ser
        115                 120

<210> 603
<211> 24
<212> DNA
<213> Artificial Sequence

<220>
<223> Synthetic

<400> 603
ggattcacgt ttagtagcta tgcc 24

<210> 604
<211> 8
<212> PRT
<213> Artificial Sequence

<220>
<223> Synthetic

<400> 604
Gly Phe Thr Phe Ser Ser Tyr Ala
1               5

<210> 605
<211> 24
<212> DNA
<213> Artificial Sequence

<220>
<223> Synthetic

<400> 605
atcagtggta atggtggtag cacc                                              24

<210> 606
<211> 8
<212> PRT
<213> Artificial Sequence

<220>
<223> Synthetic

<400> 606
Ile Ser Gly Asn Gly Gly Ser Thr
1               5


<210> 607
<211> 45
<212> DNA
<213> Artificial Sequence

<220>
<223> Synthetic

<400> 607
gcgaaagccc gttattacga tttttggggg gggaatttcg atctc                       45

<210> 608
<211> 15
<212> PRT
<213> Artificial Sequence

<220>
<223> Synthetic

<400> 608
Ala Lys Ala Arg Tyr Tyr Asp Phe Trp Gly Gly Asn Phe Asp Leu
1               5                   10                  15


<210> 609
<211> 324
<212> DNA
<213> Artificial Sequence

<220>
<223> Synthetic

<400> 609
gaaattgtgt tgacgcagtc tccaggcacc ctgtctttgt ctccagggga aagagccacc 60
ctctcctgca gggccagtca gagtgttagc atcaggtact tagcctggta tcagcagaaa 120
cctggccagg ctcccaggct cctcatctat ggtgcatcca gcagggccac tggcatccca 180
gacaggttca gtgtcagtgt gtctgggaca gacttcactc tcaccatcac tagactggag 240
cctgaagatt ttgcagtcta ttactgtcag caatatggta gttcaccgct cactttcggc 300
ggagggacca aggtggagat caaa                                              324

<210> 610
<211> 108
<212> PRT

<213> Artificial Sequence

<220>
<223> Synthetic

<400> 610
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Val Ser Val Ser Gly Thr Asp Phe Thr Leu Thr Ile Thr Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105


<210> 611
<211> 21
<212> DNA
<213> Artificial Sequence

<220>
<223> Synthetic

<400> 611
cagagtgtta gcatcaggta c                                              21

<210> 612
<211> 7
<212> PRT
<213> Artificial Sequence

<220>
<223> Synthetic

<400> 612
Gln Ser Val Ser Ile Arg Tyr
1               5


<210> 613
<211> 9
<212> DNA
<213> Artificial Sequence

<220>
<223> Synthetic

<400> 613
ggtgcatcc                                                            9

<210> 614
<211> 3
<212> PRT
<213> Artificial Sequence

<220>
<223> Synthetic

<400> 614
Gly Ala Ser
1

<210> 615
<211> 27
<212> DNA
<213> Artificial Sequence

<220>
<223> Synthetic

<400> 615
cagcaatatg gtagttcacc gctcact                                          27

<210> 616
<211> 9
<212> PRT
<213> Artificial Sequence

<220>
<223> Synthetic

<400> 616
Gln Gln Tyr Gly Ser Ser Pro Leu Thr
1               5

<210> 617
<211> 366
<212> DNA
<213> Artificial Sequence

<220>
<223> Synthetic

<400> 617
gaggtgcagt tgttggagtc tgggggaggc ttggtacagc ctggggggtc cctgagactc 60
tcctgtgcag cctctggatt cacgtttagt agctatgcca tgaactgggt ccgccaggct 120
ccagggaagg ggctggattg ggtctcaggt atcagtggta atggtggtag cacctactac 180
gcagactccg tgaagggccg gttcaccatc tccagagaca tttccaagaa cacgctgtat 240
gtgcaaatgc acagcctgag agtcgaggac acggccgttt actactgtgc gaaagcccgt 300
tattacgatt tttggggggg gaatttcgat ctctggggcc gtggcaccct ggtcactgtc 360
tcctca                                                                 366

<210> 618
<211> 122
<212> PRT
<213> Artificial Sequence

<220>
<223> Synthetic

<400> 618
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Asn Trp Val Arg Gln Ala Pro Gly Lys Gly Leu Asp Trp Val
        35                  40                  45
Ser Gly Ile Ser Gly Asn Gly Gly Ser Thr Tyr Tyr Ala Asp Ser Val
    50                  55                  60
Lys Gly Arg Phe Thr Ile Ser Arg Asp Ile Ser Lys Asn Thr Leu Tyr
65                  70                  75                  80
Val Gln Met His Ser Leu Arg Val Glu Asp Thr Ala Val Tyr Tyr Cys
                85                  90                  95
Ala Lys Ala Arg Tyr Tyr Asp Phe Trp Gly Gly Asn Phe Asp Leu Trp
            100                 105                 110
Gly Arg Gly Thr Leu Val Thr Val Ser Ser
        115                 120

<210> 619
<211> 324
<212> DNA
<213> Artificial Sequence

<220>
<223> Synthetic

<400> 619
gaaattgtgt tgacgcagtc tccaggcacc ctgtctttgt ctccagggga aagagccacc 60
ctctcctgca gggccagtca gagtgttagc atcaggtact tagcctggta tcagcagaaa 120
cctggccagg ctcccaggct cctcatctat ggtgcatcca gcagggccac tggcatccca 180
gacaggttca gtgtcagtgt gtctgggaca gacttcactc tcaccatcac tagactggag 240
cctgaagatt ttgcagtcta ttactgtcag caatatggta gttcaccgct cactttcggc 300
ggagggacca aggtggagat caaa 324

<210> 620
<211> 108
<212> PRT
<213> Artificial Sequence

<220>
<223> Synthetic

<400> 620
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Val Ser Val Ser Gly Thr Asp Phe Thr Leu Thr Ile Thr Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105

<210> 621
<211> 366
<212> DNA
<213> Artificial Sequence

<220>
<223> Synthetic

<400> 621
gaggtgcagc tgttggagtc tgggggaggc ttggtacagc ctggggggtc cctgagactc 60
tcctgtgcag cctctggatt cacgtttagt agctatgcca tgagctgggt ccgccaggct 120
ccagggaagg ggctggagtg ggtctcagct atcagtggta atggtggtag cacctactac 180
gcagactccg tgaagggccg gttcaccatc tccagagaca attccaagaa cacgctgtat 240
ctgcaaatga acagcctgag agccgaggac acggccgtat attactgtgc gaaagcccgt 300
tattacgatt tttggggggg gaatttcgat ctctggggcc gtggcaccct ggtcactgtc 360
tcctca 366

<210> 622
<211> 122
<212> PRT
<213> Artificial Sequence

<220>
<223> Synthetic

<400> 622
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45
Ser Ala Ile Ser Gly Asn Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ala Arg Tyr Tyr Asp Phe Trp Gly Gly Asn Phe Asp Leu Trp
            100                 105                 110
Gly Arg Gly Thr Leu Val Thr Val Ser Ser
        115                 120


<210> 623
<211> 324
<212> DNA
<213> Artificial Sequence

<220>
<223> Synthetic

<400> 623
gaaattgtgt tgacgcagtc tccaggcacc ctgtctttgt ctccagggga aagagccacc 60
ctctcctgca gggccagtca gagtgttagc atcaggtact tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcag caatatggta gttcaccgct cactttcggc 300
ggagggacca aggtggagat caaa                                        324

<210> 624
<211> 108
<212> PRT
<213> Artificial Sequence

<220>
<223> Synthetic

<400> 624
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ile Arg
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Gly Gly Thr Lys Val Glu Ile Lys
            100                 105


<210> 625
<211> 381
<212> DNA
<213> Artificial Sequence

<220>
<223> Synthetic

<400> 625
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180

gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattcc tactacgtta tggacgtctg gggccaaggg 360
accacggtca ccgtctcctc a 381

<210> 626
<211> 127
<212> PRT
<213> Artificial Sequence

<220>
<223> Synthetic

<400> 626
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Ser Tyr Tyr
            100                 105                 110
Val Met Asp Val Trp Gly Gln Gly Thr Thr Val Thr Val Ser Ser
        115                 120                 125

<210> 627
<211> 24
<212> DNA
<213> Artificial Sequence

<220>
<223> Synthetic

<400> 627
ggttacacct ttaccaccta tggt 24

<210> 628
<211> 8
<212> PRT
<213> Artificial Sequence

<220>
<223> Synthetic

<400> 628
Gly Tyr Thr Phe Thr Thr Tyr Gly
1               5

<210> 629
<211> 24
<212> DNA
<213> Artificial Sequence

<220>
<223> Synthetic

<400> 629
atcagcggtt acaatggtaa aaca 24

<210> 630
<211> 8

<212> PRT
<213> Artificial Sequence

<220>
<223> Synthetic

<400> 630
Ile Ser Gly Tyr Asn Gly Lys Thr
1               5


<210> 631
<211> 60
<212> DNA
<213> Artificial Sequence

<220>
<223> Synthetic

<400> 631
tcgagagatc gtttagtagt accacctgcc ctttattatt cctactacgt tatggacgtc 60


<210> 632
<211> 20
<212> PRT
<213> Artificial Sequence

<220>
<223> Synthetic

<400> 632
Ser Arg Asp Arg Leu Val Val Pro Pro Ala Leu Tyr Tyr Ser Tyr Tyr
1               5                   10                  15
Val Met Asp Val
            20


<210> 633
<211> 336
<212> DNA
<213> Artificial Sequence

<220>
<223> Synthetic

<400> 633
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                            336

<210> 634
<211> 112
<212> PRT
<213> Artificial Sequence

<220>
<223> Synthetic

<400> 634
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45

Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 635
<211> 33
<212> DNA
<213> Artificial Sequence

<220>
<223> Synthetic

<400> 635
caaagcctcg tatacagtga tggaaacacc tac                                33

<210> 636
<211> 11
<212> PRT
<213> Artificial Sequence

<220>
<223> Synthetic

<400> 636
Gln Ser Leu Val Tyr Ser Asp Gly Asn Thr Tyr
1               5                   10

<210> 637
<211> 9
<212> DNA
<213> Artificial Sequence

<220>
<223> Synthetic

<400> 637
aaggtttct                                                            9

<210> 638
<211> 3
<212> PRT
<213> Artificial Sequence

<220>
<223> Synthetic

<400> 638
Lys Val Ser
1

<210> 639
<211> 27
<212> DNA
<213> Artificial Sequence

<220>
<223> Synthetic

<400> 639
atgcaaggta cacactggcc gtacact                                       27

<210> 640
<211> 9
<212> PRT
<213> Artificial Sequence

<220>
<223> Synthetic

<400> 640
Met Gln Gly Thr His Trp Pro Tyr Thr
1               5


<210> 641
<211> 381
<212> DNA
<213> Artificial Sequence

<220>
<223> Synthetic

<400> 641
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattcc tactacgtta tggacgtctg gggccaaggg 360
accacggtca ccgtctcctc a                                           381

<210> 642
<211> 127
<212> PRT
<213> Artificial Sequence

<220>
<223> Synthetic

<400> 642
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Ser Tyr Tyr
            100                 105                 110
Val Met Asp Val Trp Gly Gln Gly Thr Thr Val Thr Val Ser Ser
        115                 120                 125


<210> 643
<211> 336
<212> DNA
<213> Artificial Sequence

<220>
<223> Synthetic

<400> 643
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180

tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 644
<211> 112
<212> PRT
<213> Artificial Sequence

<220>
<223> Synthetic

<400> 644
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 645
<211> 381
<212> DNA
<213> Artificial Sequence

<220>
<223> Synthetic

<400> 645
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct ttattattcc tactacgtta tggacgtctg ggggcaaggg 360
accacggtca ccgtctcctc a 381

<210> 646
<211> 127
<212> PRT
<213> Artificial Sequence

<220>
<223> Synthetic

<400> 646
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Tyr Tyr Ser Tyr Tyr
            100                 105                 110

Val Met Asp Val Trp Gly Gln Gly Thr Thr Val Thr Val Ser Ser
        115                 120                 125

<210> 647
<211> 336
<212> DNA
<213> Artificial Sequence

<220>
<223> Synthetic

<400> 647
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 648
<211> 112
<212> PRT
<213> Artificial Sequence

<220>
<223> Synthetic

<400> 648
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 649
<211> 381
<212> DNA
<213> Artificial Sequence

<220>
<223> Synthetic

<400> 649
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattac tactacgtta tggacgtctg gggccaaggg 360
accacggtca ccgtctcctc a                                           381

<210> 650
<211> 127
<212> PRT
<213> Artificial Sequence

<220>
<223> Synthetic

<400> 650
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Asn Tyr Tyr Tyr Tyr
            100                 105                 110
Val Met Asp Val Trp Gly Gln Gly Thr Thr Val Thr Val Ser Ser
        115                 120                 125

<210> 651
<211> 24
<212> DNA
<213> Artificial Sequence

<220>
<223> Synthetic

<400> 651
ggttacacct ttaccaccta tggt                                              24

<210> 652
<211> 8
<212> PRT
<213> Artificial Sequence

<220>
<223> Synthetic

<400> 652
Gly Tyr Thr Phe Thr Thr Tyr Gly
1               5

<210> 653
<211> 24
<212> DNA
<213> Artificial Sequence

<220>
<223> Synthetic

<400> 653
atcagcggtt acaatggtaa aaca                                              24

<210> 654
<211> 8
<212> PRT
<213> Artificial Sequence

<220>
<223> Synthetic

<400> 654
Ile Ser Gly Tyr Asn Gly Lys Thr
1               5

<210> 655

<211> 60
<212> DNA
<213> Artificial Sequence

<220>
<223> Synthetic

<400> 655
tcgagagatc gtttagtagt accacctgcc cttaattatt actactacgt tatggacgtc 60


<210> 656
<211> 20
<212> PRT
<213> Artificial Sequence

<220>
<223> Synthetic

<400> 656
Ser Arg Asp Arg Leu Val Val Pro Pro Ala Leu Asn Tyr Tyr Tyr Tyr
1               5                   10                  15
Val Met Asp Val
            20


<210> 657
<211> 336
<212> DNA
<213> Artificial Sequence

<220>
<223> Synthetic

<400> 657
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 658
<211> 112
<212> PRT
<213> Artificial Sequence

<220>
<223> Synthetic

<400> 658
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 659
<211> 33

<212> DNA
<213> Artificial Sequence

<220>
<223> Synthetic

<400> 659
caaagcctcg tatacagtga tggaaacacc tac                                    33

<210> 660
<211> 11
<212> PRT
<213> Artificial Sequence

<220>
<223> Synthetic

<400> 660
Gln Ser Leu Val Tyr Ser Asp Gly Asn Thr Tyr
1               5                   10


<210> 661
<211> 9
<212> DNA
<213> Artificial Sequence

<220>
<223> Synthetic

<400> 661
aaggtttct                                                               9

<210> 662
<211> 3
<212> PRT
<213> Artificial Sequence

<220>
<223> Synthetic

<400> 662
Lys Val Ser
1


<210> 663
<211> 27
<212> DNA
<213> Artificial Sequence

<220>
<223> Synthetic

<400> 663
atgcaaggta cacactggcc gtacact                                          27

<210> 664
<211> 9
<212> PRT
<213> Artificial Sequence

<220>
<223> Synthetic

<400> 664
Met Gln Gly Thr His Trp Pro Tyr Thr
1               5

<210> 665
<211> 381
<212> DNA
<213> Artificial Sequence

<220>
<223> Synthetic

<400> 665
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct taattattac tactacgtta tggacgtctg gggccaaggg 360
accacggtca ccgtctcctc a 381

<210> 666
<211> 127
<212> PRT
<213> Artificial Sequence

<220>
<223> Synthetic

<400> 666
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Asn Tyr Tyr Tyr Tyr
            100                 105                 110
Val Met Asp Val Trp Gly Gln Gly Thr Thr Val Thr Val Ser Ser
        115                 120                 125

<210> 667
<211> 336
<212> DNA
<213> Artificial Sequence

<220>
<223> Synthetic

<400> 667
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 668
<211> 112
<212> PRT
<213> Artificial Sequence

<220>
<223> Synthetic

<400> 668
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 669
<211> 381
<212> DNA
<213> Artificial Sequence

<220>
<223> Synthetic

<400> 669
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct taattattac tactacgtta tggacgtctg ggggcaaggg 360
accacggtca ccgtctcctc a                                           381

<210> 670
<211> 127
<212> PRT
<213> Artificial Sequence

<220>
<223> Synthetic

<400> 670
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Asn Tyr Tyr Tyr Tyr
            100                 105                 110
Val Met Asp Val Trp Gly Gln Gly Thr Thr Val Thr Val Ser Ser
        115                 120                 125

<210> 671
<211> 336
<212> DNA
<213> Artificial Sequence

<220>
<223> Synthetic

<400> 671
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 672
<211> 112
<212> PRT
<213> Artificial Sequence

<220>
<223> Synthetic

<400> 672
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110

<210> 673
<211> 381
<212> DNA
<213> Artificial Sequence

<220>
<223> Synthetic

<400> 673
caggttcagc tggtgcagtc tggacctgag gtgaagaacc ctggggcctc agtgaaggtc 60
tcctgcaagg cttctggtta cacctttacc acctatggta tcagttgggt acgacaggcc 120
cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattac tactacgtta tggacgtctg gggccaaggg 360
accacggtca ccgtctcctc a 381

<210> 674
<211> 127
<212> PRT
<213> Artificial Sequence

<220>
<223> Synthetic

<400> 674
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80

Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Tyr Tyr Tyr
            100                 105                 110
Val Met Asp Val Trp Gly Gln Gly Thr Thr Val Thr Val Ser Ser
        115                 120                 125

<210> 675
<211> 24
<212> DNA
<213> Artificial Sequence

<220>
<223> Synthetic

<400> 675
ggttacacct ttaccaccta tggt                                              24

<210> 676
<211> 8
<212> PRT
<213> Artificial Sequence

<220>
<223> Synthetic

<400> 676
Gly Tyr Thr Phe Thr Thr Tyr Gly
 1               5

<210> 677
<211> 24
<212> DNA
<213> Artificial Sequence

<220>
<223> Synthetic

<400> 677
atcagcggtt acaatggtaa aaca                                              24

<210> 678
<211> 8
<212> PRT
<213> Artificial Sequence

<220>
<223> Synthetic

<400> 678
Ile Ser Gly Tyr Asn Gly Lys Thr
 1               5

<210> 679
<211> 60
<212> DNA
<213> Artificial Sequence

<220>
<223> Synthetic

<400> 679
tcgagagatc gtttagtagt accacctgcc ctttattatt actactacgt tatggacgtc 60

<210> 680

<211> 20
<212> PRT
<213> Artificial Sequence

<220>
<223> Synthetic

<400> 680
Ser Arg Asp Arg Leu Val Val Pro Pro Ala Leu Tyr Tyr Tyr Tyr Tyr
1               5                   10                  15
Val Met Asp Val
            20


<210> 681
<211> 336
<212> DNA
<213> Artificial Sequence

<220>
<223> Synthetic

<400> 681
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 682
<211> 112
<212> PRT
<213> Artificial Sequence

<220>
<223> Synthetic

<400> 682
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 683
<211> 33
<212> DNA
<213> Artificial Sequence

<220>
<223> Synthetic

<400> 683
caaagcctcg tatacagtga tggaaacacc tac                              33

<210> 684
<211> 11
<212> PRT

<213> Artificial Sequence

<220>
<223> Synthetic

<400> 684
Gln Ser Leu Val Tyr Ser Asp Gly Asn Thr Tyr
1               5                   10

<210> 685
<211> 9
<212> DNA
<213> Artificial Sequence

<220>
<223> Synthetic

<400> 685
aaggtttct                                                                9

<210> 686
<211> 3
<212> PRT
<213> Artificial Sequence

<220>
<223> Synthetic

<400> 686
Lys Val Ser
1

<210> 687
<211> 27
<212> DNA
<213> Artificial Sequence

<220>
<223> Synthetic

<400> 687
atgcaaggta cacactggcc gtacact                                           27

<210> 688
<211> 9
<212> PRT
<213> Artificial Sequence

<220>
<223> Synthetic

<400> 688
Met Gln Gly Thr His Trp Pro Tyr Thr
1               5

<210> 689
<211> 381
<212> DNA
<213> Artificial Sequence

<220>
<223> Synthetic

<400> 689
caggttcagc tggtgcagtc tggacctgag gtgaagaacc ctggggcctc agtgaaggtc 60
tcctgcaagg cttctggtta cacctttacc acctatggta tcagttgggt acgacaggcc 120

cctggacaag ggcttgagtg gatgggatgg atcagcggtt acaatggtaa aacaaacgat 180
gcacagaagt tccaggacag agtcgccatg accacagaca catccacgag cacagcctac 240
atggagctga ggagcctgag atctgacgac acggccattt attactgttc gagagatcgt 300
ttagtagtac cacctgccct ttattattac tactacgtta tggacgtctg gggccaaggg 360
accacggtca ccgtctcctc a 381

<210> 690
<211> 127
<212> PRT
<213> Artificial Sequence

<220>
<223> Synthetic

<400> 690
Gln Val Gln Leu Val Gln Ser Gly Pro Glu Val Lys Asn Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Asp Ala Gln Lys Phe
    50                  55                  60
Gln Asp Arg Val Ala Met Thr Thr Asp Thr Ser Thr Ser Thr Ala Tyr
65                  70                  75                  80
Met Glu Leu Arg Ser Leu Arg Ser Asp Asp Thr Ala Ile Tyr Tyr Cys
                85                  90                  95
Ser Arg Asp Arg Leu Val Val Pro Pro Ala Leu Tyr Tyr Tyr Tyr Tyr
            100                 105                 110
Val Met Asp Val Trp Gly Gln Gly Thr Thr Val Thr Val Ser Ser
        115                 120                 125

<210> 691
<211> 336
<212> DNA
<213> Artificial Sequence

<220>
<223> Synthetic

<400> 691
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggtca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa 336

<210> 692
<211> 112
<212> PRT
<213> Artificial Sequence

<220>
<223> Synthetic

<400> 692
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly

Thr His Trp Pro Tyr Thr Phe Gly Gln Gly Thr Lys Leu Glu Ile Lys
                85                  90                  95
            100                 105                 110

<210> 693
<211> 381
<212> DNA
<213> Artificial Sequence

<220>
<223> Synthetic

<400> 693
caggttcagc tggtgcagtc tggagctgag gtgaagaagc ctggggcctc agtgaaggtc 60
tcctgcaagg cttctggtta cacctttacc acctatggta tcagctgggt gcgacaggcc 120
cctggacaag ggcttgagtg gatgggatgg atcagcggtt acaatggtaa aacaaactat 180
gcacagaagc tccagggcag agtcaccatg accacagaca catccacgag cacagcctac 240
atggagctga ggagcctgag atctgacgac acggccgtgt attactgttc gagagatcgt 300
ttagtagtac cacctgccct ttattattac tactacgtta tggacgtctg ggggcaaggg 360
accacggtca ccgtctcctc a                                           381

<210> 694
<211> 127
<212> PRT
<213> Artificial Sequence

<220>
<223> Synthetic

<400> 694
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Thr Tyr
            20                  25                  30
Gly Ile Ser Trp Val Arg Gln Ala Pro Gly Gln Gly Leu Glu Trp Met
        35                  40                  45
Gly Trp Ile Ser Gly Tyr Asn Gly Lys Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ser Arg Asp Arg Leu Val Val Pro Pro Ala Leu Tyr Tyr Tyr Tyr Tyr
            100                 105                 110
Val Met Asp Val Trp Gly Gln Gly Thr Thr Val Thr Val Ser Ser
        115                 120                 125

<210> 695
<211> 336
<212> DNA
<213> Artificial Sequence

<220>
<223> Synthetic

<400> 695
gatgttgtga tgactcagtc tccactctcc ctgcccgtca cccttggaca gccggcctcc 60
atctcctgca ggtctagtca aagcctcgta tacagtgatg gaaacaccta cttgaattgg 120
tttcagcaga ggccaggcca atctccaagg cgcctaattt ataaggtttc taaccgggac 180
tctggggtcc cagacagatt cagcggcagt gggtcaggca ctgatttcac actgaaaatc 240
agcagggtgg aggctgagga tgttggggtt tattactgca tgcaaggtac acactggccg 300
tacacttttg gccaggggac caagctggag atcaaa                           336

<210> 696
<211> 112
<212> PRT
<213> Artificial Sequence

<220>
<223> Synthetic

<400> 696
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Leu Val Tyr Ser
            20                  25                  30
Asp Gly Asn Thr Tyr Leu Asn Trp Phe Gln Gln Arg Pro Gly Gln Ser
        35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro Tyr Thr Phe Gly Gln Gly Thr Lys Leu Glu Ile Lys
            100                 105                 110


<210> 697
<211> 384
<212> DNA
<213> Artificial Sequence

<220>
<223> Synthetic

<400> 697
caggtgcacc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtcgc ctca                                        384

<210> 698
<211> 128
<212> PRT
<213> Artificial Sequence

<220>
<223> Synthetic

<400> 698
Gln Val His Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ala Ser
        115                 120                 125


<210> 699
<211> 24
<212> DNA
<213> Artificial Sequence

<220>
<223> Synthetic

<400> 699
ggattcacct tcagtgacca ctac                                              24

<210> 700
<211> 8
<212> PRT
<213> Artificial Sequence

<220>
<223> Synthetic

<400> 700
Gly Phe Thr Phe Ser Asp His Tyr
1               5


<210> 701
<211> 24
<212> DNA
<213> Artificial Sequence

<220>
<223> Synthetic

<400> 701
attagtaatg atggtggtac caaa                                              24

<210> 702
<211> 8
<212> PRT
<213> Artificial Sequence

<220>
<223> Synthetic

<400> 702
Ile Ser Asn Asp Gly Gly Thr Lys
1               5


<210> 703
<211> 63
<212> DNA
<213> Artificial Sequence

<220>
<223> Synthetic

<400> 703
gcgagagatc agggatatat tggctacgac tcgtattatt actattccta cggtatggac 60
gtc                                                                     63

<210> 704
<211> 21
<212> PRT
<213> Artificial Sequence

<220>
<223> Synthetic

<400> 704
Ala Arg Asp Gln Gly Tyr Ile Gly Tyr Asp Ser Tyr Tyr Tyr Tyr Ser
1               5                   10                  15
Tyr Gly Met Asp Val
            20

<210> 705
<211> 321
<212> DNA
<213> Artificial Sequence

<220>
<223> Synthetic

<400> 705
aaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tttcggcgga 300
gggaccaagg tggagatcaa g 321

<210> 706
<211> 107
<212> PRT
<213> Artificial Sequence

<220>
<223> Synthetic

<400> 706
Lys Ile Val Leu Thr Gln Ser Pro Gly Thr Leu Pro Leu Phe Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Ser Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Gly Leu Glu
65                  70                  75                  80
Pro Glu Asp Phe Glu Val Tyr Tyr Cys Gln Val Tyr Gly Asn Ser Leu
                85                  90                  95
Thr Phe Gly Gly Gly Thr Lys Val Glu Ile Lys
            100                 105

<210> 707
<211> 21
<212> DNA
<213> Artificial Sequence

<220>
<223> Synthetic

<400> 707
cagagtgtta acaacaaatt c 21

<210> 708
<211> 7
<212> PRT
<213> Artificial Sequence

<220>
<223> Synthetic

<400> 708
Gln Ser Val Asn Asn Lys Phe
1               5

<210> 709
<211> 9

<212> DNA
<213> Artificial Sequence

<220>
<223> Synthetic

<400> 709
ggtgcatcc                                                                9

<210> 710
<211> 3
<212> PRT
<213> Artificial Sequence

<220>
<223> Synthetic

<400> 710
Gly Ala Ser
1

<210> 711
<211> 24
<212> DNA
<213> Artificial Sequence

<220>
<223> Synthetic

<400> 711
caagtatatg gtaactcact cact                                               24

<210> 712
<211> 8
<212> PRT
<213> Artificial Sequence

<220>
<223> Synthetic

<400> 712
Gln Val Tyr Gly Asn Ser Leu Thr
1               5

<210> 713
<211> 384
<212> DNA
<213> Artificial Sequence

<220>
<223> Synthetic

<400> 713
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg gatttcatac attagtaatg atggtggtac caaatactat 180
gtggactctg tggagggccg attcatcatt tccagggaca acgccaagaa ctcattgtat 240
ctacatatga acagcctcag agccgacgac acggccgtgt attactgtgc gagagatcag 300
ggatatattg gctacgactc gtattattac tattcctacg gtatggacgt ctggggccaa 360
gggaccacgg tcaccgtctc ctca                                               384

<210> 714
<211> 128
<212> PRT
<213> Artificial Sequence

<220>

<223> Synthetic

<400> 714
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Ile
        35                  40                  45
Ser Tyr Ile Ser Asn Asp Gly Gly Thr Lys Tyr Tyr Val Asp Ser Val
    50                  55                  60
Glu Gly Arg Phe Ile Ile Ser Arg Asp Asn Ala Lys Asn Ser Leu Tyr
65                  70                  75                  80
Leu His Met Asn Ser Leu Arg Ala Asp Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125


<210> 715
<211> 321
<212> DNA
<213> Artificial Sequence

<220>
<223> Synthetic

<400> 715
gaaattgtgt tgacgcagtc tccaggcacc ctgcctttgt ttccagggga aagagccacc 60
ctctcctgta gggccagtca gagtgttaac aacaaattct tagcctggta ccagcagaaa 120
tctggccagg ctcccaggct cctcatctat ggtgcatcca gcagggccac tggcatccca 180
gacaggttca gtggcagtgg gtctgggacc gacttcactc tcaccatcag cggactggag 240
cctgaagatt ttgaagtgta ttattgtcaa gtatatggta actcactcac tttcggcgga 300
gggaccaagg tggagatcaa a                                           321

<210> 716
<211> 107
<212> PRT
<213> Artificial Sequence

<220>
<223> Synthetic

<400> 716
Glu Ile Val Leu Thr Gln Ser Pro Gly Thr Leu Pro Leu Phe Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Ser Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Gly Leu Glu
65                  70                  75                  80
Pro Glu Asp Phe Glu Val Tyr Tyr Cys Gln Val Tyr Gly Asn Ser Leu
                85                  90                  95
Thr Phe Gly Gly Gly Thr Lys Val Glu Ile Lys
            100                 105


<210> 717
<211> 384
<212> DNA
<213> Artificial Sequence

<220>
<223> Synthetic

<400> 717
caggtgcagc tggtggagtc tgggggaggc ttggtcaagc ctggagggtc cctgagactc 60
tcctgtgcag cctctggatt caccttcagt gaccactaca tgagctggat ccgccaggct 120
ccagggaagg ggctggagtg ggtttcatac attagtaatg atggtggtac caaatactac 180
gcagactctg tgaagggccg attcaccatc tccagggaca acgccaagaa ctcactgtat 240
ctgcaaatga acagcctgag agccgaggac acggccgtgt attactgtgc gagagatcag 300
ggatatattg gctacgactc gtattattac tattcctacg gtatggacgt ctgggggcaa 360
gggaccacgg tcaccgtctc ctca 384

<210> 718
<211> 128
<212> PRT
<213> Artificial Sequence

<220>
<223> Synthetic

<400> 718
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp His
            20                  25                  30
Tyr Met Ser Trp Ile Arg Gln Ala Pro Gly Lys Gly Leu Glu Trp Val
        35                  40                  45
Ser Tyr Ile Ser Asn Asp Gly Gly Thr Lys Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Asp Gln Gly Tyr Ile Gly Tyr Asp Ser Tyr Tyr Tyr Tyr Ser
            100                 105                 110
Tyr Gly Met Asp Val Trp Gly Gln Gly Thr Thr Val Thr Val Ser Ser
        115                 120                 125

<210> 719
<211> 321
<212> DNA
<213> Artificial Sequence

<220>
<223> Synthetic

<400> 719
gaaattgtgt tgacgcagtc tccaggcacc ctgtctttgt ctccagggga aagagccacc 60
ctctcctgca gggccagtca gagtgttaac aacaaattct tagcctggta ccagcagaaa 120
cctggccagg ctcccaggct cctcatctat ggtgcatcca gcagggccac tggcatccca 180
gacaggttca gtggcagtgg gtctgggaca gacttcactc tcaccatcag cagactggag 240
cctgaagatt ttgcagtgta ttactgtcaa gtatatggta actcactcac tttcggcgga 300
gggaccaagg tggagatcaa a 321

<210> 720
<211> 107
<212> PRT
<213> Artificial Sequence

<220>
<223> Synthetic

<400> 720
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Asn Asn Lys
            20                  25                  30
Phe Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser

    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Val Tyr Gly Asn Ser Leu
                85                  90                  95
Thr Phe Gly Gly Gly Thr Lys Val Glu Ile Lys
            100                 105

<210> 721
<211> 378
<212> DNA
<213> Artificial Sequence

<220>
<223> Synthetic

<400> 721
caaattctgc tggtgcaatc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca                                                378

<210> 722
<211> 126
<212> PRT
<213> Artificial Sequence

<220>
<223> Synthetic

<400> 722
Gln Ile Leu Leu Val Gln Ser Gly Pro Glu Val Lys Glu Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Val Pro Gly Gln Gly Leu Glu Trp Met
        35                  40                  45
Gly Trp Val Ser Ala Tyr Asn Gly His Thr Asn Tyr Ala His Glu Val
    50                  55                  60
Gln Gly Arg Val Thr Met Thr Thr Asp Thr Ser Thr Thr Thr Ala Tyr
65                  70                  75                  80
Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 723
<211> 24
<212> DNA
<213> Artificial Sequence

<220>
<223> Synthetic

<400> 723
ggttacacct ttaccaacta cgct                                         24

<210> 724
<211> 8
<212> PRT
<213> Artificial Sequence

<220>
<223> Synthetic

<400> 724
Gly Tyr Thr Phe Thr Asn Tyr Ala
1               5


<210> 725
<211> 24
<212> DNA
<213> Artificial Sequence

<220>
<223> Synthetic

<400> 725
gtcagcgctt acaatggtca caca                                              24

<210> 726
<211> 8
<212> PRT
<213> Artificial Sequence

<220>
<223> Synthetic

<400> 726
Val Ser Ala Tyr Asn Gly His Thr
1               5


<210> 727
<211> 57
<212> DNA
<213> Artificial Sequence

<220>
<223> Synthetic

<400> 727
gcgagagggg gtgtagtcgt gccagttgct ccccacttct acaacggtat ggacgtc    57

<210> 728
<211> 19
<212> PRT
<213> Artificial Sequence

<220>
<223> Synthetic

<400> 728
Ala Arg Gly Gly Val Val Val Pro Val Ala Pro His Phe Tyr Asn Gly
1               5                   10                  15
Met Asp Val



<210> 729
<211> 336
<212> DNA
<213> Artificial Sequence

<220>
<223> Synthetic

<400> 729
gatattgtga tgactcagtt tccactctcc ctgcccgtca cccctggaga gccggcctcc 60
atctcctgca ggtctagtca gagcctcctg catattaatg aatacaacta tttggattgg 120

tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttcg gccaagggac caaggtggaa atcaaa 336

<210> 730
<211> 112
<212> PRT
<213> Artificial Sequence

<220>
<223> Synthetic

<400> 730
Asp Ile Val Met Thr Gln Phe Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Lys Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Phe Gly Gln Gly Thr Lys Val Glu Ile Lys
            100                 105                 110

<210> 731
<211> 33
<212> DNA
<213> Artificial Sequence

<220>
<223> Synthetic

<400> 731
cagagcctcc tgcatattaa tgaatacaac tat 33

<210> 732
<211> 11
<212> PRT
<213> Artificial Sequence

<220>
<223> Synthetic

<400> 732
Gln Ser Leu Leu His Ile Asn Glu Tyr Asn Tyr
1               5                   10

<210> 733
<211> 9
<212> DNA
<213> Artificial Sequence

<220>
<223> Synthetic

<400> 733
ttgggtttt 9

<210> 734
<211> 3
<212> PRT
<213> Artificial Sequence

<220>
<223> Synthetic

<400> 734
Leu Gly Phe
1

<210> 735
<211> 27
<212> DNA
<213> Artificial Sequence

<220>
<223> Synthetic

<400> 735
atgcaagctc ttcaaactcc gtggacg                                          27

<210> 736
<211> 9
<212> PRT
<213> Artificial Sequence

<220>
<223> Synthetic

<400> 736
Met Gln Ala Leu Gln Thr Pro Trp Thr
1               5

<210> 737
<211> 378
<212> DNA
<213> Artificial Sequence

<220>
<223> Synthetic

<400> 737
caggttcagc tggtgcagtc tggacctgag gtgaaggagc ctggggcctc agtgaaggtc 60
tcctgcaagg cttctggtta cacctttacc aactacgcta tcagctgggt gcgacaggtc 120
cctggacaag ggcttgagtg gatgggatgg gtcagcgctt acaatggtca cacaaactat 180
gcacatgaag tccagggcag agtcaccatg accacagaca catccacgac cacagcctac 240
atggagctga ggagcctgag atctgacgac acggccatgt attactgtgc gagagggggt 300
gtagtcgtgc cagttgctcc ccacttctac aacggtatgg acgtctgggg ccaagggacc 360
acggtcaccg tctcctca                                                    378

<210> 738
<211> 126
<212> PRT
<213> Artificial Sequence

<220>
<223> Synthetic

<400> 738
Gln Val Gln Leu Val Gln Ser Gly Pro Glu Val Lys Glu Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Val Pro Gly Gln Gly Leu Glu Trp Met
        35                  40                  45
Gly Trp Val Ser Ala Tyr Asn Gly His Thr Asn Tyr Ala His Glu Val
    50                  55                  60
Gln Gly Arg Val Thr Met Thr Thr Asp Thr Ser Thr Thr Thr Ala Tyr
65                  70                  75                  80

Met Glu Leu Arg Ser Leu Arg Ser Asp Asp Thr Ala Met Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 739
<211> 336
<212> DNA
<213> Artificial Sequence

<220>
<223> Synthetic

<400> 739
gatattgtga tgactcagtc tccactctcc ctgcccgtca cccctggaga gccggcctcc 60
atctcctgca ggtctagtca gagcctcctg catattaatg aatacaacta tttggattgg 120
tacctaaaga agccagggca gtctccacag ctcctgatct atttgggttt taatcgggcc 180
tccggggtcc ctgacaggtt cagtggcagt ggatcaggca cagattttac actgaaaatc 240
agcagagtgg aggctgagga tgttggggtc tattactgca tgcaagctct tcaaactccg 300
tggacgttcg gccaagggac caaggtggaa atcaaa                           336

<210> 740
<211> 112
<212> PRT
<213> Artificial Sequence

<220>
<223> Synthetic

<400> 740
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Lys Lys Pro Gly Gln Ser
        35                  40                  45
Pro Gln Leu Leu Ile Tyr Leu Gly Phe Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Phe Gly Gln Gly Thr Lys Val Glu Ile Lys
            100                 105                 110

<210> 741
<211> 378
<212> DNA
<213> Artificial Sequence

<220>
<223> Synthetic

<400> 741
caggttcagc tggtgcagtc tggagctgag gtgaagaagc ctggggcctc agtgaaggtc 60
tcctgcaagg cttctggtta cacctttacc aactacgcta tcagctgggt gcgacaggcc 120
cctggacaag ggcttgagtg gatgggatgg gtcagcgctt acaatggtca cacaaactat 180
gcacagaagc tccagggcag agtcaccatg accacagaca catccacgag cacagcctac 240
atggagctga ggagcctgag atctgacgac acggccgtgt attactgtgc gagagggggt 300
gtagtcgtgc cagttgctcc ccacttctac aacggtatgg acgtctgggg gcaagggacc 360
acggtcaccg tctcctca                                               378

<210> 742
<211> 126
<212> PRT

<213> Artificial Sequence

<220>
<223> Synthetic

<400> 742
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Ala Ile Ser Trp Val Arg Gln Ala Pro Gly Gln Gly Leu Glu Trp Met
        35                  40                  45
Gly Trp Val Ser Ala Tyr Asn Gly His Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg Gly Gly Val Val Val Pro Val Ala Pro His Phe Tyr Asn Gly
            100                 105                 110
Met Asp Val Trp Gly Gln Gly Thr Thr Val Thr Val Ser Ser
        115                 120                 125

<210> 743
<211> 336
<212> DNA
<213> Artificial Sequence

<220>
<223> Synthetic

<400> 743
gatattgtga tgactcagtc tccactctcc ctgcccgtca cccctggaga gccggcctcc 60
atctcctgca ggtctagtca gagcctcctg catattaatg aatacaacta tttggattgg 120
tacctgcaga agccagggca gtctccacag ctcctgatct atttgggttc taatcgggcc 180
tccggggtcc ctgacaggtt cagtggcagt ggatcaggca cagattttac actgaaaatc 240
agcagagtgg aggctgagga tgttggggtt tattactgca tgcaagctct tcaaactccg 300
tggacgttcg gccaagggac caaggtggaa atcaaa                           336

<210> 744
<211> 112
<212> PRT
<213> Artificial Sequence

<220>
<223> Synthetic

<400> 744
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ile
            20                  25                  30
Asn Glu Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro Trp Thr Phe Gly Gln Gly Thr Lys Val Glu Ile Lys
            100                 105                 110

<210> 745
<211> 8
<212> PRT
<213> Artificial Sequence

<220>
<223> Synthetic

<221> VARIANT
<222> (1)...(8)
<223> Xaa = Any amino acid

<400> 745
Xaa Xaa Xaa Xaa Xaa Xaa Xaa Xaa
 1               5


<210> 746
<211> 8
<212> PRT
<213> Artificial Sequence

<220>
<223> Synthetic

<221> VARIANT
<222> (1)...(8)
<223> Xaa - Any amino acid

<400> 746
Xaa Xaa Xaa Xaa Xaa Xaa Xaa Xaa
 1               5


<210> 747
<211> 16
<212> PRT
<213> Artificial Sequence

<220>
<223> Synthetic

<221> VARIANT
<222> (1)...(16)
<223> Xaa = Any amino acid

<400> 747
Xaa Xaa Xaa Xaa Xaa Xaa Xaa Xaa Xaa Xaa Xaa Xaa Xaa Xaa Xaa Xaa
 1               5                   10                  15


<210> 748
<211> 12
<212> PRT
<213> Artificial Sequence

<220>
<223> Synthetic

<221> VARIANT
<222> (1)...(12)
<223> Xaa = Any amino acid

<400> 748
Xaa Xaa Xaa Xaa Xaa Xaa Xaa Xaa Xaa Xaa Xaa Xaa
 1               5                   10


<210> 749
<211> 3
<212> PRT
<213> Artificial Sequence

<220>
<223> Synthetic

<221> VARIANT
<222> (1)...(3)
<223> Xaa = Any amino acid

<400> 749
Xaa Xaa Xaa
1


<210> 750
<211> 9
<212> PRT
<213> Artificial Sequence

<220>
<223> Synthetic

<221> VARIANT
<222> (1)...(9)
<223> Xaa = Any amino acid

<400> 750
Xaa Xaa Xaa Xaa Xaa Xaa Xaa Xaa Xaa
 1               5


<210> 751
<211> 330
<212> PRT
<213> Artificial Sequence

<220>
<223> Synthetic

<400> 751



<210> 752
<211> 327
<212> PRT
<213> Artificial Sequence

<220>
<223> Synthetic

<400> 752
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Ser Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325



<210> 753
<211> 327
<212> PRT

<213> Artificial Sequence

<220>
<223> Synthetic

<400> 753
Ala Ser Thr Lys Gly Pro Ser Val Phe Pro Leu Ala Pro Cys Ser Arg
1               5                   10                  15
Ser Thr Ser Glu Ser Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Lys Thr
65                  70                  75                  80
Tyr Thr Cys Asn Val Asp His Lys Pro Ser Asn Thr Lys Val Asp Lys
                85                  90                  95
Arg Val Glu Ser Lys Tyr Gly Pro Pro Cys Pro Pro Cys Pro Ala Pro
            100                 105                 110
Glu Phe Leu Gly Gly Pro Ser Val Phe Leu Phe Pro Pro Lys Pro Lys
        115                 120                 125
Asp Thr Leu Met Ile Ser Arg Thr Pro Glu Val Thr Cys Val Val Val
    130                 135                 140
Asp Val Ser Gln Glu Asp Pro Glu Val Gln Phe Asn Trp Tyr Val Asp
145                 150                 155                 160
Gly Val Glu Val His Asn Ala Lys Thr Lys Pro Arg Glu Glu Gln Phe
                165                 170                 175
Asn Ser Thr Tyr Arg Val Val Ser Val Leu Thr Val Leu His Gln Asp
            180                 185                 190
Trp Leu Asn Gly Lys Glu Tyr Lys Cys Lys Val Ser Asn Lys Gly Leu
        195                 200                 205
Pro Ser Ser Ile Glu Lys Thr Ile Ser Lys Ala Lys Gly Gln Pro Arg
    210                 215                 220
Glu Pro Gln Val Tyr Thr Leu Pro Pro Ser Gln Glu Glu Met Thr Lys
225                 230                 235                 240
Asn Gln Val Ser Leu Thr Cys Leu Val Lys Gly Phe Tyr Pro Ser Asp
                245                 250                 255
Ile Ala Val Glu Trp Glu Ser Asn Gly Gln Pro Glu Asn Asn Tyr Lys
            260                 265                 270
Thr Thr Pro Pro Val Leu Asp Ser Asp Gly Ser Phe Phe Leu Tyr Ser
        275                 280                 285
Arg Leu Thr Val Asp Lys Ser Arg Trp Gln Glu Gly Asn Val Phe Ser
    290                 295                 300
Cys Ser Val Met His Glu Ala Leu His Asn His Tyr Thr Gln Lys Ser
305                 310                 315                 320
Leu Ser Leu Ser Leu Gly Lys
                325


<210> 754
<211> 2076
<212> DNA
<213> Homo sapiens

<400> 754
atgggcaccg tcagctccag gcggtcctgg tggccgctgc cactgctgct gctgctgctg 60
ctgctcctgg gtcccgcggg cgcccgtgcg caggaggacg aggacggcga ctacgaggag 120
ctggtgctag ccttgcgttc cgaggaggac ggcctggccg aagcacccga gcacggaacc 180
acagccacct tccaccgctg cgccaaggat ccgtggaggt tgcctggcac ctacgtggtg 240
gtgctgaagg aggagaccca cctctcgcag tcagagcgca ctgcccgccg cctgcaggcc 300
caggctgccc gccggggata cctcaccaag atcctgcatg tcttccatgg ccttcttcct 360
ggcttcctgg tgaagatgag tggcgacctg ctggagctgg ccttgaagtt gccccatgtc 420
gactacatcg aggaggactc ctctgtcttt gcccagagca tcccgtggaa cctggagcgg 480
attacccctc cacggtaccg ggcggatgaa taccagcccc ccgacggagg cagcctggtg 540
gaggtgtatc tcctagacac cagcatacag agtgaccacc gggaaatcga gggcagggtc 600
atggtcaccg acttcgagaa tgtgcccgag gaggacggga cccgcttcca cagacaggcc 660
agcaagtgtg acagtcatgg cacccacctg gcaggggtgg tcagcggccg ggatgccggc 720
gtggccaagg gtgccagcat gcgcagcctg cgcgtgctca actgccaagg gaagggcacg 780

gttagcggca ccctcatagg cctggagttt attcggaaaa gccagctggt ccagcctgtg 840
gggccactgg tggtgctgct gcccctggcg ggtgggtaca gccgcgtcct caacgccgcc 900
tgccagcgcc tggcgagggc tggggtcgtg ctggtcaccg ctgccggcaa cttccgggac 960
gatgcctgcc tctactcccc agcctcagct cccgaggtca tcacagttgg ggccaccaat 1020
gcccaagacc agccggtgac cctggggact ttggggacca actttggccg ctgtgtggac 1080
ctctttgccc caggggagga catcattggt gcctccagcg actgcagcac ctgctttgtg 1140
tcacagagtg ggacatcaca ggctgctgcc cacgtggctg gcattgcagc catgatgctg 1200
tctgccgagc cggagctcac cctggccgag ttgaggcaga gactgatcca cttctctgcc 1260
aaagatgtca tcaatgaggc ctggttccct gaggaccagc gggtactgac ccccaacctg 1320
gtggccgccc tgccccccag cacccatggg gcaggttggc agctgttttg caggactgta 1380
tggtcagcac actcggggcc tacacggatg gccacagccg tcgcccgctg cgccccagat 1440
gaggagctgc tgagctgctc cagtttctcc aggagtggga agcggcgggg cgagcgcatg 1500
gaggcccaag ggggcaagct ggtctgccgg gcccacaacg cttttggggg tgagggtgtc 1560
tacgccattg ccaggtgctg cctgctaccc caggccaact gcagcgtcca cacagctcca 1620
ccagctgagg ccagcatggg gacccgtgtc cactgccacc aacaggccca cgtcctcaca 1680
ggctgcagct cccactggga ggtggaggac cttggcaccc acaagccgcc tgtgctgagg 1740
ccacgaggtc agcccaacca gtgcgtgggc cacagggagg ccagcatcca cgcttcctgc 1800
tgccatgccc caggtctgga atgcaaagtc aaggagcatg gaatcccggc ccctcaggag 1860
caggtgaccg tggcctgcga ggagggctgg accctgactg gctgcagtgc cctccctggg 1920
acctcccacg tcctgggggc ctacgccgta gacaacacgt gtgtagtcag gagccgggac 1980
gtcagcacta caggcagcac cagcgaaggg gccgtgacag ccgttgccat ctgctgccgg 2040
agccggcacc tggcgcaggc ctcccaggag ctccag 2076

<210> 755
<211> 692
<212> PRT
<213> Homo sapiens

<400> 755

<210> 756
<211> 692
<212> PRT
<213> Macaca mulata

<400> 756
Met Gly Thr Val Ser Ser Arg Arg Ser Trp Trp Pro Leu Pro Leu Pro
 1               5                  10                  15
Leu Leu Leu Leu Leu Leu Leu Gly Pro Ala Gly Ala Arg Ala Gln Glu
            20                  25                  30
Asp Glu Asp Gly Asp Tyr Glu Glu Leu Val Leu Ala Leu Arg Ser Glu
        35                  40                  45
Glu Asp Gly Leu Ala Asp Ala Pro Glu His Gly Ala Thr Ala Thr Phe
    50                  55                  60
His Arg Cys Ala Lys Asp Pro Trp Arg Leu Pro Gly Thr Tyr Val Val
65                  70                  75                  80
Val Leu Lys Glu Glu Thr His Arg Ser Gln Ser Glu Arg Thr Ala Arg
                85                  90                  95
Arg Leu Gln Ala Gln Ala Ala Arg Arg Gly Tyr Leu Thr Lys Ile Leu

                100                 105                 110
His Val Phe His His Leu Leu Pro Gly Phe Leu Val Lys Met Ser Gly
            115                 120                 125
Asp Leu Leu Glu Leu Ala Leu Lys Leu Pro His Val Asp Tyr Ile Glu
    130                 135                 140
Glu Asp Ser Ser Val Phe Ala Gln Ser Ile Pro Trp Asn Leu Glu Arg
145                 150                 155                 160
Ile Thr Pro Ala Arg Tyr Arg Ala Asp Glu Tyr Gln Pro Pro Lys Gly
                165                 170                 175
Gly Ser Leu Val Glu Val Tyr Leu Leu Asp Thr Ser Ile Gln Ser Asp
            180                 185                 190
His Arg Glu Ile Glu Gly Arg Val Met Val Thr Asp Phe Glu Ser Val
        195                 200                 205
Pro Glu Glu Asp Gly Thr Arg Phe His Arg Gln Ala Ser Lys Cys Asp
    210                 215                 220
Ser His Gly Thr His Leu Ala Gly Val Val Ser Gly Arg Asp Ala Gly
225                 230                 235                 240
Val Ala Lys Gly Ala Gly Leu Arg Ser Leu Arg Val Leu Asn Cys Gln
                245                 250                 255
Gly Lys Gly Thr Val Ser Gly Thr Leu Ile Gly Leu Glu Phe Ile Arg
            260                 265                 270
Lys Ser Gln Leu Val Gln Pro Val Gly Pro Leu Val Val Leu Leu Pro
        275                 280                 285
Leu Ala Gly Gly Tyr Ser Arg Val Phe Asn Ala Ala Cys Gln Arg Leu
    290                 295                 300
Ala Arg Ala Gly Val Val Leu Val Thr Ala Ala Gly Asn Phe Arg Asp
305                 310                 315                 320
Asp Ala Cys Leu Tyr Ser Pro Ala Ser Ala Pro Glu Val Ile Thr Val
                325                 330                 335
Gly Ala Thr Asn Ala Gln Asp Gln Pro Val Thr Leu Gly Thr Leu Gly
            340                 345                 350
Thr Asn Phe Gly Arg Cys Val Asp Leu Phe Ala Pro Gly Glu Asp Ile
        355                 360                 365
Ile Gly Ala Ser Ser Asp Cys Ser Thr Cys Phe Val Ser Arg Ser Gly
    370                 375                 380
Thr Ser Gln Ala Ala Ala His Val Ala Gly Ile Ala Ala Met Met Leu
385                 390                 395                 400
Ser Ala Glu Pro Glu Leu Thr Leu Ala Glu Leu Arg Gln Arg Leu Ile
                405                 410                 415
His Phe Ser Ala Lys Asp Val Ile Asn Glu Ala Trp Phe Pro Glu Asp
            420                 425                 430
Gln Arg Val Leu Thr Pro Asn Leu Val Ala Ala Leu Pro Pro Ser Thr
        435                 440                 445
His Arg Ala Gly Trp Gln Leu Phe Cys Arg Thr Val Trp Ser Ala His
    450                 455                 460
Ser Gly Pro Thr Arg Met Ala Thr Ala Val Ala Arg Cys Ala Gln Asp
465                 470                 475                 480
Glu Glu Leu Leu Ser Cys Ser Ser Phe Ser Arg Ser Gly Lys Arg Arg
                485                 490                 495
Gly Glu Arg Ile Glu Ala Gln Gly Gly Lys Arg Val Cys Arg Ala His
            500                 505                 510
Asn Ala Phe Gly Gly Glu Gly Val Tyr Ala Ile Ala Arg Cys Cys Leu
        515                 520                 525
Leu Pro Gln Val Asn Cys Ser Val His Thr Ala Pro Pro Ala Gly Ala
    530                 535                 540
Ser Met Gly Thr Arg Val His Cys His Gln Gln Gly His Val Leu Thr
545                 550                 555                 560
Gly Cys Ser Ser His Trp Glu Val Glu Asp Leu Gly Thr His Lys Pro
                565                 570                 575
Pro Val Leu Arg Pro Arg Gly Gln Pro Asn Gln Cys Val Gly His Arg
            580                 585                 590
Glu Ala Ser Ile His Ala Ser Cys Cys His Ala Pro Gly Leu Glu Cys
        595                 600                 605
Lys Val Lys Glu His Gly Ile Pro Ala Pro Gln Glu Gln Val Ile Val
    610                 615                 620
Ala Cys Glu Asp Gly Trp Thr Leu Thr Gly Cys Ser Pro Leu Pro Gly
625                 630                 635                 640
Thr Ser His Val Leu Gly Ala Tyr Ala Val Asp Asn Thr Cys Val Val
                645                 650                 655

Arg Ser Arg Asp Val Ser Thr Thr Gly Ser Thr Ser Lys Glu Ala Val
            660                 665                 670
Ala Ala Val Ala Ile Cys Cys Arg Ser Arg His Leu Val Gln Ala Ser
        675                 680                 685
Gln Glu Leu Gln
    690

<210> 757
<211> 694
<212> PRT
<213> Mus muscular

<400> 757
   Met Gly Thr His Cys Ser Ala Trp Leu Arg Trp Pro Leu Leu Pro Leu
   1               5                   10                  15
   Leu Pro Pro Leu Leu Leu Leu Leu Leu Leu Leu Cys Pro Thr Gly Ala
               20                  25                  30
   Gly Ala Gln Asp Glu Asp Gly Asp Tyr Glu Glu Leu Met Leu Ala Leu
           35                  40                  45
   Pro Ser Gln Glu Asp Gly Leu Ala Asp Glu Ala Ala His Val Ala Thr
       50                  55                  60
   Ala Thr Phe Arg Arg Cys Ser Lys Glu Ala Trp Arg Leu Pro Gly Thr
   65                  70                  75                  80
   Tyr Ile Val Val Leu Met Glu Glu Thr Gln Arg Leu Gln Ile Glu Gln
                   85                  90                  95
   Thr Ala His Arg Leu Gln Thr Arg Ala Ala Arg Arg Gly Tyr Val Ile
               100                 105                 110
   Lys Val Leu His Ile Phe Tyr Asp Leu Phe Pro Gly Phe Leu Val Lys
           115                 120                 125
   Met Ser Ser Asp Leu Leu Gly Leu Ala Leu Lys Leu Pro His Val Glu
       130                 135                 140
   Tyr Ile Glu Glu Asp Ser Phe Val Phe Ala Gln Ser Ile Pro Trp Asn
   145                 150                 155                 160
   Leu Glu Arg Ile Ile Pro Ala Trp His Gln Thr Glu Glu Asp Arg Ser
                   165                 170                 175
   Pro Asp Gly Ser Ser Gln Val Glu Val Tyr Leu Leu Asp Thr Ser Ile
               180                 185                 190
   Gln Gly Ala His Arg Glu Ile Glu Gly Arg Val Thr Ile Thr Asp Phe
           195                 200                 205
   Asn Ser Val Pro Glu Glu Asp Gly Thr Arg Phe His Arg Gln Ala Ser
       210                 215                 220
   Lys Cys Asp Ser His Gly Thr His Leu Ala Gly Val Val Ser Gly Arg
   225                 230                 235                 240
   Asp Ala Gly Val Ala Lys Gly Thr Ser Leu His Ser Leu Arg Val Leu
                   245                 250                 255
   Asn Cys Gln Gly Lys Gly Thr Val Ser Gly Thr Leu Ile Gly Leu Glu
               260                 265                 270
   Phe Ile Arg Lys Ser Gln Leu Ile Gln Pro Ser Gly Pro Leu Val Val
           275                 280                 285
   Leu Leu Pro Leu Ala Gly Gly Tyr Ser Arg Ile Leu Asn Ala Ala Cys
       290                 295                 300
   Arg His Leu Ala Arg Thr Gly Val Val Leu Val Ala Ala Ala Gly Asn
   305                 310                 315                 320
   Phe Arg Asp Asp Ala Cys Leu Tyr Ser Pro Ala Ser Ala Pro Glu Val
                   325                 330                 335
   Ile Thr Val Gly Ala Thr Asn Ala Gln Asp Gln Pro Val Thr Leu Gly
               340                 345                 350
   Thr Leu Gly Thr Asn Phe Gly Arg Cys Val Asp Leu Phe Ala Pro Gly
           355                 360                 365
   Lys Asp Ile Ile Gly Ala Ser Ser Asp Cys Ser Thr Cys Phe Met Ser
       370                 375                 380
   Gln Ser Gly Thr Ser Gln Ala Ala Ala His Val Ala Gly Ile Val Ala
   385                 390                 395                 400
   Arg Met Leu Ser Arg Glu Pro Thr Leu Thr Leu Ala Glu Leu Arg Gln
                   405                 410                 415
   Arg Leu Ile His Phe Ser Thr Lys Asp Val Ile Asn Met Ala Trp Phe
               420                 425                 430
   Pro Glu Asp Gln Gln Val Leu Thr Pro Asn Leu Val Ala Thr Leu Pro
   435 440 445
   Pro Ser Thr His Glu Thr Gly Gly Gln Leu Leu Cys Arg Thr Val Trp
   450 455 460
   Ser Ala His Ser Gly Pro Thr Arg Thr Ala Thr Ala Thr Ala Arg Cys
   465 470 475 480
   Ala Pro Glu Glu Glu Leu Leu Ser Cys Ser Ser Phe Ser Arg Ser Gly
   485 490 495
   Arg Arg Arg Gly Asp Trp Ile Glu Ala Ile Gly Gly Gln Gln Val Cys
   500 505 510
   Lys Ala Leu Asn Ala Phe Gly Gly Glu Gly Val Tyr Ala Val Ala Arg
   515 520 525
   Cys Cys Leu Val Pro Arg Ala Asn Cys Ser Ile His Asn Thr Pro Ala
   530 535 540
   Ala Arg Ala Gly Leu Glu Thr His Val His Cys His Gln Lys Asp His
   545 550 555 560
   Val Leu Thr Gly Cys Ser Phe His Trp Glu Val Glu Asp Leu Ser Val
   565 570 575
   Arg Arg Gln Pro Ala Leu Arg Ser Arg Arg Gln Pro Gly Gln Cys Val
   580 585 590
   Gly His Gln Ala Ala Ser Val Tyr Ala Ser Cys Cys His Ala Pro Gly
   595 600 605
   Leu Glu Cys Lys Ile Lys Glu His Gly Ile Ser Gly Pro Ser Glu Gln
   610 615 620
   Val Thr Val Ala Cys Glu Ala Gly Trp Thr Leu Thr Gly Cys Asn Val
   625 630 635 640
   Leu Pro Gly Ala Ser Leu Thr Leu Gly Ala Tyr Ser Val Asp Asn Leu
   645 650 655
   Cys Val Ala Arg Val His Asp Thr Ala Arg Ala Asp Arg Thr Ser Gly
   660 665 670
   Glu Ala Thr Val Ala Ala Ala Ile Cys Cys Arg Ser Arg Pro Ser Ala
   675 680 685
   Lys Ala Ser Trp Val Gln
   690

<210> 758
<211> 653
<212> PRT
<213> Homo sapiens

<400> 758
Glu Phe Arg Cys His Asp Gly Lys Cys Ile Ser Arg Gln Phe Val Cys
1               5                   10                  15
Asp Ser Asp Arg Asp Cys Leu Asp Gly Ser Asp Glu Ala Ser Cys Pro
            20                  25                  30
Val Leu Thr Cys Gly Pro Ala Ser Phe Gln Cys Asn Ser Ser Thr Cys
        35                  40                  45
Ile Pro Gln Leu Trp Ala Cys Asp Asn Asp Pro Asp Cys Glu Asp Gly
    50                  55                  60
Ser Asp Glu Trp Pro Gln Arg Cys Arg Gly Leu Tyr Val Phe Gln Gly
65                  70                  75                  80
Asp Ser Ser Pro Cys Ser Ala Phe Glu Phe His Cys Leu Ser Gly Glu
                85                  90                  95
Cys Ile His Ser Ser Trp Arg Cys Asp Gly Gly Pro Asp Cys Lys Asp
            100                 105                 110
Lys Ser Asp Glu Glu Asn Cys Ala Val Ala Thr Cys Arg Pro Asp Glu
        115                 120                 125
Phe Gln Cys Ser Asp Gly Asn Cys Ile His Gly Ser Arg Gln Cys Asp
    130                 135                 140
Arg Glu Tyr Asp Cys Lys Asp Met Ser Asp Glu Val Gly Cys Val Asn
145                 150                 155                 160
Val Thr Leu Cys Glu Gly Pro Asn Lys Phe Lys Cys His Ser Gly Glu
                165                 170                 175
Cys Ile Thr Leu Asp Lys Val Cys Asn Met Ala Arg Asp Cys Arg Asp
            180                 185                 190
Trp Ser Asp Glu Pro Ile Lys Glu Cys Gly Thr Asn Glu Cys Leu Asp
        195                 200                 205
Asn Asn Gly Gly Cys Ser His Val Cys Asn Asp Leu Lys Ile Gly Tyr
    210                 215                 220

Glu Cys Leu Cys Pro Asp Gly Phe Gln Leu Val Ala Gln Arg Arg Cys
225                 230                 235                 240
Glu Asp Ile Asp Glu Cys Gln Asp Pro Asp Thr Cys Ser Gln Leu Cys
                245                 250                 255
Val Asn Leu Glu Gly Gly Tyr Lys Cys Gln Cys Glu Glu Gly Phe Gln
            260                 265                 270
Leu Asp Pro His Thr Lys Ala Cys Lys Ala Val Gly Ser Ile Ala Tyr
        275                 280                 285
Leu Phe Phe Thr Asn Arg His Glu Val Arg Lys Met Thr Leu Asp Arg
    290                 295                 300
Ser Glu Tyr Thr Ser Leu Ile Pro Asn Leu Arg Asn Val Val Ala Leu
305                 310                 315                 320
Asp Thr Glu Val Ala Ser Asn Arg Ile Tyr Trp Ser Asp Leu Ser Gln
                325                 330                 335
Arg Met Ile Cys Ser Thr Gln Leu Asp Arg Ala His Gly Val Ser Ser
            340                 345                 350
Tyr Asp Thr Val Ile Ser Arg Asp Ile Gln Ala Pro Asp Gly Leu Ala
        355                 360                 365
Val Asp Trp Ile His Ser Asn Ile Tyr Trp Thr Asp Ser Val Leu Gly
    370                 375                 380
Thr Val Ser Val Ala Asp Thr Lys Gly Val Lys Arg Lys Thr Leu Phe
385                 390                 395                 400
Arg Glu Asn Gly Ser Lys Pro Arg Ala Ile Val Val Asp Pro Val His
                405                 410                 415
Gly Phe Met Tyr Trp Thr Asp Trp Gly Thr Pro Ala Lys Ile Lys Lys
            420                 425                 430
Gly Gly Leu Asn Gly Val Asp Ile Tyr Ser Leu Val Thr Glu Asn Ile
        435                 440                 445
Gln Trp Pro Asn Gly Ile Thr Leu Asp Leu Leu Ser Gly Arg Leu Tyr
    450                 455                 460
Trp Val Asp Ser Lys Leu His Ser Ile Ser Ser Ile Asp Val Asn Gly
465                 470                 475                 480
Gly Asn Arg Lys Thr Ile Leu Glu Asp Glu Lys Arg Leu Ala His Pro
                485                 490                 495
Phe Ser Leu Ala Val Phe Glu Asp Lys Val Phe Trp Thr Asp Ile Ile
            500                 505                 510
Asn Glu Ala Ile Phe Ser Ala Asn Arg Leu Thr Gly Ser Asp Val Asn
        515                 520                 525
Leu Leu Ala Glu Asn Leu Leu Ser Pro Glu Asp Met Val Leu Phe His
    530                 535                 540
Asn Leu Thr Gln Pro Arg Gly Val Asn Trp Cys Glu Arg Thr Thr Leu
545                 550                 555                 560
Ser Asn Gly Gly Cys Gln Tyr Leu Cys Leu Pro Ala Pro Gln Ile Asn
                565                 570                 575
Pro His Ser Pro Lys Phe Thr Cys Ala Cys Pro Asp Gly Met Leu Leu
            580                 585                 590
Ala Arg Asp Met Arg Ser Cys Leu Thr Glu Ala Glu Ala Ala Val Ala
        595                 600                 605
Thr Gln Glu Thr Ser Thr Val Arg Leu Lys Val Ser Ser Thr Ala Val
    610                 615                 620
Arg Thr Gln His Thr Thr Thr Arg Pro Val Pro Asp Thr Ser Arg Leu
625                 630                 635                 640
Pro Gly Ala Thr Pro Gly Leu Thr Thr Val Glu Ile Val
                645                 650

1